(19) Europäisches Patentamt
European Patent Office
Office européen des brevets

(11) EP 4 480 507 A1

(12) EUROPEAN PATENT APPLICATION
published in accordance with Art. 153(4) EPC

(43) Date of publication:
**25.12.2024 Bulletin 2024/52**

(21) Application number: **22927052.5**

(22) Date of filing: **16.02.2022**

(51) International Patent Classification (IPC):
***A61L 24/08*** $^{(2006.01)}$ ***C08B 37/02*** $^{(2006.01)}$
***C08B 37/04*** $^{(2006.01)}$ ***C08B 37/08*** $^{(2006.01)}$

(52) Cooperative Patent Classification (CPC):
**A61L 24/08; C08B 37/0021; C08B 37/003; C08B 37/0084**

(86) International application number:
**PCT/JP2022/006265**

(87) International publication number:
**WO 2023/157150 (24.08.2023 Gazette 2023/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Mochida Pharmaceutical Co., Ltd.**
  **Tokyo 160-8515 (JP)**
- **The University of Tokyo**
  **Bunkyo-ku, Tokyo 113-8654 (JP)**

(72) Inventors:
- **ITO Taichi**
  **Tokyo 113-8654 (JP)**
- **CHANDEL Arvind Kumar Singh**
  **Tokyo 113-8654 (JP)**
- **ISAJI Mitsuko**
  **Tokyo 160-8515 (JP)**

(74) Representative: **Hoffmann Eitle**
**Patent- und Rechtsanwälte PartmbB**
**Arabellastraße 30**
**81925 München (DE)**

# (54) USE OF POLYSACCHARIDE DERIVATIVE

(57) [Problem] To provide an additional use of a polysaccharide derivative such as a tissue adhesive material.

[Solution] According to some embodiments, a tissue adhesive material is provided, which is intended to be used for a hard and thick tissue and/or a connective tissue and comprises a polysaccharide derivative having such a structure that a group represented by formula (A) is introduced in an acidic, basic or amphoteric polysaccharide. According to some embodiments, a crosslinked structure and a tissue adhesive material comprising the crosslinked structure are provided, in which the crosslinked structure comprises (a) a polysaccharide derivative having such a structure that a group represented by formula (A) is introduced into an acidic, basic or amphoteric polysaccharide and (b) at least one of an amino-group-containing polymer and an amino-group-containing low-molecular-weight compound each containing at least two primary amino groups, hydrazide groups or aminooxy groups and a group represented by formula (x1), and the crosslinking is achieved by the covalent bonding between a primary amino group, a hydrazide group or an aminooxy group contained in the amino-group-containing polymer and the amino-group-containing low-molecular-weight compound and the group represented by formula (A) contained in the polysaccharide derivative through a Schiff base. According to some embodiments, a tissue adhesive material having a gel-like form is provided, which comprises a polysaccharide derivative having such a structure that a group represented by formula (A-2) is introduced into a polysaccharide containing an amino group, in which the group represented by formula (A-2) is introduced into the polysaccharide by substituting for a hydrogen atom in the amino group in the polysaccharide to form an amide bond.

EP 4 480 507 A1

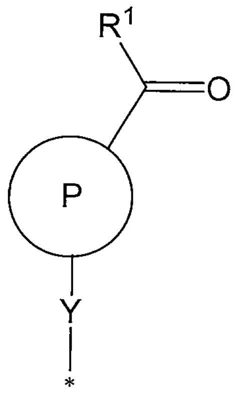

(A)

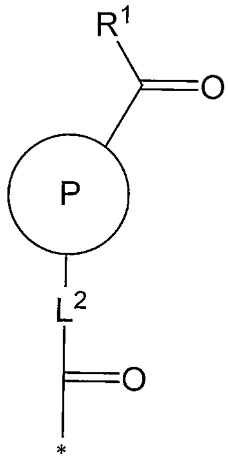

(A-2)

## Description

[Technical Field]

The present invention relates to a use of a polysaccharide derivative, for example, a use as a tissue adhesive material.

[Background Art]

Because polysaccharides such as hyaluronic acid and alginic acid generally exhibit superior water solubility and water retention and suitable viscosity, adhesiveness and biocompatibility, they are widely used as medical materials and food additives, as additives in cosmetics and everyday goods, and as thickening agents. For example, hyaluronic acid (HA) is a biodegradable and biocompatible natural polymer that is abundant in the body, and is widely used in pharmaceuticals for orthopedics, ophthalmology and the like, in medical devices and cosmetics, and as a material in health care products such as contact lenses. Alginic acid (AL) is a natural polysaccharide found in brown algae and the like, and because it has properties such as high biocompatibility and the ability to be crosslinked by polyvalent metal ions such as Ca, it is used in pharmaceuticals including hemostats and wound dressings, in medical devices and pharmaceutical additives, and in foods, supplements and food additives.

Recently research has being done into applying these polysaccharides as drug carrier materials in drug delivery systems (DDS). Because HA binds specifically to the CD44 receptor, is overexpressed on the surface of various tumor cells, and can be easily derivatized using its hydroxyl and carboxyl groups, it is attracting attention as a drug carrier material. For example, there have been multiple reports of HA-drug conjugates using Schiff bases (NPL 1: Materials Science and Engineering: C, 2014, 36: 287-293; NPL 2: Carbohydrate Polymers 134 (2015) 293-299; NPL 3: Carbohydrate Polymers 189 (2018) 273-279; NPL 4: Carbohydrate Polymers 216 (2019) 63-71). Schiff bases are imine group-containing compounds formed by condensation reactions between primary imines and reactive carbonyl compounds. Because Schiff bases exhibit reversible pH-responsive dissociation, they are expected to be able to selectively release drugs. However, Schiff bases formed by common aldehydes or ketones with amines are poorly stable in water due to rapid reverse reactions. Drug conjugates using Schiff bases therefore have rapid drug release rates, and often adequate performance has not been obtained. On the other hand, biocompatible in situ crosslinked hydrogels with hyaluronic acid or alginic acid frameworks have been achieved by using hydrazide groups or the like in place of amino groups to slow down the hydrolysis rate.

Because of its aromatic structure, benzaldehyde has reportedly been able to form stable Schiff bases with amine groups contained in various drugs (NPL 5: Materials Chemistry Frontiers, 2018, 2(10): 1765-1778). NPL 6 (J. Biomed. Nanotechnol. 2017, 13, 1647-1659) discloses conjugates of carboxymethyl chitosan and daunorubicin using Schiff base formation between the aldehyde groups of triazole benzylaldehyde and the amino groups of daunorubicin. Conjugate formation in NPL 6 is complex and involves multiple steps in which the drug is reacted with azido benzylaldehyde, and this is then reacted with alkynemodified carboxymethyl chitosan to form triazole rings and bind the drug to the chitosan, and conjugate formation also requires that the drug and the azido benzylaldehyde be reacted together in advance. This reaction also uses a copper catalyst, which is a problem from a manufacturing and safety standpoint.

In addition, NPL 7 (Polymer Bulletin 31 (1993) 145-149) discloses dextran derivatives obtained by introducing benzaldehydes via ester bonds at the hydroxyl group position of dextran. Because these polysaccharide derivatives use the neutral polysaccharide dextran as the polysaccharide, it has been difficult to introduce hydrophobic benzaldehydes at a high modification rate. Furthermore, the resulting polysaccharide derivatives also have poor water solubility, which is a problem in terms of application. This literature also discloses derivatives obtained by introducing benzaldehydes into dextran which has been derivatized with epichlorohydrin or epoxy. With these derivatives the aim is to improve hydrophilicity by introducing hydroxy groups from the epichlorohydrin or epoxy into the side chains, but synthesis of these derivatives requires multiple reaction steps, and there is room for improvement from the standpoint of industrial manufacture and benzaldehyde modification rates.

There have been multiple reports of conjugates between drugs and hyaluronic acid without Schiff bases. For example, these include conjugates of HA and proteins (NPL 8: Carbohydrate Polymers 92 (2013) 2163-2170), an HA-IFN$\alpha$2a conjugate (NPL 9: Journal of Controlled Release 236 (2016) 79-89), and a conjugate of HA and pemetrexed (NPL 10: European Journal of Pharmaceutical Sciences 138 (2019) 105008).

[Citation List]

[Non Patent Literature]

[NPL 1] Materials Science and Engineering: C, 2014, 36: 287-293
[NPL 2] Carbohydrate Polymers 134 (2015) 293-299
[NPL 3] Carbohydrate Polymers 189 (2018) 273-279
[NPL 4] Carbohydrate Polymers 216 (2019) 63-71
[NPL 5] Materials Chemistry Frontiers 2 (2018) 1765-1778
[NPL 6] J. Biomed. Nanotechnol. 13 (2017) 1647-1659
[NPL 7] Polymer Bulletin 31 (1993) 145-149
[NPL 8] Carbohydrate Polymers 92 (2013) 2163-2170
[NPL 9] Journal of Controlled Release 236 (2016) 79-89
[NPL 10] European Journal of Pharmaceutical Sciences 138 (2019) 105008

[Summary of Invention]

In PCT/JP2021/029573 (filing date: August 10, 2021), the inventors provide a novel polysaccharide derivative having a modifying group which can form a Schiff base with an amino group such as benzaldehyde. The present invention relates to further improvement of the polysaccharide derivative, and particularly, relates to an additional use of a polysaccharide derivative for a tissue adhesive material, and a use of a polysaccharide derivative for an improved crosslinked structure.

A first aspect of the present invention provides a tissue adhesive material which has adhesion not only to soft tissue in vivo but also to relatively hard and thick tissue and/or connective tissue such as the dura mater, perichondrium, and synovium.

A second aspect of the present invention provides a crosslinked structure of a polysaccharide derivative and an amino group-containing material having a polyhydroxyphenyl group, and a tissue adhesive material containing the same.

A third aspect of the present invention provides a tissue adhesive material including a polysaccharide derivative containing an amino group.

In some aspects, the tissue adhesive material is used to repair/restore biological tissue.

The present invention provides, for example, following aspects.

[1] A tissue adhesive material used for hard and thick tissue and/or connective tissue, including a polysaccharide derivative in which a group represented by Formula (A) below is introduced into an acidic, basic or amphoteric polysaccharide:

[C1]

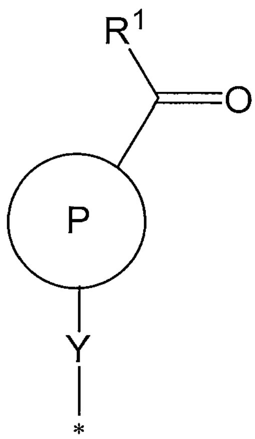

(A)

(in the formula, $R^1$ represents a hydrogen atom or $C_{1-4}$ alkyl,

ring P is a phenyl ring or pyridine ring, and the phenyl ring and pyridine ring are optionally substituted with one or more substituents independently selected from the halogen atoms and $-CF_3$, $-NO_2$, carboxyl and $-SO_3H$ groups,

Y represents a divalent group selected from the group consisting of -NH-, -C(=O)-, - S-, -O-, the $C_{1-6}$ alkylenes and $-(CH_2CH_2O)_n-$ and any combinations of these, with n being an integer from 1 to 9, and

* represents a linkage with the polysaccharide).

[2] The tissue adhesive material according to [1], wherein the polysaccharide is selected from alginic acid, derivatives thereof or salts of these, hyaluronic acid, derivatives thereof or salts of these, carboxymethyl cellulose, derivatives thereof or salts of these, carboxymethyl dextran, derivatives thereof or salts of these, carboxymethyl starch, derivatives thereof or salts of these, heparin, derivatives thereof or salts of these, heparan sulfate, derivatives thereof or salts of these, chondroitin sulfate, derivatives thereof or salts of these, dermatan sulfate, derivatives thereof or salts of these, chitosan, derivatives thereof or salts of these, regenerated oxidized cellulose, derivatives thereof or salts of these, and pectic acid, derivatives thereof or salts of these.

[3] The tissue adhesive material according to [1] or [2], wherein

the polysaccharide is a polysaccharide containing a carboxyl group, the group represented by Formula (A) is a group represented by Formula (A-1) below, and the group represented by Formula (A-1) is introduced into the polysaccharide by substituting -OH of the carboxyl group of the polysaccharide to form an amide bond; or;

the polysaccharide is a polysaccharide containing an amino group, the group represented by Formula (A) is a group represented by Formula (A-2) below, and the group represented by Formula (A-2) is introduced into the polysaccharide by substituting a hydrogen atom of the amino group of the polysaccharide to form an amide bond:

[C2]

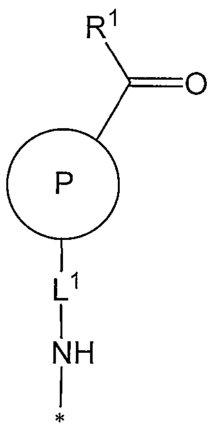

(A-1)

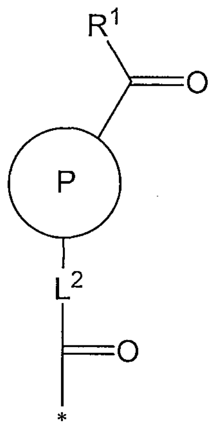

(A-2)

(in formulae (A-1) and (A-2), $R^1$ represents a hydrogen atom or $C_{1-4}$ alkyl,

ring P is a phenyl ring or pyridine ring, and the phenyl ring or pyridine ring is optionally substituted with one or more substituents independently selected from the halogen atoms and $-CF_3$, $-NO_2$, carboxyl and $-SO_3H$ groups,

$L^1$ is a single bond or represents a divalent group selected from the group consisting of -C(=O)-, -S-, -O-, the $C_{1-6}$ alkylenes and $-(CH_2CH_2O)_n-$ and any combinations of these,

$L^2$ is a single bond or represents a divalent group selected from the group consisting of -NH-, -S-, -O-, the $C_{1-6}$ alkylenes and $-(CH_2CH_2O)_n-$ and any combinations of these,

n is an integer from 1 to 9, and

* represents a linkage with the polysaccharide).

[4] The tissue adhesive material according to any one of [1] to [3], wherein

the polysaccharide is alginic acid, a derivative thereof or a salt thereof, and

the polysaccharide derivative contains a constituent unit represented by Formula (c11) and/or (c12) below:

[C3]

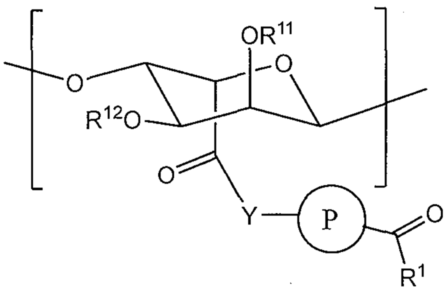

(c11)

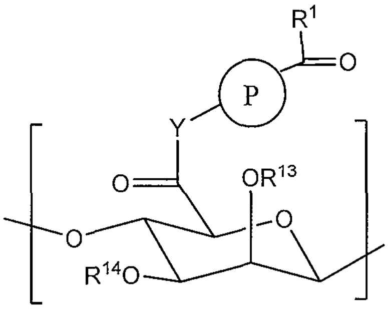

(c12)

(in the formulae, $R^{11}$, $R^{12}$, $R^{13}$, and $R^{14}$ are each independently selected from among a hydrogen atom, a $C_{1-6}$ alkyl group, and a -C(=O)-Ci-6 alkyl group,

$R^1$ represents a hydrogen atom or a $C_{1-4}$ alkyl group,
the ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
Y represents $-L^1-NH-$, in which $L^1$ is bound to the ring P,
$L^1$ is selected from among a single bond, a $C_{1-6}$ alkylene group, and $-(CH_2CH_2O)_n-$, and
n is an integer from 1 to 9).

[5] The tissue adhesive material according to any one of [1] to [4], wherein
the group represented by Formula (A) is selected from the group consisting of the following formulae:

[C4]

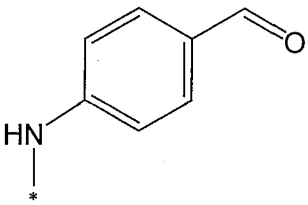

,

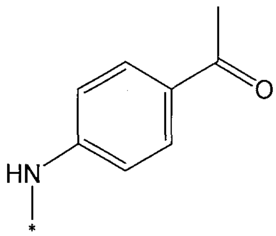

,

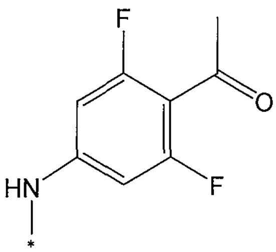

,

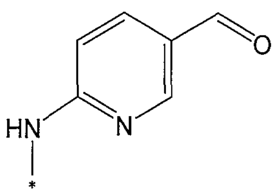

, and

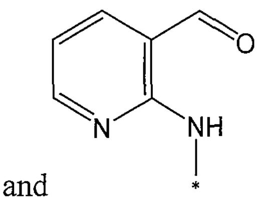

,

(in the formulae, * represents a linkage with the polysaccharide).

[5-1] The tissue adhesive material according to any one of [1] to [4], wherein
the hard and thick tissue and/or connective tissue is selected from the group consisting of dura mater tissue, perichondrium tissue, synovium tissue and periodontal ligament tissue.
[5-2] The tissue adhesive material according to any one of [1] to [4], wherein
the hard and thick tissue and/or connective tissue is dura mater tissue.
[5-3] The tissue adhesive material according to any one of [1] to [4], wherein
the hard and thick tissue and/or connective tissue is hard and thick tissue.

[5-4] The tissue adhesive material according to any one of [1] to [4], wherein the hard and thick tissue and/or connective tissue is connective tissue.

[6] A tissue adhesive material used for hard and thick tissue and/or connective tissue, having a crosslinked structure in which at least a part of a polysaccharide derivative in which a group represented by Formula (A) below is introduced into an acidic, basic or amphoteric polysaccharide is crosslinked:

[C5]

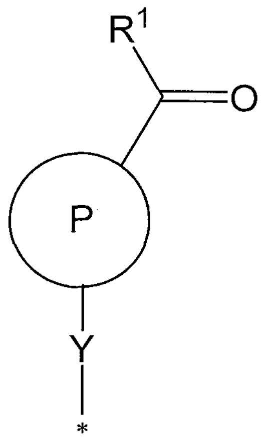

(A)

(in the formula, $R^1$ represents a hydrogen atom or a $C_{1-4}$ alkyl group,

the ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
Y represents a divalent group selected from the group consisting of -NH-, -C(=O)-, - S-, -O-, a $C_{1-6}$ alkylene group, $-(CH_2CH_2O)_n-$, and any combinations of these, and n is an integer from 1 to 9, and
* represents a linkage with the polysaccharide).

[6-1] The tissue adhesive material according to [6], wherein
the crosslinked structure has a structure in which the polysaccharide derivative is crosslinked with a crosslinking agent selected from among divalent metal ions; an amino group-containing polymer and an amino group-containing low-molecular-weight compound, which contain two or more primary amino groups, a hydrazide group, or an aminoxy group; and a compound containing two or more aldehyde groups such as glutaraldehyde.
[6-2] The tissue adhesive material according to [6] or [6-1], wherein
the polysaccharide is selected from among alginic acid, a derivative thereof or a salt thereof, hyaluronic acid, a derivative thereof or a salt thereof, carboxymethylcellulose, a derivative thereof or a salt thereof, carboxymethyl dextran, a derivative thereof or a salt thereof, carboxymethyl starch, a derivative thereof or a salt thereof, heparin, a derivative thereof or a salt thereof, heparan sulfate, a derivative thereof or a salt thereof, chondroitin sulfate, a derivative thereof or a salt thereof, dermatan sulfate, a derivative thereof or a salt thereof, chitosan, a derivative thereof or a salt thereof, regenerated oxidized cellulose, a derivative thereof or a salt thereof, and pectic acid, a derivative thereof or a salt thereof.
[6-3] The tissue adhesive material according to [6], [6-1] or [6-2], wherein

the polysaccharide is a polysaccharide containing a carboxyl group, the group represented by Formula (A) is a group represented by Formula (A-1) below, and the group represented by Formula (A-1) is introduced into the polysaccharide by substituting -OH of the carboxyl group of the polysaccharide to form an amide bond; or;
the polysaccharide is a polysaccharide containing an amino group, the group represented by Formula (A) is a group represented by Formula (A-2) below, and the group represented by Formula (A-2) is introduced into the polysaccharide by substituting a hydrogen atom of the amino group of the polysaccharide to form an amide bond:

[C6]

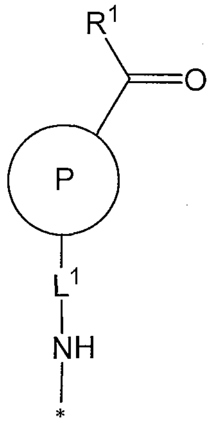

(A-1)

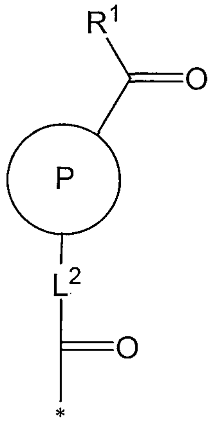

(A-2)

(in Formulae (A-1) and (A-2),

$R^1$ represents a hydrogen atom or a $C_{1-4}$ alkyl group,
the ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
$L^1$ is a single bond or a divalent group selected from the group consisting of - C(=O)-, -S-, -O-, a $C_{1-6}$ alkylene group, $-(CH_2CH_2O)_n-$, and any combinations of these,
$L^2$ is a single bond or represents a divalent group selected from the group consisting of -NH-, -S-, -O-, a $C_{1-6}$ alkylene group, $-(CH_2CH_2O)_n-$, and any combinations of these,
n is an integer from 1 to 9, and
* represents a linkage with the polysaccharide).

[6-4] The tissue adhesive material according to any one of [6], and [6-1] to [6-3], wherein

the polysaccharide is alginic acid, a derivative thereof or a salt thereof, and
the polysaccharide derivative contains a constituent unit represented by Formula (c11) and/or (c12) below:

[C7]

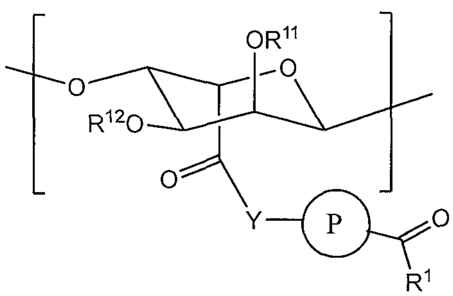

(c11)

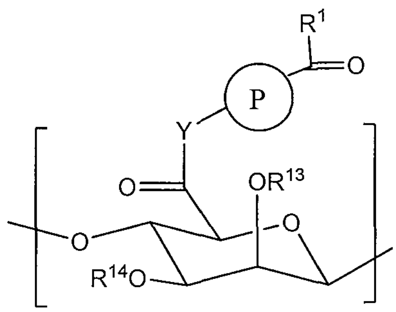

(c12)

(in the formulae, $R^{11}$, $R^{12}$, $R^{13}$, and $R^{14}$ are each independently selected from among a hydrogen atom, a $C_{1-6}$ alkyl group, and a $-C(=O)-C_{1-6}$ alkyl group,

$R^1$ represents a hydrogen atom or a $C_{1-4}$ alkyl group,
the ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,

Y represents $-L^1-NH-$, in which $L^1$ is bound to the ring P,

$L^1$ is selected from among a single bond, a $C_{1-6}$ alkylene group, and $-(CH_2CH_2O)_n-$, and

n is an integer from 1 to 9).

[6-5] The tissue adhesive material according to any one of [6], and [6-1] to [6-4], wherein the group represented by Formula (A) is selected from the group consisting of the following formulae:

[C8]

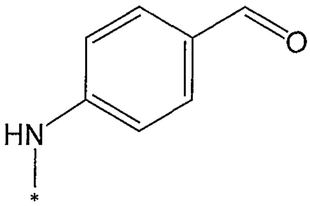
,
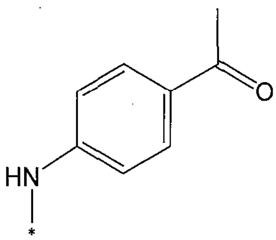
,
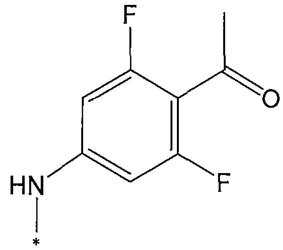
,

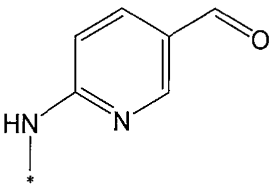
, and
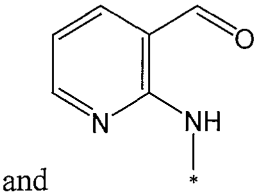
,

(in the formulae, * represents a linkage with the polysaccharide).

[7] The tissue adhesive material according to any one of [1] to [6], which is in the form of a gel, sponge, sheet, or film.

[8] The tissue adhesive material according to any one of [1] to [7], further including a nonwoven fabric, sheet, or film.

[9] A crosslinked structure, including:

(a) a polysaccharide derivative in which a group represented by Formula (A) below is introduced into an acidic, basic or amphoteric polysaccharide; and

(b) at least one of an amino group-containing polymer and an amino group-containing low-molecular-weight compound, which contain two or more primary amino groups, a hydrazide group, or an aminoxy group, and a group represented by Formula (x1) below, wherein

the primary amino groups, the hydrazide group, or the aminoxy group contained in the amino group-containing polymer and the amino group-containing low-molecular-weight compound and the group represented by Formula (A) contained in the polysaccharide derivative are crosslinked by a covalent bond via a Schiff base:

[C9]

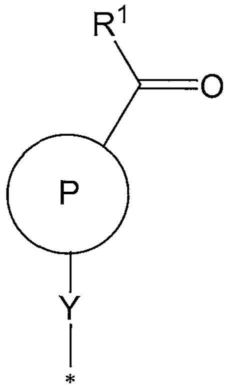
(A)

(in Formula (A), $R^1$ represents a hydrogen atom or a $C_{1-4}$ alkyl group,

the ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
Y represents a divalent group selected from the group consisting of -NH-, -C(=O)-, - S-, -O-, a $C_{1-6}$ alkylene group, $-(CH_2CH_2O)_n-$, and any combinations of these, and n is an integer from 1 to 9, and
* represents a linkage with the polysaccharide).

[C10]

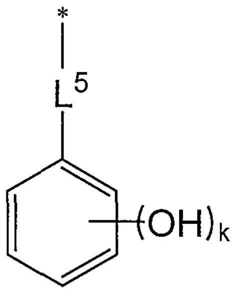

(x1)

(in Formula (x1), k is an integer from 2 to 5,

$L^5$ is a single bond or a divalent group selected from the group consisting of - C(=O)-, -S-, -O-, an alkylene group, $-(CH_2CH_2O)_n-$, and any combinations of these,
n is an integer from 1 to 9, and
* represents a linkage with the amino group-containing polymer or the amino group-containing low-molecular-weight compound).

[10] The crosslinked structure according to [9], wherein
the amino group-containing polymer is at least one selected from among linear, branched or dendritic polyamines; polyalkylene glycols substituted with an amino group, a hydrazide group, or an aminoxy group; polyallylamines; polyvinylamines; polyacrylamines; amino group-containing polysaccharides; amino group-containing proteins; and polyamino acids.
[11] The crosslinked structure according to [9] or [10], wherein

the polysaccharide is alginic acid, a derivative thereof or a salt thereof, and
the polysaccharide derivative contains a constituent unit represented by Formula (c11) and/or (c12) below:

[C11]

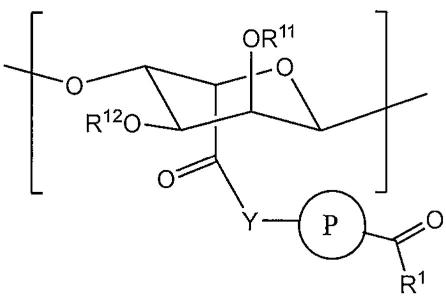

(c11)

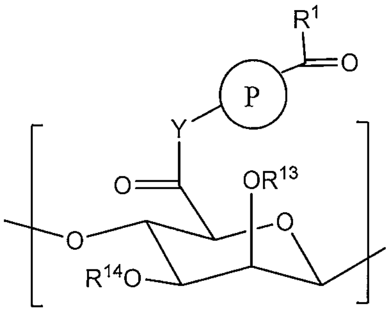

(c12)

(in the formulae, $R^{11}$, $R^{12}$, $R^{13}$, and $R^{14}$ are each independently selected from among a hydrogen atom, a $C_{1-6}$ alkyl group, and a $-C(=O)-C_{1-6}$ alkyl group,

$R^1$ represents a hydrogen atom or a $C_{1-4}$ alkyl group,
the ring P is a phenyl ring or pyridine ring, and the phenyl ring or pyridine ring is optionally substituted with one

or more substituents independently selected from among a halogen atom, $-CF_3$, $-NO_2$, a carboxyl group and $-SO_3H$,
Y represents $-L^1-NH-$, in which $L^1$ is bound to the ring P,
$L^1$ is selected from among a single bond, a $C_{1-6}$ alkylene group, and $-(CH_2CH_2O)_n-$, and
n is an integer from 1 to 9).

[12] A tissue adhesive material having the crosslinked structure according to any one of [9] to [11].
[13] A tissue adhesive material containing a polysaccharide derivative in which a group represented by Formula (A-2) below is introduced into a polysaccharide containing an amino group, wherein

the group represented by Formula (A-2) is introduced into the polysaccharide by substituting a hydrogen atom of the amino group of the polysaccharide to form an amide bond, and
the tissue adhesive material is in the form of a gel:

[C12]

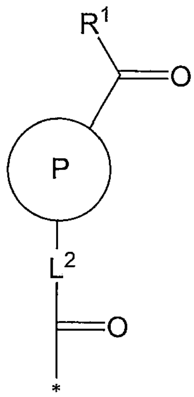

(A-2)

(in Formula (A-2),

$R^1$ represents a hydrogen atom or a $C_{1-4}$ alkyl group,
the ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
$L^2$ is a single bond or represents a divalent group selected from the group consisting of -NH-, -S-, -O-, a $C_{1-6}$ alkylene group, $-(CH_2CH_2O)_n-$, and any combinations of these,
n is an integer from 1 to 9, and
* represents a linkage with the polysaccharide).

[14] The composition according to [13], wherein
the ratio ($\eta_{0.001}/\eta_{1000}$) of a viscosity ($\eta_{0.001}$) at a shear rate of 0.001 $s^{-1}$ to a viscosity ($\eta_{1000}$) at a shear rate of 1,000 $s^{-1}$ measured at 25°C is 10 to $10^9$.
[15] The tissue adhesive material according to [13] or [14], wherein
the polysaccharide derivative forms a crosslinked structure when the amino group contained in the polysaccharide derivative and the group represented by Formula (A-2) are crosslinked by forming a covalent bond via a Schiff base.
[16] The tissue adhesive material according to any one of [13] to [15], wherein

the polysaccharide is chitosan, a derivative thereof or a salt thereof, and
the polysaccharide derivative contains a constituent unit represented by Formula (c16) below:

[C13]

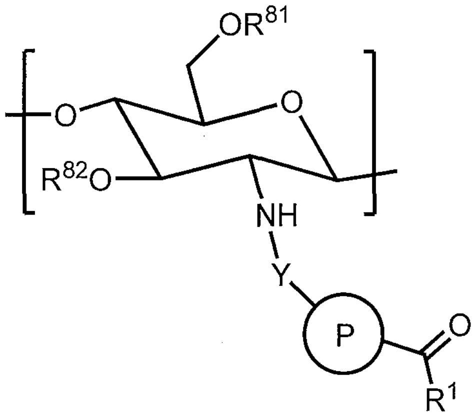

(c16)

(in the formula,

$R^{81}$ and $R^{82}$ are each independently selected from among a hydrogen atom, a $C_{1-6}$ alkyl group, and a -C(=O)-$C_{1-6}$ alkyl group,
$R^1$ represents a hydrogen atom or a $C_{1-4}$ alkyl group,
the ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
Y represents $-L^2-C(=O)-$, in which $L^2$ is bound to the ring P,
$L^2$ is selected from among a single bond, a $C_{1-6}$ alkylene group, $-(CH_2CH_2O)_n-$, $-(CH_2)_{m1}-(CH_2CH_2O)_n-(CH_2)_{m2}-$, and $-(CH_2)_{m1}-O-(CH_2CH_2O)_n-(CH_2)_{m2}-$,
n is an integer from 1 to 9, and
m1 and m2 are each independently an integer from 1 to 9).

According to the first aspect of the present invention, there is provided a tissue adhesive material used for repairing/restoring biological tissue, which contains a polysaccharide derivative, and which has adhesion not only to soft tissue in vivo but also to relatively hard and thick tissue and/or connective tissue such as the dura mater, perichondrium and synovium. The tissue adhesive material has favorable adhesion to tissue due to formation of a Schiff base between amino groups of the tissue and aldehyde of the polysaccharide derivative. According to some aspects, the adhesion to tissue can be improved when hydrophobic interactions and hydrogen bonds are formed between tissue proteins constituting the tissue and the polysaccharide derivative. The material adhered to the tissue serves to protect and repair the tissue, and for example, in the case of the dura mater, it can prevent the cerebrospinal fluid from leaking.

According to the second aspect of the present invention, there is provided a crosslinked structure of a polysaccharide derivative and an amino group-containing material having a polyhydroxyphenyl group. The crosslinked structure has favorable adhesion to tissue and can be used for applications such as a tissue adhesive material.

According to the third aspect of the present invention, there is provided a tissue adhesive material in the form of a gel, including a polysaccharide derivative containing an amino group. The tissue adhesive material exhibits favorable adhesion to tissue when the aldehyde group or ketone group ($-C(=O)R^1$) of the modifying group (A-2) of the polysaccharide derivative forms a Schiff base between the amino group of the biological tissue and the aldehyde of the polysaccharide derivative. In addition, in some aspects, an interaction occurs between the amino group of the polysaccharide derivative and the carboxyl group of the biological tissue, and the adhesion to tissue can be improved. In some aspects, the polysaccharide derivative containing an amino group has a pH-responsive self-crosslinking property.

[Brief Description of Drawings]

[Fig. 1]
Fig. 1A shows the $^1H$ NMR spectra of a benzaldehyde-modified alginic acid (AL-ABA) synthesized in Example 1-1 and

alginic acid (AL), and Fig. 1B shows the sites corresponding to peaks b, c and d in Fig. 1A.

[Fig. 2]

Fig. 2 shows the UV-visible light absorption spectra (UV-vis) of the benzaldehyde-modified alginic acid (AL-ABA) synthesized in Example 1-1, alginic acid (AL), and 4-aminobenzaldehyde (ABA).

[Fig. 3]

Fig. 3 shows the FT-IR spectra of the benzaldehyde-modified alginic acid (AL-ABA) synthesized in Example I-1 and alginic acid (AL).

[Fig. 4]

Fig. 4 shows cytotoxicity test results (WST assay results) for the benzaldehyde-modified alginic acid (AL-ABA) synthesized in Example I-1 and alginic acid (AL) at different concentrations (0.01 mg/mL, 0.1 mg/mL and 1 mg/mL) with respect to MeT-5A (human mesothelial cell line), NIH/3T3 (mouse embryo fibroblasts), HUVEC (human umbilical vein endothelial cells) and RAW264.7 cells (mouse macrophage-like cell line).

[Fig. 5]

Fig. 5 shows the $^1$H NMR spectrum of an AAP-modified alginic acid (AL-AAP) synthesized in Example 1-3.

[Fig. 6]

Fig. 6 shows the FT-IR spectrum of the AAP-modified alginic acid (AL-AAP) synthesized in Example I-3.

[Fig. 7]

Fig. 7 shows the $^1$H NMR spectrum of an ADFBA-modified alginic acid (AL-ADFBA) synthesized in Example 1-4.

[Fig. 8]

Fig. 8 shows the FT-IR spectrum of the ADFBA-modified alginic acid (AL-ADFBA) synthesized in Example I-4.

[Fig. 9]

Fig. 9 shows the $^1$H NMR spectrum of an AAP-modified alginic acid (AL-APCA) synthesized in Example I-5.

[Fig. 10]

Fig. 10 shows the FT-IR spectrum of the AAP-modified alginic acid (AL-APCA) synthesized in Example I-5.

[Fig. 11]

Fig. 11 shows the $^1$H NMR spectrum of an ANA-modified alginic acid (AL-ANA) synthesized in Example 1-6.

[Fig. 12]

Fig. 12 shows the FT-IR spectrum of the ANA-modified alginic acid (AL-ANA) synthesized in Example 1-6.

[Fig. 13]

Fig. 13 shows the $^1$H NMR spectra of a benzaldehyde-modified alginic acid-vancomycin conjugate (AL-ABA-Van) synthesized in Example 1-7, the benzaldehyde-modified alginic acid (AL-ABA) synthesized in Example I-1, vancomycin (Van) and alginic acid (AL).

[Fig. 14]

Fig. 14 shows the UV-visible light absorption spectra (UV-vis) of the benzaldehyde-modified alginic acid-vancomycin conjugate (AL-ABA-Van) synthesized in Example 1-7, the benzaldehyde-modified alginic acid (AL-ABA) synthesized in Example I-1 and vancomycin (Van).

[Fig. 15]

Fig. 15 shows the FT-IR spectra of the benzaldehyde-modified alginic acid-vancomycin conjugate (AL-ABA-Van) synthesized in Example 1-7, the benzaldehyde-modified alginic acid (AL-ABA) synthesized in Example I-1, vancomycin (Van) and alginic acid (AL).

[Fig. 16A]

Fig. 16A shows the release behavior of vancomycin (Van) from a solution of the benzaldehyde-modified alginic acid-vancomycin conjugate (AL-ABA-Van) synthesized in Example I-7 under different pH conditions (pH = 5.0, 6.0, 7.4). The Van release rate (Cumulative Release %) shown on the vertical axis is the cumulative Van release rate (%), which was calculated from the absorbance of the UV-vis spectrum measurement.

[Fig. 16B]

Fig. 16B shows the release behavior of Van from a mixed solution (AL+Van) of sodium alginate and vancomycin under different pH conditions (pH = 5.0, 6.0, 7.4). The Van release rate (Cumulative Release %) shown on the vertical axis is the cumulative Van release rate (%), which was calculated from the absorbance of the UV-vis spectrum measurement.

[Fig. 16C]

Fig. 16C shows the release behavior of Van from a vancomycin solution (Van only) under different pH conditions (pH = 5.0, 6.0, 7.4). The Van release rate (Cumulative Release %) shown on the vertical axis is the cumulative Van release rate (%), which is calculated from the absorbance of the UV-vis spectrum measurement.

[Fig. 17]

Fig. 17 shows confocal microscope images of an FTSC-loaded AL-ABA capsule prepared in Example I-8 and an FTSC-loaded AL capsule. The top row shows confocal microscope images of the AL capsule (Alg microcapsule) in FTSC-containing physiological saline, the middle row shows confocal microscope images of the AL-ABA capsule (Alg-ABA microcapsule) in FTSC-containing physiological saline, and the bottom row shows confocal microscope

images of the AL-ABA capsule (Alg-ABA microcapsule) in FTSC-containing DMEM. In each row, the lefthand image is a transmitted image (Transmitted), the middle image is a fluorescent image (Amine-Fluorescein), and the righthand image shows the two superimposed (Merged).

[Fig. 18]

Fig. 18 shows vancomycin (Van) release behavior from a Van-loaded AL-ABA microcapsule (AL-ABA-Van capsule) prepared in Example I-9 and a Van-loaded AL microcapsule (AL-Van capsule) as a control. Fig. 18A shows the cumulative release rate (%) of Van on the vertical axis, while Fig. 18B shows the Van release rate (Vancomycin Release %) at each time point on the vertical axis. The Van release rate (Vancomycin Release %) is calculated from the absorbance in UV-vis spectrum measurement.

[Fig. 19]

Fig. 19 shows photographs illustrating the growth of Staphylococcus aureus around filter paper impregnated with sustained release solution from vancomycin-loaded capsules. The results using sustained release solution from a vancomycin-loaded AL capsule (AL-Van) are shown in (a), while the results using sustained release solution from a vancomycin-loaded AL-ABA capsule (AL-ABA-Van) are shown in (b). The top images show results immediately after addition of the sustained release solution, while the bottom images show results 24 hours after addition.

[Fig. 20]

Fig. 20 shows a comparison of Staphylococcus aureus growth inhibition areas using release liquids from vancomycin-loaded AL capsules (AL-Van) and vancomycin-loaded AL-ABA capsules (AL-ABA-Van) at different release times (n=3).

[Fig. 21]

Fig. 21 shows the UV-visible light absorption spectra (UV-vis) of a benzaldehyde-modified alginic acid-bacitracin conjugate (AL-ABA-Bac) synthesized in Example 1-10, the benzaldehyde-modified alginic acid (AL-ABA) synthesized in Example 1, and bacitracin (Bac).

[Fig. 22]

Fig. 22 shows the FT-IR spectra of the benzaldehyde-modified alginic acid-bacitracin conjugate (AL-ABA-Bac) synthesized in Example 1-10, bacitracin (Bac), the benzaldehyde-modified alginic acid (AL-ABA) synthesized in Example 1 and alginic acid (AL).

[Fig. 23]

Fig. 23 shows the $^1$H NMR spectrum of a benzaldehyde-modified alginic acid-dopamine conjugate (AL-ABA-DOPA) synthesized in Example 1-11.

[Fig. 24]

Fig. 24 shows the FT-IR spectrum of the benzaldehyde-modified alginic acid-dopamine conjugate (AL-ABA-DOPA) synthesized in Example 1-11.

[Fig. 25]

Fig. 25 shows the $^1$H NMR spectrum of a benzaldehyde-modified alginic acid-serotonin conjugate (AL-ABA-Serotonin) synthesized in Example I-13.

[Fig. 26]

Fig. 26 shows the FT-IR spectrum of the benzaldehyde-modified alginic acid-serotonin conjugate (AL-ABA-Serotonin) synthesized in Example 1-13.

[Fig. 27]

Fig. 27 shows the $^1$H NMR spectrum of a benzaldehyde-modified alginic acid-celecoxib conjugate (AL-ABA-Celecoxib) synthesized in Example 1-14.

[Fig. 28]

Fig. 28 shows methods for preparing AL-ABA-Apt in Example 1-15 and test procedures for release testing of the AL-ABA-Apt.

[Fig. 29]

Fig. 29 shows the release behavior of an HGF aptamer (Apt) from the AL-ABA-HGF aptamer (AL-ABA-Apt) synthesized in Example 1-15, a mixture of alginic acid and the HGF aptamer (ALG-Apt) and the HGF aptamer by itself (Apt) under physiological pH conditions (pH 7.4). The HGF aptamer release rate (cumulative release rate %) on the vertical axis is the cumulative release rate (%) of the HGF aptamer as calculated from the absorbance in UV-vis spectrum measurement.

[Fig. 30]

Fig. 30 shows optical photographs of benzaldehyde-modified alginic acid sponges prepared in Example I-16. Control indicates an AL sponge prepared as a control, and A, B and C indicate AL/AL-ABA mixed sponges in which the compounding ratios (weight ratios) of AL and AL-ABA are 75:25, 50:50 and 25:75, respectively.

[Fig. 31]

Fig. 31 shows SEM photographs of the benzaldehyde-modified alginic acid sponges prepared in Example I-16. Control indicates an AL sponge prepared as a control, and A, B and C indicate AL/AL-ABA mixed sponges in which the

compounding ratios (weight ratios) of AL and AL-ABA are 75:25, 50:50 and 25:75, respectively.

[Fig. 32]

Fig. 32 shows the swelling and degradation profile (hydrogel weight change) of a Ca-crosslinked AL-ABA hydrogel.

[Fig. 33]

Fig. 33 is an SEM photograph (500x; 24 hours after start of swelling) of a sponge obtained by freeze drying a Ca-crosslinked AL-ABA hydrogel, confirming a porous structure of a dried hydrogel.

[Fig. 34]

Fig. 34 shows a schematic diagram of a crosslinked structure of AL-ABA with an amino group-containing polymer (DPI: dendritic polyethyleneimine), prepared in Example 1-18.

[Fig. 35]

Fig. 35 shows a photograph of a hydrogel consisting of a crosslinked structure of AL-ABA and DPI.

[Fig. 36A]

Fig. 36A shows the $^1$H NMR spectrum of PEG-COOH prepared in Example 1-19.

[Fig. 36B]

Fig. 36B shows the $^1$H NMR spectrum of PEGDH prepared in Example 1-19.

[Fig. 36C]

Fig. 36C shows a photograph of a hydrogel consisting of a crosslinked structure of AL-ABA and PEGDH prepared in Example 1-19.

[Fig. 37]

Fig. 37 shows dynamic viscoelasticity measurement results for a hydrogel consisting of a crosslinked structure of AL-ABA and an amino group-containing polymer (PEGDH), prepared in Example 1-19.

[Fig. 38]

Fig. 38 shows a photograph of a hydrogel of AL-ABA and polyallylamine prepared in Example 1-21.

[Fig. 39]

Fig. 39 shows changes in appearance over time when calcium-crosslinked gels (AL-ABA gel, AL gel) of benzaldehyde-modified alginic acid (AL-ABA) and alginic acid (AL) were each administered to a pig esophagus and immersed in physiological saline in Example 1-22. In Fig. 39, ALG (IL-6G) represents the AL gel, while ALG-ABA represents the AL-ABA gel.

[Fig. 40]

Fig. 40 shows changes over time in gel weight (adhesion rate, %) when calcium-crosslinked gels (AL-ABA gel, AL gel) of benzaldehyde-modified alginic acid (AL-ABA) and alginic acid (AL) were each administered to a pig esophagus and immersed in physiological saline in Example I-22. In Fig. 40, ALG (IL-6G) represents the AL gel, while ALG-ABA represents the AL-ABA gel.

[Fig. 41]

Fig. 41 is a schematic diagram illustrating binding of AL-ABA and AL bind to the esophageal mucosal layer (mucosa) and the submucosal layer (submucosa).

[Fig. 42]

Fig. 42 shows procedures for evaluating the adhesiveness of AL-ABA and AL to the mucosa and submucosa in Example 1-23.

[Fig. 43]

Fig. 43 shows changes in appearance over time when gels of benzaldehyde-modified alginic acid (AL-ABA) and alginic acid (AL) calcium crosslinked at a concentration of 50 mM or 100 mM $Ca^{2+}$ (AL-ABA, AL) were each applied to pig esophageal mucosa (Control) and submucosa (ESD) and immersed in physiological saline in Example 1-23. The circles (dotted lines) in the figure indicate areas where the material persists.

[Fig. 44]

Fig. 44 shows changes over time in gel weight (adhesion rate, %) when gels of benzaldehyde-modified alginic acid (AL-ABA) and alginic acid (AL) calcium crosslinked at a concentration of 50 mM or 100 mM $Ca^{2+}$ (AL-ABA, AL) were each applied to pig esophageal mucosa (Con) and submucosa (ESD) and immersed in physiological saline in Example 1-23.

[Fig. 45]

Fig. 45 is a diagram illustrating methods of tensile testing in Example 1-23.

[Fig. 46]

Fig. 46 shows results of tensile testing of Ca-crosslinked hydrogels of AL-ABA and AL adhering to mucosa (Control) and submucosa (ESD) in Example 1-23.

[Fig. 47]

Fig. 47(A) and Fig. 47(B) are diagrams illustrating test procedures for the lap shear method in Example I-24.

[Fig. 48]

Fig. 48 shows lap shear measurement results for adhesion strength (Pa) of conventional tissue adhesive materials

and Ca-crosslinked hydrogels of AL-ABA and AL adhering to submucosa and skin in Example I-24.

[Fig. 49]

Fig. 49(A) is a diagram illustrating a device used for burst testing in Example I-25. Fig. 49(B) is a diagram illustrating the methods of burst testing.

[Fig. 50]

Fig. 50 shows the results of measurement of burst pressure (mmHg) in burst testing of a conventional tissue adhesive materials and Ca-crosslinked hydrogels of AL-ABA and AL adhering to submucosa and skin in Example I-25.

[Fig. 51]

Fig. 51 shows the $^1$H NMR spectrum of a benzaldehyde-modified hyaluronic acid (HA-ABA) synthesized in Example II-1.

[Fig. 52]

Fig. 52 shows the UV-visible light absorption spectra (UV-vis) of the benzaldehyde-modified hyaluronic acid (HA-ABA) synthesized in Example II-1, hyaluronic acid (HA) and 4-aminobenzaldehyde (ABA).

[Fig. 53]

Fig. 53 shows the FT-IR spectra of the benzaldehyde-modified hyaluronic acid (HA-ABA) synthesized in Example II-1, hyaluronic acid (HA) and 4-aminobenzaldehyde (ABA).

[Fig. 54]

Fig. 54 shows cytotoxicity testing results (WST assay results) for hyaluronic acid (HA) and the benzaldehyde-modified hyaluronic acid (HA-ABA) synthesized in Example II-1 at different concentrations (0.01 mg/mL, 0.1 mg/mL and 1 mg/mL) with respect to MeT-5A cells (human mesothelial cell line), HUVEC cells (human umbilical vein endothelial cells), RAW264.7 cells (mouse macrophage-like cell line), NIH/3T3 cells (mouse embryo fibroblasts) and AB22 cells (mouse mesothelioma cells).

[Fig. 55]

Fig. 55 shows the $^1$H NMR spectra of a benzaldehyde-modified hyaluronic acid-pemetrexed conjugate (HA-ABA-PMX) synthesized in Example II-2, benzaldehyde-modified hyaluronic acid (HA-ABA), hyaluronic acid (HA) and pemetrexed (PMX).

[Fig. 56]

Fig. 56 shows the UV-visible light absorption spectra (UV-vis) of the benzaldehyde-modified hyaluronic acid-pemetrexed conjugate (HA-ABA-PMX) synthesized in Example II-2 and benzaldehyde-modified hyaluronic acid (HA-ABA).

[Fig. 57]

Fig. 57 shows the release behavior of pemetrexed (PMX) from a benzaldehyde-modified hyaluronic acid-pemetrexed conjugate solution (HA-ABA-PMX) under different pH conditions (pH = 5.0, 6.0, 7.4), the release behavior of PMX from a PMX solution (Free PMX), and the release behavior of PMX from a mixed solution of HA and PMX (Free PMX mixed with HA). The vertical axis shows the cumulative drug release rate (%) of PMX, which is calculated from the absorbance in UV-vis spectrum measurement.

[Fig. 58]

Fig. 58A and Fig. 58B show the cell growth inhibition effects of the benzaldehyde-modified hyaluronic acid-pemetrexed conjugate (HA-ABA-PMX) synthesized in Example II-2 with different PMX concentrations ($10^{-4}$ μg/mL, $10^{-3}$ μg/mL, $10^{-2}$ μg/mL, $10^{-1}$ μg/mL, 1 μg/mL, 10 μg/mL), free pemetrexed (PMX), and HA-ADH-PMX comprising PMX bound to HA by irreversible amide bonds with respect to AB22 cells (mouse mesothelioma cells) and MeT-5A (human mesothelial cell line), respectively. Cell viability (%) is shown on the vertical axis.

[Fig. 59]

Fig. 59 shows the UV-visible light absorption spectra (UV-vis) of a benzaldehyde-modified hyaluronic acid-doxorubicin conjugate synthesized in Example II-3 (HA-ABA-DOX), the benzaldehyde-modified hyaluronic acid synthesized in Example II-1 (HA-ABA), and doxorubicin (DOX).

[Fig. 60]

Fig. 60 shows the release behavior of doxorubicin (DOX) from a benzaldehyde-modified hyaluronic acid-doxorubicin conjugate (HA-ABA-DOX) under different pH conditions (pH = 5.0, 6.0, 7.4). The vertical axis shows the cumulative drug release rate (%) of DOX, which is calculated from the absorbance in UV-vis spectrum measurement.

[Fig. 61]

Fig. 61 shows the cell growth inhibition effects of the benzaldehyde-modified hyaluronic acid-doxorubicin conjugate (HA-ABA-DOX) synthesized in Example II-3 at different DOX concentrations ($10^{-3}$ μg/mL, $10^{-2}$ μg/mL, $10^{-1}$ μg/mL, 1 μg/mL, 10 μg/mL, 100 μg/mL) and free doxorubicin (DOX) with respect to AB22 cells (mouse mesothelioma cells). Cell viability (%) is shown on the vertical axis.

[Fig. 62]

Fig. 62 shows the UV-visible light spectra (UV-vis) of a benzaldehyde-modified hyaluronic acid-gemcitabine conjugate synthesized in Example II-4 (HA-ABA-GEM), the benzaldehyde-modified hyaluronic acid (HA-ABA) synthesized in Example II-1 and gemcitabine (GEM).

[Fig. 63]

Fig. 63 shows the $^1H$ NMR spectrum of a benzaldehyde-modified hyaluronic acid-adenine conjugate (HA-ABA-adenine) synthesized in Example 11-5.

[Fig. 64]

Fig. 64 shows the $^1H$ NMR spectrum of a benzaldehyde-modified hyaluronic acid-cytosine conjugate (HA-ABA-cytosine) synthesized in Example II-5.

[Fig. 65]

Fig. 65 shows the $^1H$ NMR spectrum of a benzaldehyde-modified hyaluronic acid-guanine conjugate (HA-ABA-guanine) synthesized in Example II-5.

[Fig. 66]

Fig. 66 shows the FT-IR spectra of the benzaldehyde-modified hyaluronic acid- DNA nucleotide conjugates (HA-ABA-adenine, HA-ABA-cytosine, HA-ABA-guanine) synthesized in Example II-5 and the benzaldehyde-modified hyaluronic acid (HA-ABA) synthesized in Example II-1.

[Fig. 67]

Fig. 67 shows the $^1H$ NMR spectrum of the benzaldehyde-modified carboxymethyl cellulose (CMC-ABA) synthesized in Example III-1.

[Fig. 68]

Fig. 68 shows the FT-IR spectra of the benzaldehyde-modified carboxymethyl cellulose (CMC-ABA) synthesized in Example III-1 and carboxymethyl cellulose (CMC).

[Fig. 69]

Fig. 69 shows the $^1H$ NMR spectrum of benzaldehyde-modified carboxymethyl dextran (CMDX-ABA) synthesized in Example IV-1.

[Fig. 70]

Fig. 70 shows the FT-IR spectra of benzaldehyde-modified carboxymethyl dextran (CMD-ABA) synthesized in Example IV-1 and carboxymethyl dextran (CMD).

[Fig. 71]

Fig. 71 shows the FT-IR spectra of benzaldehyde-modified carboxymethyl dextran-DNA nucleotide conjugates synthesized in Example IV-2 (CMD-ABA-adenine, CMD-ABA-cytosine, CMD-ABA-guanine), the benzaldehyde-modified carboxymethyl dextran (CMD-ABA) synthesized in Example IV-1 and carboxymethyl dextran (CMD).

[Fig. 72]

Fig. 72 shows the $^1H$ NMR spectrum of benzaldehyde-modified chitosan (Chitosan-CBA) synthesized in Example V-1.

[Fig. 73]

Fig. 73 shows the FT-IR spectrum of benzaldehyde-modified chitosan (Chitosan-CBA) synthesized in Example V-1.

[Fig. 74]

Fig. 74(A) is a schematic diagram showing a test procedure for evaluating adhesion to dura mater tissue according to a lap shear method in Example I-26. Fig. 74(B) shows the results obtained by measuring the adhesion strength (Pa) of various alginic acid derivatives (AL-ABA, AL-AFA, AL-AAP, AL-APA, and AL-ANA) and conventional tissue adhesives to dura mater tissue according to a lap shear method.

[Fig. 75A]

Fig. 75(A) is a schematic diagram showing a burst test method in Example 1-27. Fig. 75(B) shows the results obtained by measuring the burst pressure (mmHg) in a burst test for various alginic acid derivatives (AL-ABA, AL-AFA, AL-AAP, AL-APA, and AL-ANA) and conventional tissue adhesives adhered to dura mater tissue in Example I-27. Samples ending with "Neovil" are samples in which Neovil (registered trademark) was used as a nonwoven fabric, and samples ending with "Double Vicryl mesh" and "Single Vicryl mesh" are samples in which two sheets and one sheet of "Ethicon Vicryl Mesh" were used as a nonwoven fabric.

[Fig. 75B]

Fig. 75(C) shows the results obtained by measuring the burst pressure (mmHg) in a burst test for various alginic acid derivatives (AL-ABA, AL-AFA, AL-AAP, AL-APA, and AL-ANA) and conventional tissue adhesives adhered to dura mater tissue in Example I-27. Samples ending with "Neovil" are samples in which Neovil (registered trademark) was used as a nonwoven fabric, and samples ending with "Double Vicryl mesh" and "Single Vicryl mesh" are samples in which two sheets and one sheet of "Ethicon Vicryl Mesh" were used as a nonwoven fabric.

[Fig. 76]

Fig. 76 shows the results of viscoelastic analysis (flow curve) in Example 1-28.

[Fig. 77]

Fig. 77 shows the results of a frequency sweep test in Example 1-28.

[Fig. 78]

Fig. 78 shows the results of a hemolysis test in Example I-29.
[Fig. 79]
Fig. 79 shows the $^1$H NMR spectrum of dendritic poly(ethyleneimine) (DPIGA) conjugated with gallic acid synthesized in Example 1-30. The spectra of gallic acid (GA) and dendritic poly(ethyleneimine) (DPI) are also shown.
[Fig. 80]
Fig. 80 shows the FT-IR spectrum of DPIGA synthesized in Example 1-30. The spectra of GA and DPI are also shown.
[Fig. 81]
Fig. 81 shows the UV-visible light absorption spectrum (UV-vis) of DPIGA synthesized in Example 1-30. The spectra of GA and DPI are also shown.
[Fig. 82]
Fig. 82(A) shows the results obtained by measuring the adhesion strength (Pa) of the conjugate (crosslinked structure) (AL-ABA/DPI-GA) of AL-ABA and a GA-modified amino group-containing polymer prepared in Example I-30 to skin tissue or submucosa according to a lap shear method. AL-ABA and DPI-GA were used as controls. Fig. 82(B) is a schematic diagram showing a binding form of a conjugate (crosslinked structure) of AL-ABA and a GA-modified amino group-containing polymer to tissue.
[Fig. 83]
Fig. 83(A) shows the $^1$H NMR spectrum of benzaldehyde-modified chitosan (CH-CBA) synthesized in Example V-2. Fig. 83(B) shows the FT-IR spectrum of CH-CBA prepared in Example V-2. Fig. 83(C) shows the UV-visible light absorption spectrum (UV-vis) of CH-CBA prepared in Example V-2.
[Fig. 84]
Fig. 84 shows the results of a gelling test at different pH values measured by a vial inversion method for CH-CBA prepared in Example V-2.
[Fig. 85]
Fig. 85 shows the results of viscoelastic analysis for CH-CBA with different pH values prepared in Example V-2. Fig. 85(A) is a diagram showing a storage modulus (G') and a loss modulus (G") of CH-CBA hydrogel with different pH values, Fig. 85(B) is a diagram in which the loss factor (tan$\delta$) of CH-CBA hydrogel with different pH values is plotted, and Fig. 85(C) is a diagram in which the viscosity ($\eta$) when the shear rate for CH-CBA hydrogel with different pH values is changed is plotted.
[Fig. 86]
Fig. 86 is a diagram showing the relationship between the pH of CH-CBA prepared in Example V-2 and the viscosity ratio ($\eta_{0.001}/\eta_{1000}$) of a viscosity ($\eta_{0.001}$) at a shear rate of $10^{-3}$ s' $^1$ to a viscosity ($\eta_{1000}$) at a shear rate of $10^3$ $s^{-1}$.
[Fig. 87]
Fig. 87 shows SEM images of CH-CBA hydrogels gelled at different pH values prepared in Example V-2.
[Fig. 88]

Fig. 88(A) is a schematic diagram showing the pH-responsive self-crosslinking property of CH-CBA hydrogel.
Fig. 88(B) is a schematic diagram showing adhesion of CH-CBA hydrogel to tissue.

[Fig. 89]
Fig. 89(A) shows the cell toxicity test results (WST-8 assay results) of CH-CBA prepared in Example V-2. Fig. 89(B) shows the Live/Dead cell viability assay results of CH-CBA prepared in Example V-2. The results for control groups, Fibrin Glue and albumin/glutaraldehyde (Albumin/GTA), are also shown.
[Fig. 90]
Fig. 90 is a schematic diagram showing a method for a lap shear test using CH-CBA hydrogel.
[Fig. 91A]
Figs. 91(A) and 91(B) show the results of a tack test of CH-CBA hydrogel with different pH values prepared in Example V-2. Fig. 91(C) shows the results of a lap shear test of CH-CBA hydrogel with different pH values prepared in Example V-2 with respect to mucosa.
[Fig. 91B]
Figs. 91(D) to 91(E) show the results of a lap shear test of CH-CBA hydrogel with different pH values prepared in Example V-2 with respect to submucosa tissue and skin.

[Description of Embodiments]

In this Description, a "halogen atom" is a fluorine atom (F), chlorine atom (Cl), bromine atom (Br) or iodine atom (I).
In this Description, a "$C_{1-k}$ alkyl" is a linear or branched alkyl group with 1 to k carbon atoms.
In this Description, a "$C_{1-k}$ alkylene" is a linear or branched alkylene group with 1 to k carbon atoms.
In this Description, a "polysaccharide" means a compound consisting of two or more monosaccharides linked by

glycoside bonds, a derivative thereof (for example a polysaccharide modified by esterification, maleimide modification, thiol modification, acrylate modification, aldehyde modification, disulfide (such as pyridyl disulfide) modification, alkyne (including cyclic alkyne) modification, tetrazine modification, furan ring modification or the like), or a salt or crosslinked body thereof. One consisting of 2 to 10 linked monosaccharides may be called an oligosaccharide, but in this Description the term "polysaccharide" encompasses oligosaccharides in addition to the narrow definition of "polysaccharide" meaning more than 10 linked monosaccharides.

In this Description, a "polysaccharide derivative-drug conjugate" is a complex formed by linking a drug to a polysaccharide derivative via bonds.

I. First aspect

A first aspect of the present invention relates to a tissue adhesive material used for tissue that has adhesion not only to soft tissue in vivo but also relatively hard and thick tissue and/or connective tissue such as dura mater. The tissue adhesive material of this aspect is a tissue adhesive material used for hard and thick tissue and/or connective tissue, including a polysaccharide derivative in which a group represented by Formula (A) below is introduced into an acidic, basic or amphoteric polysaccharide (hereinafter simply referred to as "polysaccharide derivatives"). In certain embodiments, the tissue adhesive material is used to repair/restore biological tissue.

[C14]

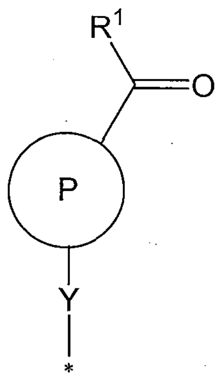

(A)

(in the formula, $R^1$ represents a hydrogen atom or a $C_{1-4}$ alkyl group,

the ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
Y represents a divalent group selected from the group consisting of -NH-, -C(=O)-, -S-, -O-, a $C_{1-6}$ alkylene group, $-(CH_2CH_2O)_n-$, and any combinations of these, and n is an integer from 1 to 9, and
* represents a linkage with the polysaccharide).

In this specification, "hard and thick tissue" is a tissue other than solid biological components containing calcium and phosphate such as bones and teeth and refers to a membrane-like, tubular, or flat tissue that contains collagen such as dura mater, perichondrium, periodontal ligament, and cartilage or a tissue present at the epiphysis. The thickness of such a "hard and thick tissue" is, for example, in a range of 0.1 to 10 mm.

"Connective tissue" is derived from the mesenchyme, and is tissue that connects various organs and tissues, fills the gaps and supports them. Connective tissue is made up of fibers such as collagen fibers and elastic fibers and connective tissue cells such as fibrocytes, lymphocytes, plasma cells, and white blood cells. Depending on the types of constituting fibers and cells, they are classified into gelatinous tissue, reticular tissue, fibrillar connective tissue, elastic tissue, adipose tissue, pigment tissue or the like, and the shapes and structures of connective tissue are diverse. Connective tissues include, for example, fascia, tendons, ligaments, bursae, cartilage, periosteum, dura mater, sclera, and dermis.

Here, although there are some tissues that overlap between "hard and thick tissue" and "connective tissue," the tissue adhesive material in the first aspect can be preferably applied if the tissue falls into either group.

The target hard and thick tissue and connective tissue are found throughout the body.

In specific embodiments, the hard and thick tissue and/or connective tissue is selected from among dura mater, perichondrium, synovium, periodontal ligament, and elastic cartilage.

In some embodiments, the hard and thick tissue and/or connective tissue is selected from among dura mater, perichondrium, synovium, and periodontal ligament.

One of the representative hard and thick tissue and/or connective tissue is dura mater tissue. The dura mater is a hard membrane that covers the brain and spinal cord, and is divided into the cerebral dura mater, which covers the brain, and the spinal dura mater, which covers the spinal cord. The structures beneath the dura mater include the arachnoid mater, pia mater, brain/spinal cord parenchyma. The dura mater, arachnoid mater and pia mater are collectively called the meninges, and the dura mater has a function of protecting the brain and spinal cord from injury and infection. The dura mater is composed of two layers: an inner layer (also called the inner leaf or inner plate) and an outer layer (also called the outer leaf or outer plate). In the spinal cord, there is a space called the epidural cavity (epidural space) between the outer layer and the inner layer, but in the brain, they are in close contact in most areas except for the cranial sinus. There is a space called the subdural cavity between the dura mater and the inner arachnoid membrane. In this specification, the dura mater may be either the cerebral dura mater or the spinal dura mater. The perichondrium is a membrane that covers the cartilage of knees, elbows, hip joints, fingers and the like, and the synovium is a membrane that particularly covers the joints of the knees, elbows, fingers and the like. Both are membranes that contain the synovial fluid in the joint cavity.

The dura mater tissue has primary amino groups on its surface. Specifically, the main component of the dura mater is collagen, and there are many lysine residues (Lys) containing amino groups at crosslinking sites of collagen. According to the tissue adhesive material of this aspect, when a Schiff base is formed between the amino group of the lysine residue (Lys) of an amino acid abundant in collagen and the modifying group (A) of the polysaccharide derivative (the aldehyde group or the ketone group ($-C(=O)R^1$)), favorable adhesion to dura mater tissue is exhibited.

In preferable embodiments, the tissue adhesive material is used for dura mater tissue.

Collagen is a so-called membrane-like main component in vivo, and the fact that the tissue adhesive material of this aspect exhibits adhesion to the dura mater indicates that the tissue adhesive material of this aspect has adhesion to membrane components other than the dura mater. In examples to be described below, dura mater is used to exhibit adhesion to hard and thick tissue and/or connective tissue. The reason for focusing on dura mater is that the dura mater is a relatively hard and thick material among soft tissues and membrane-like components in the body, and stronger adhesion is required to withstand the leakage pressure of the spinal fluid under the dura mater. Since the tissue adhesive material of this aspect has appropriate flexibility in addition to favorable adhesion, it has excellent resistance to the leakage pressure of the spinal fluid.

In addition, the polysaccharide derivative such as an alginic acid derivative, a hyaluronic acid derivative, and a carboxymethyl dextran derivative forms a hydrophobic interaction and a hydrogen bond (for example, a hydrogen bond between the hydroxyl group of the hydroxyproline residue in collagen and the hydroxyl group or carboxyl group of the polysaccharide derivative) with tissue proteins of the tissue, and the adhesion to tissue can be improved.

Other examples of representative hard and thick tissue and/or connective tissue include perichondrium and periodontal ligament.

Perichondrium is connective tissue that covers the surface of cartilage. For example, when a cartilage fragment is transplanted in order to fill a cartilage defect, it can be used as a substitute for perichondrium or to cover the perichondrium defect with a tissue adhesive material of this aspect. Not only donor cartilage but also cartilage implants cultured in vitro, stem cells and the like can be mixed with the tissue adhesive material, and filled into the defect and simultaneously adhered to the defect, and there is an advantage that there is no need to use a covering material in addition to the filler.

The periodontal ligament is a membrane that connects the alveolar bone and the tooth root, has a thickness of about 0.3 mm, and contains a large amount of collagen. The tissue adhesive material of this aspect can also be used to adhere the alveolar bone and the periodontal ligament and adhere the gums and the periodontal ligament in dentistry.

In the first aspect, the tissue adhesive material is a material used to repair/restore biological tissue, has adhesion not only to soft tissue in vivo but also relatively hard and thick tissue and/or connective tissue such as the dura mater, is used to restore/reinforce tissue and its injury site, and has a function of preventing tissue break/damage from expanding and increasing the physical strength of the reinforced site.

The application of the tissue adhesive material is not particularly limited, and for example, the tissue adhesive material is used to repair the dura mater, which is the outermost layer of the meninges, during surgery for brain injury, brain tumor, or cerebral hemorrhage in brain surgery, that is, to repair and restore incisions in the cerebral dura mater, and used as an adhesive when filling defects with artificial dura mater (dura mater repair and protection). In orthopedics, during spinal and spinal cord surgery, it is used to repair and protect the spinal cord membrane, particularly, the dura mater. In addition, in otolaryngology, it is also used for adhesion when transplanting and repairing elastic cartilage such as conchal cartilage, nasal cartilage, and epiglottic cartilage.

The specific site of the biological tissue is not particularly limited, and sites where the tissue adhesive material has been conventionally used may be appropriately used. Examples of such application sites include injury sites, suture sites after surgery, and adhesion sites at bleeding sites.

In certain embodiments, the tissue adhesive material is used to restore and reproduce dura mater damage and prevent spinal fluid fistula.

In certain embodiments, the tissue adhesive material is used to prevent restoring and reproduction of perichondrium damage and to fill a cartilage defect when a cartilage fragment is transplanted. In addition, in certain embodiments, the tissue adhesive material is used in the damaged site of synovium in order to restore and protect joints with respect to damage to joints in the knees, elbows, fingers and the like.

In certain embodiments, the tissue adhesive material is used to prevent restoring and reproduction of periodontal ligament damage, to adhere the alveolar bone and periodontal ligament in dentistry, or to adhere the gums and periodontal ligament.

Conventional tissue adhesive materials include 1) a cyanoacrylate adhesive (Dermabond, Liquiband), 2) a gelatin-aldehyde adhesive (Bioglue), 3) a fibrin glue adhesive (Veriplast, Bolheal), and 4) other materials (hydrofit (matsudaito)).

The 1) cyanoacrylate adhesive utilizes a polymerization reaction of cyanoacrylate monomers and has high adhesion strength. In addition, it has an advantage of a short time required for adhesion. However, after the material is fixed, the polymer is hydrolyzed in vivo, and thus formaldehyde is generated, which is highly toxic to the body. Curing of the fixed site may be inhibited. Therefore, there is a problem that it is necessary to avoid use of the adhesive in sites that come into direct contact with the central nervous system or blood vessels in the body.

The 2) gelatin-aldehyde adhesive is an adhesive that utilizes a crosslinking reaction of the biopolymer gelatin with formaldehyde and glutaraldehyde. The adhesive has high adhesive power and polymer strength after fixing, but it is biotoxic because it utilizes a toxic formaldehyde compound as a crosslinking agent.

The 3) fibrin glue adhesive is an adhesive that utilizes a biological component in a blood coagulation reaction. It utilizes fibrin formation in blood, is non-toxic to biological tissue, and most commonly used as a tissue adhesive used in vivo. Since it is derived from a biological component, it has high affinity with biological tissue and does not inhibit tissue reproduction at the site of use, but it is soft as an adhesive, and has weak adhesive power. In addition, the adhesive is mixed immediately before use, but there are problems that it requires much effort and preparation time in advance such as the need to prepare two types of solutions at the same time and assemble a dedicated filling instrument. In addition, another problem is that the adhesive that has dissolved once cannot be left because its use time is short. In addition, since the source of raw fibrin is human blood, it is difficult to secure raw blood and avoid the risk of contamination with pathogenic viruses.

The 4) other materials include, for example, a urethane material adhesive. The urethane material is non-absorbable, and is mainly used for localized hemostasis. It is a viscous liquid composed of a polyether-based fluorine-containing urethane prepolymer, reacts with water in blood and biological tissue at vascular anastomosis to polymerize and becomes a gel-like polymer that adheres closely to vascular anastomosis, and stops bleeding.

On the other hand, the tissue adhesive material of this aspect utilizes a polysaccharide component such as alginic acid, and has advantages of being able to avoid the use of toxic formaldehyde for 1), 2), being able to avoid the blood-borne infection risk for 3), and being able to prevent the material from remaining in vivo because it is non-absorbable for 4). In addition, in contrast to the 1) cyanoacrylate adhesive material, which adheres and fixes strongly and quickly, the tissue adhesive material of this aspect has appropriate flexibility (viscosity) even after adhesion. Thereby, the tissue adhesive material of this aspect has an advantage of excellent resistance to the leakage pressure while preventing leakage of a liquid from the adhesion site (space).

Thus, the tissue adhesive material of this aspect is excellent in terms of biocompatibility and safety when considering its use in vivo. In addition, it is thought that, depending on the site of use, tissue that is very wet and rich in blood and body fluids should be adhered, but the tissue adhesive material of this aspect can exhibit its function by directly adhering to proteins constituting the tissue even under such wet conditions. In addition, conventional tissue adhesives are used to adhere soft tissue to soft tissue in vivo (for example, for vascular anastomosis or to attach a bioabsorbable material to repair tissue removed by surgery), but it is found that they can also adhere to hard tissue such as the dura mater which is the outermost layer of the meninges, which indicates an improvement as a new tissue adhesive material, and will broaden the range of use in the medical field.

The favorable tissue adhesive of the tissue adhesive material of this aspect to dura mater tissue is shown in, for example, Example I-26 and Example 1-27 to be described below. Particularly, as shown in Example I-26 to be described below (Fig. 74(B)), many conventional tissue adhesives (DuraSeal (registered trademark) (a gel of activated esterterminated PEG and trilysin using NHS) and fibrin glue) have poor adhesion to the dura mater, and the tissue adhesive material of this aspect having excellent adhesion to dura mater tissue is highly useful. In the tissue adhesive material of this aspect, it is presumed that the benzaldehyde group of the polysaccharide derivative has appropriate hydrophobicity, abundant hydroxyl groups in the polysaccharides contribute to hydrophilicity, and such excellent properties such as adhesion are exhibited.

1. Polysaccharide derivative

In the present invention, in the polysaccharide derivative, a group represented by Formula (A) below (hereinafter

simply referred to as a "modifying group (A)") is introduced into an acidic, basic, or amphoteric polysaccharide.

[C15]

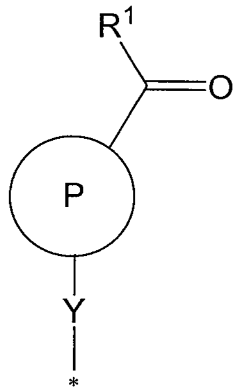

(A)

The polysaccharide derivative can react with a primary amino group to form a Schiff base under neutral to basic pH conditions. While the Schiff base formed between the polysaccharide derivative and the primary amino group is stable under neutral to basic pH conditions, it can dissociate under low pH conditions. This property makes it useful as a drug delivery carrier, a bioabsorbable material, a medical device, or a separation material device.

The polysaccharide derivative of this embodiment has at least one of the following advantages.

(1) The polysaccharide derivative can form a stable conjugate under neutral to basic pH conditions with various drugs having primary amino groups, and can also release drugs having primary amino groups. In particular, because of its aromatic structure the modifying group (A) is capable of forming stable Schiff bases with amine groups contained in various drugs even in water.

(2) Because the polysaccharide derivative is made from an acidic, basic or amphoteric polysaccharide, the modifying group (A) can be introduced at a good modification rate even if the modifying group (A) has a hydrophobic benzene ring or pyridine ring. Furthermore, even if the modifying group (A) has been introduced at a high modification rate, the resulting polysaccharide derivative has good water solubility due to the presence of unmodified acidic or basic functional groups (anionic groups or cationic functional groups). In particular, a polysaccharide having anionic functional groups (such as carboxyl groups) can yield a polysaccharide derivative with especially good water solubility even if the modification rate by the modifying group (A) is high.

(3) Because the polysaccharide has functional groups that are acidic, basic or both (amphoteric) (that is, functional groups having charge), it can be reacted with a highly hydrophobic phenyl aldehyde or pyridyl aldehyde compound in one stage under mild and safe conditions to easily introduce the modifying group (A) into the polysaccharide.

(4) In certain embodiments, a stable polysaccharide derivative-drug conjugate can be obtained easily and in one pot under one-stage mild and highly safe reaction conditions. Because there is no need to modify the drug before conjugate formation, and since complex experimental equipment and catalysts are also unnecessary for conjugate formation, in-situ preparation of the polysaccharide derivative-drug conjugate from the polysaccharide derivative and the drug is also possible.

(5) In certain embodiments, the polysaccharide derivative and the conjugate of the polysaccharide derivative and the drug can be subjected to a crosslinking reaction with a crosslinking agent to form crosslinked structures in a variety of forms (such as tube structures, fiber structures, fibers, beads, gels, semispherical gels, capsules, sponges and sheets). Furthermore, in certain embodiments the polysaccharide derivative and the conjugate of the polysaccharide derivative and the drug can form crosslinked structures of various forms with amino group-containing polymers.

(Modifying group (A): group represented by formula (A))

In the formula (A), $R^1$ represents a hydrogen atom or $C_{1-4}$ alkyl. Preferably $R^1$ is a hydrogen atom or a methyl, ethyl, n-propyl, isopropyl, tert-butyl, sec-butyl, iso-butyl or n-butyl group, or more preferably a hydrogen atom or a methyl group. In one embodiment, $R^1$ is a hydrogen atom.

In the formula (A), * is a linkage with the polysaccharide.

In the formula (A), ring P is a phenyl ring or pyridine ring. This phenyl ring or pyridine ring is optionally substituted with one or more substituents independently selected from the halogen atoms (F, Cl, Br and/or I) and $-CF_3$, $-NO_2$, carboxyl and $-SO_3H$ groups.

In one embodiment, ring P is a phenyl ring, and this phenyl ring is optionally substituted with 1 to 4 substituents selected from the halogen atoms (F, Cl, Br and/or I) and - $CF_3$, $-NO_2$, carboxyl and $-SO_3H$ groups. In one embodiment, ring P is an unsubstituted phenyl ring.

In one embodiment, ring P is a pyridine ring, and the pyridine ring is optionally substituted with 1 to 3 substituents independently selected from the halogen atoms (F, Cl, Br and/or I) and $-CF_3$, $-NO_2$, carboxyl and $-SO_3H$ groups. In one embodiment, ring P is an unsubstituted pyridine ring.

In the formula (A), Y represents a divalent group selected from the group consisting of -NH-, -C(=O)-, -S-, -O-, the alkylenes and $-(CH_2CH_2O)_n-$ and any combinations of these, with n being an integer from 1 to 9 (preferably from 1 to 4, or more preferably from 1 to 2). From the standpoint of hydrophilicity, the carbon number of the alkylene group of Y is 1 to 6, or preferably 1 to 4, or more preferably 1 or 2, or still more preferably 1.

In certain embodiments, Y is selected from $-L^1$-NH- or $-L^2$-C(=O), in which case $L^1$ and $L^2$ bind to ring P. That is, the modifying group (A) is selected from the following formulae (A-1) and (A-2):

[C16]

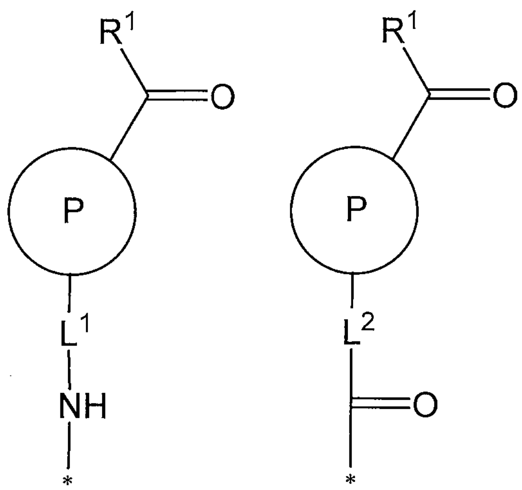

(A-1) (A-2)

In the formulae (A-1) and (A-2), * represents a linkage with the polysaccharide. The definitions and preferred embodiments of $R^1$ and ring P in the formulae (A-1) and (A-2) are the same as the definitions and preferred embodiments of $R^1$ and ring P in the formula (A) above.

In formula (A-1), $L^1$ is a single bond or represents a divalent group selected from the group consisting of -C(=O)-, -S-, -O-, the alkylenes and $-(CH_2CH_2O)_n-$ and any combinations of these, and n is an integer from 1 to 9 (preferably from 1 to 4, or more preferably from 1 to 2). From the standpoint of hydrophilicity, the carbon number of the alkylene group of $L^1$ is preferably 1 to 6, or more preferably 1 or 4, or still more preferably 1 or 2, or yet more preferably 1.

In particular, $L^1$ is selected from a single bond, a $C_{1-6}$ alkylene, $-(CH_2CH_2O)_n-$, - $(CH_2)_{m1}-(CH_2CH_2O)_n-(CH_2)_{m2}-$ and $(CH_2)_{m1}-O-(CH_2CH_2O)_n-(CH_2)_{m2}-$, with n being an integer from 1 to 9 (preferably from 1 to 4, or more preferably from 1 to 2). m1 and m2 are each independently integers from 1 to 9 (preferably from 1 to 4, or more preferably from 1 to 2). From the standpoint of hydrophilicity, the $C_{1-6}$ alkylene group of $L^1$ is preferably a $C_{1-4}$ alkylene, or more preferably a $C_{1-2}$ alkylene, or still more preferably methylene.

In one embodiment, $L^1$ is a single bond or a $C_{1-2}$ alkylene. In one embodiment, $L^1$ is a single bond.

In formula (A-2), $L^2$ is a single bond or represents a divalent group selected from the group consisting of -NH-, -S-, -O-, the alkylenes and $-(CH_2CH_2O)_n-$ and any combinations of these, with n being an integer from 1 to 9 (preferably from 1 to 4, or more preferably from 1 to 2). From the standpoint of hydrophilicity, the carbon number of the alkylene group in $L^2$ is 1 to 6, or preferably 1 to 4, or more preferably 1 or 2, or still more preferably 1.

In particular, $L^2$ is selected from a single bond, a $C_{1-6}$ alkylene, $-(CH_2CH_2O)_n-$, - $(CH_2)_{m1}-(CH_2CH_2O)_n-(CH_2)_{m2}-$ and $(CH_2)m1-O-(CH_2CH_2O)_n-(CH_2)_{m2}-$, with n being an integer from 1 to 9 (preferably from 1 to 4, or more preferably from 1 to 2). m1 and m2 are each independently integers from 1 to 9 (preferably from 1 to 4, or more preferably from 1 to 2). From the standpoint of hydrophilicity, the $C_{1-6}$ alkylene group of $L^2$ is preferably a $C_{1-4}$ alkylene, or more preferably a $C_{1-2}$ alkylene, or still more preferably methylene.

In one embodiment, $L^2$ is a single bond or a $C_{1-2}$ alkylene. In one embodiment, $L^2$ is a single bond.

In certain embodiments, the modifying group (A) is a group represented by formula (A-1) above, ring P is a phenyl

ring, and this phenyl ring is optionally substituted with 1 to 4 substituents independently selected from the halogen atoms (F, Cl, Br and/or I) and $-CF_3$, $-NO_2$, carboxyl and $-SO_3H$ groups.

For example, modifying group (A) is selected from the following formulae (1), (2) and (3) (sometimes called "formulae (1) to (3)" below):

[C17]

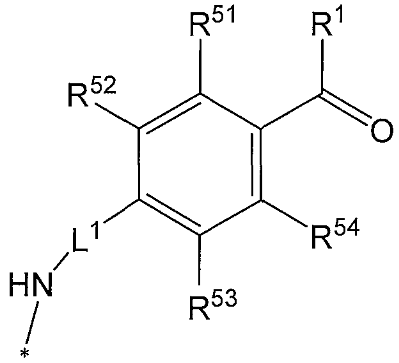

(1)

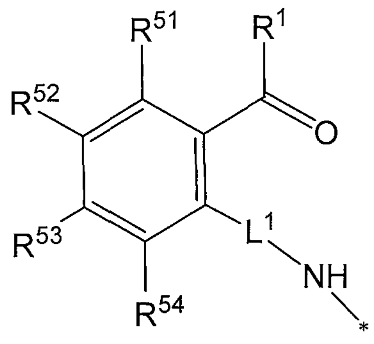

(2)

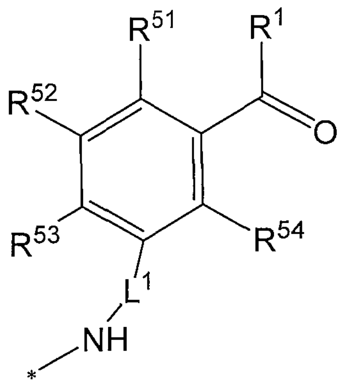

(3)

In formulae (1) to (3), * is a linkage with the polysaccharide. Furthermore, in formulae (1) to (3) the definitions and preferred embodiments of $R^1$ are the same as the definitions and preferred embodiments of $R^1$ in the formula (A), and the definitions and preferred embodiments of $L^1$ are the same as the definitions and preferred embodiments of $L^1$ in the formula (A-1).

In formulae (1) to (3) above, each of $R^{51}$, $R^{52}$, $R^{53}$ and $R^{54}$ is independently selected from a hydrogen atom, a halogen atom (F, Cl, Br, I), $-CF_3$, $-NO_2$, a carboxyl group and $-SO_3H$. In one embodiment, $R^{51}$, $R^{52}$, $R^{53}$ and $R^{54}$ are all hydrogen atoms. In one embodiment, each of $R^{51}$, $R^{52}$, $R^{53}$ and $R^{54}$ is independently selected from a hydrogen atom and a halogen atom (F, Cl, Br, or I; preferably F).

In specific embodiments, in formulae (1) to (3) above $R^1$ is a hydrogen atom, $L^1$ is a single bond, and each of $R^{51}$, $R^{52}$, $R^{53}$ and $R^{54}$ is independently selected from a hydrogen atom, a halogen atom (F, Cl, Br, I), $-CF_3$, $-NO_2$, a carboxyl group and $-SO_3H$. In one embodiment, $R^1$ in formulae (1) to (3) above is a hydrogen atom, $L^1$ is a single bond, and $R^{51}$, $R^{52}$, $R^{53}$ and $R^{54}$ are all hydrogen atoms. In one embodiment, $R^1$ in formulae (1) to (3) above is a hydrogen atom, $L^1$ is a single bond, and each of $R^{51}$, $R^{52}$, $R^{53}$ and $R^{54}$ is independently a hydrogen atom or a halogen atom (F, Cl, Br, or I; preferably F).

In specific embodiments, in formulae (1) to (3) $R^1$ is a methyl group, $L^1$ is a single bond, and each of $R^{51}$, $R^{52}$, $R^{53}$ and $R^{54}$ is independently selected from a hydrogen atom, a halogen atom (F, Cl, Br, I), $-CF_3$, $-NO_2$, a carboxyl group and $-SO_3H$. In one embodiment, $R^1$ in formulae (1) to (3) is a methyl group, $L^1$ is a single bond, and $R^{51}$, $R^{52}$, $R^{53}$ and $R^{54}$ are all hydrogen atoms. In one embodiment, $R^1$ in formulae (1) to (3) is a methyl group, $L^1$ is a single bond, and each of $R^{51}$, $R^{52}$, $R^{53}$ and $R^{54}$ is independently a hydrogen atom or a halogen atom (F, Cl, Br, or I; preferably F).

In specific embodiments, in formulae (1) to (3) above $R^1$ is a hydrogen atom, $L^1$ is a methylene ($-CH_2$) or ethylene ($-C_2H_4$), and each of $R^{51}$, $R^{52}$, $R^{53}$ and $R^{54}$ is independently selected from a hydrogen atom, a halogen atom (F, Cl, Br, I), $-CF_3$, $-NO_2$, a carboxyl group and $-SO_3H$. In one embodiment, $R^1$ in formulae (1) to (3) is a hydrogen atom, $L^1$ is a methylene ($-CH_2$) or ethylene ($-C_2H_4$), and $R^{51}$, $R^{52}$, $R^{53}$ and $R^{54}$ are all hydrogen atoms. In one embodiment, $R^1$ in formulae (1) to (3) is a hydrogen atom, $L^1$ is a methylene ($-CH_2$) or ethylene ($-C_2H_4$), and each of $R^{51}$, $R^{52}$, $R^{53}$ and $R^{54}$ is independently a hydrogen atom or a halogen atom (F, Cl, Br, or I; preferably F).

In specific embodiments, in formulae (1) to (3) $R^1$ is a methyl, $L^1$ is a methylene ($-CH_2$) or ethylene ($-C_2H_4$), and each of $R^{51}$, $R^{52}$, $R^{53}$ and $R^{54}$ is independently selected from a hydrogen atom, a halogen atom (F, Cl, Br, I), $-CF_3$, $-NO_2$, a carboxyl group and $-SO_3H$. In one embodiment, $R^1$ in formulae (1) to (3) is a methyl, $L^1$ is a methylene ($-CH_2$) or ethylene ($-C_2H_4$), and $R^{51}$, $R^{52}$, $R^{53}$ and $R^{54}$ are all hydrogen atoms. In one embodiment, $R^1$ in formulae (1) to (3) is a methyl, $L^1$ is a methylene ($-CH_2$) or ethylene ($-C_2H_4$), and each of $R^{51}$, $R^{52}$, $R^{53}$ and $R^{54}$ is independently a hydrogen atom or a halogen atom (F, Cl, Br, or I; preferably F).

In one embodiment, the modifying group (A) is selected from the groups represented by the following formulae:

[C18]

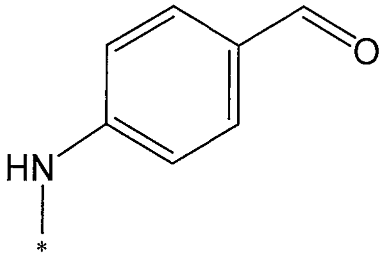
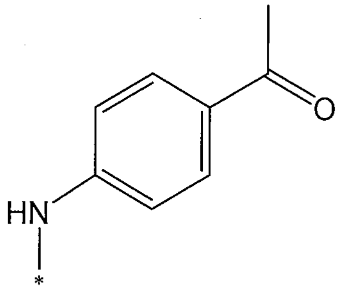
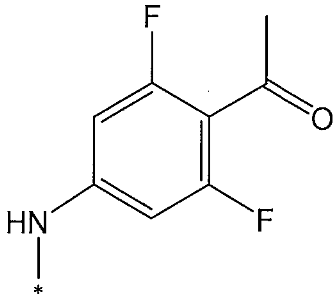

ABA AAP ADFBA (AFA)

(in the formulae, * represents a linkage with the polysaccharide).

In one embodiment, the modifying group (A) is a group represented by the following formula:

[C19]

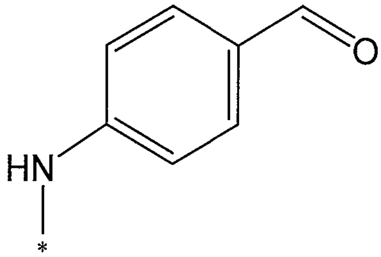

(in the formula, * represents a linkage with the polysaccharide).

In certain embodiments, the modifying group (A) is a group represented by formula (A-1) above, ring P is a pyridine ring, and the pyridine ring is optionally substituted with 1 to 3 substituents independently selected from the halogen atoms (F, Cl, Br and/or I) and $-CF_3$, $-NO_2$, carboxyl and $-SO_3H$ groups.

For example, the modifying group (A) is selected from the following formulae (4), (5), (6), (7), (8), (9), (10), (11), (12) and (13) (hereunder sometimes called "formulae (4) to (13)"):

[C20]

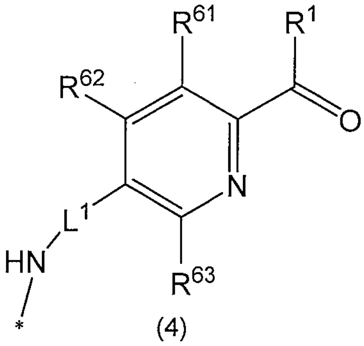
(4)

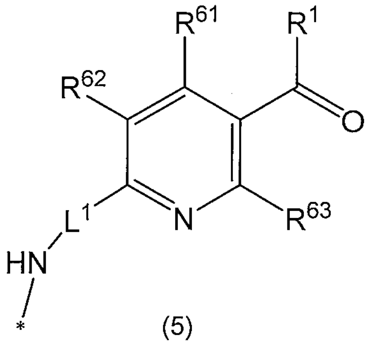
(5)

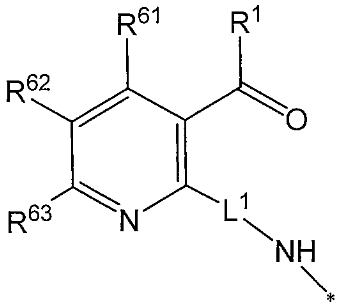
(6)

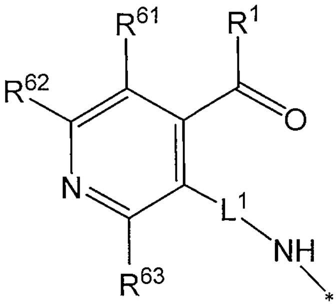
(7)

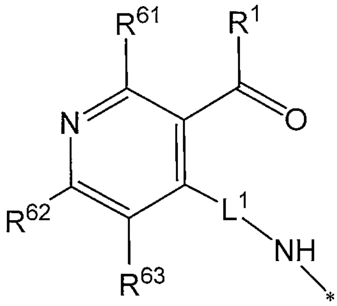
(8)

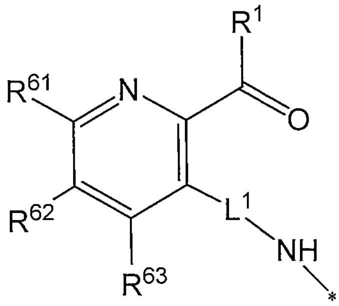
(9)

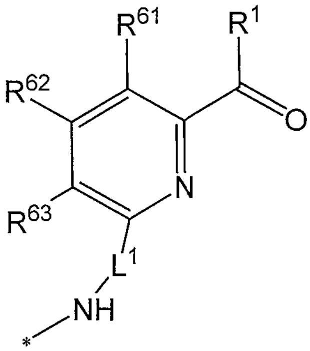
(10)

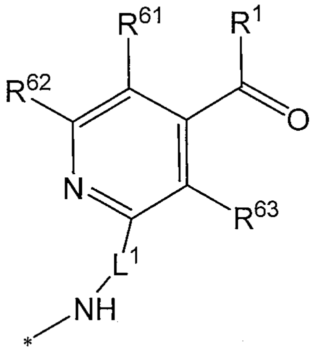
(11)

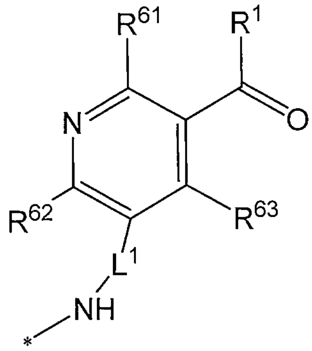
(12)

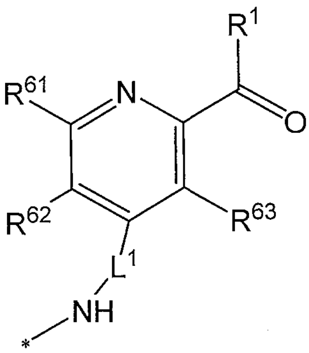
(13)

In formulae (4) to (13) above, * represents a linkage with the polysaccharide. The definition and preferred embodiments of $R^1$ in formulae (4) to (13) above are the same as the definition and preferred embodiments of $R^1$ in the formula (A), and the definition and preferred embodiments of $L^1$ are the same as the definition and preferred embodiments of $L^1$ in the formula (A-1).

In the formulae (4) to (13) above, each of $R^{61}$, $R^{62}$ and $R^{63}$ is independently selected from a hydrogen atom, a halogen atom (F, Cl, Br, I), $-CF_3$, $-NO_2$, a carboxyl group and $-SO_3H$. In one embodiment, $R^{61}$, $R^{62}$ and $R^{63}$ are all hydrogen atoms. In one embodiment, each of $R^{61}$, $R^{62}$ and $R^{63}$ is independently selected from a hydrogen atom and a halogen atom

(F, Cl, Br, I).

In specific embodiments, in the formulae (4) to (13) $R^1$ is a hydrogen atom, $L^1$ is a single bond, and each of $R^{61}$, $R^{62}$ and $R^{63}$ is independently selected from a hydrogen atom, a halogen atom (F, Cl, Br, I), $-CF_3$, $-NO_2$, a carboxyl group and $-SO_3H$. In one embodiment, $R^1$ in the formulae (4) to (13) is a hydrogen atom, $L^1$ is a single bond, and $R^{61}$, $R^{62}$ and $R^{63}$ are all hydrogen atoms. In one embodiment, $R^1$ in the formulae (4) to (13) is a hydrogen atom, $L^1$ is a single bond, and each of $R^{61}$, $R^{62}$ and $R^{63}$ is independently a hydrogen atom or a halogen atom (F, Cl, Br, or I; preferably F).

In specific embodiments, in the formulae (4) to (13) $R^1$ is a methyl group, $L^1$ is a single bond, and each of $R^{61}$, $R^{62}$ and $R^{63}$ is independently selected from a hydrogen atom, a halogen atom (F, Cl, Br, I), $-CF_3$, $-NO_2$, a carboxyl group and $-SO_3H$. In one embodiment, $R^1$ in the formulae (4) to (13) is a methyl group, $L^1$ is a single bond, and $R^{61}$, $R^{62}$ and $R^{63}$ are all hydrogen atoms. In one embodiment, $R^1$ in the formulae (4) to (13) is a methyl group, $L^1$ is a single bond, and each of $R^{61}$, $R^{62}$ and $R^{63}$ is independently a hydrogen atom or a halogen atom (F, Cl, Br, or I; preferably F).

In specific embodiments, in the formulae (4) to (13) $R^1$ is a hydrogen atom, $L^1$ is a methylene ($-CH_2$) or ethylene ($-C_2H_4$), and each of $R^{61}$, $R^{62}$ and $R^{63}$ is independently selected from a hydrogen atom, a halogen atom (F, Cl, Br, I), $-CF_3$, $-NO_2$, a carboxyl group and - $SO_3H$. In one embodiment, $R^1$ in the formulae (4) to (13) is a hydrogen atom, $L^1$ is a methylene ($-CH_2$) or ethylene ($-C_2H_4$), and $R^{61}$, $R^{62}$ and $R^{63}$ are all hydrogen atoms. In one embodiment, $R^1$ in the formulae (4) to (13) is a hydrogen atom, $L^1$ is a methylene ($-CH_2$) or ethylene ($-C_2H_4$), and each of $R^{61}$, $R^{62}$ and $R^{63}$ is independently a hydrogen atom or a halogen atom (F, Cl, Br, or I; preferably F).

In specific embodiments, in the formulae (4) to (13) $R^1$ is a methyl, $L^1$ is a methylene ($-CH_2$) or ethylene ($-C_2H_4$), and each of $R^{61}$, $R^{62}$ and $R^{63}$ is independently selected from a hydrogen atom, a halogen atom (F, Cl, Br, I), $-CF_3$, $-NO_2$, a carboxyl group and - $SO_3H$. In one embodiment, $R^1$ in the formulae (1) to (3) is a methyl, $L^1$ is a methylene ($-CH_2$) or ethylene ($-C_2H_4$), and $R^{61}$, $R^{62}$ and $R^{63}$ are all hydrogen atoms. In one embodiment, $R^1$ in the formulae (1) to (3) is a methyl, $L^1$ is a methylene ($-CH_2$) or ethylene ($-C_2H_4$), and each of $R^{61}$, $R^{62}$ and $R^{63}$ is independently a hydrogen atom or a halogen atom (F, Cl, Br, or I; preferably F).

In one embodiment, the modifying group (A) is selected from the groups represented by the following formulae:

[C21]

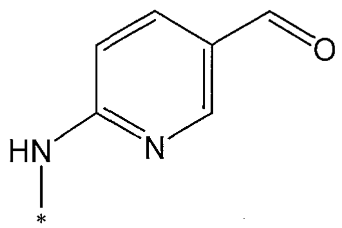

,

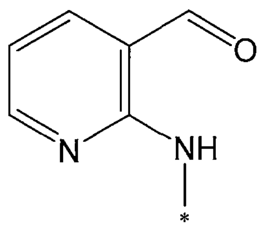

ANA APCA(APA)

(in the formulae, * represents a linkage with the polysaccharide).

In one embodiment, the modifying group (A) is selected from the groups represented by the following formulae.

[C22]

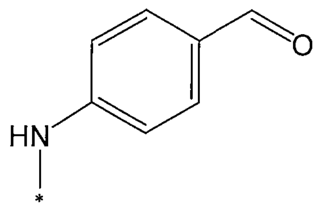, 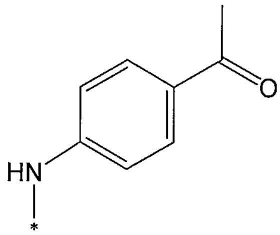, 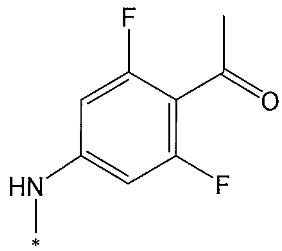,

ABA AAP ADFBA(AFA)

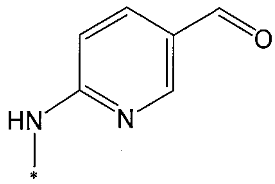, and 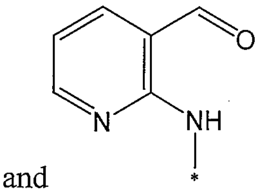

ANA APCA(APA)

(in the formulae, * represents a linkage with the polysaccharide).

In one embodiment, the modifying group (A) is a group represented by the following formula.

[C23]

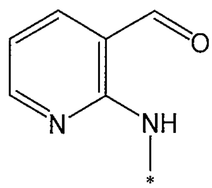

According to the aspect, the adhesion strength of the tissue adhesive material can be improved.

In certain embodiments, the modifying group (A) is a group represented by formula (A-2) above, ring P is a phenyl ring, and the phenyl ring is optionally substituted with 1 to 4 substituents independently selected from the halogen atoms (F, Cl, Br and/or I) and the $-CF_3$, $-NO_2$, carboxyl and $-SO_3H$ groups.

For example, the modifying group (A) is selected from the following formulae (14), (15) and (16) (hereunder sometimes called "formulae (14) to (16)"):

[C24]

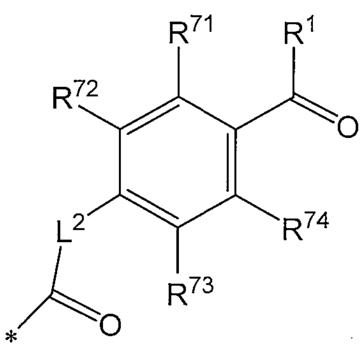

(14)

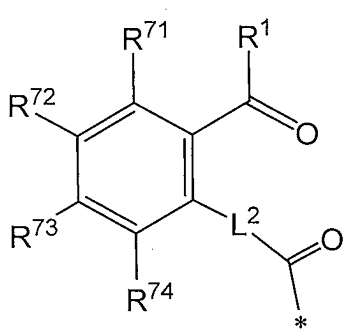

(15)

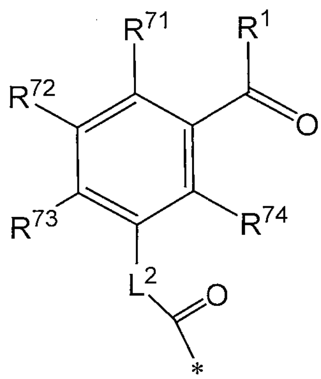

(16)

In formulae (14) to (16) above, * represents a linkage with the polysaccharide. The definition and preferred embodiments of $R^1$ in formulae (14) to (16) above are the same as the definition and preferred embodiments of $R^1$ in the formula (A), and the definition and preferred embodiments of $L^2$ are the same as the definition and preferred embodiments of $L^2$ in the formula (A-2).

In the formulae (1) to (3) above, each of $R^{71}$, $R^{72}$, $R^{73}$ and $R^{74}$ is independently selected from a hydrogen atom, a halogen atom (F, Cl, Br, I), $-CF_3$, $-NO_2$, a carboxyl group and $-SO_3H$. In one embodiment, $R^{71}$, $R^{72}$, $R^{73}$ and $R^{74}$ are all hydrogen atoms. In one embodiment, each of $R^{71}$, $R^{72}$, $R^{73}$ and $R^{74}$ is independently selected from a hydrogen atom and a halogen atom (F, Cl, Br, or I; preferably F).

In specific embodiments, in the formulae (14) to (16) $R^1$ is a hydrogen atom, $L^2$ is a single bond, and each of $R^{71}$, $R^{72}$, $R^{73}$ and $R^{74}$ is independently selected from a hydrogen atom, a halogen atom (F, Cl, Br, I), $-CF_3$, $-NO_2$, a carboxyl group and $-SO_3H$. In one embodiment, $R^1$ in the formulae (14) to (16) is a hydrogen atom, $L^2$ is a single bond, and $R^{71}$, $R^{72}$, $R^{73}$ and $R^{744}$ are all hydrogen atoms. In one embodiment, $R^1$ in the formulae (14) to (16) is a hydrogen atom, $L^2$ is a single bond, and each of $R^{71}$, $R^{72}$, $R^{73}$ and $R^{74}$ is independently selected from a hydrogen atom and a halogen atom (F, Cl, Br, or I; preferably F).

In specific embodiments, in the formulae (14) to (16) $R^1$ is a methyl, $L^2$ is a single bond, and each of $R^{71}$, $R^{72}$, $R^{73}$ and $R^{74}$ is independently selected from a hydrogen atom, a halogen atom (F, Cl, Br, I), $-CF_3$, $-NO_2$, a carboxyl group and $-SO_3H$. In one embodiment, $R^1$ in the formulae (14) to (16) is a methyl, $L^2$ is a single bond, and $R^{71}$, $R^{72}$, $R^{73}$ and $R^{74}$ are all hydrogen atoms. In one embodiment, $R^1$ in the formulae (14) to (16) is a methyl, $L^2$ is a single bond, and each of $R^{71}$, $R^{72}$, $R^{73}$ and $R^{74}$ is independently selected from a hydrogen atom and a halogen atom (F, Cl, Br, or I; preferably F).

In specific embodiments, in the formulae (14) to (16) $R^1$ is a hydrogen atom, $L^2$ is a methylene ($-CH_2$) or ethylene ($-C_2H_4$), and each of $R^{71}$, $R^{72}$, $R^{73}$ and $R^{74}$ is independently selected from a hydrogen atom, a halogen atom (F, Cl, Br, I), $-CF_3$, $-NO_2$, a carboxyl group and $-SO_3H$. In one embodiment, $R^1$ in the formulae (14) to (16) is a hydrogen atom, $L^2$ is a methylene ($-CH_2$) or ethylene ($-C_2H_4$), and $R^{71}$, $R^{72}$, $R^{73}$ and $R^{74}$ are all hydrogen atoms. In one embodiment, $R^1$ in the formulae (14) to (16) is a hydrogen atom, $L^2$ is a methylene ($-CH_2$) or ethylene ($-C_2H_4$), and each of $R^{71}$, $R^{72}$, $R^{73}$ and $R^{74}$ is independently selected from a hydrogen atom and a halogen atom (F, Cl, Br, or I; preferably F).

In specific embodiments, in the formulae (14) to (16) $R^1$ is a methyl, $L^2$ is a methylene ($-CH_2$) or ethylene ($-C_2H_4$), and each of $R^{71}$, $R^{72}$, $R^{73}$ and $R^{74}$ is independently selected from a hydrogen atom, a halogen atom (F, Cl, Br, I), $-CF_3$, $-NO_2$, a carboxyl group and $-SO_3H$. In one embodiment, $R^1$ in the formulae (14) to (16) is a methyl, $L^2$ is a methylene ($-CH_2$) or ethylene ($-C_2H_4$), and $R^{71}$, $R^{72}$, $R^{73}$ and $R^{74}$ are all hydrogen atoms. In one embodiment, $R^1$ in the formulae (14) to (16) is a methyl, $L^2$ is a methylene ($-CH_2$) or ethylene ($-C_2H_4$), and each of $R^{71}$, $R^{72}$, $R^{73}$ and $R^{74}$ is independently selected from a hydrogen atom and a halogen atom (F, Cl, Br, or I; preferably F).

In one embodiment, the modifying group (A) is a group represented by the following formula:

[C25]

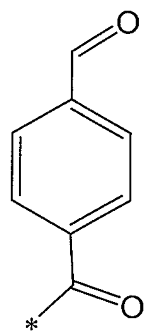

(in the formula * represents a linkage with the polysaccharide).

In certain embodiments, Y is selected from $-L^3-S-$ or $-L^4-$, in which case $L^3$ and $L^4$ are bound to ring P. That is, the modifying group (A) is selected from the following formulae (A-3) and (A-4):

[C26]

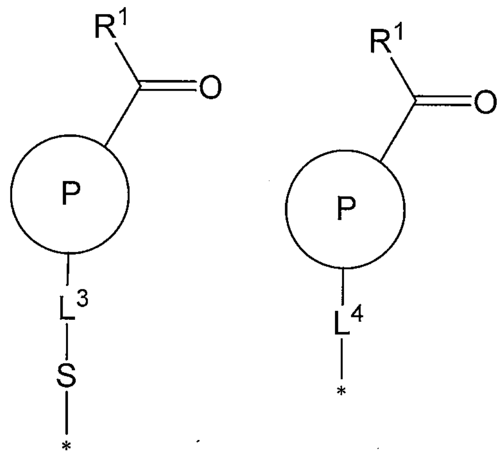

(A-3) (A-4)

In the formulae (A-3) and (A-4), * represents a linkage with the polysaccharide. Furthermore, the definitions and preferred embodiments of $R^1$ and ring P in the formulae (A-3) and (A-4) are the same as the definitions and preferred embodiments of $R^1$ and ring P in the formula (A) above.

In the formula (A-3), $L^3$ is a single bond or represents divalent group selected from the group consisting of -NH-, -C(=O)-, -O-, the alkylenes and $-(CH_2CH_2O)_n-$ and any combinations of these, with n being an integer from 1 to 9 (preferably 1 to 4, more preferably 1-2). From the standpoint of hydrophilicity, the carbon number of the alkylene group of $L^3$ is 1 to 6, or preferably 1 to 4, or more preferably 1 to 2, or still more preferably 1.

In particular, $L^3$ is selected from a single bond, the $C_{1-6}$ alkylenes, $-(CH_2CH_2O)_n-$, $-(CH_2)_{m1}-(CH_2CH_2O)_n-(CH_2)_{m2}-$ and $(CH_2)_{m1}-O-(CH_2CH_2O)_n-(CH_2)_{m2}-$, with n being an integer from 1 to 9 (preferably from 1 to 4, or more preferably from 1 to 2). m1 and m2 are each independently integers from 1 to 9 (preferably from 1 to 4, or more preferably from 1 to 2). From the standpoint of hydrophilicity, the $C_{1-6}$ alkylene group of $L^3$ is preferably a $C_{1-4}$ alkylene, or more preferably a $C_{1-2}$ alkylene, or still more preferably methylene.

In one embodiment, $L^3$ is a single bond or $C_{1-2}$ alkylene. In one embodiment, $L^3$ is a single bond.

In the formula (A-4), $L^4$ is a single bond, or represents divalent group selected from the group consisting of -NH-, -C(=O)-, -S-, -O-, the alkylenes and $-(CH_2CH_2O)_n-$ and any combinations of these, with n being an integer from 1 to 9 (preferably 1 to 4, more preferably 1-2). From the standpoint of hydrophilicity, the carbon number of the alkylene group of $L^4$ is 1 to 6, or preferably 1 to 4, or more preferably 1 to 2, or still more preferably 1.

In particular, $L^4$ is selected from a single bond, the $C_{1-6}$ alkylenes, $-(CH_2CH_2O)_n-$, $-(CH_2)_{m1}-(CH_2CH_2O)_n-(CH2)_{m2}-$ and $(CH_2)_{m1}-O-(CH_2CH_2O)_n-(CH_2)_{m2}-$, with n being an integer from 1 to 9 (preferably from 1 to 4, or more preferably from 1 to 2). m1 and m2 are each independently integers from 1 to 9 (preferably from 1 to 4, or more preferably from 1 to 2). From the standpoint of hydrophilicity, the $C_{1-6}$ alkylene of $L^4$ is preferably a $C_{1-4}$ alkylene, or more preferably a $C_{1-2}$ alkylene, or still more preferably methylene.

In one embodiment, $L^4$ is a $C_{1-2}$ alkylene.

The modifying group (A) has $-C(=O)R^1$ (aldehyde group or ketone group) in the head part, and this $-C(=O)R^1$ can form a Schiff base by reacting with a primary amino group. In the present invention, $-C(=O)R^1$ may also form a superconjugate structure with an adjacent phenyl ring or pyridine ring, which can stabilize the formed Schiff base and increase binding stability with the amino group.

(Polysaccharide)

The polysaccharide is not particularly limited as long as it is acidic, basic or amphoteric and permits introduction of the modifying group (A), and may be a polysaccharide that has been extracted and isolated from natural plant or animal sources, or a polysaccharide that has been genetically modified and produced in a microorganism, or a chemically synthesized polysaccharide.

An acidic polysaccharide is a polysaccharide having anionic functional groups (such as carboxyl groups, sulfate groups, phosphate groups or the like) in its structure. Examples of acidic polysaccharides include polysaccharides HAVING uronic acids (such as guluronic acid, mannuronic acid, glucuronic acid, iduronic acid, galacturonic acid, etc.), polysaccharides having sulfuric acid groups or phosphoric acid groups in part of their structures, and polysaccharides

having both these kinds of structures. In addition to polysaccharides that intrinsically have anionic functional groups in their structures, acidic polysaccharides include polysaccharides (for example, neutral polysaccharides) that intrinsically have no anionic functional groups but have had anionic functional groups introduced by substituting anionic functional groups such as carboxyalkyl groups for the hydrogen atoms of some or all of the hydroxyl groups.

Specific examples of acidic polysaccharides include alginic acid, hyaluronic acid, carboxymethyl cellulose, carboxymethyl dextran, carboxymethyl starch, heparin, heparan sulfate, chondroitin sulfate, dermatan sulfate, regenerated oxidized cellulose, pectic acid, gellan gum, gum arabic, xanthan gum, agar, agaropectin, carrageenan, and derivatives thereof or salts of these.

A basic polysaccharide is a polysaccharide having cationic functional groups (such as amino groups) in its structure. In addition to polysaccharides that intrinsically have cationic functional groups in their structures, basic polysaccharides include polysaccharides obtained by introducing cationic functional groups into polysaccharides (for example, neutral polysaccharides) that intrinsically have no cationic functional groups.

Specific examples of basic polysaccharides include chitosan and its derivatives and salts of these.

An amphoteric polysaccharide is a polysaccharide having both anionic functional groups (such as carboxyl groups, sulfate groups and phosphate groups) and cationic functional groups (such as amino groups) in its structure. Specific examples of amphoteric polysaccharides include anion-modified basic polysaccharides such as succinate-modified chitosan; deacetylated polysaccharides such as deacetylated hyaluronic acid; amine denatured acidic polysaccharides such as cation-modified alginic acid and cation-modified hyaluronic acid; phosphocholine-modified polysaccharides such as phosphocholine-modified hyaluronic acid; carbobetaine-modified polysaccharides; and sulfobetaine-modified polysaccharides and the like.

Using an acidic, basic or amphoteric polysaccharide, it is possible to introduce the modifying group (A) at a good modification rate even when the modifying group (A) has a hydrophobic benzene ring or pyridyl ring. It is also possible to obtain a polysaccharide derivative with excellent water solubility even when a hydrophobic modifying group (A) is introduced at a high modification rate due to the presence of unmodified acidic or basic functional groups (anionic groups or cationic functional groups).

In certain embodiments, the polysaccharide is selected from alginic acid, derivatives thereof or salts of these, hyaluronic acid, derivatives thereof or salts of these, carboxymethyl cellulose, derivatives thereof or salts of these, carboxymethyl dextran, derivatives thereof or salts of these, carboxymethyl starch, derivatives thereof or salts of these, heparin, derivatives thereof or salts of these, heparan sulfate, derivatives thereof or salts of these, chondroitin sulfate, derivatives thereof or salts of these, dermatan sulfate, derivatives thereof or salts of these, chitosan, derivatives thereof or salts of these, regenerated oxidized cellulose, derivatives thereof or salts of these, and pectic acid, derivatives thereof or salts of these.

In the present invention, a polysaccharide derivative may be a polysaccharide which has been modified in any way. Examples include polysaccharides that have been modified by esterification, maleimide modification, thiol modification, acrylate modification, aldehyde modification, disulfide (such as pyridyl disulfide) modification, alkyne (including cyclic alkyne) modification, tetrazine modification, furan ring modification and the like. Methods of such modification are known to those skilled in the art, and the polysaccharide may be modified by a conventionally known method such as a carbodiimide reaction. For example, methods of maleimide modification are described in WO 2019/189330. The methods of Akira Takahashi et al., Biomacromolecules, 2013, (14)10, 3581-3588 for example may be used for alkyne (including cyclic alkyne) modification. For tetrazine modification, the methods described in Vianney Delplace et al., Nonswelling, Ultralow Content Inverse Electron-Demand Diels-Alder Hyaluronan Hydrogels with Tunable Gelation Time: Synthesis and In Vitro Evaluation, Advanced Functional Materials, 2020, 30(14) may be used for example. Methods of furan ring modification include those described in Nimmo, Chelsea M. et al., Biomacromolecules, 2011, 12(3) 824-830 and RSC Adv., 2018, 8, 11036-11042 for example. By applying these modifications in combination with a copper free click reaction (such as a reaction between a cyclic alkyne and an azide), an inverse electron demand type Diels-Alder reaction (for example, a reaction between tetrazine and a cyclic alkene) or a Diels-Alder reaction (for example, a reaction between furan and maleimide), it is possible to introduce a second drug (an additional drug of a different kind) into the polysaccharide derivative or perform crosslinking.

In certain embodiments, the polysaccharide is an acidic polysaccharide. A polysaccharide having anionic functional groups can yield a polysaccharide derivative with particularly excellent water solubility even if the modification rate by the modifying group (A) is high.

In certain embodiments, the polysaccharide is a polysaccharide containing a carboxyl group. A polysaccharide containing a carboxyl group can form an amide bond by a reaction between the carboxyl group and an amino group, allowing the modifying group (A) to be introduced into the polysaccharide.

In specific embodiments, the polysaccharide is a polysaccharide having a carboxyl group, the group represented by the formula (A) is a group represented by the formula (A-1), and the group represented by the formula (A-1) is introduced into the polysaccharide by substitution for the -OH of the carboxyl group of the polysaccharide, forming an amide bond. A polysaccharide derivative with even greater stability can be obtained by forming an amide bond.

The polysaccharide having a carboxyl group may be any having at least one or more unmodified carboxyl groups.

The polysaccharide having a carboxyl group may be a polysaccharide having a carboxyl group in its structure, or a derivative thereof or a salt of these (for example, alginic acid, derivatives thereof or salts of these, hyaluronic acid, derivatives thereof or salts of these, carboxymethyl cellulose, derivatives thereof or salts of these, carboxymethyl dextran, derivatives thereof or salts of these, carboxymethyl starch, derivatives thereof or salts of these, heparin, derivatives thereof or salts of these, heparan sulfate, derivatives thereof or salts of these, chondroitin sulfate, derivatives thereof or salts of these, dermatan sulfate, derivatives thereof or salts of these, pectic acid, derivatives thereof or salts of these, regenerated oxidized cellulose, derivatives thereof or salts of these, gellan gum, derivatives thereof or salts of these, gum arabic, derivatives thereof or salts of these, xanthan gum, derivatives thereof or salts of these, agarose, derivatives thereof or salts of these, and agaropectin, derivatives thereof or salts of these; or preferably alginic acid, derivatives thereof or salts of these, hyaluronic acid, derivatives thereof or salts of these, carboxymethyl cellulose, derivatives thereof or salts of these, carboxymethyl dextran, derivatives thereof or salts of these, carboxymethyl starch, derivatives thereof or salts of these, heparin, derivatives thereof or salts of these, heparan sulfate, derivatives thereof or salts of these, chondroitin sulfate, derivatives thereof or salts of these, dermatan sulfate, derivatives thereof or salts of these, pectic acid, derivatives thereof or salts of these, and regenerated oxidized cellulose, derivatives thereof or salts of these) or else a polysaccharide having no carboxyl group (for example, chitosan, derivatives thereof or salts of these, curdlan, derivatives thereof or salts of these, agar, derivatives thereof or salts of these, carrageenan, derivatives thereof or salts of these, guar gum, derivatives thereof or salts of these, locust bean gum, derivatives thereof or salts of these, and tamarind seed gum, derivatives thereof or salts of these; or preferably chitosan, derivatives thereof or salts of these) into which a carboxyl group has been introduced by substitution of a carboxyalkyl group for the hydrogen atoms of some or all of the hydroxyl groups.

In specific embodiments, the polysaccharide having a carboxyl group is selected from alginic acid or its salts, hyaluronic acid or its salts, carboxymethyl cellulose or its salts, carboxymethyl dextran or its salts, carboxymethyl starch or its salts, heparin or its salts, heparan sulfate or its salts, chondroitin sulfate or its salts, dermatan sulfate or its salts, pectin or its salts, and regenerated oxidized cellulose or its salts.

In specific embodiments, the polysaccharide is selected from alginic acid, derivatives thereof or salts of these, hyaluronic acid, derivatives thereof or salts of these, carboxymethyl cellulose, derivatives thereof or salts of these, and carboxymethyl dextran, derivatives thereof or salts of these.

In specific embodiments, the polysaccharide is selected from alginic acid or its salts, hyaluronic acid or its salts, carboxymethyl cellulose or its salts, and carboxymethyl dextran or its salts.

In specific embodiments, the polysaccharide is alginic acid or a derivative thereof or a salt of these, or hyaluronic acid or a derivative thereof or a salt of these.

In specific embodiments, the polysaccharide is alginic acid or a salt thereof or hyaluronic acid or a salt of these.

(Alginate)

In one embodiment, the polysaccharide is alginic acid or a derivative thereof or a salt of these (hereunder also called an "alginate").

Alginic acid is a linear heteropolymer of two kinds of uronic acid, D-mannuronic acid (M) and L-guluronic acid (G). Specifically, it is a block copolymer comprising a homopolymer fraction of D-mannuronic acid (MM fraction), a homopolymer fraction of L-guluronic (GG fraction) and a fraction of randomly arranged D-mannuronic acid and L-guluronic acid (M/G fraction) in arbitrary combination. The ratio of D-mannuronic acid to L-guluronic acid in alginic acid (M/G ratio) differs principally according to the type of seaweed or other organism from which it is derived, and may also be affected by the organism's habitat and season, with a wide range from high-G alginic acid (M/G ratio about 0.2) to high-M alginic (M/G ratio about 5)

A derivative of alginic acid is alginic acid with any modification, or a salt thereof, with no particular limitations. Any modifications to the alginic acid may include esterification, sulfation, maleimide modification, thiol modification, acrylate modification, aldehyde modification, disulfide (for example, pyridyl disulfide) modification and cation modification (for example, heparin-bound alginate) and the like. In one embodiment, the alginic acid derivative is an alginic acid ester, propylene glycol alginate, sulfated alginic acid in which the hydroxyl groups are sulfated, maleimide modified alginic acid, thiol modified alginic acid, acrylate modified alginic acid or cation modified alginic acid (such as the heparin-bound alginate described in Japanese Patent Application Publication No. 2001-233786). Such modification of alginic acid may be accomplished by known methods or analogous methods.

Salts of alginic acid or its derivatives include metal salts of alginic acid or its derivatives for example. For example, a hydrogen atom (hydrogen ion) of carboxylic acid at position 6 of alginic acid or its derivative may be ion substituted with a monovalent metal ion (for example, and alkali metal ion such as $Na^+$ or $K^+$) to create a water-soluble salt. Specific examples include sodium alginate, potassium alginate, sodium salts of alginic acid derivatives and potassium salts of alginic acid derivatives. In specific embodiments, the salt of alginic acid or its derivative is sodium alginate or a sodium salt of an alginic acid derivative. A solution of a monovalent metal salt of alginic acid or its derivative forms a gel when mixed with

a crosslinking agent. Alternatively, a metal salt of alginic acid or its derivative may be a salt (crosslinked body) formed by ion substitution of a divalent metal ion (for example, an alkali earth metal ion such as $Mg^{2+}$ or $Ca^{2+}$) for a hydrogen atom (hydrogen ion) of carboxylic acid at position 6 of alginic acid or its derivative.

When originally extracted from brown algae, alginic acid has a high molecular weight and high viscosity, but the molecular weight falls and viscosity is reduced by processes such as heat drying, freeze drying and purification. Consequently, alginates with different molecular weights can be manufactured by appropriately controlling temperature at each stage of manufacture. An alginate with a high molecular weight can be obtained by controlling temperature at a low level at each stage of manufacture, while an alginate with a low molecular weight can be obtained by increasing the temperature. Alginates with different molecular weights can also be manufactured by methods such as appropriately selecting the type of brown algae used as a raw material or fractioning molecular weights during the manufacturing process. Furthermore, the molecular weights or viscosities of alginates produced by various methods can be measured, and separate lots of alginate with different molecular weights or viscosities can be mixed to obtain an alginate with the target molecular weight.

The viscosity of the alginate used in not particularly limited, but when measured in a 1 w/w% aqueous solution of the alginate, it is preferably from 10 mPa·s to 1,000 mPa·s, or more preferably from 50 mPa·s to 800 mPa·s.

The viscosity of the alginate in an aqueous solution can be measured by conventional methods. For example, it can be measured using by rotation viscometry using a coaxial double-cylinder rotational viscometer, a single-cylinder rotational viscometer (Brookfield viscometer), or a cone plate rotational viscometer. Preferably it is measured according to the viscosity measurement methods of the Japanese Pharmacopoeia (16th Edition). More preferably, a cone plate viscometer is used.

The position where the modifying group (A) is introduced into the alginate is not particularly limited, but preferably it is introduced at the position of the 6-position carboxyl groups of the D-mannuronic acid (M) and L-guluronic acid (G) (uronic acid) constituting the alginate.

In specific embodiments, the group represented by the formula (A-1) is introduced into the polysaccharide by substitution for the -OH of a carboxyl group of alginic acid.

In one embodiment, the polysaccharide derivative contains constituent units represent by formula (c11) and/or formula (c12) below:

[C27]

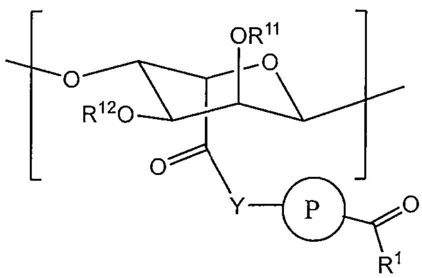

(c11)

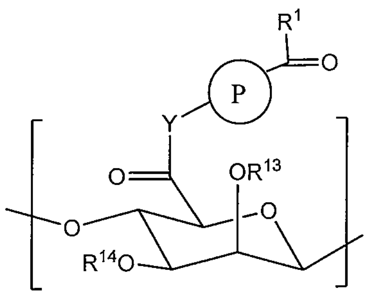

(c12)

In the formulae (c11) and (c12), the definitions and preferred embodiments of $R^1$ and ring P are the same as the definitions and preferred embodiments of $R^1$ and ring P in the formula (A).

In the formulae (c11) and (c12), Y represents -$L^1$-NH-, in which case $L^1$ is bound to the ring P. The definitions and preferred embodiments of $L^1$ are the same as the definitions and preferred embodiments of $L^1$ in the formula (A-1).

In the formulae (c11) and (c12), each of $R^{11}$, $R^{12}$, $R^{13}$ and $R^{14}$ is independently selected from a hydrogen atom, a $C_{1-6}$ alkyl and a -C(=O)-$C_{1-6}$ alkyl. In one embodiment, $R^{11}$, $R^{12}$, $R^{13}$ and $R^{14}$ are all hydrogen atoms.

(Hyaluronate)

In one embodiment, the polysaccharide is hyaluronic acid or a derivative thereof or a salt of these (hereunder also called a "hyaluronate").

Hyaluronic acid is a biodegradable and biocompatible polymer having a repeating structure consisting of linear chains of two sugars, D-glucuronic acid and N-acetyl-D-glucosamine.

A derivative of hyaluronic acid is hyaluronic acid with any modification, or a salt thereof, with no particular limitations. Any modifications to the alginic acid may include esterification, sulfation, maleimide modification, thiol

modification, acrylate modification, aldehyde modification and disulfide (for example, pyridyl disulfide) modification. In one embodiment, the hyaluronic acid derivative is a hyaluronic acid ester, propylene glycol hyaluronate, sulfated hyaluronic acid in which the hydroxyl groups are sulfated, maleimide modified hyaluronic acid, thiol modified hyaluronic acid or acrylate modified hyaluronic acid. Such modification of hyaluronic acid may be accomplished by known methods or analogous methods.

Salts of hyaluronic acid or its derivatives include metal salts of hyaluronic acid or its derivatives for example. For example, a hydrogen atom (hydrogen ion) of carboxylic acid at position 6 of hyaluronic acid or its derivative may be ion substituted with a monovalent metal ion (for example, and alkali metal ion such as $Na^+$ or $K^+$) to create a water-soluble salt. Specific examples include sodium hyaluronate, potassium hyaluronate, sodium salts of hyaluronic acid derivatives and potassium salts of hyaluronic acid derivatives and the like. In specific embodiments, the salt of hyaluronic acid or its derivative is sodium hyaluronate or a sodium salt of a hyaluronic acid derivative. A solution of a monovalent metal salt of hyaluronic acid or its derivative forms a gel when mixed with a crosslinking agent. Alternatively, a metal salt of hyaluronic acid or its derivative may be a salt (crosslinked body) formed by ion exchange of a divalent metal ion (for example, an alkali earth metal ion such as $Mg^{2+}$ or $Ca^{2+}$) for a hydrogen atom (hydrogen ion) of carboxylic acid at position 6 of hyaluronic acid or its derivative.

The position where the modifying group (A) is introduced into the hyaluronate is not particularly limited, but preferably it is introduced at the position of a carboxyl group of the D-glucuronic acid constituting the hyaluronic acid.

In specific embodiments, the group represented by the formula (A-1) is introduced into the polysaccharide by substitution for the -OH of a carboxyl group of hyaluronic acid.

In one embodiment, the polysaccharide derivative contains constituent units represent by formula (c13) below:

[C28]

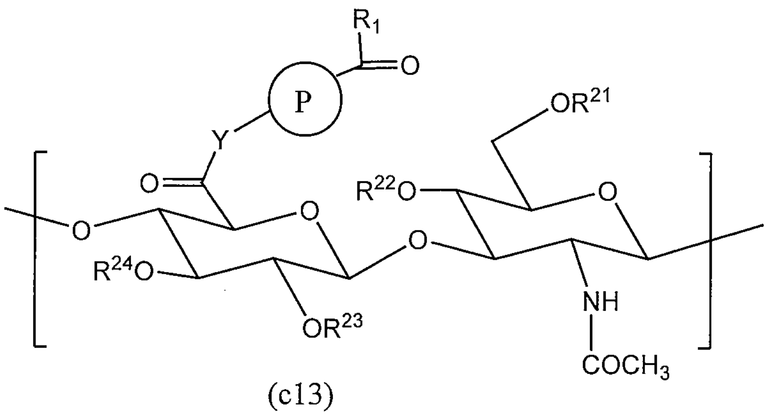

(c13)

In formula (c13), the definitions and preferred embodiments of $R^1$ and ring P are the same as the definitions and preferred embodiments of $R^1$ and ring P in the formula (A).

In formula (c13), Y represents -$L^1$-NH-, in which case $L^1$ is bound to the ring P. The definitions and preferred embodiments of $L^1$ are the same as the definitions and preferred embodiments of $L^1$ in formula (A-1).

In formula (c13), each of $R^{21}$, $R^{22}$, $R^{23}$ and $R^{24}$ is independently selected from a hydrogen atom, a $C_{1-6}$ alkyl and a -C(=O)-$C_{1-6}$ alkyl. In one embodiment, $R^{21}$, $R^{22}$, $R^{23}$ and $R^{24}$ are all hydrogen atoms.

(Carboxymethyl cellulose)

In one embodiment, the polysaccharide is carboxymethyl cellulose or a derivative thereof or a salt of these (hereunder called "the carboxymethyl cellulose").

Carboxymethyl cellulose is a derivative of cellulose that has been solubilized by introducing carboxymethyl groups into cellulose, and has excellent thickening, water absorption and water retention properties.

A derivative of carboxymethyl cellulose is carboxymethyl cellulose with any modification, or a salt thereof, with no particular limitations. Any modifications to the carboxymethyl cellulose may include esterification, sulfation, maleimide modification, thiol modification, acrylate modification, aldehyde modification and disulfide (for example, pyridyl disulfide) modification. Such modification of carboxymethyl cellulose may be accomplished by known methods or analogous methods.

Salts of carboxymethyl cellulose or its derivatives include metal salts of carboxymethyl cellulose or its derivatives for example. For example, a hydrogen atom (hydrogen ion) of carboxylic acid contained in carboxymethyl cellulose or its

derivative may be ion substituted with a monovalent metal ion (for example, and alkali metal ion such as $Na^+$ or $K^+$) to create a water-soluble salt. Specific examples include sodium carboxymethyl cellulose, potassium carboxymethyl cellulose, sodium salts of carboxymethyl cellulose derivatives and potassium salts of carboxymethyl cellulose derivatives. In specific embodiments, the salt of carboxymethyl cellulose or its derivative is sodium carboxymethyl cellulose or a sodium salt of a carboxymethyl cellulose derivative. A solution of a monovalent metal salt of carboxymethyl cellulose or its derivative forms a gel when mixed with a crosslinking agent. Alternatively, a metal salt of carboxymethyl cellulose or its derivative may be a salt (crosslinked body) formed by ion exchange of a divalent metal ion (for example, an alkali earth metal ion such as $Mg^{2+}$ or $Ca^{2+}$) for a hydrogen atom (hydrogen ion) of carboxylic acid contained in the carboxymethyl cellulose or its derivative.

The position where the modifying group (A) is introduced into the carboxymethyl cellulose is not particularly limited, but preferably it is introduced at the position of a carboxyl group contained in the carboxymethyl cellulose.

In specific embodiments, the group represented by the formula (A-1) is introduced into the polysaccharide by substitution for the -OH of a carboxyl group of carboxymethyl cellulose.

In one embodiment, the polysaccharide derivative contains constituent units represent by formula (c14) below:

[C29]

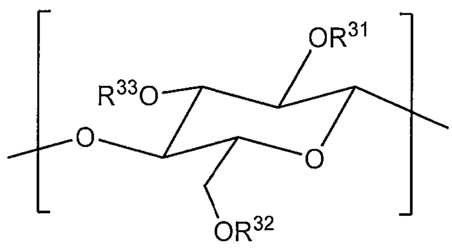

(c14)

In formula (c14), 1 to 3 of $R^{31}$, $R^{32}$ and $R^{33}$ are groups represented by formula (i) below:

[C30]

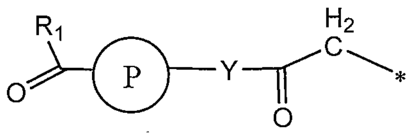

(i)

In the formula (i), the definitions and preferred embodiments of $R^1$ and ring P are the same as the definitions and preferred embodiments of $R^1$ and ring P in the formula (A).

In the formula (i), Y represents $-L^1-NH-$, in which case $L^1$ is bound to the ring P. The definition and preferred embodiments of $L^1$ are the same as the definition and preferred embodiments of $L^1$ in the formula (A-1).

The rest of $R^{31}$, $R^{32}$ and $R^{33}$ (substituents not represented by formula (i)) are each independently selected from a hydrogen atom, a $C_{1-6}$ alkyl, a $-C(=O)-C_{1-6}$ alkyl and $-CH_2COOH$, and preferably are selected from a hydrogen atom and $-CH_2COOH$, or more preferably are hydrogen atoms.

In one embodiment, $R^{31}$ is a group represented by formula (i) above, and $R^{32}$ and $R^{33}$ are hydrogen atoms.

(Carboxymethyl dextran)

In one embodiment, the polysaccharide is carboxymethyl dextran or a derivative thereof or a salt of these (hereunder called "the carboxymethyl dextran").

Carboxymethyl dextran is a carboxymethyl ether of dextran.

A derivative of carboxymethyl dextran is carboxymethyl dextran with any modification, or a salt thereof, with no particular limitations. Any modifications to the carboxymethyl dextran may include esterification, sulfation, maleimide modification, thiol modification, acrylate modification, aldehyde modification and disulfide (for example, pyridyl disulfide) modification. Such modification of carboxymethyl dextran may be accomplished by known methods or analogous methods.

Salts of carboxymethyl dextran or its derivatives include metal salts of carboxymethyl dextran or its derivatives for example. For example, a hydrogen atom (hydrogen ion) of carboxylic acid contained in carboxymethyl dextran or its derivative may be ion substituted with a monovalent metal ion (for example, and alkali metal ion such as $Na^+$ or $K^+$) to create a water-soluble salt. Specific examples include sodium carboxymethyl dextran, potassium carboxymethyl dextran, and sodium salts of carboxymethyl dextran derivatives and potassium salts of carboxymethyl dextran derivatives. In specific embodiments, the salt of carboxymethyl dextran or its derivative is a sodium salt of carboxymethyl dextran or a carboxymethyl dextran derivative. A solution of a monovalent metal salt of carboxymethyl dextran or its derivative forms a gel when mixed with a crosslinking agent. Alternatively, a metal salt of carboxymethyl dextran or its derivative may be a salt (crosslinked body) formed by ion exchange of a divalent metal ion (for example, an alkali earth metal ion such as $Mg^{2+}$ or $Ca^{2+}$) for a hydrogen atom (hydrogen ion) of carboxylic acid contained in the carboxymethyl dextran or its derivative.

The position where the modifying group (A) is introduced into the carboxymethyl dextran is not particularly limited, but preferably it is introduced at the position of a carboxyl group contained in the carboxymethyl dextran.

In specific embodiments, the group represented by the formula (A-1) is introduced into the polysaccharide by substitution for the -OH of a carboxyl group of the carboxymethyl dextran.

In one embodiment, the polysaccharide derivative contains constituent units represent by formula (c15) below:

[C31]

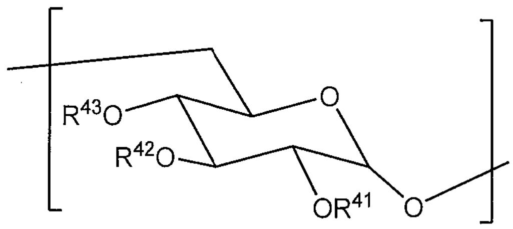

(c15)

In formula (c15), 1 to 3 of $R^{41}$, $R^{42}$ and $R^{43}$ are groups represented by formula (i) below:

[C32]

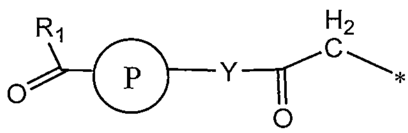

(i)

In formula (i), the definitions and preferred embodiments of $R^1$ and ring P are the same as the definitions and preferred embodiments of $R^1$ and ring P in the formula (A).

In formula (i), Y represents $-L^1$-NH-, in which case $L^1$ is bound to the ring P. The definitions and preferred embodiments of $L^1$ are the same as the definitions and preferred embodiments of $L^1$ in the formula (A-1).

The rest of $R^{41}$, $R^{42}$ and $R^{43}$ (substituents not represented by formula (i)) are each independently selected from a hydrogen atom, a $C_{1-6}$ alkyl, a $-C(=O)-C_{1-6}$ alkyl and - $CH_2COOH$, and preferably are selected from a hydrogen atom and $-CH_2COOH$, or more preferably are hydrogen atoms.

In one embodiment, $R^{41}$ is a group represented by formula (i) above, and $R^{42}$ and $R^{43}$ are hydrogen atoms.

The polysaccharide may also be heparin or a derivative thereof or a salt of these, heparan sulfate or a derivative thereof or a salt of these, chondroitin sulfate or a derivative thereof or a salt of these, dermatan sulfate or a derivative thereof or a salt of these, regenerated oxidized cellulose or a derivative thereof or a salt of these, or pectic acid or a derivative thereof or a salt of these. In one embodiment of the polysaccharide derivative, the polysaccharide is heparin or a derivative thereof or a salt of these, heparan sulfate or a derivative thereof or a salt of these, chondroitin sulfate or a derivative thereof or a salt of these, dermatan sulfate or a derivative thereof or a salt of these, regenerated oxidized cellulose or a derivative thereof or a salt of these or pectic acid or a derivative thereof or a salt of these, and the group represented by the formula (A-1) is introduced by substitution for the -OH of a carboxyl group contained in the polysaccharide.

In certain embodiments, the polysaccharide is a polysaccharide containing an amino group. A polysaccharide containing an amino group can form an amide bond by a reaction between the amino group and a carboxyl group, allowing

the modifying group (A) to be introduced into the polysaccharide.

In specific embodiments, the polysaccharide is a polysaccharide containing an amino group, the group represented by the formula (A) is a group represented by the formula (A-2), and the group represented by the formula (A-2) is introduced into the polysaccharide by substitution for a hydrogen atom of the amino groups of the polysaccharide, forming an amide bond. A polysaccharide derivative with even greater stability can be obtained by forming an amide bond.

The polysaccharide having an amino group may be a polysaccharide that intrinsically has an amino group in its structure, or a derivative thereof or a salt of these (for example, chitosan or a derivative thereof or a salt of these), or a polysaccharide obtained by introducing an amino group into part of a polysaccharide having no amino groups.

The following is an example of a polysaccharide derivative obtained by introducing a group represented by the formula (A-2) into a polysaccharide having an amino group:

[C33]

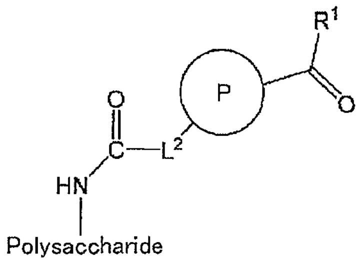

In the formula above, the definitions and preferred embodiments of $R^1$ and ring P are the same as the definitions and preferred embodiments of $R^1$ and ring P in the formula (A).

In the formula above, moreover, the definition and preferred embodiments of $L^2$ are the same as the definition and preferred embodiments of $L^2$ in the formula (A-2).

(Chitosan)

The polysaccharide may also be chitosan or a derivative thereof or a salt of these (hereunder also called "the chitosan"). Chitosan is a polysaccharide composed of D-glucosamine units and N-acetyl-D-glucosamine units bound by beta-(1-4) glycoside bonds. Chitosan can be obtained by partial deacetylation of the polysaccharide chitin. The degree of deacetylation is not particularly limited. In certain embodiments, the chitosan is deacetylated to a degree exceeding about 50% (typically exceeding about 75%).

The chitosan derivative is not particularly limited, and may be chitosan with any modification, or a salt thereof. Any modifications to the chitosan may include esterification, sulfation, maleimide modification, thiol modification, acrylate modification, aldehyde modification and disulfide (for example, pyridyl disulfide) modification. Examples of chitosan derivatives include carboxymethyl chitosan, hydroxybutyl chitin, N-acyl chitosan, O-acyl chitosan, N-alkyl chitosan, O-alkyl chitosan, N-alkylidene chitosan, O-sulfonyl chitosan, sulfated chitosan, phosphorylated chitosan, nitrated chitosan, alkali chitosan, and metal chelates of chitosan and the like. Such modification of chitosan may be accomplished by known methods or analogous methods. A commercial product may also be used.

Chitosan has a primary amino group. In one embodiment of the polysaccharide derivative, the polysaccharide is chitosan or its derivative or a salt of these, and the group represented by the formula (A-2) is introduced by substitution for a hydrogen atom of the amino group of the polysaccharide.

The position where the modifying group (A) is introduced into the chitosan is not particularly limited, but preferably it is introduced at the position of an amino group of the D-glucosamine units constituting the chitosan. In specific embodiments, a group represented by the formula (A-2) is introduced into the polysaccharide by substitution for a hydrogen atom of the amino group of chitosan.

In one embodiment, the polysaccharide derivative contains constituent units represented by the following formula (c16):

[C34]

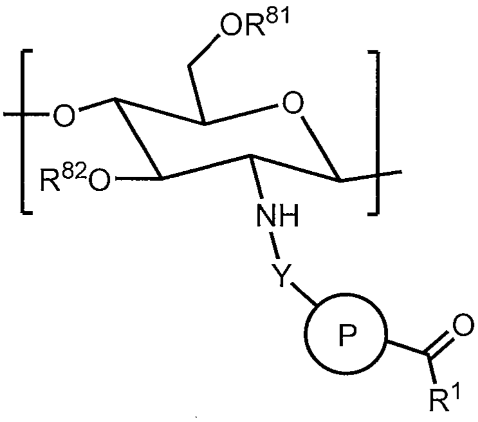

(c16)

In the formula (c16), the definitions and preferred embodiments of $R^1$ and ring P are the same as the definitions and preferred embodiments of $R^1$ and ring P in the formula (A).

In the formula (c16), Y represents $-L^2-C(=O)-$, in which case $L^2$ binds to the ring P. The definition and preferred embodiments of $L^2$ are the same as the definition and preferred embodiments of $L^2$ in the formula (A-2).

In formula (c16), each of $R^{81}$ and $R^{82}$ is independently selected from a hydrogen atom, a $C_{1-6}$ alkyl and a $-C(=O)-C_{1-6}$ alkyl. In one embodiment, $R^{81}$ and $R^{82}$ are both hydrogen atoms.

In specific embodiments, the polysaccharide is a polysaccharide having an ethylenic double bond group. A polysaccharide having an ethylenic double bond group can form a sulfide bond (-S-) by a reaction between the ethylenic double bond group and a thiol group (-SH), thereby allowing the modifying group (A) to be introduced into the polysaccharide. In specific embodiments, the polysaccharide is a polysaccharide containing an ethylenic double bond group, the group represented by the formula (A) is a group represented by the formula (A-3), and the group represented by the formula (A) is introduced into the polysaccharide by reacting with the ethylenic double bond group of the polysaccharide.

The polysaccharide having an ethylenic double bond is not particularly limited, but examples include polysaccharides (such as alginic acid, hyaluronic acid, carboxymethyl cellulose, carboxymethyl dextran, carboxymethyl starch, pectin, regenerated oxidized cellulose and chitosan) that have been modified by maleimide modification or acrylate modification, or salts of these.

For example, the following is an example of a polysaccharide derivative obtained by introducing a group represented by the formula (A-3) into a maleimide modified polysaccharide:

[C35]

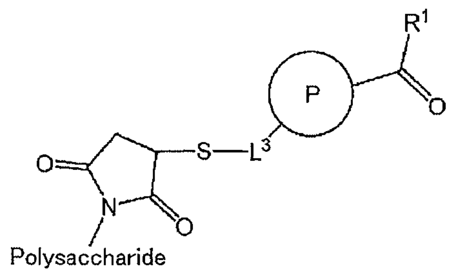

In the above formula, the definitions and preferred embodiments of $R^1$ and ring P are the same as the definitions and preferred embodiments of $R^1$ and ring P in the formula (A).

In the above formula, furthermore, the definition and preferred embodiments of $L^3$ are the same as the definition and preferred embodiments of $L^3$ in the formula (A-3).

The polysaccharide derivative may also have a structure in which the modifying group (A) is introduced at the position of a hydroxyl group contained in the polysaccharide.

In specific embodiments, the group represented by the formula (A-4) is introduced by substitution for the

hydrogen atom of the hydroxyl group contained in the polysaccharide. In this embodiment, as shown below, an ether bonds is formed in the polysaccharide derivative by an oxygen atom derived from a hydroxyl group contained in the polysaccharide.

[C36]

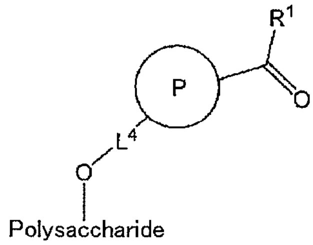

(Molecular weight and viscosity of polysaccharide derivative)

High-molecular-weight polysaccharides generally have a weight-average molecular weight in the range of 1,000 to 10,000,000, although the weight-average molecular weight is often difficult to determine exactly.

For example, for the molecular weight of a polysaccharide derivative, the weight-average molecular weight (Mw) as measured by gel filtration chromatography (GFC) is preferably from 1,000 to 5,000,000, or more preferably from 1,000 to 3,000,000.

The preferred range of the molecular weight of a polysaccharide derivative differs depending on the type of polysaccharide making up the polysaccharide derivative (presence or absence of degradation enzymes, inflammatory response or the like) and the use of the polysaccharide derivative.

For example, for DDS applications this is partly affected by the charge of the polysaccharide derivative, but considering that the kidney excretion limit is said to be 40,000 to 50,000, in general the weight-average molecular weight (Mw) of a polysaccharide derivative composed of a (non-biodegradable) polysaccharide having no in vivo degradation enzyme (such as dextran, cellulose derivatives, alginic acid and salts thereof and derivatives of these) is preferably in the range of 1,000 to 50,000, or more preferably in the range of 1,000 to 40,000.

However, since alginic acid has no in vivo degradation enzyme but also very little immunogenicity, a higher weight-average molecular weight (such as Mw 1,000 to 5,000,000, or 1,000 to 2,000,000) is possible in this case.

Because degradation enzymes for hyaluronic acid are abundant in the body, high weight-average molecular weights (such as up to 2,000,000, or up to 5,000,000 Mw) are possible, and at least 500,000 Mw is desirable because low-molecular-weight hyaluronic acid (around 100,000 Mw) is known to cause inflammation.

A measurement error of 10 to 20% or more is normal when the molecular weight of a high-molecular-weight polysaccharide is calculated by gel filtration chromatography. For example, the value may vary in the range of 320,000 to 480,000 in the case of a molecular weight of 400,000, or in the range of 400,000 to 600,000 in the case of 500,000, or in the range of 800,000 to 1,200,000 in the case of 1,000,000. Consequently, in the case of a polysaccharide the preferred weight-average molecular weight range is at least 1,000. Because manufacture is more difficult and the viscosity of an aqueous solution increases if the weight is too high, making it difficult to maintain the properties during long-term storage among other problems, the weight-average molecular weight is preferably not more than 5,000,000, or preferably not more than 3,000,000.

The weight-average molecular weight of the polysaccharide derivative with the introduced modifying group (A) is higher than the molecular weight of the polysaccharide before introduction of the modifying group (A) due to introduction of the modifying group (A). A polysaccharide derivative with the desired molecular weight can be obtained by selecting a polysaccharide with an appropriate molecular weight as the raw material.

Because in general naturally derived polymeric substances do not have a single molecular weight, but are aggregates of molecules with various molecular weights, the molecular weight is measured as a molecular weight distribution with a certain width. Gel filtration chromatography is a typical measurement method. Typical information about molecular weight distribution obtained from gel filtration chromatography includes the weight-average molecular weight (Mw), number-average molecular weight (Mn) and variance ratio (Mw/Mn).

The weight-average molecular weight is emphasized as a contributor to the average molecular weight of molecules with high molecular weights, and is represented by the following formula.

$$Mw = \Sigma(WiMi)/W = \Sigma(HiMi)/ \Sigma(Hi)$$

The number-average molecular weight is calculated by dividing the total weight of the macromolecules by the number of macromolecules.

$$Mn = W/ \Sigma Ni = \Sigma(MiNi)/ \Sigma Ni = \Sigma(Hi)/ \Sigma(Hi/Mi)$$

In the formulae, W is the total weight of the macromolecules, Wi is the weight of the i-th macromolecule, Mi is the molecular weight at the i-th elution time, Ni is the number of molecular weights Mi, and Hi is the height at the i-th elution time.

In measuring the molecular weights of naturally derived high-molecular-weight substances, the values are known to vary depending on the measurement method (for hyaluronic acid: Chikako Yomota et al., Bull. Natl. Health Sci., Vol. 117, pp 135-139 (1999), Chikako Yomota et al., Bull. Natl. Inst. Health Sci., Vol. 121, pp 30-33 (2003)). Published methods for measuring the molecular weights of alginate salts include methods of calculation from intrinsic viscosity and methods of calculation by SEC-MALLS (Size Exclusion Chromatography with Multiple Angle Laser Light Scattering Detection) as described in ASTM F2064-00 (2006), ASTM International Pub.. This publication recommends using a Multi Angle Light Scattering (MALS) detection device in combination with a calibration curve using pullulan as a standard substance (SEC-MALS measurement) when measuring molecular weight by size exclusion chromatography (gel filtration chromatography). SEC-MALS is described in ASTM F2065-16. Molecular weights obtained by SEC-MALS are also sometimes used as standard values in catalogues of alginate salts (FMC Biopolymer Co., PRONOVA™ sodium alginates catalogue).

In the present invention, unless otherwise specified, the molecular weight of a polysaccharide derivative is the weight-average molecular weight as calculated by gel filtration chromatography.

Typical conditions for gel filtration chromatography include the use of a calibration curve based on pullulan as a standard substance. The molecular weights of the pullulan used as a standard substance preferably include at least 1,600,000, 788,000, 404,000, 212,000 and 112,000. In addition, the eluent (200 mM sodium nitrate solution), column conditions and the like may also be specified. For the column conditions, it is desirable to use a polymethacrylate resin filler with at least 1 to 3 columns with exclusion limit molecular weights of at least 10,000,000. Typical columns include TSKgel $GMPW_{x1}$ (diameter 7.8 mm × 300 mm) and G2500 $PW_{XL}$ (diameter 7.8 mm × 300 mm) (manufactured by Tosoh Corp.).

(Modification rate)

The modification rate indicates the number of modifying groups (A) relative to constituent monosaccharide units contained in the polysaccharide derivative.

Modification rate = Number of modifying groups (A) contained in polysaccharide derivative/number of monosaccharide units contained in polysaccharide derivative [Math. 1]

The modification rate by the modifying group (A) in the polysaccharide derivative is not particularly limited, and may be adjusted according to the purpose. For example, the modification rate by the modifying group (A) in the polysaccharide derivative can be adjusted to the desired range by adjusting the type of modifying group (A), the number of functional groups reacting with the modifying group (A) in the polysaccharide, and the reaction conditions when introducing the modifying group (A) and the like.

The modification rate by the modifying group (A) in the polysaccharide derivative may be for example in the range of from 0.01 to 1, from 0.02 to 0.6, from 0.05 to 0.2 or from 0.1 to 0.2. Such a range of modification rates may be useful for example when the polysaccharide derivative is used as a drug delivery carrier.

In another embodiment, the modification rate by the modifying group (A) in the polysaccharide derivative may be in the range of from 0.1 to 1, or from 0.1 to 0.8, or from 0.15 to 0.6 for example. Such a range of modification rates may be useful when the polysaccharide derivative is used in hydrogel form or the like.

In another embodiment, the modification rate by the modifying group (A) in the polysaccharide derivative may be in the range of from 0.00001 to 0.01 for example.

When the polysaccharide is alginic acid for example, because it has one carboxyl group per monosaccharide unit, the modification rate by the modifying group (A) in the polysaccharide derivative when the modifying group (A) is introduced into the carboxyl groups is a maximum of 1, and may be in the range of from 0.01 to 1, or from 0.01 to 0.6, or from 0.05 to 0.2, or from 0.00001 to 0.01 for example.

When the polysaccharide is hyaluronic acid for example, because hyaluronic acid is composed of a monosaccharide unit (D-glucuronic acid) having one carboxyl group and a monosaccharide unit (N-acetyl-D-glucosamine) having no carboxyl group, the modification rate by the modifying group (A) in the polysaccharide derivative when the

modifying group (A) is introduced into the carboxyl groups is a maximum of 0.5, and may be in the range of from 0.01 to 0.5, or from 0.05 to 0.2, or from 0.00001 to 0.01 for example.

When the polysaccharide is carboxymethyl cellulose for example, because the monosaccharide units have from 1 to 3 carboxyl groups, the modification rate by the modifying group (A) in the polysaccharide derivative when the modifying group (A) is introduced into the carboxyl groups is a maximum of 3 (carboxyl substitution at all 3 positions), and may be in the range of from 0.01 to 3, or from 0.05 to 1.5, or from 0.05 to 1, or from 0.00001 to 0.01 for example.

When the polysaccharide is carboxymethyl dextran for example, because the monosaccharide units have from 1 to 3 carboxyl groups, the modification rate by the modifying group (A) in the polysaccharide derivative when the modifying group (A) is introduced into the carboxyl groups is a maximum of 3 (carboxyl substitution at all 3 positions), and may be in the range of from 0.01 to 3, or from 0.05 to 1.5, or from 0.05 to 1, or from 0.00001 to 0.01 for example.

The modification rate can be calculated by $^1H$ NMR measurement ($D_2O$).

The modification rate can also be calculated by a colorimetric aldehyde assay.

In some cases, the modification rates calculated by $^1H$ NMR measurement and colorimetric aldehyde assay will not match exactly and there may be a difference between the two, but in the present invention it is sufficient that the modification rate as calculated by either $^1H$ NMR measurement or colorimetric aldehyde assay be within the above range.

A spacer may also be used when introducing the modifying group (A). The spacer may be one commonly used in the field (such as those described in Greg T. Hermanson, Bioconjugate Techniques, Third Edition (2013)), and specific examples include polyethylene glycol, polyamides and other peptides, and hydrocarbons.

2. Method for manufacturing polysaccharide derivative

The method for manufacturing the polysaccharide derivative is not particularly limited, but the polysaccharide derivative can be manufactured by a method that includes reacting a compound (a) represented by the following formula (a) with a polysaccharide having a functional group capable of reacting with the -X group of the compound (a). This yields a polysaccharide derivative (C) comprising a modifying group (A) corresponding to the compound (a) (a group represented by the formula (A) as explained above) introduced into a polysaccharide.

[C37]

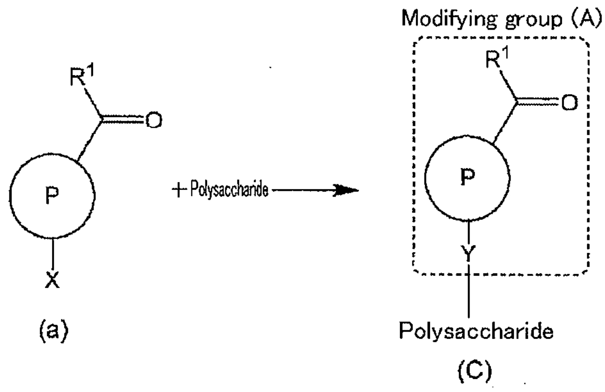


In formula (a) above, X is selected according to the Y of the formula (C).

When Y is $-L^1$-NH- (and $L^1$ binds to ring P), X is $-L^1-NH_2$.

When Y is $-L^2$-C(=O)- (and $L^2$ binds to ring P), X is $-L^2$-C(=O)OH.

When Y is $-L^3$-S- (and $L^3$ binds to ring P), X is $-L^3$-SH.

When Y is $L^4$- (and $L^4$ binds to ring P), X is $-L^4-R^L$, in which $R^L$ is selected from the halogen atoms (F, Cl, Br or I).

The definitions and preferred embodiments of the ring P and $R^1$ in the formulae (a) and (C) above and Y in the formula (C) are the same as the definitions and preferred embodiments in the formula (A) and the formulae (A-1), (A-2), (A-3) and (A-4).

In the manufacturing method of this embodiment, because the polysaccharide has a functional group that is acidic, basic or both (amphoteric) (that is, a functional group having charge), the modifying group (A) can be introduced into the polysaccharide by a one-stage reaction performed under mild and highly safe conditions with the compound (a) represented by formula (a), which has a highly hydrophobic phenyl or pyridyl ring.

In specific embodiments, the method for manufacturing the polysaccharide derivative represented by formula (C) includes reacting an acidic, basic or amphoteric polysaccharide with a compound (a) represented by the formula (a) by

adding the compound (a) in an aqueous solvent, a polar solvent or a higher alcohol to a solution containing the polysaccharide.

The modifying group (A) can be introduced into the polysaccharide at a high modification rate by means of such a reaction.

The method for manufacturing the polysaccharide derivative is explained below using an example in which Y is $-L^1-NH-$ (and $L^1$ binds to ring P) and X is $-L^1-NH_2$. Even if Y is not $-L^1-NH-$, a polysaccharide derivative represented by the formula (C) can still be manufactured by methods known in the literature.

One aspect of the present invention relates to a method for manufacturing a polysaccharide derivative represented by formula (C1) below.

The polysaccharide derivative represented by formula (C1) can be obtained by a condensation reaction between the carboxyl groups of the polysaccharide and the amino groups of the compound (a1) represented by the formula (a1) below. This reaction forms amide bonds between the amino groups of the compound (a1) and the carboxyl groups of the polysaccharide, yielding a polysaccharide derivative represented by formula (C1) below comprising a modifying group (A) corresponding to the compound (a1) (group represented by formula (A-1) as explained above) introduced into the polysaccharide.

[C38]

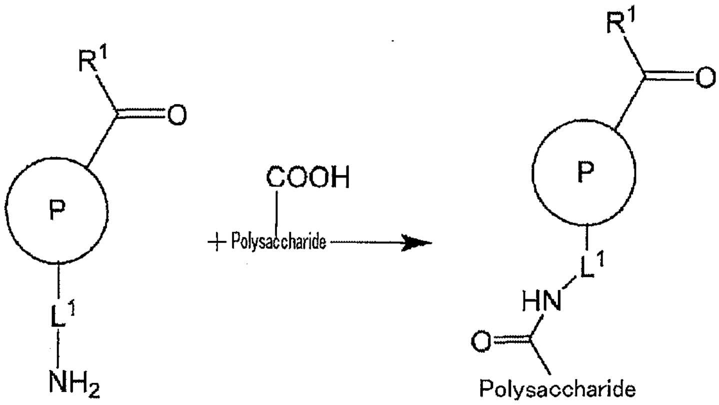


(a1) (C1)

The definitions and preferred embodiments of the ring P and $R^1$ in the formulae (a1) and (C1) above and $L^1$ in the formula (C1) are the same as the definitions and preferred embodiments in the formula (A) and formula (A-1).

The method of the condensation reaction is not particularly limited, and it can be accomplished by methods known in the literature, such as for example the methods described in "Experimental Chemistry Course 5th Edition 16, Synthesis of Organic Compounds IV, Carboxylic Acids and Derivatives, Esters, pp 35-70, Acid Amides and Acid Imides, pp 118-154, Amino Acids and Peptides, pp 258-283, 2007, Maruzen" and the like. The reaction is normally performed in a solvent.

In specific embodiments, the method for manufacturing the polysaccharide derivative represented by formula (C1) includes performing a condensation reaction in a solvent (such as an aqueous solvent) between a polysaccharide having carboxyl groups and a compound (a1) represented by the formula (a1) below.

In one embodiment, the compound (a1) is dissolved or dispersed in an aqueous solvent, a polar solvent or a higher alcohol solvent and then added to a solution containing the polysaccharide and reacted with the polysaccharide. The modifying group (A) can thus be introduced into the polysaccharide at a high modification rate.

Because the compound (a1) represented by the formula (a1) generally has low solubility in aqueous solvents, a reaction can be accomplished with the compound (a1) in a suspended state by adding the compound (a1) to an aqueous solvent containing the polysaccharide and reacting it with the polysaccharide under stirring.

In one embodiment, the compound (a1) is dissolved in a polar solvent (preferably DMSO) or a higher alcohol solvent, and the resulting solution of the compound (a1) is added to an aqueous solvent containing the polysaccharide. The modification rate by the compound (a1) can be increased in this way.

It is surprising that the modification rate could be dramatically improved by the simple method of dissolving or dispersing the compound (a1) in a specific solvent and then adding it to the polysaccharide. The exact reason why the modification rate was improved is unknown, but the inventors hypothesize that because there is an excess of carboxyl groups relative to the compound (a1), the poorly soluble (hydrophobic) compound (a1) is consumed in the reaction in such

an environment, promoting dissolution of subsequent compound (a1) and leading to an improved modification rate. In addition, it is thought that the modification rate is also improved because the reaction between the carboxyl groups of the polysaccharide and the amino groups of the compound (a) is more efficient than the reaction (Schiff base forming reaction) between the amino groups of the compound (a) and the aldehyde groups of the compound (a). However, the manufacturing method of this embodiment is not dependent on this mechanism.

Examples of polar solvents include N,N-dimethylformamide, dimethylsulfoxide (DMSO), dioxane, N-methyl-2-pyrrolidone (NMP) and the like.

Examples of aqueous solvents include water and mixed solvents of water with solvents selected from ether solvents such as tetrahydrofuran (THF) and 1,4-dioxane, alcohol solvents such as methanol, ethanol and 2-propanol, and polar solvents such as N,N-dimethylformamide, dimethylsulfoxide (DMSO), dioxane, N-methyl-2-pyrrolidone (NMP) and the like to the extent that the polysaccharide does not precipitate.

The condensation reaction between the compound (a1) and the polysaccharide is preferably performed under conditions of pH 5 to 10 (more preferably pH 5.5 to 8.5, or still more preferably pH 7.5 to 8.0). The modification rate by the modifying group (A) can be further improved by promoting a reaction while adjusting the pH within the above range. pH adjustment is preferably performed using a sodium hydroxide aqueous solution or a hydrochloric acid aqueous solution. A buffer may also be used in the reaction.

The condensation reaction between the compound (a1) and the polysaccharide is preferably performed in the presence of a condensing agent selected from 1,3-dicyclohexyl carbodiimide (DCC), 1-ethyl-3-(3-dimethylaminopropyl) carbodiimide hydrochloride (WSC·HCl), benzotriazole-1-yloxytris(dimethylamino) phosphonium hexafluorophosphate (BOP reagent), bis(2-oxo-3-oxazolidinyl) phosphinic chloride (BOP-Cl), 2-chloro-1,3-dimethylimidazolinium hexafluorophosphate (CIP), 4-(4,6-dimethoxy-1,3,5-triazine-2-yl)-4-methylmorpholinium chloride (DMT-MM) or the like, with or without an inorganic base such as sodium hydrogen carbonate or sodium carbonate or an organic base such as triethylamine or pyridine.

Such a condensation reaction may be performed at from 0°C to 50°C for example. A preferred temperature range is from 15°C to 40°C. By performing the reaction at a temperature close to room temperature, it is possible to shorten the reaction time and prevent a decline in the molecular weight of the polysaccharide. Side reactions (Schiff base formation reactions) between the amino groups of the compound (a) and the aldehyde groups of the compound (a) can also be suppressed by using such a temperature range.

An additive such as 1-hydroxy-1H-benzotriazole (HOBt) or 1-hydroxy-7-azabenzotriazole (HOAt) may also be added to suppress production of by-products in the condensation reaction.

Following the reaction, purification treatment may be performed by filtration and/or dialysis.

One embodiment of the present invention relates to a method for manufacturing the polysaccharide derivative represented by the following formula (C2). In certain embodiments, the method for manufacturing the polysaccharide derivative represented by formula (C2) includes performing a condensation reaction between a polysaccharide having amino groups and a compound (a2) represented by the following formula (a2).

The polysaccharide derivative represented by the formula (C2) can be obtained by performing a condensation reaction between the amino groups of the polysaccharide and the carboxyl groups of the compound (a2) represented by the formula (a2). This reaction forms amide bonds between the carboxyl groups of the compound (a2) and the amino groups of the polysaccharide, thereby yielding a polysaccharide derivative represented by the formula (C2) comprising a modifying group (A) corresponding to the compound (a2) (group represented by formula (A-2) as explained above) introduced into the polysaccharide.

[C39]

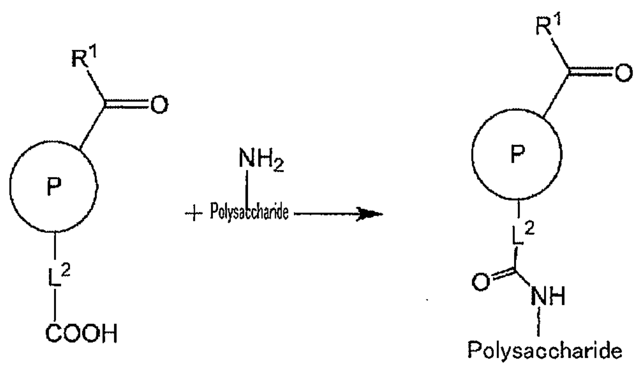

(a2) (C2)

The definitions and preferred embodiments of the ring P and $R^1$ in the formulae (a2) and (C2) above and $L^2$ in the formula (C2) are the same as the definitions and preferred embodiments in the formula (A) and formula (A-2).

The method of the condensation reaction is not particularly limited, and it can be accomplished by a conventional known method such as a method using a carbodiimide condensation agent.

The condensation reaction between the compound (a2) and the polysaccharide is preferably performed under conditions of pH 4 to 7 (more preferably pH 5 to 6). By performing a reaction with the pH adjusted within this range, it is possible to suppress precipitation of the reaction product and efficiently promote the reaction. pH adjustment is preferably performed using a sodium hydroxide aqueous solution or a hydrochloric acid aqueous solution. A buffer may also be used in the reaction.

Moreover, the ratio (molar ratio) of the carboxyl groups of the compound (a2) to the amino groups of the polysaccharide is preferably from 0.8:1 to 1.2:1 in the reaction between the compound (a2) and the polysaccharide. In such cases, it is possible to suppress precipitation of the reaction product and efficiently promote the reaction.

3. Polysaccharide derivative-drug conjugate

The polysaccharide derivative may form a polysaccharide derivative-drug conjugate with a drug containing a primary amino group and the polysaccharide derivative according to the above aspect. Therefore, in certain embodiments, the tissue adhesive material includes a polysaccharide derivative-drug conjugate. The polysaccharide derivative is capable of a conjugate by forming a Schiff base with a drug having a primary amino group. Specifically, an aldehyde group or ketone group (-$CR^1$(=O)) of the polysaccharide derivative reacts with the primary amino group of the drug to form a Schiff base.

Consequently, in the polysaccharide derivative-drug conjugate of this embodiment the drug and the group represented by formula (A) in the polysaccharide (modifying group (A)) are covalently bonded via a Schiff base, forming the structure represented by formula (D) below.

[C40]

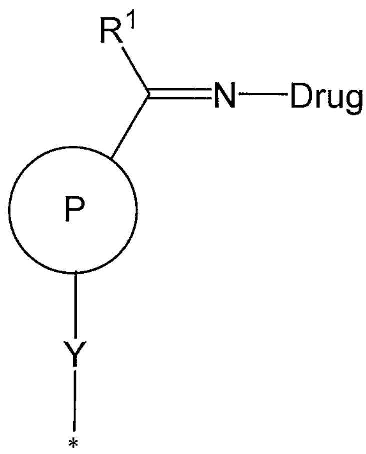

(D)

In the formula (D), "Drug" represents the part of the drug excluding the primary amino group.

In the formula (D), the definitions and preferred embodiments of $R^1$, ring P and Y are the same as the definitions and preferred embodiments of $R^1$ and ring P in the formula (A).

(Drug having primary amino group)

The drug is not particularly limited as long as it has one or more primary amino groups in the molecule. The drug may be either a synthetic product or a natural product. Examples include low-molecular-weight compounds, middle-molecular-weight compounds, peptides, nucleic acids, nucleic acid derivatives, aptamers, vitamins, monoamines, amino acids, polyamines, antibodies, fluorescent dyes, contrast agents and the like.

The primary amino group may also be a hydrazide group (-C(=O)-NH-$NH_2$). A hydrazide group can react with an aldehyde group or ketone group (-$CR^1$(=O)) of the polysaccharide derivative to form a hydrazone bond (-C(=O)-NH-N=CH-). A hydrazone bond is normally more stable than a Schiff base, so slower sustained release can be expected.

Examples of low-molecular-weight compounds (molecular weight up to 500 for example) and middle-molecular-weight compounds (molecular weight 500 to 2,000 for example) having primary amino groups include doxorubicin,

gemcitabine, aminosalicylic acid, pemetrexed, methotrexate, alendronate, eribulin, memantine, remdesivir, ampicillin, amoxicillin, aztreonam, tigemonam, vancomycin, cephalosporin C, gentamycin, trimethoprim, sulfamethoxazole, oseltamivir, mexiletine, midodrine, levodopa, tenofovir, darunavir, procaine, ethyl aminobenzoate, procainamide, fluvoxamine, milnacipran, baclofen, benserazide, carbidopa, droxidopa, gusperimus, ubenimex, fingolimod, amfenac, sulfamine, triamterene, mexiletine, amlodipine, azelnidipine, methyldopa, hydralazine, mosapride, sitagliptin phosphate hydrate, valacyclovir, acyclovir, celecoxib and the like.

The peptide is not particularly limited as long as it has a primary amino group at the N-end or the like. Examples of peptides include methionine enkephalin, leucine enkephalin, dynorphin A, beta-endorphin, bacitracin, daptomycin, colistin, elcatonin, oxytocin and the like.

The nucleic acid bases adenine, thymine, guanine and cytosine have primary amino groups, so nucleic acids and nucleic acid derivatives containing these nucleic acid bases have primary amino groups and can be used in the present invention. Examples of nucleic acid derivatives include cytarabine, cladribine, fludarabine and the like.

Nucleic acid (DNA, RNA) aptamers and peptide aptamers can be used as aptamers.

Examples of vitamins having primary amino groups include folic acid, vitamin B1 (thiamine), vitamin B6 (pyridoxamine), vitamin B12 (cyanocobalamin), nicotinamide and the like.

Examples of monoamines having primary amino groups include dopamine, noradrenaline, serotonin, histamine, thiamine, octopamine and the like.

Examples of amino acids include various amino acids having primary amino groups in the molecule. The amino acid may be a natural amino acid or an artificial amino acid such as tranexamic acid.

Examples of polyamines include molecules existing in vivo that have multiple primary amines in the molecule, such as spermine, spermidine and putrescine.

Examples of fluorescent dyes include fluorescein-5-thiosemicarbazide (FTSC) and the like.

One drug alone or a mixture of two or more may be used.

The drug may be water soluble or water insoluble.

As long as it has a primary amino group, the drug may be in the form of a salt or in the form of a hydrate or solvate.

(Drug introduction rate)

The drug introduction rate of the modifying group (A) in the polysaccharide derivative-drug conjugate is not particularly limited, but is for example the ratio of the modifying groups (A) introduced into the polysaccharide derivative which have formed bonds (Schiff bases) with the drug, represented as a percentage. The drug introduction rate is not particularly limited, but may be from 1% to 100% for example.

(Sustained release properties)

The Schiff bases formed between the aldehyde or ketone groups ($-CR^1(=O)$) of the polysaccharide derivative and the primary amino groups of the drug exist stably under neutral to basic pH conditions, but are dissociated under low pH conditions, releasing the drug.

In this Description, "low pH conditions" mean conditions of less than pH 7, or typically pH 3.5 to 7.0.

Although some drug release does occur in neutral to alkaline (high pH) environments (such as pH 7.4 or more), this release is gradual. Under low pH conditions, on the other hand, drug release occurs rapidly, and a large quantity of the drug is released.

While the physiological pH in blood and the like is about pH 7.4, inflamed tissue and tumor tissue (pH 6.5 to 7.2) and the interior of lysosomes and endosomes (pH 4.5 to 5.5) are known as low pH environments. Since the polysaccharide derivative-drug conjugate of this embodiment can release drugs in response to low pH, it is capable for example of suppressing drug release and maintaining a drug stably at pH 7.4 (corresponding to blood pH), and efficiently releasing the drug at target sites with low pH environments.

[C41]

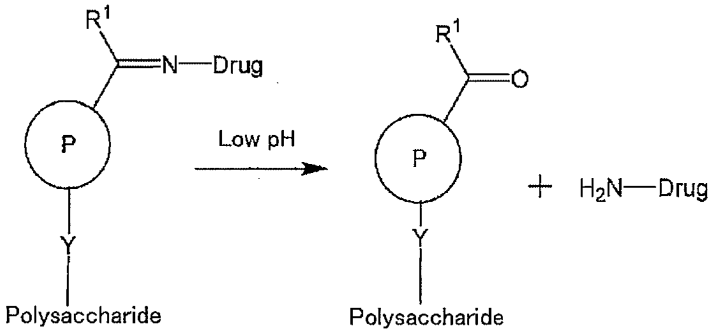


In the formula above, "Drug" represents the part of the drug apart from the primary amino group.
The definitions and preferred embodiments of $R^1$, ring P and Y in the formula above are the same as the definitions and preferred embodiments of $R^1$ and ring P in the formula (A).

(Manufacturing method)

Another aspect of the present invention relates to a method for manufacturing a polysaccharide derivative-drug conjugate. The manufacturing method of this embodiment includes mixing the polysaccharide derivative described above with a drug containing a primary amino group in a solvent. Mixing yields a polysaccharide derivative-drug conjugate by forming Schiff bases by a reaction between the aldehyde or ketone groups ($-CR^1(=O)$) of the polysaccharide derivative and the amino groups of the drug.
The manufacturing method of this embodiment can easily yield a stable polysaccharide derivative drug-conjugate in one pot under one-stage mild and highly stable reaction conditions. Because there is no need to modify the drug before conjugate formation, and since complex experimental equipment and catalysts are also unnecessary for conjugate formation in the manufacturing method of this embodiment, in-situ preparation of the polysaccharide derivative-drug conjugate from the polysaccharide derivative and the drug is also possible.
The solvent is not particularly limited, and one capable of dissolving polysaccharides may be selected appropriately. For example, an aqueous solvent or a polar solvent such as dimethylsulfoxide (DMSO) is preferred for its excellent ability to dissolve polysaccharides.
Examples of aqueous solvents include water and mixed solvents of water with solvents selected from ether solvents such as tetrahydrofuran (THF) and 1,4-dioxane, alcohol solvents such as methanol, ethanol and 2-propanol, and polar solvents such as N,N-dimethylformamide, dimethylsulfoxide (DMSO), and the like to the extent that the polysaccharide does not precipitate.
The temperature during mixing is also not particularly limited, and may be in the range of from 0°C to 50°C for example. A temperature range of from 15°C to 40°C is preferred. It is possible to both achieve an appropriate reaction speed and prevent reduced portion loss by performing the reaction at a temperature near room temperature.
The pH in the reaction between the polysaccharide derivative and the drug may be any at which the drug dissolves but does not denature, and from the standpoint of the reaction speed, conditions of pH 4.0 to 11.0 (more preferably pH 6.0 to 9.0, or still more preferably pH 7.0 to 8.0) are preferred. By appropriately adjusting the pH while allowing the reaction to proceed, it is possible to further improve the introduction efficiency of the drug into the modifying group (A). pH adjustment is preferably performed used hydrochloric acid or sodium hydroxide.
The reaction between the polysaccharide derivative and the drug is also preferably performed under shaded conditions if the drug has poor photostability.
The reaction time is not particularly limited, but is about 1 minute to 24 hours, or preferably 10 minutes to two hours.
The mixing ratio of the polysaccharide derivative and the drug is set appropriately according to the number of modifying groups (A) introduced into the polysaccharide derivative, the desired drug introduction rate, and the solubility of the resulting polysaccharide derivative drug-conjugate. For example, the drug may be mixed at a rate of 0.001 to 10 moles, or preferably 0.05 to 3 moles, or more preferably 0.1 to 1 mole per 1 mole of the introduced modifying groups (A) in the polysaccharide derivative.
The method for mixing the polysaccharide derivative and the drug is not particularly limited as long as both components can be mixed uniformly.
The polysaccharide derivative-drug conjugate (reaction liquid) obtained by mixing may be used as is without

purification. After conjugate formation, it may be subjected to purification treatment such as dialysis, salting out, gel filtration, ion exchange chromatography or electrophoresis, and may also be freeze dried.

4. Composition

Another aspect of the present invention relates to a composition containing a polysaccharide derivative of the above aspect or a polysaccharide derivative-drug conjugate of the above aspect.

Certain embodiments provide a composition containing a polysaccharide derivative of the above aspect, or a polysaccharide derivative-drug conjugate of the above aspect, together with an aqueous solvent. The weight ratio of the polysaccharide derivative or conjugate and the aqueous solvent in the composition is from 0.0001:1 to 0.3:1 for example, or preferably from 0.001:1 to 0.1:1.

A composition containing a polysaccharide derivative of the above aspect together with a drug having a primary amino group is provided in certain embodiments. The molar ratio of the polysaccharide derivative and the drug in this composition is for example a ratio (A:drug) of from 1:0.001 to 1:10, or preferably from 1:0.05 to 1:3, or more preferably from 1:0.1 to 1:1.

Certain embodiments provide a composition containing a polysaccharide derivative of the above aspect, a drug having a primary amino group, and an aqueous solvent. The ratio of the total weight of the polysaccharide derivative and the drug to the weight of the aqueous solvent in this composition is for example from 0.0001:1 to 0.3:1, or preferably from 0.001:1 to 0.1:1. The molar ratio of the polysaccharide derivative and the drug is for example a ratio (A:drug) of 1:0.001 to 1:10, or preferably from 1:0.05 to 1:3, or more preferably from 1:0.1 to 1:1.

Certain embodiments provide a composition containing the crosslinked structure of the polysaccharide derivative described below together with an aqueous solvent. The weight ratio of the crosslinked structure to the aqueous solvent in this composition is for example from 0.0001:1 to 0.3:1, or preferably from 0.001:1 to 0.1:1.

Certain embodiments provide a composition containing the crosslinked structure of the polysaccharide derivative-drug conjugate described below (crosslinked structure-drug conjugate) together with an aqueous solvent. The weight ratio of the crosslinked structure to the aqueous solvent in this composition is for example from 0.0001:1 to 0.3:1, or preferably from 0.001:1 to 0.1:1.

The aqueous solvent may be similar to those mentioned in the context of the polysaccharide derivative manufacturing method and conjugate manufacturing method.

The polysaccharide derivative or polysaccharide derivative-drug conjugate of the present invention may be made into a medical composition together with generally known medical carriers and diluents and other additives, and administered either orally or non-orally to non-mammals or mammals including humans.

Examples of mammals are not limited but include humans, chimpanzees, apes, monkeys, cows, horses, sheep, goats, pigs, rabbits, dogs, cats, rats, mice, guinea pigs and the like. Examples of non-mammals are not limited but include birds, fish and insects.

One embodiment provides a medical composition containing a polysaccharide derivative of the above aspect, a drug, and pharmaceutically acceptable additives.

One embodiment provides a medical composition containing a polysaccharide derivative-drug conjugate of the above aspect together with pharmaceutically acceptable additives.

Examples of the pharmaceutically acceptable additives include excipients, fillers, bulking agents, binders, humectants, disintegrants, lubricants, surfactants, dispersants, buffers, preservatives, solubilizers, antiseptics, flavoring agents, analgesics, stabilizers and isotonic agents and the like commonly used in drug manufacture. These additives may be selected appropriately and used to prepare the drug composition by ordinary methods.

The polysaccharide derivative or polysaccharide derivative-drug conjugate of the present invention may be compounded into a food composition together with additives commonly used in the food sector (for example, at least one ingredient selected from the various nutritional supplements (amino acids, vitamins, minerals, etc.), sugars, milk products, sweeteners, flavorings, fragrances, antioxidants, preservatives, colorings, emulsifiers, pH adjusters, organic acids, buffers, fruit juices and the like).

The composition according to the above aspect can be used as a tissue adhesive material or to produce a tissue adhesive material.

5. Crosslinked structure

The polysaccharide derivative may form a crosslinked structure. One aspect of the present invention relates to a crosslinked structure containing a polysaccharide derivative.

(1) Crosslinked structure of polysaccharide derivative crosslinked by crosslinking agent

In certain aspects, the crosslinked structure is a crosslinked structure of a polysaccharide derivative or a crosslinked structure of a polysaccharide derivative-drug conjugate (also called a "crosslinked structure-drug conjugate"). The crosslinked structure of a polysaccharide derivative is one in which the polysaccharide derivative is crosslinked via crosslinking groups to form a three-dimensional network structure. The crosslinked structure of a polysaccharide derivative-drug conjugate (crosslinked structure-drug conjugate) is one in which the polysaccharide derivative-drug conjugate is crosslinked via crosslinking groups to form a three-dimensional network structure. The crosslinked structure of the polysaccharide derivative can be obtained by performing a crosslinking reaction using a crosslinking agent on a polysaccharide derivative having crosslinking groups. The crosslinked structure of a polysaccharide derivative-drug conjugate (crosslinked structure-drug conjugate) can typically be obtained by a method of performing a crosslinking reaction with a crosslinking agent on a polysaccharide derivative-drug conjugate having crosslinking groups, or by a method of first manufacturing a crosslinked structure of a polysaccharide derivative, and then binding the crosslinked structure to a drug via Schiff bases.

Examples of crosslinking agents include metal ions; an amino group-containing polymer and an amino group-containing low-molecular-weight compound, which contain two or more primary amino groups, a hydrazide group, or an aminoxy group; and a compound containing two or more aldehyde groups such as glutaraldehyde.

For example, unmodified carboxyl groups may function as crosslinking groups to form a crosslinked structure via a crosslinking agent such as a divalent metal ion. A composition containing a polysaccharide derivative or polysaccharide derivative-drug conjugate containing unmodified carboxyl groups can be made into a crosslinked structure by reacting it in a solution containing a crosslinking agent.

The forms of these crosslinked structures are not particularly limited, but examples include tube structures, fibrous structures, fibers, beads, gels, semispherical gels, capsules, sponges, sheets, films and the like.

Specific embodiments provide a gel, capsule, sponge, bead, fiber, tube, sheet or film containing a polysaccharide derivative, polysaccharide derivative-drug conjugate, crosslinked structure or crosslinked structure-drug conjugate of the above aspects.

Specifically, a solution containing a polysaccharide derivative having unmodified carboxyl groups can be added dropwise or the like to a solution containing a divalent metal ion as a crosslinking agent, and partially crosslinked to obtain a crosslinked structure. Similarly, a solution containing a polysaccharide derivative-drug conjugate having unmodified carboxyl groups can be added dropwise or the like to a solution containing a divalent metal ion as a crosslinking agent, and partially crosslinked to obtain a crosslinked structure.

A crosslinked structure manufactured by such methods may be in the form of a capsule, bead, fiber, tube or film for example.

Alternatively, a solution containing a polysaccharide derivative having unmodified carboxyl groups and a solution containing a divalent metal ion as a crosslinking agent can each be applied to a base material to partially crosslink the polysaccharide derivative and obtain a crosslinked structure. A crosslinked structure manufactured by such methods may be in the form of a gel (hydrogel).

A crosslinked structure in the form of a sponge can then be obtained by freeze drying the gel.

After the crosslinked structure is formed, the drug can be bound to the crosslinked structure via a Schiff base by a method similar to the method of manufacturing the polysaccharide derivative-drug conjugate, such as for example by a method that includes first forming a crosslinked structure and then mixing the crosslinked structure in a solvent with a drug containing a primary amino group. Mixing causes the aldehyde or ketone groups ($-CR^1(=O)$) of the crosslinked structure to form Schiff bases by reacting with the amino groups of the drug, yielding a crosslinked structure-drug conjugate.

The drug can also be attached physically or by chemical bonds to the crosslinked structure of the polysaccharide derivative to thereby obtain a drug carried on a crosslinked structure of a polysaccharide derivative.

For example, a solution containing the drug can be applied to the crosslinked structure of the polysaccharide derivative, or the crosslinked structure of the polysaccharide derivative can be immersed in this solution, or the drug can be printed on the crosslinked structure of the polysaccharide derivative to thereby obtain a drug carried on a crosslinked structure of a polysaccharide derivative.

Specific examples of the divalent metal ion used as the crosslinking agent in the crosslinking reaction include calcium ions, magnesium ions, barium ions, strontium ions, zinc ions and the like, and a calcium ion is preferred. More specifically, $CaCl_2$, $MgCl_2$, $CaSO_4$, $ZnCl_2$, BaCh or $SrCl_2$ or the like (preferably $CaCl_2$, $CaSO_4$, $ZnCl_2$, $SrCl_2$ or $BaCl_2$) may be used as a divalent metal ion compound.

In addition to this divalent metal ion (divalent metal ion compound), a trivalent metal ion compound ($FeCl_3$) such as $Fe^{3+}$ or a crosslinking reagent having 2 to 4 amino groups in the molecule or the like may also be used as a crosslinking agent. Examples of crosslinking reagents having 2 to 4 amino groups in the molecule include diaminoalkanes which may have lysyl groups ($-COCH(NH_2)-(CH_2)_4-NH_2$) on nitrogen atoms, or in other words diaminoalkanes and derivatives thereof having lysylamino groups formed by substitution of amino groups with lysyl groups, and specific examples include

diaminoethane, diaminopropane, N-(lysyl)-diaminoethane and the like.

In addition to these, when the drug has multiple primary amino groups the drug itself may function as a crosslinking agent when introduced into the polysaccharide. For example, the polysaccharide derivative may form a three-dimensional mesh structure when crosslinked by an intermediate-molecular-weight compound (peptide) (for example, bacitracin) having multiple primary amino groups.

In addition, as will be described below, the amino group-containing polymer and the amino group-containing low-molecular-weight compound, which contain two or more primary amino groups, a hydrazide group, or an aminoxy group, can function as a crosslinking agent, and be crosslinked by a covalent bond to the modifying group (A) of the polysaccharide derivative via a Schiff base.

In addition, a compound containing two or more aldehyde groups such as glutaraldehyde can function as a crosslinking agent and form a crosslinked structure (crosslinked structure component) via the hydroxyl group of the polysaccharide derivative.

The amount of the crosslinking agent used is preferably adjusted appropriately according to the amount and molecular weight of the polysaccharide derivative and the types of polysaccharides constituting the polysaccharide derivative. For example, when a calcium ion is used as the crosslinking agent, the calcium ion concentration of a solution containing the crosslinking agent is not particularly limited, but may be from 1 mM to 1 M, or preferably from 5 mM to 500 mM, or more preferably from 10 mM to 300 mM.

The degree of crosslinking may also be adjusted by adjusting the amount of the crosslinking agent used.

(2) Crosslinked structure of polysaccharide derivative and amino group-containing polymer/amino group-containing low-molecular-weight compound

In certain embodiments, the crosslinked structure contains a polysaccharide derivative and at least one of an amino group-containing polymer and an amino group-containing low-molecular-weight compound containing two or more primary amino groups, hydrazide groups or aminooxy groups, and is crosslinked by covalent bonds formed via Schiff bases between the primary amino groups, hydrazide groups or aminooxy groups of the amino group-containing polymer and amino group-containing low-molecular-weight compound and a group represented by the formula (A) (modifying group (A)) in the polysaccharide derivative. That is, the amino group-containing polymer and the amino group-containing low-molecular-weight compound function as a crosslinking agent and form a crosslinked structure (crosslinked structure component) via the crosslinking group of the polysaccharide derivative (for example, the aldehyde group or carbonyl group of the modifying group (A)). Specifically, an aldehyde group or ketone group ($-CR^1(=O)$) of the modifying group (A) of the polysaccharide derivative reacts with the primary amino groups, hydrazide groups or aminooxy groups of the amino group-containing polymer and amino group-containing low-molecular-weight compound to form Schiff bases ($-CR^1=N-$). The hydrazide groups are represented by $-C(=O)-NH-NH_2$, and form hydrazone bonds by reacting with the aldehyde groups or ketone groups of the modifying group (A). The aminooxy groups are represented by $-O-NH_2$, and form oxime bonds by a reaction between the terminal $-NH_2$ groups and the aldehyde groups or ketone groups of the modifying groups (A).

The amino group-containing polymer is a substance with a molecular weight of at least 1,000 containing two or more primary amino groups, hydrazide groups or aminooxy groups. In this Description, an "amino group-containing polymer" also includes having a molecular weight of about 1,000 to 10,000, commonly called oligomers.

The amino group-containing low-molecular-weight compound is a substance with a molecular weight of less than 1,000 containing two or more primary amino groups, hydrazide groups or aminooxy groups.

The amino group-containing polymer and amino group-containing low-molecular-weight compound may have been manufactured by synthesis, or may be naturally occurring substances.

The amino group-containing polymer containing primary amino groups, hydrazide groups or aminooxy groups (hereunder also called simply the "amino group-containing polymer") may be any polymer containing a total of two or more of at least kind selected from a primary amino group, a hydrazide group and an aminooxy group, without any particular limitations. The primary amino groups, hydrazide groups or aminooxy groups may be present at the end of the polymer, in the side chains of the polymer, or in pendant groups in the polymer.

The amino group-containing polymer may be of at least one kind selected from the polyamines; polyalkylene glycols substituted with amino groups, hydrazide groups or aminooxy groups; polyallylamines; polyvinylamines; polyacrylamines; amino group-containing polysaccharides; amino group-containing proteins; and polyamino acids. Commercial products may be used for these, or they may be synthesized by conventional known methods.

A polyamine may be either linear, branched or dendritic. "Dendritic" means that the polyamine has a dendritic hyperbranched morphology, and is a polymer having multiple arms of equal or unequal length.

The polyamine is not particularly limited, but examples include linear, branched or dendritic polyalkyleneimines and branched or dendritic polyetheramines.

A polyalkyleneimine is a polymer having an alkylene imine structures as a repeating unit, and having a terminal primary amino group. The alkylene part and imine part of the alkyleneimine structure may each be substituted. Linear

polyalkyleneimines contain alkyleneimine structures having secondary amino groups. Branched polyalkyleneimines and dendritic polyalkyleneimines contain alkyleneimine structures having primary amino groups, secondary amino groups or tertiary amino groups. The polyalkyleneimine preferably has a lower (such as C1 to C6, or C1 to C3) alkyleneimine structure, and specific examples include, but are not limited, to polyethylenimine, polypropylenimine and the like.

A branched on dendritic polyetheramine is a polymer having an alkylene oxide structure as a repeating unit, and having a terminal primary amino group. Examples include, but are not limited to, amino-terminated star-shaped polyethylene oxide, amino-terminated dendritic polyethylene oxide, amino-terminated comb-shaped polyethylene oxide, amino-terminated star-shaped polypropylene oxide, amino-terminated dendritic polypropylene oxide, amino-terminated comb-shaped polypropylene oxide, amino-terminated star-shaped polyethylene oxide-polypropylene oxide copolymer, amino-terminated dendritic polyethylene oxide-polypropylene oxide copolymer, amino-terminated comb-shaped polyethylene oxide-polypropylene oxide copolymer and the like. An amino-terminated star-shaped polymer may be a polymer having 3, 4, 6 or 8 arms terminated with primary amines. For example, amino-terminated star-shaped polyethylene glycol is star-shaped polyethylene glycol having 3, 4, 6 or 8 arms terminated with primary amines (3-, 4-, 6- or 8-arm star PEG amine). However, examples are not limited to these. A commercial product such as polyoxyalkylene triamine sold under the trade name of Jeffamine® triamine by Huntsman LLC (Houston, TX) may be used as a branched or dendritic polyetheramine.

Examples of polyalkylene glycols substituted with amino, hydrazide or aminooxy groups include polyethylene glycol (PEG), polypropylene glycol or polyethylene oxide-polypropylene oxide copolymers substituted with multiple (two or more) amino groups, hydrazide groups or aminooxy groups.

A polyallylamine is a polymer having an allylamine structure as a repeating unit. The allyl part in the allylamine structure may also be substituted. In addition to the repeating units of the amine structure, the polyallylamine may also have another repeating unit as a copolymerization component.

A polyvinylamine is a polymer having a vinylamine structure as a repeating unit, and the vinyl part in the vinylamine structure may be substituted. In addition to the repeating units of the vinylamine structure, the polyvinylamine may also have another repeating unit as a copolymerization component.

A polyacrylamine is a polymer containing an acrylic structure with side chains containing primary amino groups, and the vinyl part in the acrylic structure may be substituted. For example, this may be an acrylic polymer containing an amine structure with a polyalkyelenimine grafted to the side chains. In addition to the repeating units of the acrylic structure having side chains containing primary amino groups, the polyacrylamine may also another repeating unit as a copolymerization component. A commercial product such as an aminoethylated acrylic polymer sold under the trade name Polyment® (Nippon Shokubai) may be used.

Examples of amino group-containing polysaccharides include polysaccharides such as chitosan containing amino groups. An amino group-containing polysaccharide may also be obtained by modifying a polysaccharide lacking amino groups to introduce amino groups into the polysaccharide (aminated polysaccharide). Examples of aminated polysaccharides include aminodextran or the like with introduced amino groups.

Examples of amino group-containing proteins include fibrinogen, albumin, gelatins, collagens and the like.

Examples of polyamino acids (polypeptides) include polylysine, polyarginine, polyglutamic acid, polyaspartic acid and the like.

The weight-average molecular weight of an amino group-containing polymer is not particularly limited, but is generally at least 1,00. There is no particular upper limit to the weight-average molecular weight of an amino group-containing polymer, but generally it is not more than 1,000,000.

An amino group-containing low-molecular-weight compound may be any low-molecular-weight compound containing a total of at least two of at least one kind of group selected from a primary amino group, a hydrazide group and an aminooxy group, without any particular limitations. Examples include hydrazide crosslinking agents such as adipic dihydrazide, sebacic dihydrazide, dodecanedioic dihydrazide and isophthalic dihydrazide.

A salt may also be formed by the amino group in an amino group-containing polymer or amino group-containing low-molecular weight compound. Salts of amino group-containing polymers or amino group-containing low-molecular-weight compounds include halide salts (such as hydrochlorides), phosphate salts, phosphite salts, carbonate salts, bicarbonate salts, sulfate salts, hydrogen sulfate salts, hydroxides, nitrate salts, persulfate salts, sulfite salts, acetates, ascorbate salts, citrate salts, oxalate salts, succinate salts, tartrate salts, and taurocholate or cholate salts.

Fig. 34 shows a schematic diagram of a crosslinked structure prepared in Example I-17 below, which is formed from a polysaccharide derivative (AL-ABA) and an amino group-containing polymer (DPI: polyethyleneimine). As shown in Fig. 34, the crosslinked structure has a structure in which the primary amino groups contained in the amino group-containing polymer (polyethyleneimine) are crosslinked by covalent binding via Schiff bases (-C=N-) with the benzaldehyde groups contained in the polysaccharide derivative.

The crosslinked structure of the polysaccharide derivative with the amino group-containing polymer or amino group-containing low-molecular-weight compound is typically manufactured by mixing the polysaccharide derivative with the amino group-containing polymer or amino group-containing low-molecular-weight compound in a solvent. From the

standpoint of biocompatibility, an aqueous solvent (such as water or physiological saline) is preferred as the solvent.

Once a crosslinked structure of the polysaccharide derivative and the amino group-containing polymer or amino group-containing low-molecular-weight compound has been obtained, a crosslinking agent (such as a divalent metal ion) different from the amino group-containing low-molecular weight compound may be used to obtain a structure crosslinked via crosslinking groups (for example, unmodified carboxyl groups) contained in the crosslinked structure. Specifically, crosslinked structures can be obtained by first mixing the polysaccharide derivative and the amino group-containing polymer or amino group-containing low-molecular-weight compound in a solvent, and then dripping a solution containing a divalent metal ion as a crosslinking agent into the solution of the crosslinked structure; or by first mixing the polysaccharide derivative and the amino group-containing polymer or amino group-containing low-molecular-weight compound in a solvent, and then dripping the solution containing the crosslinked structure into a solution containing a divalent metal ion as a crosslinking agent to perform partial crosslinking.

The type and amount of the crosslinking agent are not particularly limited, and may be similar to the type and amount of the crosslinking agent in the crosslinking reaction of the polysaccharide derivative using a crosslinking agent above.

The form of the crosslinked structure of the polysaccharide derivative and the amino group-containing polymer or amino group-containing low-molecular-weight compound is not particularly limited, and examples include tube structures, fibrous structures, fibers, beads, gels, semispherical gels, capsules, sponges, sheets, films and the like.

In one embodiment, the crosslinked structure of the polysaccharide derivative and the amino group-containing polymer or amino group-containing low-molecular-weight compound may be in the form of a gel (hydrogel). The gel may also be freeze dried to form a crosslinked structure in sponge form.

6. Tissue adhesive material

In certain embodiments, the tissue adhesive material includes the above polysaccharide derivative, polysaccharide derivative-drug conjugate, crosslinked structure, and crosslinked structure-drug conjugate. In specific embodiments, the tissue adhesive material is a biological tissue adhesive or sealing agent (sealant) used to adhere biological tissue.

A tissue adhesive material containing the above polysaccharide derivative or crosslinked structure is provided by certain embodiments. The polysaccharide derivative or crosslinked structure can attach to biological tissue through interactions between the aldehyde groups or ketone groups ($-C(=O)R^1$) of the introduced modifying group (A) and functional groups in the biological tissue.

In specific embodiments, the polysaccharide derivative or crosslinked structure can attach to biological tissue by forming Schiff bases by a reaction between the aldehyde groups or ketone groups ($-C(=O)R^1$) of the introduced modifying group (A) and primary amino groups in the biological tissue.

In certain embodiments, the tissue adhesive material contains a polysaccharide derivative-drug conjugate or crosslinked structure-drug conjugate. Preferably, the polysaccharide derivative-drug conjugate or crosslinked structure-drug conjugate has a drug having a primary amino group bound to some of the modifying groups (A), and also has unreacted modifying groups (A) with no drug bound thereto. With this embodiment, the drug can be delivered to an adhesion site while at the same time the introduced aldehyde groups or ketone groups ($-C(=O)R^1$) of the unmodified modifying groups (A) react with the primary amino groups of the biological tissue to form Schiff bases and attach the material to the biological tissue.

The form of the tissue adhesive material is not particularly limited, and examples include tube structures, fibrous structures, fibers, beads, capsules, gels, semispherical gels, sponges, sheets, films and the like, but a bead, capsule, gel, semispherical gel or sponge is preferred, a capsule, hydrogel or sponge is more preferred, and a hydrogel is especially preferred. A hydrogel is useful as a tissue adhesive or sealant for medical applications when rapid decomposition is required to prevent undesirable tissue-tissue adhesion due to injury, surgery or the like for example.

The method for using the tissue adhesive material is not particularly limited.

In certain embodiments, the tissue adhesive material is a tissue adhesive used for attaching two biological tissues together.

In certain embodiments, a tissue adhesive containing the above polysaccharide derivative or crosslinked structure is applied to at least one attachment site of biological tissue, and two or more sites are brought into contact to attach two biological tissues.

In certain embodiments, a tissue adhesive containing the above polysaccharide derivative or crosslinked structure is applied to at least one attachment site of biological tissue, a crosslinking agent (such as a solution containing a divalent metal ion) is then applied to the same attachment site, and two or more sites are brought into contact to attach two biological tissues (layering method). In certain embodiments, a crosslinking agent (for example, a solution containing divalent metal ions) is applied to at least one adhesion site of the biological tissue, a tissue adhesive containing the polysaccharide derivative or crosslinked structure is then applied to the adhesion site, two or more sites are brought into

contact with each other to adhere two biological tissues to each other (layering method). In certain aspects, a tissue adhesive containing the polysaccharide derivative or crosslinked structure and a crosslinking agent (for example, a solution containing divalent metal ions) are mixed, the mixture is applied to biological tissues, and two or more sites are brought into contact with each other to adhere two biological tissues (mixing method). In the form of the mixing method, in order to obtain sufficient adhesive ability, it is preferable to apply the mixture to the biological tissue immediately after mixing. When the crosslinking agent is applied, a hydrogel can be formed or the degree of hydrogel crosslinking can be increased.

In certain embodiments, the tissue adhesive material is a sealant used to seal air/fluid leaks in biological tissue or seal or pack small voids or defects in biological tissue.

In certain embodiments, a sealant containing the polysaccharide derivative or crosslinked structure is applied to biological tissue and left there to seal air/fluid leaks in the biological tissue, or to seal or pack small voids or defects in the biological tissue.

In certain embodiments, a sealant containing the polysaccharide derivative or crosslinked structure is applied to biological tissue, and a crosslinking agent (such as a solution containing a divalent metal ion) is then applied to the same application site and left there to seal air/fluid leaks in the biological tissue, or to seal or pack small voids or defects in the biological tissue (layering method). In certain embodiments, a crosslinking agent (for example, a solution containing divalent metal ions) is applied to biological tissue, and a sealing agent containing the polysaccharide derivative or crosslinked structure is then applied to the application site, and left, and thus seals air/fluid leakage in the biological tissue or seals or fills small cavities or defects in the biological tissue (layering method). In certain aspects, a sealing agent containing the polysaccharide derivative or crosslinked structure and a crosslinking agent (for example, a solution containing divalent metal ions) are mixed, the mixture is applied to biological tissue, and left, and thus seals air/fluid leakage in the biological tissue or to seal or fill small cavities or defects in the biological tissue (mixing method). In the form of the mixing method, in order to obtain sufficient adhesive and sealing ability, it is preferable to apply the mixture to biological tissue immediately after mixing. When the crosslinking agent is applied, a hydrogel can be formed or the degree of hydrogel crosslinking can be increased.

In certain embodiments, the sealant may be combined with a tissue section derived from biological tissue, and skin flap, periosteum or the like may be used for the tissue section. A tissue section may be attached to tissue in combination with these sealants.

In certain embodiments, the sealant may also be combined with a biodegradable or non-biodegradable non-woven fabric, sheet, film or the like, and used to attach the nonwoven fabric, sheet or film. The biodegradable nonwoven fabric, sheet or film may be made of a material such as polyglycolic acid, L-lactide/ε-caprolactone polymer, polylactic acid, glycolic acid/lactic acid polyester or sodium alginate, while the non-biodegradable nonwoven fabric, sheet or film may be made of a material such as polytetrafluoroethylene (PTFE). The sealants may be attached to tissue in combination with such a nonwoven fabric, sheet or film.

When the polysaccharide derivative is used in combination with a nonwoven fabric, sheet, film or the like, the mechanical strength of the polysaccharide derivative structure (such as hydrogel) can be improved, and it is possible to prevent burst even at a higher pressure level.

In certain embodiments, the tissue adhesive material further includes a nonwoven fabric, sheet, or film. In specific embodiments, the tissue adhesive material is a sealing agent, and the sealing agent contains the polysaccharide derivative and a nonwoven fabric, sheet, or film. In one embodiment, the tissue adhesive material is a sealing agent used to restore dura mater damage, and the sealing agent contains the polysaccharide derivative and a nonwoven fabric, sheet, or film.

As the nonwoven fabric, sheet, or film, commercial products may be used, and for example, Neovil (registered trademark) from Gunze Medical Japan Ltd (GMJ) and Vicryl (registered trademark) Mesh from Ethicon can be used.

When used in combination with a nonwoven fabric, sheet, or film, the method of use is not particularly limited, for example, a method in which first, a nonwoven fabric, sheet, or film is placed or attached onto a tissue (for example, tissue injury site), a tissue adhesive or sealing agent containing the polysaccharide derivative or crosslinked structure is then applied onto the nonwoven fabric, sheet, or film, or between the nonwoven fabric, sheet, or film and the tissue, and a crosslinking agent (for example, a solution containing divalent metal ions) is then applied to the application site and left can be used. According to the method, it is possible to easily and reliably restore the tissue injury site. As other methods, a method in which a tissue adhesive or sealing agent containing the polysaccharide derivative or crosslinked structure is applied onto tissue (for example, tissue injury site), the nonwoven fabric, sheet, or film is then placed or attached onto the application site, and a crosslinking agent (for example, a solution containing divalent metal ions) is then applied and left; and a method in which a tissue adhesive or sealing agent containing the polysaccharide derivative or crosslinked structure is applied onto tissue (for example, tissue injury site), a crosslinking agent (for example, a solution containing divalent metal ions) is then applied, and the nonwoven fabric, sheet, or film is then placed or attached to the application site before gelling occurs may be used.

In specific embodiments, the tissue adhesive material (tissue adhesive, sealant) is attached to biological tissue in

the form of an aqueous solution or dispersion.

In specific embodiments, the tissue adhesive material (tissue adhesive, sealant) is attached to biological tissue in the form of a gel, sponge, sheet or film.

The tissue adhesive material may also contain various additives according to the intended use. These various additives may include one or more selected from the pH adjusters, antibiotics, colorants and surfactants.

As described above, in the first aspect, the tissue adhesive material is preferably used for hard and thick tissue and/or connective tissue, and can also be used for a wide range of biological tissues other than hard and thick tissue and connective tissue.

The biological tissue to which the tissue adhesive material is applied is not particularly limited, and examples thereof include dura mater, skin, oral cavity, periodontal ligament, gums, alveolar bone, esophagus, stomach, small intestine, large intestine (rectum), bone, nerve, exon, cartilage, perichondrium, elastic cartilage (for example, conchal cartilage, nasal cartilage, and epiglottic cartilage), blood vessel, cornea, muscle, fascia, brain, prostate, breast, endometrium, lung, spleen, liver, testis, ovary, neck, lymph node, bone marrow, and kidney. Among these, dura mater, perichondrium, periodontal ligament, elastic cartilage (for example, conchal cartilage, nasal cartilage, and epiglottic cartilage), skin, the mucosa of digestive tract (oral cavity, esophagus, stomach, small intestine, large intestine), and the submucosa of digestive tract (mucosa injury site) are preferable.

In certain aspects, the biological tissue to which the tissue adhesive material is applied is biological tissue having an amino group exposed on the surface. In such a case, a covalent bond is formed between the aldehyde group or ketone group (-C(=O)$R^1$) of the modifying group (A) of the tissue adhesive material and the amino group via a Schiff base. The adhesion strength can be improved. Examples of biological tissues having an amino group exposed on the surface include skin (particularly, collagen-exposed site after surgery), healthy mucosa, the submucosa of digestive tract (mucosa injury site), dura mater, perichondrium, and periodontal ligament. In addition, application to biological tissue such as lung, heart, pancreas, kidney, liver, and bladder is also preferable.

The specific site of the biological tissue is also not particularly limited, and sites where tissue adhesive materials have been conventionally used may be adopted appropriately. Examples of such application sites include suture sites following surgery, bleeding sites, fracture fragment fixing sites, anastomosis sites of peripheral nerves and microvessels, sites affected by tendon adhesion and tendon sutures, and adhesion sites of organ injuries and the like.

The tissue adhesive material can be used in applications such as dura mater adhesion, nerve anastomosis, microvascular anastomosis, preservation of vascular sutures, closure of traumatic tympanic membrane defects, sealing of vascular substitute and preservation of suture sites, liver destruction, adhesion of liver test incision and gallbladder incision, partial nephrectomy, wound management during renal destruction and prostatectomy, preservation of intestinal anastomosis, adhesion of split skin flaps, closure of bone and cartilage wound cavities (particularly in patients at risk of bleeding), cartilage and bone adhesion, tendon suture reinforcement, pleural adhesion in pneumothorax, sealing an alveolar cavity after tooth extraction when there is a risk of bleeding, during sealing the tonsillar fossa with collagen fiber bundles (particularly in patients at risk of bleeding), and free skin grafting for burn patients.

As described above, the application of the tissue adhesive material is not particularly limited, and for example, in brain surgery, it is used to repair the dura mater, which is the outermost layer of the meninges, during surgery for brain injury, brain tumor, or cerebral hemorrhage, that is, to repair and restore incisions in the cerebral dura mater, and used as an adhesive when filling defects with artificial dura mater (dura mater repairing and protection). In orthopedics, it is used to repair and protect the spinal cord membrane, particularly, the dura mater, during spinal and spinal cord surgery. In addition, in otolaryngology, it is also used for adhesion when transplanting and repairing elastic cartilage such as conchal cartilage, nasal cartilage, and epiglottic cartilage.

In addition, in gastrointestinal surgery, it is used to adhere tissue for adhesion at digestive tract anastomosis, for tissue restoration during anal fistula or anal formation, and for preventing pancreatic fistula and bile leakage during pancreatic and liver resection and used to protect and restore damaged sites of the digestive tract inner wall during endoscopic surgery. In respiratory surgery, it is used as a lung sealant material in order to prevent air leakage during lung resection (lung tissue protection) and to adhere tissue such as tracheal cartilage, and in urological surgery, it is used to protect tissue during surgery, to prevent urine leakage by adhering ureteral anastomosis sites, to restore anastomosis, and in gynecology, it is used to protect and restore tissue during surgery.

Certain embodiments provide a kit containing a tissue adhesive material for application to a biological material to attach the biological material, together with instructions for use of the tissue adhesive material.

Certain embodiments provide a kit containing a precursor of a tissue adhesive material for application to a biological material to attach the biological material, together with instructions for use of the tissue adhesive material. In one embodiment, the precursor of the tissue adhesive material includes an un-crosslinked polysaccharide derivative or partially crosslinked polysaccharide derivative, or a conjugate of these with a drug, together with a crosslinking agent (such as a solution containing a divalent metal ion).

II. Second aspect

A second aspect of the present invention provides a crosslinked structure including (a) a polysaccharide derivative in which a group represented by Formula (A) below is introduced into an acidic, basic or amphoteric polysaccharide, and (b) at least one of an amino group-containing polymer and an amino group-containing low-molecular-weight compound, which contain two or more primary amino groups, a hydrazide group, or an aminoxy group and a group represented by Formula (x1) below (hereinafter referred to as a "modifying group (x1)") (hereinafter referred to as an "amino group-containing substance (b)"), wherein the primary amino groups, the hydrazide group, or the aminoxy group contained in the amino group-containing polymer and the amino group-containing low-molecular-weight compound and the group represented by Formula (A) contained in the polysaccharide derivative are crosslinked by a covalent bond via a Schiff base.

[C42]

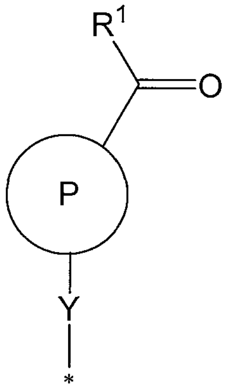

(A)

(in Formula (A), $R^1$ represents a hydrogen atom or a $C_{1-4}$ alkyl group,

the ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
Y represents a divalent group selected from the group consisting of -NH-, -C(=O)-, -S-, -O-, a $C_{1-6}$ alkylene group, $-(CH_2CH_2O)_n-$, and any combinations of these, and n is an integer from 1 to 9, and
* represents a linkage with the polysaccharide).

[C43]

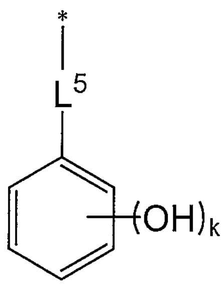

(x1)

In Formula (x1), k is an integer from 2 to 5 and preferably an integer from 2 to 3.
In Formula (x1), $L^5$ is a linker group. $L^5$ is a single bond or a divalent group selected from the group consisting of -C(=O)-, -S-, -O-, an alkylene group, $-(CH_2CH_2O)_n-$, and any combinations of these, and n is an integer from 1 to 9. In certain embodiments, $L^5$ is -C(=O)- or an alkylene group with 1 to 5 carbon atoms. In one embodiment, $L^5$ is -C(=O)-.
In Formula (x1), * represents a linkage with the amino group-containing polymer or the amino group-containing low-molecular-weight compound.
In the crosslinked structure, the aldehyde group or ketone group ($-CR^1(=O)$) of the modifying group (A) of the polysaccharide derivative reacts with the primary amino groups, hydrazide group, or aminoxy group of the amino group-containing substance (b) to form a covalent bond (a Schiff base ($-CR^1=N-$), a hydrazone bond, or an oxime bond).

In the crosslinked structure, the modifying group (x1) such as a catechol (1,2-dihydroxybenzene) group or a pyrogallol(1,2,3-trihydroxybenzene) group can form a hydrogen bond with a hydrogen atom on the surface of the tissue to promote adhesion.

The modifying group (x1) in the crosslinked structure and the modifying group (A) of the unreacted polysaccharide derivative can function as functional groups for tissue adhesion or the like.

In the second aspect, as the (a) polysaccharide derivative, those described in the section "1. Polysaccharide derivative" in the first aspect can be used in the same manner.

In the second aspect, as the amino group-containing substance (b), one obtained by introducing a modifying group (x1) into the "amino group-containing polymer" and "amino group-containing low-molecular-weight compound" in the first aspect can be used.

The method of introducing the modifying group (x1) into the amino group-containing substance (b) is not particularly limited, and for example, when a precursor compound having a modifying group (x1) such as gallic acid and a carboxyl group is subjected to a carbodiimide reaction with the amino group-containing substance (b) to bond the carboxyl group and the amino group of the amino group-containing substance (b), the modifying group (x1) can be introduced.

In certain embodiments, the amino group-containing substance (b) is a polyalkyleneimine modified with a modifying group (x1).

In a second embodiment, the ratio of the polysaccharide derivative to the amino group-containing substance (b) is not particularly limited. In certain embodiments, the ratio of the polysaccharide derivative to the amino group-containing substance (b) is in a range of 1:1000 to 1000:1 (equivalent ratio) between the modifying group (A) of the polysaccharide derivative and the amino group of the amino group-containing substance (b).

When the reactivity of the amino group of the amino group-containing substance (b) is high, it tends to quickly react with the modifying group (A) (aldehyde group or ketone group) of the polysaccharide derivative to form a Schiff base. In this case, when the equivalent of the modifying group (A) of the polysaccharide derivative is less than that of the amino group of the amino group-containing substance (b), almost all of the modifying group (A) of the polysaccharide derivative may react with the amino group of the amino group-containing substance (b) to form a Schiff base, and the amount of the unreacted modifying group (A) may be small or zero. In such a viewpoint, when the crosslinked structure of this aspect is used for applications such as the tissue adhesive material to be described below, it is preferable to combine the polysaccharide derivative and the amino group-containing substance (b) so that a sufficient amount of unreacted modifying groups (A) for reaction with the amino groups of the tissue remains. For example, the ratio of the polysaccharide derivative to the amino group-containing substance (b) is in a range of 1:100 to 100:1 (equivalent ratio) between the modifying group (A) of the polysaccharide derivative and the amino group of the amino group-containing substance (b).

The method of producing a crosslinked structure and the form thereof are the same as those of "(2) Crosslinked structure of polysaccharide derivative and amino group-containing polymer/amino group-containing low-molecular-weight compound" in "4. Crosslinked structure" in the first aspect except that the amino group-containing substance (b) has a modifying group (x1).

Similar to the first aspect, the crosslinked structure of the second aspect may be a crosslinked structure formed of a polysaccharide derivative-drug conjugate obtained by conjugating a drug to a polysaccharide derivative in advance and an amino group-containing substance (b). As the "polysaccharide derivative-drug conjugate," those described in the section "3. Polysaccharide derivative-drug conjugate" in the first aspect can be used in the same manner.

In certain embodiments, there is provided a composition containing a crosslinked structure of the second aspect. The form of "composition" is the same as that described in the section "4. Composition" in the first aspect.

(Use)

The use of the crosslinked structure according to the second aspect is not particularly limited.

The crosslinked structure, the crosslinked structure-drug conjugate, and the composition can be preferably used as a tissue adhesive material used for the biological tissue. The embodiment (a shape, a method of use, a specific form of use, or the like) of "Tissue adhesive material" is the same as that in the section "6. Tissue adhesive material" in the first aspect. Similar to the first aspect, the tissue adhesive material of the second aspect can be applied for a wide range of biological tissues. That is, the tissue adhesive material of the second aspect can be used for a wide range of biological tissues such as soft tissue in vivo and hard and thick tissue and/or connective tissue, as described in the section "6. Tissue adhesive material" in the first aspect.

In addition, the crosslinked structure, crosslinked structure-drug conjugate, or composition can be used in a wide range of fields including food, medicine, cosmetics, household goods, textiles, and papermaking. The form thereof is not particularly limited, and is selected according to the use. Examples thereof include tubular structures, fibrous structures, fibers, beads, gels, semispherical gels, capsules, sponges, sheets, and films. In one embodiment, the crosslinked structure, crosslinked structure-drug conjugate, or composition may be in the form of a gel, sponge, film or capsule.

The crosslinked structure can be used in place of conventional polysaccharides materials in a wide range of fields

including food, medicine, cosmetics, household goods, textiles, and papermaking.

The crosslinked structure, crosslinked structure-drug conjugate, or composition can be suitably used as a medical material because it has excellent biodegradability and biocompatibility.

When used as a medical material, examples of medical materials include drug delivery devices, sutures, materials for hemostasis, adhesion, and adhesion prevention within the body, tissue adhesive materials, wound dressings, materials for leakage or preventing leakage of tissue fluids, lung sealants for preventing air leakage, cell culture substrates, cell transplant substrate reproducing engineering materials, antithrombotic materials, diagnostic drugs, and dialysis carriers. The medical materials can be used in mammals or non-mammals.

Particularly, the polysaccharide derivative can be suitably used as a drug transport carrier (drug delivery carrier) because it can bind to a drug having a primary amino group under neutral pH and high pH conditions and release it under low pH conditions (pH-responsive drug release). One aspect of the present invention provides a drug delivery device containing the above crosslinked structure or crosslinked structure-drug conjugate, or a composition containing the same. The drug delivery device can be used as a means for selectively and efficiently introducing a drug loaded on the polysaccharide derivative into target tissue. In addition, another aspect provides a method of releasing an encapsulated desired drug in target tissue or a method of controlling release.

When used as a medical material, examples of shapes of the crosslinked structure include a tubular shape, a fibrous shape, a fiber, a bead, a capsule, a gel, a semispherical gel, a sponge, a sheet, and a film, and a bead, a capsule, a gel, a semispherical gel and a sponge are preferable, and a capsule, a hydrogel, and a sponge are more preferable.

According to certain embodiments, there is provided an anti-adhesion material containing the crosslinked structure and crosslinked structure-drug conjugate.

The crosslinked structure-drug conjugate can be used as a bioabsorbable material and a medical device since it has excellent biocompatibility.

The crosslinked structure can be used as a separation material since it binds with a drug having a primary amino group. Examples of separation materials include chromatography carriers, nonwoven fabrics, and membrane materials.

The crosslinked structure and the crosslinked structure-drug conjugate can be used as food, supplements and food additives.

An example of the crosslinked structure of this aspect is described in, for example, Example 1-30 to be described below.

III. Third aspect

The third aspect of the present invention relates to a tissue adhesive material containing a polysaccharide derivative containing an amino group. In certain embodiments of the present invention, the tissue adhesive material contains a polysaccharide derivative in which a group represented by Formula (A-2) below (hereinafter referred to as a "modifying group (A-2)") is introduced into a polysaccharide containing an amino group, and the group represented by Formula (A-2) is introduced into the polysaccharide by substituting a hydrogen atom of the amino group of the polysaccharide to form an amide bond.

In certain embodiments, the tissue adhesive material of the third aspect is in the form of a gel.

[C44]

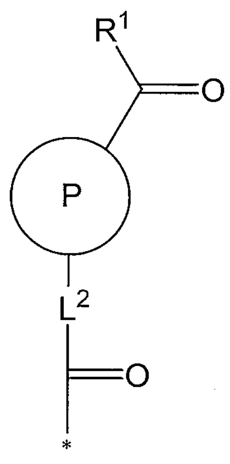

(A-2)

(in Formula (A-2),

$R^1$ represents a hydrogen atom or a $C_{1-4}$ alkyl group,
the ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
$L^2$ is a single bond or represents a divalent group selected from the group consisting of -NH-, -S-, -O-, a $C_{1-6}$ alkylene group, $-(CH_2CH_2O)_n-$, and any combinations of these,
n is an integer from 1 to 9, and
* represents a linkage with the polysaccharide).

In the third aspect, as the polysaccharide derivative containing an amino group, those described in "Polysaccharide derivative containing an amino group" in the first aspect can be used. The "polysaccharide containing an amino group" and "group represented by Formula (A-2)" are the same as those in the first aspect.
The tissue adhesive material of the third aspect has favorable adhesion due to the modifying group (A-2) and/or amino group of the polysaccharide derivative. That is, when the aldehyde group or ketone group ($-C(=O)R^1$) of the modifying group (A-2) of the polysaccharide derivative forms a Schiff base between the amino group of the biological tissue and the aldehyde of the polysaccharide derivative, favorable adhesion to tissue is exhibited. In addition, between the amino group of the polysaccharide derivative and the carboxyl group of the biological tissue, an electrostatic interaction or an interaction such as a hydrogen bond occurs, and the adhesion to tissue can be improved. In certain embodiments, the amino group of the polysaccharide derivative becomes ammonium ions ($NH^{3+}$) at a low pH (for example, a pH of 5 or less, a pH of less than 5, a pH of 1 to 5, a pH of 4 or less, or a pH of 1 to 4), an electrostatic interaction occurs between the carboxyl groups of the biological tissue, and the adhesion to tissue can be improved. In certain embodiments, the amino group of the polysaccharide derivative interacts with the hydroxyl group or carboxyl group of the biological tissue via a hydrogen bond at a high pH (for example, a pH of 5 or more, a pH of more than 5, a pH of 5 to 10, a pH of 5 to 8, a pH of 6 to 10, or a pH of 6 to 8), and the adhesion to tissue can be improved. Thus, when the tissue adhesive material of the third aspect contains a polysaccharide derivative containing an amino group, it can exhibit excellent adhesion at various pH values, and can be applied to various sites of the biological tissue.
The shape of the tissue adhesive material of the third aspect is not particularly limited, and examples thereof include a tubular shape, a fibrous shape, a fiber, a bead, a capsule, a gel, a semispherical gel, a sponge, a sheet, and a film.
In certain embodiments, the tissue adhesive material is in the form of a gel.

(Gel form)

The shape of the tissue adhesive material in the form of a gel is not particularly limited, and examples thereof include a tube, a fiber, a disk, a bead, a sheet, and a film. Whether the tissue adhesive material is in the form of a gel, for example, can be confirmed by a test according to the vial inversion method in Example V-2 to be described below.
In certain embodiments, the tissue adhesive material is self-crosslinking. In certain embodiments, the amino group contained in the polysaccharide derivative and the group represented by Formula (A-2) are crosslinked by forming a covalent bond via a Schiff base to form a crosslinked structure, and a gel form is exhibited.
In certain embodiments, the tissue adhesive material has a self-crosslinking property in response to pH (pH-responsive self-crosslinking property). As shown in Fig. 88(A), at a high pH, the polysaccharide derivative containing an amino group forms a covalent bond between the amino group contained in the polysaccharide derivative and the aldehyde group or ketone group ($-C(=O)R^1$) of the group represented by Formula (A-2) via a Schiff base, and a gel form is exhibited. On the other hand, at a low pH, in the polysaccharide derivative containing an amino group, the amino group contained in the polysaccharide derivative becomes ammonium ions (cations), and it becomes a solution.
In certain embodiments, in the tissue adhesive material, at a pH of 5 or more (for example, a pH of 5 to 10 or a pH of 5 to 8), the polysaccharide derivative undergoes self-crosslinking, and a gel form is exhibited.
The pH of the tissue adhesive material may be adjusted using an acid (for example, hydrochloric acid) or an alkali (for example, sodium hydroxide). When the pH of the solution containing the polysaccharide derivative is adjusted to, for example, a pH of 5 or more (for example, a pH of 5 to 10 or a pH of 5 to 8), a polysaccharide derivative hydrogel can be obtained, and a tissue adhesive material containing a polysaccharide derivative hydrogel can be obtained.
In certain embodiments, the tissue adhesive material of the third aspect can be produced by a method according to "Method of producing polysaccharide derivative represented by Formula (C2)" in "2. Method of producing polysaccharide derivative." Specifically, the tissue adhesive material of the third aspect can be produced by reacting a polysaccharide containing an amino group with a compound (a2) represented by Formula (a2) to obtain a polysaccharide derivative represented by Formula (C2) below in which a group represented by Formula (A-2) is introduced into a polysaccharide, then adjusting the pH to, for example, a pH of 5 or more (for example, a pH of 5 to 10 or a pH of 5 to 8), and causing gelling.

In certain embodiments, the tissue adhesive material has a structure (crosslinked structure) crosslinked via a crosslinking group of the polysaccharide derivative (for example, a carboxyl group, an aldehyde group, a carbonyl group, an amino group, and a hydroxyl group) using a crosslinking agent (for example, divalent metal ions such as calcium ions, magnesium ions, barium ions, strontium ions, and zinc ions; an amino group-containing polymer and an amino group-containing low-molecular-weight compound, which contain two or more primary amino groups, a hydrazide group, or an aminoxy group; and a compound containing two or more aldehyde groups such as glutaraldehyde), and exhibits a gel form.
In certain embodiments, in the tissue adhesive material, the viscosity ($\eta_{0.001}$) of the composition at a shear rate of 0.001 $s^{-1}$ measured at 25°C is in a range of preferably $10^9$ to 1 mPa·s, more preferably $10^9$ to $10^3$ mPa·s, and still more preferably $10^9$ to $10^4$ mPa·s.
In the tissue adhesive material of certain embodiments, the viscosity ($\eta_{1000}$) of the composition at a shear rate of 1,000 $s^{-1}$ measured at 25°C is in a range of preferably 0.1 to $10^4$ mPa·s, more preferably 1 to $10^3$ mPa·s, and still more preferably 1 to 100 mPa·s.
In the tissue adhesive material of certain embodiments, the ratio ($\eta_{0.001}/\eta_{1000}$) of a viscosity ($\eta_{0.001}$) at a shear rate of 0.001 $s^{-1}$ to a viscosity ($\eta_{1000}$) at a shear rate of 1,000 $s^{-1}$ measured at 25°C is preferably 10 or more, more preferably 100 or more, still more preferably $10^4$ or more, and particularly preferably $10^5$ or more. A higher $\eta_{0.001}/\eta_{1000}$ is preferable, and the upper limit is not particularly limited, and is, for example, $10^9$ or less. The $\eta_{0.001}/\eta_{1000}$ is, for example, in a range of preferably 10 to $10^9$, more preferably 100 to $10^9$, still more preferably $10^4$ to $10^9$, and particularly preferably $10^5$ to $10^9$.
The tissue adhesive material having a ratio ($\eta_{0.001}/\eta_{1000}$) of a viscosity ($\eta_{0.001}$) at a shear rate of 0.001 $s^{-1}$ to a viscosity ($\eta_{1000}$) at a shear rate of 1,000 $s^{-1}$ within the above range has a shear thinning property, for example, an advantage of reducing the injection resistance in catheters and injection needles.
In the tissue adhesive material of the third aspect, similar to the first aspect, the polysaccharide derivative may be a polysaccharide derivative-drug conjugate obtained by conjugating a drug. As the "polysaccharide derivative-drug conjugate," those described in the section "3. Polysaccharide derivative-drug conjugate" in the first aspect can be used in the same manner.

(Tissue adhesive material)

The tissue adhesive material of the third aspect is applied to biological tissue
The embodiment (a shape, a method of use, a specific form of use, or the like) of the "tissue adhesive material" is the same as that in the section "6. Tissue adhesive material" in the first aspect. The tissue adhesive material of the third aspect can be applied for a wide range of biological tissues. That is, the tissue adhesive material of the third aspect can be used for a wide range of biological tissues such as soft tissue in vivo and hard and thick tissue and/or connective tissue, as described in the section "6. Tissue adhesive material" in the first aspect.
The hydrogel (a composition in the form of a gel) containing the polysaccharide derivative containing an amino group can be used for applications other than the tissue adhesive material (for example, a wide range of applications described in the second aspect).
Here, all publications cited in this specification, for example, prior art documents, patent applications, patent publications, and other patent documents are incorporated herein by reference.
Furthermore, the objects, features, advantages and ideas of the present invention are clear to a person skilled in the art from the descriptions of this Description, and a person skilled in the art can easily implement the present invention based on the descriptions of this Description. The best mode and specific examples for implementing the present invention illustrate preferred embodiments of the present invention, and are presented in order to exemplify or explain the invention, not to limit its scope. Based on the description herein, it will be apparent to those skilled in the art that various modifications can be made within the spirit and scope of the invention disclosed herein.

Examples

The present invention will be described in more detail below with reference to Examples and Comparative Examples, but the present invention is not limited to these Examples.
In this Description, "room temperature" normally means about 10°C to 35°C. "%" means percent by weight unless otherwise specified.
In this Description, the term "about" may mean ±10%.
Unless otherwise specified, measurement of the nuclear magnetic resonance spectrum ($^1$H NMR) was performed using a JEOL JNM-A500 Alpha FT-NMR spectrometer (500 MHz) (JEOL) with a heavy solvent ($D_2O$).
The abbreviations used in the examples are commonly used abbreviations known to those skilled in the art. Certain abbreviations are shown below.

AL: Alginic acid (sodium)

Alg: Alginic acid (sodium)
ABA: 4-aminobenzaldehyde
AL-ABA: Benazaldehyde-modified alginic acid
HOBt: 1-hydroxybenzotriazole
WSCD/HCl, EDC·HCl: 1-ethyl-3-(3-dimethylaminopropyl) carbodiimide hydrochloride
THF: Tetrahydrofuran
DMSO: Dimethylsulfoxide
NaCl: Sodium chloride
eq: Equivalent
Van: Vancomycin
PBS: Phosphate-buffered saline
FTSC: Fluorescein-5-thiosemicarbazide
FBS: Fetal bovine serum
DMEM: Dulbecco's modified Eagle Medium
Bac: Bacitracin
HA: Hyaluronic acid
PMX: Pemetrexed
CMC: Carboxymethyl cellulose
DCC: N,N'-dicyclohexyl carbodiimide
CMD, CMDX: Carboxymethyl dextran
Mw: Weight-average molecular weight

In the synthesis schemes and reaction formulae shown in the examples below, the polysaccharides (AL, HA, CMC, CMDX) are shown in free form (with carboxyl groups), but the carboxyl groups (-COOH) in the polysaccharides (AL, HA, CMC, CMDX) shown in the synthesis schemes and reaction formulae may also be in an ionized state ($-COO^-$) or salt state (-COOX). For example, in Example 1 below the AL is used in the form of a sodium salt (sodium alginate), so in the synthesis scheme 1 the carboxyl groups (-COOH) in the AL may be in an ionized state ($-COO^-$) or salt state (-COONa).

I. Alginic acid derivative

1. AL-ABA

[Example 1-1] Benzaldehyde modified alginic acid (AL-ABA)

<Synthesis of AL-ABA (1)>

[C45]

Synthesis scheme 1

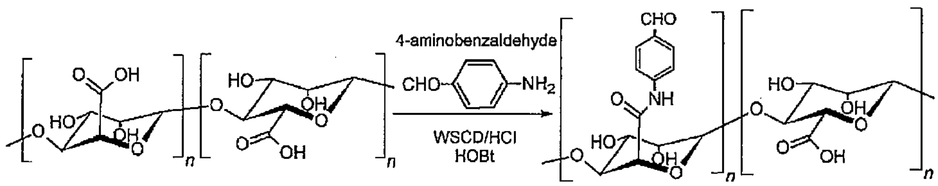


AL AL-ABA

For purposes of convenience, the synthesis scheme 1 above shows a reaction to introduce a modifying group derived from 4-aminobenzaldehyde (ABA) into a carboxyl group of the guluronic acid unit (lefthand monosaccharide unit), but the modifying group derived from ABA may also be introduced into a carboxyl group of the mannuronic acid unit (righthand monosaccharide unit). In the scheme 1, the AL-ABA has modifying groups derived from ABA introduced into the carboxyl groups of both the guluronic acid unit (lefthand monosaccharide unit) and the mannuronic acid unit (righthand monosaccharide unit).

AL-ABA (1) was synthesized according to the following steps (1) to (6) by an amidation reaction via carbodiimide

according to the above synthesis scheme.

(Synthesis procedures)

(1) 200 mg (1 mmol) of sodium alginate (AL) (IL-6G, viscosity 50 to 80 mPa·s (1%), weight-average molecular weight Mw = 750,000 to 850,000, manufactured by Kimica Co., Ltd.) was dissolved in 50 mL of pure water, and stirred overnight to prepare an alginic acid solution.
(2) 242.9 mg (2 mmol, 1 eq) of ABA was dissolved in 20 mL of THF, and stirred overnight to prepare an ABA solution.
(3) 270.3 mg (2 mmol, 2 eq) of 1-hydroxybenzotriazole (HOBt) was dissolved in 10 mL of DMSO, and added dropwise one drop at a time to the alginic acid solution prepared in (1) above. Next, 383.7 mg (2 mmol, 2 eq) of 1-ethyl-3-(3-dimethylaminopropyl) carbodiimide hydrochloride (WSCD/HCl) was dissolved in 5 mL of pure water, and added dropwise one drop at a time to the alginic acid solution.
(4) The ABA solution prepared in (2) above was added dropwise one drop at a time to the alginic acid solution obtained in (3) above.
(5) The solution obtained in (4) above was adjusted to pH 5.5, stirred for 3 to 4 hours, and then diluted with 100 mL of pure water.
(6) The solution obtained in (5) above was filtered under reduced pressure, and dialyzed for 2 days with NaCl and for 2 days with pure water. This was then frozen with liquid nitrogen and freeze dried for 3 days to obtain AL-ABA (1).

<$^1$H NMR spectrum measurement, UV-visible light absorption spectrum (UV-vis) measurement and FT-IR spectrum measurement>

The AL-ABA (1) was subjected to $^1$H NMR spectrum measurement, UV-visible light absorption spectrum (UV-vis) measurement and Fourier transform infrared (FT-IR) spectrum measurement. The results are shown in Figs. 1 to 3.

In the $^1$H NMR spectrum (Fig. 1), peaks derived from ABA (peaks b and c derived from benzene rings and peak d derived from aldehyde) were observed in the AL-ABA after modification. Characteristic peaks derived from ABA (at about 235 nm and about 330 nm) were also observed in the UV-vis spectrum (Fig. 2) of the AL-ABA after modification, while in the FT-IR spectrum (Fig. 3) absorption (peak at about 1740 $cm^{-1}$) derived from the C=O of the amide, which is the attachment point of ABA, was observed in the AL-ABA after modification. These results confirmed synthesis of benzaldehyde modified alginic acid (AL-ABA) formed by binding between amino groups of 4-aminobenzaldehyde and carboxyl groups of alginic acid. Based on the $^1$H NMR spectrum, the ABA modification rate of the carboxyl groups (-COOH) of the alginic acid was calculated to be 0.069.

<Cell toxicity evaluation (WST assay) of benzaldehyde-modified alginic acid (AL-ABA)>

(Test procedures)

MeT-5A (human mesothelial cell line), NIH-3T3 (mouse embryo fibroblasts), HUVEC (human umbilical vein endothelial cells) and RAW264.7 cells (mouse macrophage-like cell line) were seeded on 24-well plates and incubated for 24 hours. The medium was then replaced with media having AL-ABA and AL dissolved at different concentrations (0.01 mg/mL, 0.1 mg/mL, 1 mg/mL). Cell viability (%) 48 hours after sample addition was then measured by WST-8 assay (Cell Counting Kit-8, Dojindo). The AL-ABA (1) synthesized in Example 1-1 was used as the AL-ABA.

(Results)

The results are shown in Fig. 4. Cell viability was equivalent with the AL-ABA and the AL. This confirms that AL-ABA has low cell toxicity and high biocompatibility.

[Example 1-2] Benzaldehyde-modified alginic acids (AL-ABA)

AL-ABA (2) and AL-ABA (3) were synthesized by the different methods described below.

<Synthesis of AL-ABA (2)>

1 g (0.0046 mol) of AL-500 (sodium alginate, viscosity 400 to 600 mPa·s, manufactured by Mochida Pharmaceutical Co., Ltd.) was dissolved in 300 mL of distilled water, and stirred for 6 to 8 hours. 2.81 g (0.020 mol) of HOBt was

dissolved in 10 mL of DMSO, and added dropwise to the AL-500 solution. Next, 3.97 g (0.020 mol) of WSCD/HCl was dissolved in 10 mL of distilled water, and added dropwise to the AL-500 solution. The AL-500 solution was stirred for 10 minutes to obtain an alginic acid solution. 0.84 g (0.0039 mol) of 4-aminobenzaldehyde (ABA) in 10 mL of DMSO was added dropwise to the alginic acid solution. The pH of the reaction mixture was maintained at pH 7.5 with 1N NaOH as the mixture was stirred for 16 to 20 hours at room temperature. The reaction mixture was transferred to a 50 mL centrifuge tube and centrifuged at room temperature for 10 minutes at 3,000 rpm, and the supernatant was collected. The solution was then dialyzed for 72 hours with deionized water using a dialysis tube (MWCO: 6-8 kDa, Spectra/Pro®) and freeze dried to obtain AL-ABA (2).

<Synthesis of AL-ABA (3)>

AL-ABA (3) was obtained by the same methods as the AL-ABA (2) except that the amount of HOBt was changed to 1.04 g (0.007 mol), the amount of WSCD/HCl was changed to 1.98 g (0.010 mol), and the pH of the reaction mixture was changed to 5.5.

<$^1$H NMR spectrum measurement and FT-IR spectrum measurement>

The AL-ABA (2) and AL-ABA (3) were subjected to $^1$H NMR spectrum measurement and FT-IR spectrum measurement (not shown). These results confirmed synthesis of ABA-modified alginic acids (AL-ABA) formed by binding between amino groups of ABA and carboxyl groups of alginic acid.

Based on the $^1$H NMR spectrum, the ABA modification rate of the carboxyl groups (-COOH) of the alginic acid was calculated for the AL-ABA (2) and AL-ABA (3). The results are shown in Table 1 below together with the reaction conditions for the AL-ABA (2) and AL-ABA (3).

<Aldehyde measurement by colorimetric aldehyde assay>

Using a blue (MAK140) colorimetric aldehyde assay kit, the aldehyde amounts in AL-ABA (2) and AL-ABA (3) were measured and used to calculate the ABA modification rates of the carboxyl groups (-COOH) of the alginic acids.

(Assay procedures)

(1) 10 μL of 10 mM standard solution was diluted with 990 μL of assay buffer to prepare a 100 μM standard solution. The 100 μM standard solution was also subjected to 2-fold serial dilution with assay buffer. 50 μL of diluted standard solution was added to a 96-well plate to produce 0 (blank), 1.56, 3.125, 6.25, 12.5, 25, 50 and 100 μM standards.
(2) 50 μL of Master Reaction Mix was added to each well. This was mixed thoroughly with a horizontal shaker or by pipetting, and the reaction mixture was incubated for 20 to 30 minutes at room temperature. The plate was protected from light during incubation.
(3) 50 μL of Blue Enhancer was added to each well.
(4) The reaction mixture was protected from light while being incubated for a further 20 minutes at room temperature.
(5) After the incubation period, absorbance was measured at 620 to 660 nm.

(Results)

The ABA modification rate of the carboxyl groups (-COOH) of the alginic acid as calculated from the amount of aldehyde was 0.56 or 0.64 for the AL-ABA (2) and 0.33 for the AL-ABA (3). The results are shown in Table 1 below.

[Table 1]

| Table 1 Reaction conditions and ABA modification rates for AL-ABA (2) and AL-ABA (3) | | | | | | | |
|---|---|---|---|---|---|---|---|
| | ABA | ABA Solution | WSCD | HOBt | Reaction pH | DS NMR | DS Colorimetric |
| AL-ABA(2) | 1.5 mol eq. | DMSO | 8 mol eq. | 8 mol eq. | 7.5 | 64%*1<br>78%*2 | 56%*1<br>64%*2 |
| AL-ABA(3) | 1.5 mol eq. | DMSO | 4.5 mol eq. | 4.5 mol eq. | 5.5 | 21% | 33% |
| *1: Low DS, *2: High DS | | | | | | | |

In Table 1, DS represents the degree of substitution, which is the ABA modification rate converted to a percentage (ABA modification rate × 100). DS NMR is the value calculated from the $^{1}H$ NMR spectrum, while DS Colorimetric is the value calculated from the colorimetric aldehyde assay.

*1 and *2 of the AL-ABA (2) represent AL-ABA prepared in different batches. In the examples below, * 1 may be called AL-ABA (2) (Low DS) and *2 may be called AL-ABA (2) (High DS).

2. AL-AAP, AL-ADFBA, AL-APCA, AL-ANA

Alginic acids modified with 4-aminoacetophenone (AAP), 4-amino-2,6-difluorobenzaldehyde (ADFBA), 2-amino-3-pyridinecarboxyaldehyde (APCA) or 6-aminonicotinealdehyde (ANA) were obtained in the same manner as Example 1-1. In the schemes below, the modifying groups derived from AAP, ADFBA, APCA or ANA are shown introduced into the carboxyl groups of the guluronic acid units (lefthand monosaccharide units) for purposes of convenience, but these modifying groups may also be introduced into the carboxyl groups of the mannuronic acid units (righthand monosaccharide units). That is, the AL-AAP, AL-ADFBA, AL-APCA and AL-ANA may have modifying groups derived from AAP, ADFBA, APCA or ANA introduced into both the carboxyl groups of the guluronic acid units (lefthand monosaccharide units) and the carboxyl groups of the mannuronic acid units (righthand monosaccharide units).

[Example 1-3] AAP-modified alginic acid (AL-AAP)

[C46]

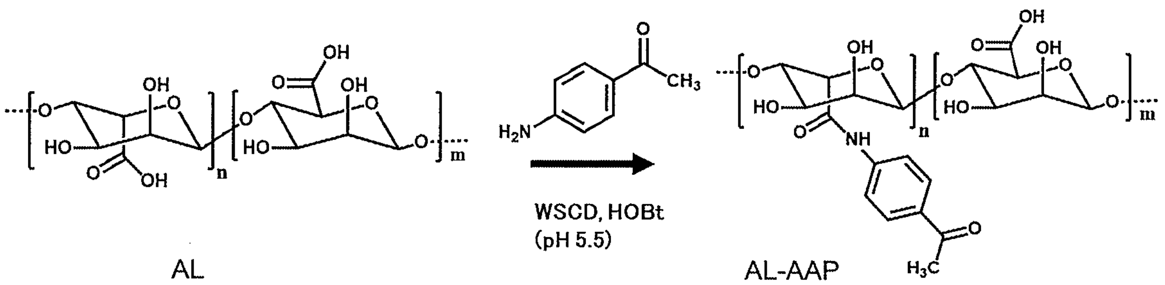


AL　　　AL-AAP

0.5 g (0.0023 mol) of AL-500 (sodium alginate, viscosity 400 to 600 mPa·s, manufactured by Mochida Pharmaceutical Co., Ltd.) was dissolved in 100 mL of distilled water, and stirred for 6 to 8 hours. 1.4 g (0.010 mol) of HOBt was dissolved in 10 mL of DMSO, and added dropwise to the AL-500 solution. Next, 1.97 g (0.010 mol) of WSCD/HCl was dissolved in 10 mL of distilled water, and added dropwise to the AL-500 solution. The AL-500 solution was stirred for 10 minutes to obtain an alginic acid solution. 0.310 g (0.0023 mol) of 4'-aminoacetophenone (AAP) in 10 mL of DMSO was added dropwise to the alginic acid solution. The pH of the reaction mixture was maintained at pH 5.5 with 1N NaOH as the mixture was stirred for 16 to 20 hours at room temperature. The reaction mixture was transferred to a 50 mL centrifuge tube and centrifuged at room temperature for 10 minutes at 3,000 rpm, and the supernatant was collected. The solution was then dialyzed for 72 hours with deionized water using a dialysis tube (MWCO: 6-8 kDa, Spectra/Pro®) and freeze dried to obtain AL-AAP.

<$^{1}H$ NMR spectrum measurement and FT-IR spectrum measurement>

The AL-AAP was subjected to $^{1}H$ NMR spectrum measurement and FT-IR spectrum measurement, with the results shown in Fig. 5 and Fig. 6. These results confirmed synthesis of AAP-modified alginic acid (AL-AAP) formed by binding between amino groups of AAP and carboxyl groups of alginic acid.

Based on the $^{1}H$ NMR spectrum, the AAP modification rate of the carboxyl groups (-COOH) of the alginic acid was calculated to be 0.31.

<Aldehyde measurement by colorimetric aldehyde assay>

The AAP modification rate of the carboxyl groups (-COOH) of the alginic acid was 0.34 when calculated based on the aldehyde amount as measured with a blue (MAK140) colorimetric aldehyde assay kit.

[Example 1-4] ADFBA-modified alginic acid (AL-ADFBA)

[C47]

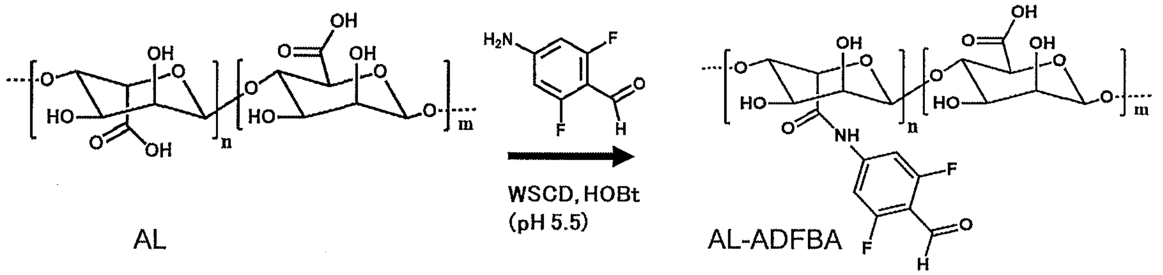


AL AL-ADFBA

An alginic acid solution was obtained as in the Example 1-3. 0.361 g (0.0023 mol) of 4'-4-amino-2,6-difluorobenzaldehyde (ADFBA) in 10 mL of DMSO was added dropwise to the alginic acid solution. The pH of the reaction mixture was maintained at pH 5.5 with 1N NaOH as the mixture was stirred for 16 to 20 hours at room temperature. The reaction mixture was transferred to a 50 mL centrifuge tube and centrifuged at room temperature for 10 minutes at 3,000 rpm, and the supernatant was collected. The solution was then dialyzed for 72 hours with deionized water using a dialysis tube (MWCO: 6-8 kDa, Spectra/Pro®) and freeze dried to obtain AL-ADFBA.

<$^1$H NMR spectrum measurement and FT-IR spectrum measurement>

The AL-ADFBA was subjected to $^1$H NMR spectrum measurement and FT-IR spectrum measurement, with the results shown in Fig. 7 and Fig. 8. These results confirmed synthesis of ADFBA-modified alginic acid (AL-ADFBA) formed by binding between amino groups of ADFBA and carboxyl groups of alginic acid.

Based on the $^1$H NMR spectrum, the ADFBA modification rate of the carboxyl groups (-COOH) of the alginic acid was calculated to be 0.12.

<Aldehyde measurement by colorimetric aldehyde assay>

The ADFBA modification rate of the carboxyl groups (-COOH) of the alginic acid was 0.18 when calculated based on the aldehyde amount as measured with a blue (MAK140) colorimetric aldehyde assay kit.

[Example 1-5] APCA-modified alginic acid (AL-APCA)

[C48]

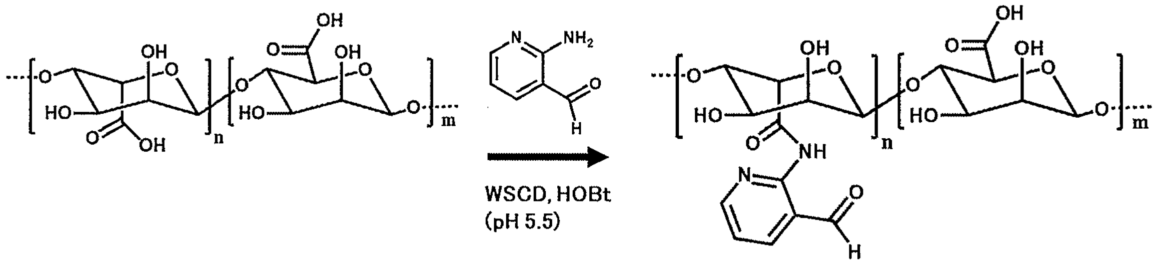


AL AL-APCA

An alginic acid solution was obtained as in the Example 1-3. 0.28 g (0.0023 mol) of 2-amino-3-pyridinecarboxyaldehyde (APCA) in 10 mL of DMSO was added dropwise to the alginic acid solution. The pH of the reaction mixture was maintained at pH 5.5 with 1N NaOH as the mixture was stirred for 16 to 20 hours at room temperature. The reaction mixture was transferred to a 50 mL centrifuge tube and centrifuged at room temperature for 10 minutes at 3,000 rpm, and the supernatant was collected. The solution was then dialyzed for 72 hours with deionized water using a dialysis tube (MWCO:

6-8 kDa, Spectra/Pro®) and freeze dried to obtain AL-APCA.

<$^1$H NMR spectrum measurement and FT-IR spectrum measurement>

The AL-APCA was subjected to $^1$H NMR spectrum measurement and FT-IR spectrum measurement, with the results shown in Fig. 9 and Fig. 10. These results confirmed synthesis of APCA-modified alginic acid (AL-APCA) formed by binding between amino groups of APCA and carboxyl groups of alginic acid.

Based on the $^1$H NMR spectrum, the APCA modification rate of the carboxyl groups (-COOH) of the alginic acid was calculated to be 0.41.

<Aldehyde measurement by colorimetric aldehyde assay>

The APCA modification rate of the carboxyl groups (-COOH) of the alginic acid was 0.33 when calculated based on the aldehyde amount as measured with a blue (MAK140) colorimetric aldehyde assay kit.

[Example 1-6] ANA-modified alginic acid (AL-ANA)

[C49]

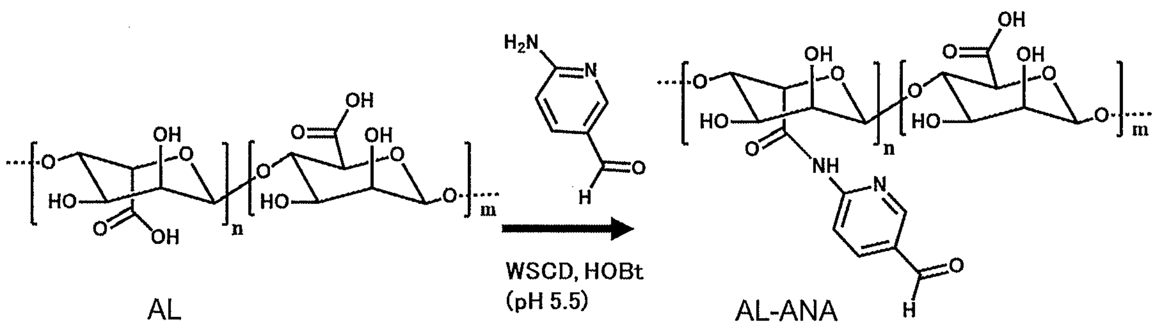


An alginic acid solution was obtained as in the Example 1-3. 0.280 g (0.0023 mol) of 6-aminonicotinealdehyde (ANA) in 10 mL of DMSO was added dropwise to the alginic acid solution. The pH of the final reaction mixture was maintained at pH 5.5 with 1N NaOH as the mixture was stirred for 16 to 20 hours at room temperature. The reaction mixture was transferred to a 50 mL centrifuge tube and centrifuged at room temperature for 10 minutes at 3,000 rpm, and the supernatant was collected. The solution was then dialyzed for 72 hours with deionized water using a dialysis tube (MWCO: 6-8 kDa, Spectra/Pro®) and freeze dried to obtain AL-ANA.

<$^1$H NMR spectrum measurement and FT-IR spectrum measurement>

The AL-ANA was subjected to $^1$H NMR spectrum measurement and FT-IR spectrum measurement, with the results shown in Fig. 11 and Fig. 12. These results confirmed synthesis of ANA-modified alginic acid (AL-ANA) formed by binding between amino groups of ANA and carboxyl groups of alginic acid.

Based on the $^1$H NMR spectrum, the ANA modification rate of the carboxyl groups (-COOH) of the alginic acid was calculated to be 0.17.

<Aldehyde measurement by colorimetric aldehyde assay>

The ANA modification rate of the carboxyl groups (-COOH) of the alginic acid was 0.26 when calculated based on the aldehyde amount as measured with a blue (MAK140) colorimetric aldehyde assay kit.

The following table shows the modification rates by the modifying groups (A) as determined from both the $^1$H NMR spectrum (NMR) and a colorimetric aldehyde assay (Colorimetric).

[Table 2]

| Table 2 Modification rates of alginic acid derivatives | | | | |
|---|---|---|---|---|
| Example | Alginic acid derivative | Modifying group (A) | Modification rate by modifying group (A) | |
| | | | NMR | Colorimetric |
| I-1 | AL-ABA(1) | ABA | 0.069 | - |
| I-2 | AL-ABA(2)High DS | ABA | 0.78 | 0.64 |
| I-2 | A1-ABA(2) Low DS | ABA | 0.64 | 0.56 |
| I-2 | AL-ABA(3) | ABA | 0.21 | 0.33 |
| I-5 | AL-APCA | APCA | 0.41 | 0.33 |
| I-3 | AL-AAP | AAP | 0.31 | 0.34 |
| I-6 | AL-ANA | ANA | 0.17 | 0.26 |
| I-4 | AL-ADFBA | ADFBA | 0.12 | 0.18 |

3. AL-ABA drug conjugate

[Example 1-7] Benzaldehyde-modified alginic acid-vancomycin conjugate (AL-ABA-Van)

<Synthesis of AL-ABA-Van>

[C50]

Synthesis scheme 2

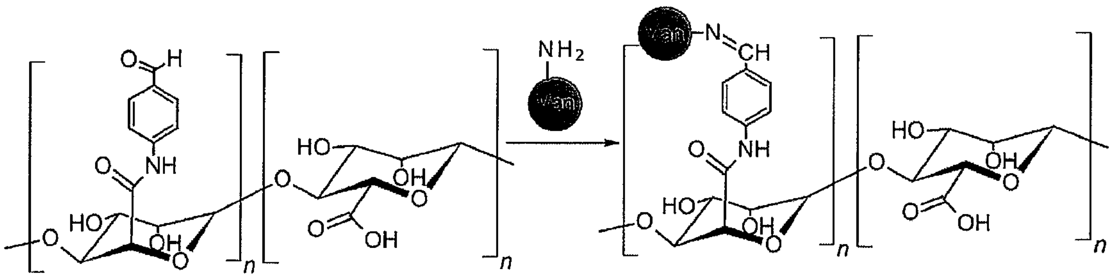

AL-ABA AL-ABA-Van

Vancomycin (Van)

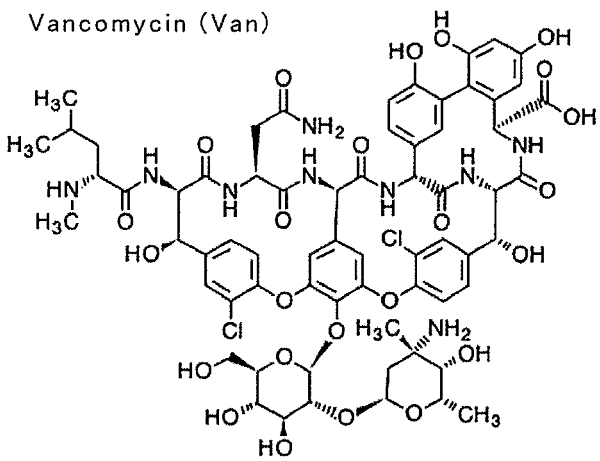

Vancomycin (Van) is an antibiotic having a primary amino group. A conjugate (AL-ABA-Van) of Van bound to AL-ABA was synthesized by the following steps according to the above synthesis scheme by performing a Schiff base reaction between the aldehyde groups of the AL-ABA and the primary amino groups of the vancomycin.

Specifically, 50 mg of the AL-ABA (1) obtained in Example 1-1 was dissolved in 70 mL of pure water, and stirred for at least 1 hour. 60 mg of vancomycin hydrochloride was dissolved in 60 mL of pure water, and added to the AL-ABA solution. 0.1 M NaOH was added dropwise to adjust the pH to near 7.0, and the mixture was reacted overnight under stirring at room temperature under shaded conditions. This was diluted with 150 mL of pure water, dialyzed for 2 days with pure water (equipment: Spectra/Pro® 1 Dialysis Membrane Standard RC Tubing, MWCO: 6-8 kD) and freeze-dried for 3 days, and 86.8 mg of white AL-ABA-Van was collected (yield: 78.9%).

<$^1$H NMR spectrum measurement, UV-visual light absorption spectrum (UV-vis) measurement and FT-IR spectrum measurement>

The resulting AL-ABA-Van was subjected to $^1$H NMR spectrum measurement, UV-visual light absorption spectrum (UV-vis) measurement and FT-IR spectrum measurement, with the results shown in Figs. 13 to 15.

A peak derived from ABA and a peak derived from vancomycin (Van) were observed in the $^1$H NMR spectrum of the AL-ABA-Van (Fig. 13). A peak derived from ABA and a peak derived from vancomycin (Van) (arrows in figure; a shift to lower wavelengths was observed) were also observed in the UV-vis spectrum of the AL-ABA-Van (Fig. 14). In the FT-IR spectrum (Fig. 15), the AL-ABA-Van exhibited a peak of absorption (at about 1740 cm$^{-1}$) derived from the C=O of the amide, which is the binding point between AL and ABA.

These results confirm formation of a conjugate between AL-ABA and Van. Based on the $^1$H NMR spectrum, the conjugate rate of the drug (Van) to the carboxyl groups (-COOH) of the alginic acid was 5.9%. This shows that 82.4% of the ABA in the AL-ABA reacted with the Van.

These results show that conjugation can be instantly completely simply by mixing AL-ABA and Van in an aqueous solution. Another advantage is that no further purification is necessary because the reaction produces only water. Since this reaction can be applied to a variety of drugs containing primary amines, AL-ABA is expected to provide a simple and general-purpose platform for developing alginic acid-drug conjugates.

<Release test of Van from benzaldehyde-modified alginic acid-vancomycin conjugate (AL-ABA-Van)>

(Test procedures)

The release behavior or Van from an AL-ABA-Van solution at different pH values was investigated using a dialysis membrane (pore size: 50 kDa). Specifically, 1 mL of an AL-ABA-Van solution of the AL-ABA-Van obtained in Example 1-7 above dissolved in 3 mg/mL of pure water was injected into a dialysis membrane cut to 9 cm, and both ends of the dialysis membrane were tied with kite thread. This was placed in an 80 mL security container, 79 mL of PBS solution was added, and the mixture was stirred at the maximum rotational speed with a 1 cm stirrer tip in a 37°C thermostatic tank. 1.0 mL samples were taken from the solution in the security container at specific time points (1, 2, 3, 4, 6, 8, 12, 24, and 48 hours). The sample solutions were subjected to UV-vis spectrum measurement, the drug concentration in the solutions was assayed based on absorbance, and the release rate of the drug Van (vancomycin release %) was calculated. The above measurements were conducted with pH 5.0, pH 6.0 and pH 7.4 PBS solutions (n = 4 at each pH).

For purposes of comparison as control tests, the same release testing was also performed with the AL-ABA-Van solution replaced by a mixed solution (AL+Van) of sodium alginate and vancomycin obtained by dissolving 3 mg/mL of sodium alginate (AL) (IL-6G, viscosity 50 to 80 mPa·s (1%), manufactured by Kimica Co., Ltd.) and 8.6 mg/mL of vancomycin (Van) in pure water, and by a solution (Van only) obtained by dissolving 8.6 mg/mL of vancomycin (Van) in pure water.

(Results)

The results are shown in Fig. 16. Fig. 16A shows the release behavior of Van from the AL-ABA-Van solution (Van cumulative release rate, %), Fig. 16B shows the release behavior of Van from an AL+Van mixed solution (Van cumulative release rate, %), and Fig. 16C shows the release behavior of Van from a Van solution (Van cumulative release rate, %).

While over 80% of the Van was released in the AL-ABA-Van solution at pH 5.0 after 10 hours, almost 50% had not been released at pH 7.4. These results show that Van is released more rapidly the lower the pH. This is thought to be because that the imine bonds between AL-ABA and Van are more easily dissociated at a low pH.

In the AL+Van solution and Van solution (control tests), on the other hand, almost all of the Van was released in the first 10 hours, and there was no difference in release behavior depending on pH.

These results show that an AL-ABA-Van conjugate can release Van continuously in contrast to the control groups

(AL+Van, Van only), and also that the drug is released selectively at low pH, allowing the Van release speed to be varied pH-dependently.

[C51]

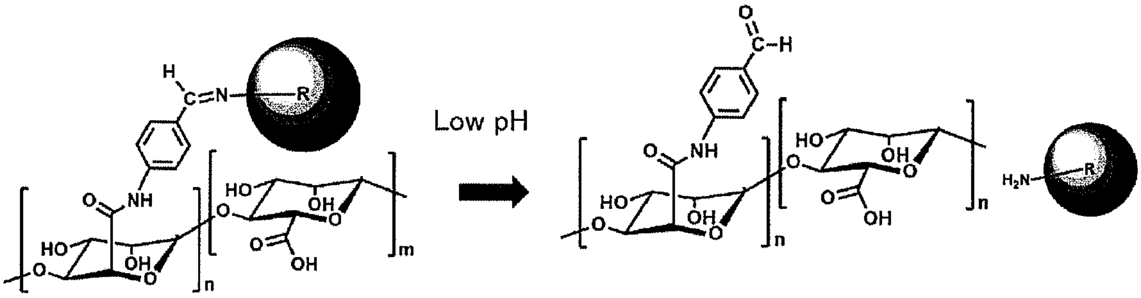


AL-ABA-Van AL-ABA Van

[Example 1-8] FTSC-loaded AL-ABA microcapsule (AL-ABA-FTSC capsule)

(Capsule preparation)

[C52]

Fluorescein-5-thiosemicarbazide (FTSC)

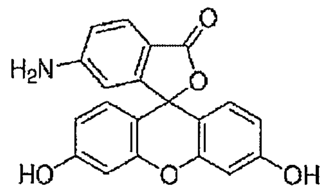

Fluorescein-5-thiosemicarbazide (FTSC) is a fluorescein dye having a primary amino group. An FTSC-loaded AL-ABA capsule was prepared.

Specifically, the AL-ABA (1) prepared in Example 1-1 was dissolved in pure water to prepare a 2 wt% AL-ABA solution. This was added dropwise to a 50 mM $CaCl_2$ aqueous solution to obtain a capsule (AL-ABA capsule). The FTSC was dissolved in DMEM (Dulbecco's modified Eagle medium) containing saline and 10% FBS (fetal bovine serum), and the prepared AL-ABA capsule was immersed in this to load the FTSC into the capsule by reactive dispersion.

For purposes of comparison as a control test, a capsule (AL capsule) was obtained as described above except that the AL-ABA was replaced with sodium alginate (AL) (IL-6G, viscosity 50 to 80 mPa·s (1%), manufactured by Kimica Co., Ltd.). The resulting AL capsule was immersed similarly in physiological saline containing FTSC to verify whether or not the FTSC could be loaded.

(Results)

After being immersed in the FTSC solution, the AL and AL-ABA capsules were observed by confocal microscopy. Fig. 17 shows transmitted images (Transmitted), fluorescent images (Amine-Fluorescein), and merged images (Merged).

It can be seen from Fig. 17 that micro-scale capsules were obtained using AL-ABA as well as AL. While almost no fluorescence from the FTSC was observed with the AL capsule, obvious fluorescence from the FTSC was observed with the AL-ABA capsule, showing that the capsule was loaded with FTSC in situ. This confirms that benzaldehyde-modified alginic acid (AL-ABA) retains the same $Ca^{2+}$ crosslinking ability as alginic acid (AL), allowing it to easily form capsules, and can also be loaded with an amine compound by simple immersion.

[Example 1-9] Van-loaded AL-ABA microcapsule (AL-ABA-Van capsule)

(Capsule preparation)

The AL-ABA (1) prepared in Example 1-1 was dissolved in pure water to prepare a 2 wt% AL-ABA solution. 15 mg of vancomycin (Van) was added to obtain a mixed solution of AL-ABA and Van. The mixed solution of AL-ABA and Van was added dropwise to a 50 mM $CaCl_2$ aqueous solution to obtain a capsule (AL-ABA-Van capsule).
For purposes of comparison as a control test, a capsule (AL-Van capsule) was obtained as described above except that the AL-ABA was replaced with sodium alginate (AL) (IL-6G, viscosity 50 to 80 mPa·s (1%), manufactured by Kimica Co., Ltd.).

(Results)

It was confirmed that even using a mixed solution of AL-ABA and Van, the benzaldehyde-modified alginic acid (AL-ABA) retained $Ca^{2+}$ crosslinking ability, and a capsule could be prepared as easily as using alginic acid (AL).

<Release test of Van from AL-ABA-Van capsule>

(Test procedures)

The AL-ABA-Van capsule prepared above was collected and added to a solution of 10 mM $CaCl_2$ in 1 mL of saline. External solution (sustained-release liquid) was collected at specific time points (3, 24 and 48 hours), and the entire amount of the external solution was replaced with a solution of 10 mM $CaCl_2$ in 1 mL of physiological saline.
The external solution (sustained-release liquid) collected at each time point was subjected to UV-vis spectrum measurement, the drug concentration in the solution was assayed based on absorbance, and the release rate of the drug Van (vancomycin release %) was calculated).
For purposes of comparison as a control test, a release test was performed using an AL-Van capsule instead of the AL-ABA-Van capsule.

(Results)

The results are shown in Fig. 18. Fig. 18A shows the cumulative release rate (%) of Van from each capsule, and Fig. 18B shows the change in the release rate (%) of Van from each capsule at each point in time.
Fig. 18A and Fig. 18B confirm that both the AL-Van capsule and AL-ABA-Van capsule gradually release vancomycin, with 60% of the Van being released after 48 hours. There was no great difference between the two in the cumulative released amount (Fig. 18A), but this may be because the effect of loading by the Schiff bases was obscured by the fact that the speed of release of Van from the microcapsules by subsequent dispersion was slower than the speed of release of Van by dissociation of the Schiff bases with the ABA.

<Bacterial growth inhibition test>

To examine the antibacterial effects of a vancomycin-loaded AL-ABA microcapsule, sustained release solution collected at each time point from the above test of Van release from the AL-ABA-Van capsule was used to evaluate the growth inhibition effects of each sustained release solution by a halo test using Staphylococcus aureus. The specific test procedures are as follows.

(Test procedures)

(1) Inoculation of Staphylococcus aureus on agar medium

1.1 15 g of agar was added to 1 liter of Mueller Hinton medium (BD Co.) and dissolved and sterilized with an autoclave, after which 15 mL was added per 100 mm dish and cooled to room temperature to prepare agar medium.
1.2 700 μL of Mueller Hinton medium was added to a 1.5 mL microtube (Asnol sterilization tube), inoculated with a toothpick with Staphylococcus aureus stock, and cultured overnight in a 37°C incubator.
1.3 The bacterial liquid was diluted to $1.0 \times 10^7$ cells/mL, and 100 μL was added to the agar medium and spread

evenly with a spreading stick.

(2) Impregnation of filter paper with sustained-release liquid and administration on agar medium

2.1 1 mL of each sustained-release liquid collected in the release test was filter sterilized with a syringe filter (0.22 μm).
2.2 Filter paper (As One: MFWG 4780) was cut into a circle 16 mm in diameter.
2.3 The cut-out filter paper was placed in the center of agar medium inoculated with Staphylococcus aureus.
2.4 40 μL of sustained-release liquid was evenly dripped onto the filter paper to impregnate the paper.
2.5 The dish was transferred to an incubator and cultured for 24 hours at 37°C.
2.6 The dish was removed from the incubator and the appearance of the proliferated bacteria was photographed with a camera and image analyzed with Image J (provided by NIH) to measure the area of the part where proliferation was inhibited.

(Results)

Fig. 19 shows the results of observation of Staphylococcus aureus growth on agar medium under each condition. A circular region without bacterial proliferation was observed on the edge of the filter paper with both the Van-loaded AL capsule (AL-Van) and the Van-loaded AL-ABA capsule (AL-ABA-Van), confirming a growth inhibition effect.

The area where growth was inhibited was also calculated by image analysis, and compared under each condition with the results shown in Fig. 20. While the growth inhibition area decreased over time with the AL capsule (AL-Van) from 3 hours to 24 hours to 48 hours after the start of sustained release, this decrease was suppressed with the AL-ABA capsule (AL-ABA-Van), and the difference in area tended to be less after 3 hours and 48 hours. These results suggest that with the AL-Van capsule most of the vancomycin was released during the initial period, but the released amount decreased, and the antibacterial effect was reduced over time, while with the AL-ABA-Van initial release of the vancomycin was controlled, so that an effective concentration could be maintained for a longer period of time.

[Example I-10] Benzaldehyde-modified alginic acid-bacitracin conjugate (AL-ABA-Bac)

<Synthesis of AL-ABA-Bac>

[C53]

Bacitracin (Bac)

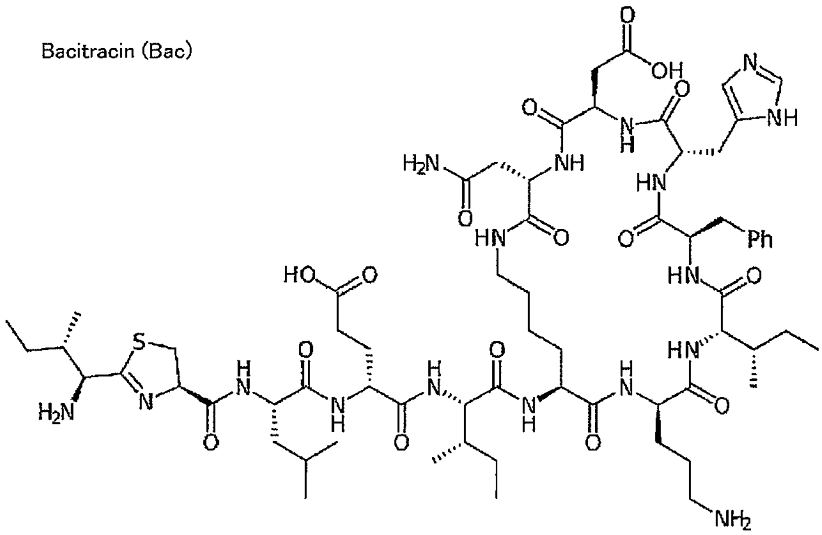

Bacitracin (Bac) is a polypeptide antibiotic of intermediate molecular weight having two primary amino groups. A benzaldehyde-modified alginic acid-bacitracin conjugate (AL-ABA-Bac) was synthesized by the same methods used in the Example 1-7.

Specifically, 50 mg of the AL-ABA (1) obtained in Example 1-1 was dissolved in 70 mL of pure water, and stirred for

at least 1 hour. 15 mg of bacitracin (Bac) was dissolved in 15 mL of pure water, and added to the AL-ABA solution. 0.1 M NaOH was dripped in to adjust the pH to near 7.0, and the mixture was reacted overnight under stirring at room temperature under shaded conditions. This was diluted with 150 mL of pure water, dialyzed for 2 days with pure water (equipment: Spectra/Pro® 1 Dialysis Membrane Standard RC Tubing, MWCO: 6-8 kD) and freeze-dried for 3 days, and 47.6 mg of AL-ABA-Bac in gel form was collected (yield: 73.2%).

<UV-visual light absorption spectrum (UV-vis) measurement and FT-IR spectrum measurement>

The resulting AL-ABA-Bac was subjected to UV-visual light absorption spectrum (UV-vis) measurement and FT-IR spectrum measurement, with the results shown in Fig. 21 and Fig. 22.

In the UV-vis spectrum (Fig. 21), the AL-ABA-Bac exhibited peaks derived from bacitracin (Bac) and ABA (arrows in figure; a shift to lower wavelengths was observed). In the FT-IR spectrum (Fig. 16), the AL-ABA-Bac exhibited a peak of absorption (at about 1740 $cm^{-1}$) derived from the C=O of the amide, which is the binding point between AL and ABA.

These results confirm formation of a conjugate between AL-ABA and Bac.

The collected AL-ABA-Bac had formed a hydrogel. It is thought that because the two amino groups in the Bac formed bonds with the aldehyde groups of the ABA, the Bac itself functioned as a crosslinking agent (gelling agent) to form a gel. Long-term sustained release can be expected due to this gel structure.

Apart from bacitracin (Bac), the polypeptide antibiotics include many other drugs (such as daptomycin, colistin and elcatonin) having two or more amino groups, and it is expected that with these drugs also the drug itself will be able to function as a crosslinking agent to form a gel structure.

[Example 1-11] Benzaldehyde-modified alginic acid-dopamine conjugate (AL-ABA-DOPA)

<Synthesis of AL-ABA-DOPA>

[C54]

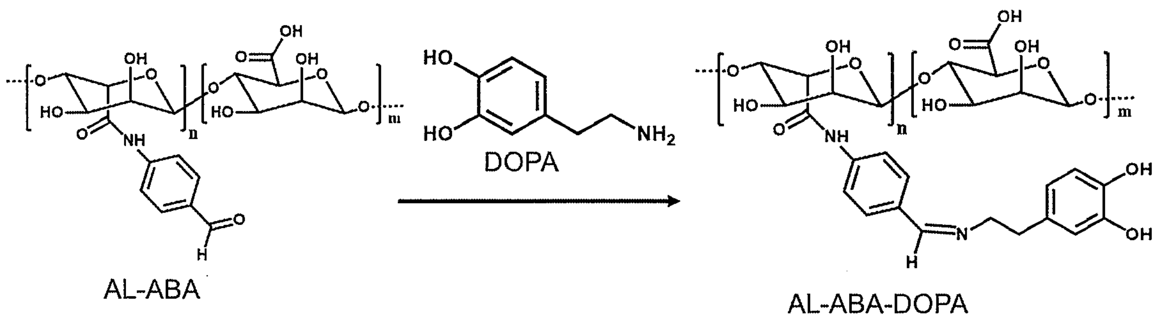


Dopamine (DOPA) is a neurotransmitter that plays an important role in the brain and body, and is an organic chemical in the catecholamine and phenethylamine families. DOPA has a primary amine group. A benzaldehyde-modified alginic acid-dopamine conjugate (AL-ABA-DOPA) was synthesized according to the following procedures.

Dopamine (DOPA) is a neurotransmitter that plays an important role in the brain and body, and has a primary amino group. A benzaldehyde-modified alginic acid-dopamine conjugate (AL-ABA-DOPA) was synthesized according to the following procedures.

0.25 g (0.00115 moles) of the AL-ABA (2) prepared in Example 1-2 (Low DS; modification rate (colorimetric): 0.56) was dissolved in 100 mL of PBS (pH 7.4), and stirred for 6 to 8 hours. 0.43 g (0.0023 moles) of dopamine hydrochloride (Sigma Aldrich) was dissolved in 10 mL of PBS (pH 7.4), and added dropwise to the AL-ABA solution. The solution was dialyzed for 72 hours with deionized water using a dialysis tube (Spectra/Pro®, MWCO: 6-8 kDa) to obtain an AL-ABA-DOPA solution, which was then freeze dried.

<$^1$H NMR spectrum measurement and FT-IR spectrum measurement>

The AL-ABA-DOPA was subjected to $^1$H NMR spectrum measurement and FT-IR spectrum measurement to confirm that a conjugate of AL-ABA and dopamine had formed. The results are shown in Fig. 23 and Fig. 24.

Based on the $^1$H NMR spectrum, the conjugate rate of the DOPA drug to the ABA in the AL-ABA was 47% (ABA modification rate by DOPA: 0.47). Because the modification rate (colorimetric) calculated by colorimetric aldehyde assay

is more accurate than the modification rate (NMR) calculated from the $^1$H NMR spectrum as the ABA modification rate in the AL-ABA, the DOPA conjugate rate was calculated based on the modification rate (colorimetric) from the colorimetric aldehyde assay. In the examples below, when the modification rate (colorimetric) has been measured, the conjugate rate of the drug with ABA is calculated based on the modification rate (colorimetric) value.

[Example 1-12] Sponge loaded with AL-ABA-DOPA (AL-ABA-DOPA sponge)

(Preparation procedures)

AL-ABA (modification rate (NMR): 0.60) was synthesized under the same conditions as the AL-ABA (2) of Example 1-2. The AL-ABA was dissolved in PBS (pH 7.4) to prepare a 0.5% AL-ABA solution. Dopamine (DOPA) was added in the amount of 2 molar equivalents of the ABA, and the solution was stirred for 16 to 20 minutes at room temperature. The solution was then dialyzed for 72 hours with pure water, and then freeze dried to obtain AL-ABA-DOPA.

The AL-ABA-DOPA was dissolved in PBS to obtain a 1.0 wt% AL-ABA-DOPA solution. The AL-ABA-DOPA solution was mixed with 1.0 mL of a 10 mM $CaCl_2$ aqueous solution in a 35 mm petri dish. After hydrogel formation, this was frozen overnight at -20°C and then freeze dried. Freeze drying yielded an AL-ABA-DOPA sponge.

A hydrogel and sponge of the polysaccharide derivative-drug conjugate AL-ABA-DOPA could be formed by crosslinking with a calcium ion.

[Example 1-13] Benzaldehyde-modified alginic acid-serotonin conjugate (AL-ABA-Serotonin)

<Synthesis of AL-ABA-Serotonin>

[C55]

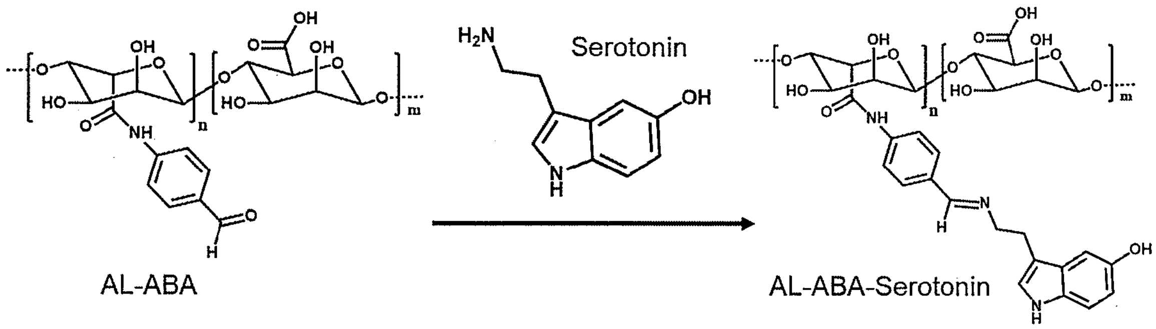

Serotonin (5-hydroxytryptamine) is a monoamine neurotransmitter having a primary amino group. A benzaldehyde-modified alginic acid-serotonin conjugate (AL-ABA-Serotonin) was synthesized by the following procedures.

0.25 g (0.00115 moles) of the AL-ABA (2) prepared in Example 1-2 (Low DS; modification rate (colorimetric): 0.56) prepared in Example 1-2 was dissolved in 100 mL of PBS (pH 7.4), and stirred for 6 to 8 hours. 0.489 g (0.0023 moles) of serotonin hydrochloride (Sigma Aldrich) was dissolved in 10 mL of PBS (pH 7.4), and added dropwise to the AL-ABA solution. The solution was dialyzed for 72 hours with deionized water using a dialysis tube (Spectra/Pro®, MWCO: 6-8 kDa), and then freeze dried.

<$^1$H NMR spectrum measurement and FT-IR spectrum measurement>

The AL-ABA-Serotonin was subjected to $^1$H NMR spectrum measurement and FT-IR spectrum measurement to confirm that a conjugate of AL-ABA and serotonin had formed. The results are shown in Fig. 25 and Fig. 26.

Based on the $^1$H NMR spectrum, the conjugate rate of the serotonin drug to the ABA in the AL-ABA was 46% (ABA modification rate by serotonin: 0.46).

[Example 1-14] Benzaldehyde-modified alginic acid-celecoxib conjugate (AL-ABA-Celecoxib)

<Synthesis of AL-ABA-Celecoxib>

[C56]

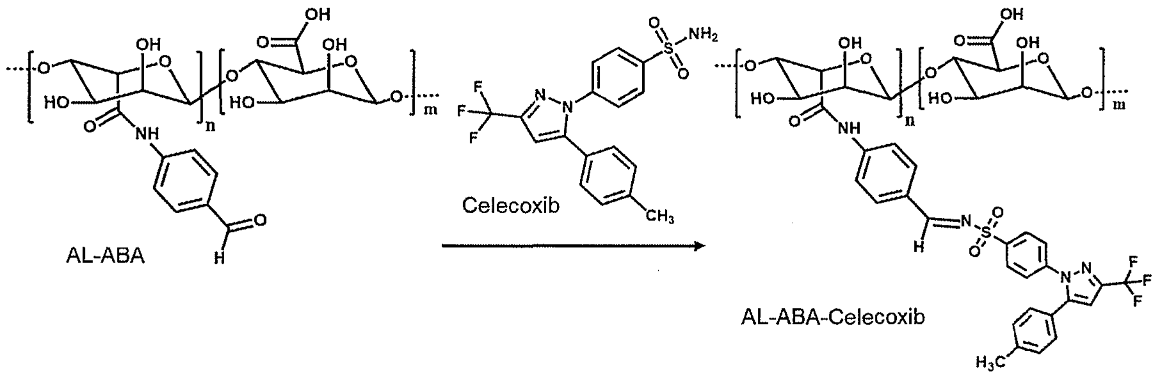


Celecoxib is a non-steroidal anti-inflammatory/analgesic having a primary amino group. A benzaldehyde-modified alginic acid-celecoxib conjugate (AL-ABA-Celecoxib) was synthesized by the following procedures.

0.25 g (0.00115 moles) of the AL-ABA (2) prepared in Example 1-2 (Low DS; modification rate (colorimetric): 0.56) was dissolved in 100 mL of PBS (pH 7.4), and stirred for 6 to 8 hours. 0.877 g (0.0023 mol) of celecoxib hydrochloride (TCI) was dissolved in 10 mL of DMSO, and added dropwise to the AL-ABA solution. The solution was dialyzed for 72 hours with deionized water using a dialysis tube (Spectra/Pro®, MWCO: 6-8 kDa), and then freeze dried.

<$^1$H NMR spectrum measurement and FT-IR spectrum measurement>

The AL-ABA-Celecoxib was subjected to $^1$H NMR spectrum measurement and FT-IR spectrum measurement (not shown) to confirm that a conjugate of AL-ABA and celecoxib had formed. The $^1$H NMR spectrum measurement results are shown in Fig. 27.

Based on the $^1$H NMR spectrum, the conjugate rate of the celecoxib drug to the ABA in the AL-ABA was 11% (ABA modification rate by celecoxib: 0.11).

[Example I-15] Benzaldehyde-modified alginic acid-HGF aptamer conjugate (AL-ABA-Apt)

With the aim of developing a novel anti-adhesion hydrogel using AL-ABA having a sustained release function with an HGF aptamer having a mesothelial cell growth promoting effect (manufactured by Eurofins Genomics Co., Ltd., Lot: 1649399-3), a benzaldehyde-modified alginic acid-HGF aptamer conjugate (AL-ABA-Apt) was synthesized and sustained release of the HGF aptamer from the AL-ABA-Apt was tested. The test procedures are shown in Fig. 28.

(Test)

<Synthesis of AL-ABA-Apt>

AL-ABA (modification rate (NMR) 4.0%) was synthesized under the same conditions as the AL-ABA (1) of Example 1-1. The AL-ABA was dissolved in phosphate-buffered saline (PBS, pH = 7.4) to obtain a 0.4 wt% AL-ABA solution. HGF aptamer containing amino groups in the amount of 1.0 equivalents of the aldehyde groups of the AL-ABA was added to the AL-ABA solution, and stirred for 1 hour. This was then dialyzed for 3 days with pure water to remove unreacted HGF aptamer, and freeze dried.

The AL-ABA-HGF aptamer conjugate (AL-ABA-Apt) and the HGF aptamer were each subjected to UV-visual light absorption spectrum (UV-vis) measurement, and loading of the HGF aptamer (Apt) by the AL-ABA was evaluated based on the peak value at 256 nm in the UV-vis spectrum.

<Release test of Apt from AL-ABA-Apt>

1.0 wt% of AL-ABA was dissolved in PBS (pH = 7.4). HGF aptamer containing amino groups in the amount of 1.0 equivalents of the aldehyde groups in the AL-ABA was then added to the AL-ABA aqueous solution. This was then stirred for 1 hour to synthesize an AL-ABA-HGF aptamer conjugate (AL-ABA-Apt). The AL-ABA-HGF aptamer was then placed in a dialysis membrane (MWCO = 50 kDa), and stirred in PBS (pH = 7.4). 1 mL samples of the external solution were taken at specific time points after the start of stirring, and the cumulative release rate (%) of the HGF aptamer was determined by UV-vis measurement. As a control test, a mixture of ordinary alginic acid and HGF aptamer (ALG-Apt) and the HGF aptamer by itself (Apt) were each dissolved in PBS, placed in a dialysis membrane and subjected to the same sustained release testing, and the sustained release speeds were compared.

<Preparation of AL-ABA-HGF aptamer/$Ca^{2+}$ gel>

The AL-ABA-HGF aptamer (AL-ABA-APt) prepared above was dissolved in pure water to a concentration of 1.0 wt% to obtain an AL-ABA-HGF aptamer aqueous solution. The AL-ABA-HGF aptamer aqueous solution and a 100 mM $CaCl_2$ aqueous solution were mixed 300 μL each in a microtube, and the presence or absence of gelling was examined.

(Results)

<Synthesis of AL-ABA-HGF aptamer>

A peak derived from the HGF aptamer was confirmed near 256 nm in the UV-vis spectrum (not shown) of the synthesized AL-ABA-HGF aptamer, indicating that synthesis of the AL-ABA-HGF aptamer (AL-ABA-Apt) was successful.

<Sustained release of HGF aptamer from AL-ABA-HGF aptamer>

Fig. 29 shows the results of sustained release testing of the HGF aptamer (Apt) from the AL-ABA-HGF aptamer (AL-ABA-Apt), the mixture of alginic acid and HGF aptamer (ALG-Apt), and the HGF aptamer by itself (Apt). In the AL-ABA-Apt group, release of the HGF aptamer (Apt) was more gradual than in the ALG-Apt and Apt groups. This shows that sustained release of the HGF aptamer can be achieved under physiological pH conditions by forming a conjugate of the HGF aptamer with AL-ABA.

<Preparation of AL-ABA-HGF aptamer/$Ca^{2+}$ gel>

The AL-ABA-HGF aptamer was gelled immediately by $Ca^{2+}$, and gel preparation was successful. Instantaneous gelling of AL-ABA by $Ca^{2+}$ crosslinking has been confirmed as shown in Example 1-16 and Example 1-17 below. This suggest that conjugation of the HGF aptamer with AL-ABA has almost no effect on the gelling performance of AL-ABA.

Synthesis of an AL-ABA-HGF aptamer loaded with the HGF aptamer having a mesothelial cell growth promotion effect was successful, and sustained release of the HGF aptamer from the AL-ABA-HGF aptamer was shown to be possible. Preparation of a gel of the AL-ABA-HGF aptamer (AL-ABA-Apt) by $Ca^{2+}$ crosslinking was also successful, suggesting that this could be used as an anti-adhesion material.

4. Sponge and hydrogel of AL-ABA

[Example 1-16] Sponge of calcium-crosslinked benzaldehyde-modified alginic acid (AL-ABA sponge)

(Preparation procedures)

A total of 20 mg of sodium alginate (AL) (IL-6G, viscosity 50 to 80 mPa·s (1%), manufactured by Kimica Co., Ltd.) and the AL-ABA (1) obtained in Example 1-1 were dissolved in 2 mL of pure water at weight ratios of 75:25, 50:50 and 25:75 to prepare AL-ABA aqueous solutions. Each of these was mixed in a dish with 2 mL of 10 mM $CaCl_2$ aqueous solution to obtain an AL-ABA hydrogel. This was then frozen overnight in a -20°C refrigerator and freeze dried for 3 days in a freeze drier to obtain an AL-ABA sponge.

For purposes of comparison as a control test, a sponge (AL sponge) was obtained in the same way except that the AL-ABA was replaced with AL alone (AL 100 wt%).

(Results)

Optical photographs of the AL-ABA sponges obtained in the above example and the Control AL sponge are shown in Fig. 30, and SEM photographs in Fig. 31.

It was confirmed that benzaldehyde-modified alginic acid sponges (AL-ABA sponges) could be prepared by the same simple methods used for AL. Fig. 31 shows that the AL-ABA sponges had porous structures similar to that of the AL sponge. It is believed that the unmodified carboxyl groups of the AL and AL-ABA were crosslinked via calcium (Ca) ions, forming crosslinked structures. The content and crosslinking density of the benzaldehyde (ABA), which is the modifying group contained in the resulting sponges, can be controlled by mixing AL with AL-ABA.

[Example I-17] Calcium-crosslinked benzaldehyde-modified alginic acid hydrogel (Ca crosslinked AL-ABA hydrogel)

The AL-ABA (2) prepared in Example 1-2 (High DS, modification rate (colorimetric): 0.64) was crosslinked with calcium to prepare a hydrogel (calcium-crosslinked AL-ABA hydrogel). Specifically, 20 mg of the AL-ABA (2) prepared in Example 1-2 (High DS, modification rate (colorimetric): 0.64) was dissolved in 1 mL of pure water to prepare an AL-ABA aqueous solution. This was mixed with 1 mL of 100 mM $CaCl_2$ aqueous solution in a dish to obtain a calcium-crosslinked AL-ABA hydrogel.

<In vitro swelling and degradation test>

The Ca-crosslinked AL-ABA hydrogel obtained above was swelled in pH 7.4 phosphate-buffered saline (PBS). Specifically, 4 new hydrogel samples were prepared, and each sample was weighed, added to 25 mL of PBS solution at 37°C, and swelled for 72 hours. Degradation of each sample after 72 hours of swelling was observed. The weights of the hydrogel samples were measured every 24 hours for 2 weeks. The PBS was exchanged every week for new PBS that had been equilibrated at 37°C. A completely swollen Ca-crosslinked AL-ABA hydrogel was freeze dried, and its morphology was observed with an SEM unit.

(Results)

Fig. 32 shows the swelling and degradation profile (hydrogel weight changes) of the Ca-crosslinked AL-ABA hydrogel. The weight change value is an average of 4 samples.

Fig. 33 shows an SEM photograph of a cross-section cut with a knife from a sponge obtained by freeze drying a Ca-crosslinked AL-ABA hydrogel (500x; 24 hours after start of swelling), confirming the porous structure of the dried hydrogel.

5. Hydrogel containing AL-ABA and amino group-containing polymer

[Example 1-18] Hydrogel of AL-ABA and DPI (AL-ABA-DPI)

[C57]

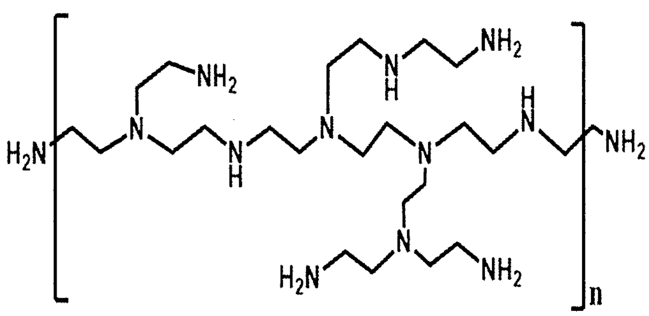

**DPI**

The AL-ABA (2) prepared in Example 1-2 (Low DS, modification rate (colorimetric): 0.56) was dissolved in saline

as a solvent to prepare a 2% w/v AL-ABA solution. Dendritic polyethyleneimine (DPI) (BASF Co., product name PS, weight-average molecular weight M = 750,000) was dissolved in water to prepare a 10% w/v DPI solution. The two solutions were mixed so that the aldehyde groups of the AL-ABA solution and the primary amino groups ($-NH_2$) of the DPI solution were in equal molar amounts (1:1). Specifically, 250 μL of the 10% w/v DPI solution (amino groups: $2.59 \times 10^{-4}$ mol) was mixed mechanically with a pipette into 5 mL of the 2% w/v AL-ABA solution (aldehyde groups: $2.59 \times 10^{-4}$ mol). A hydrogel formed within 20 to 30 seconds (Fig. 35). The hydrogel was stable for 2 weeks in water.

[Example 1-19] Hydrogel of AL-ABA and PEG(4k)-dihydrazide (AL-ABA-PEGDH)

<Synthesis of PEGDH>

[C58]

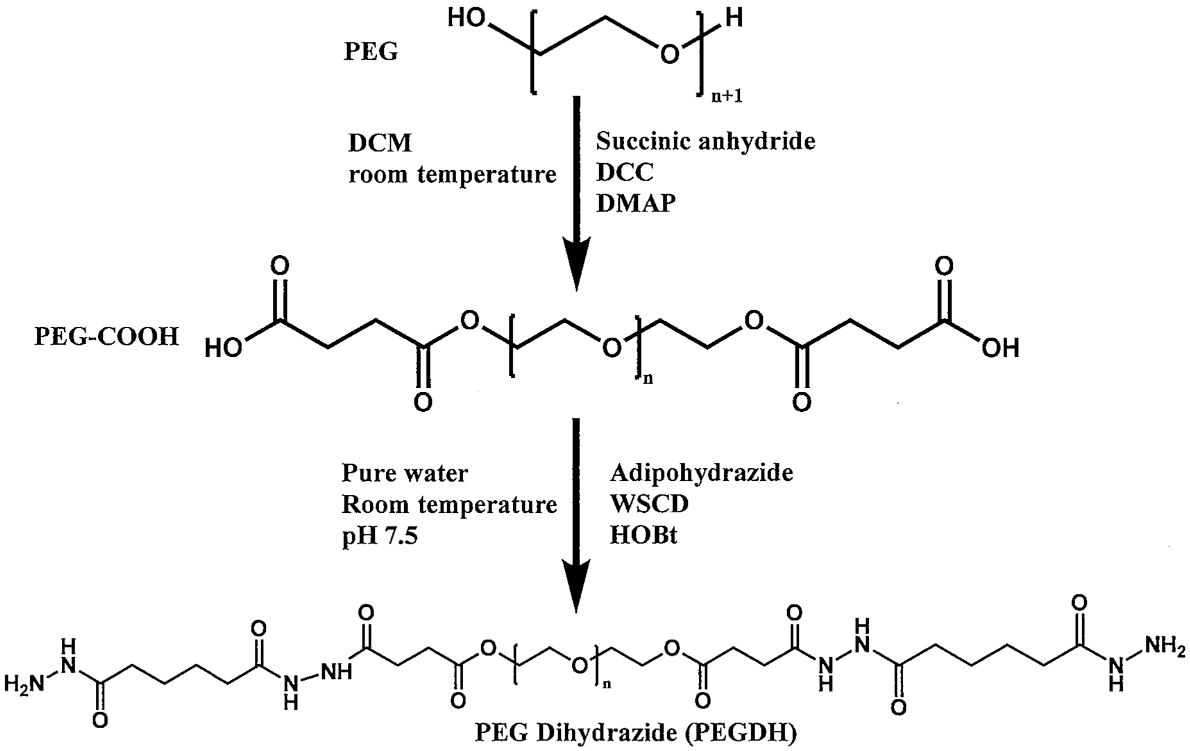


The PEGDH was prepared by a two-stage reaction.

First, 10 g (0.0025 mol) of PEG (Mw 4,000, 162-09115, Wako) was dissolved at room temperature in a 300 mL round-bottom flask in 100 mL of dichloromethane (DCM) solvent. 2.0 g (0.02 mol) of succinic anhydride was added to the PEG solution, followed by 2.57 g (0.0125 mol) of N,N'-dicyclohexyl carbodiimide and 1.83 g (0.015 mol) of 4-dimethylaminopyridine, and the flask was closed with a rubber stopper and stirred for 24 hours at room temperature. The reaction mixture was then filtered with filter paper, and the filtrate was evaporated at 50°C with a rotary evaporator. Once the DCM had completely evaporated, the viscous reaction mixture was left in the flask, 100 mL of distilled water was added, this was stirred for a further 1 hour, and the solution was dialyzed for 48 hours with pure water using a dialysis tube (MWCO: 1 kDa, Spectra/Pro®). After dialysis, the solution was freeze dried and characterized by $^1H$ NMR ($D_2O$ solvent). The yield was about 73%, and the conversion rate of PEG to PEG-COOH was about 100%.

The second stage is a carbodiimide reaction. 4 g (0.00097 mol) of newly prepared PEG-COOH was taken in a 200 mL triangular flask, and dissolved for 2 to 4 hours in 50 mL of distilled water. 1.05 g (0.0070 mol) of HOBt was dissolved in 10 mL of DMSO (Wako), after which 1.49 g (0.0.0078 mol) of WSCD HCl (Peptide Institute, Inc.) was dissolved in 10 mL of distilled water and added dropwise to the PEG-COOH solution. The solution was stirred for a further 10 minutes. 3.399 g (0.019 mol) of adipohydrazide was dissolved in 15 mL of pure water, and added to the PEG-COOH solution. The pH of the final reaction mixture was maintained around 7.5 pH with 1 N NaOH as the mixture was stirred for 16 to 20 hours at room temperature. The solution was then dialyzed extensively for 48 hours with deionized water using a dialysis tube (MWCO 1 kDa, Spectra/Pro®), and then freeze dried. Binding of PEGDH was confirmed by $^1H$ NMR. The degree of substitution was

about 91%.

<Synthesis of AL-ABA-PEGDH>

The AL-ABA (2) prepared in Example 1-2 (High DS, modification rate (colorimetric): 0.64) was dissolved in physiological saline as a solvent to prepare a 1.5% w/v AL-ABA (2) solution.

The PEGDH prepared above was dissolved in water to prepare a 10% w/v PEGDH solution. The two solutions were mixed so that the aldehyde groups of the AL-ABA (2) solution and the hydrazide groups of the PEGDH solution were in equal molar amounts (1:1). Specifically, 0.5 mL of 10% w/v PEGDH solution (hydrazide groups: $2.27 \times 10^{-5}$ mol) was mixed mechanically with a pipette into 1 mL of 1.5% w/v AL-ABA solution (aldehyde groups: $6.9 \times 10^{-5}$ mol). A hydrogel formed within 30 seconds (Fig. 36).

<Dynamic viscoelasticity measurement>

The storage modulus G' and loss modulus G" were measured with a rheometer.

The results are shown in Fig. 37. Within the measurement frequency range, the G' of the AL-ABA-PEGDH was confirmed to be greater than the G", confirming actual formation of a hydrogel.

[Example I-20] Benzaldehyde-modified alginic acid films (non-crosslinked and crosslinked AL-ABA films)

<Film preparation>

(1) Non-crosslinked AL-ABA film

A non-crosslinked AL-ABA film was prepared by solution casting and a vacuum heat drying process. Specifically, the AL-ABA (2) prepared in Example 1-2 (High DS, modification rate (colorimetric): 0.64) was dissolved in distilled water to prepare a 1.5% w/v AL-ABA solution. After this had completely dissolved, bubbles were removed for 15 minutes by ultrasound treatment. The solution was then poured into a 60 mm petri dish, and the petri dish was kept for 40 to 48 hours in a vacuum drier at 50°C, after which the film was detached and stored at 4°C. The film was a transparent, non-porous film with a thickness of about 63 μm.

(2) Crosslinked AL-ABA film

The non-crosslinked AL-ABA film obtained in (1) above was crosslinked with PEGDH to obtain a crosslinked AL-ABA film. Specifically, the PEGDH synthesized in Example 1-19 above was dissolved in an aqueous butanol solution (butanol:water = 9:1), and the film obtained in (1) above was immersed in this solution and maintained for 6 to 8 hours at room temperature. The film was removed from the PEGDH solution, dried in atmosphere, and stored at 4°C. The resulting crosslinked AL-ABA film was a transparent non-porous film.

<Swelling and dissolution test of films>

(Test procedures)

The non-crosslinked AL-ABA film obtained in (1) above, the crosslinked AL-ABA film obtained in (2) above and an AL film were each immersed for 2 to 4 weeks in PBS (pH = 7.4), and swelling and dissolution of the films were observed. The AL film was prepared by the methods of (1) above except that AL-500 (sodium alginate, viscosity 400 to 600 mPa·s, manufactured by Mochida Pharmaceutical Co., Ltd.) was used in place of the AL-ABA (2) prepared in Example 1-2.

(Results)

The results are shown in the following Table.

[Table 3]

| Table 3 Film swelling and dissolution test results | | |
|---|---|---|
| | Swelling after 6 days | Dissolution |
| AL Film | - | Dissolved |

(continued)

| Table 3 Film swelling and dissolution test results | | |
|---|---|---|
| | Swelling after 6 days | Dissolution |
| Non-crosslinked AL-ABA film | Large swelling | Dissolved after 1 week |
| Crosslinked AL-ABA film | Swelling somewhat less than with non-crosslinked AL-ABA film | No dissolution after 20 days |

[Example 1-21] Hydrogels of AL-ABA with polyallylamines (AL-ABA-PAA1, AL-ABA-PAA2, AL-ABA-PAA3, AL-ABA-PAA4)

[C59]

(i)

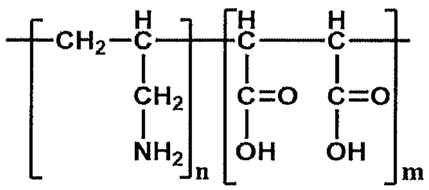

PAA1

(ii)

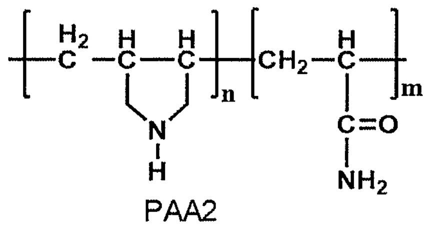

PAA2

(iii)

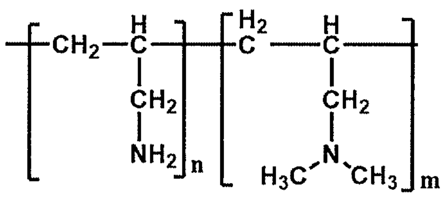

PAA3

(iv)

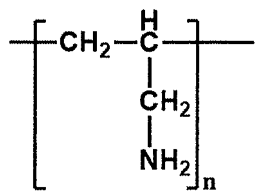

PAA4

(i) PAA1: Product name allylamine maleic acid copolymer (Product No. PAA-1151, Nittobo Medical Co., Ltd., 20 wt% viscosity 20 cp at 20°C)
(ii) PAA2: Diallyl dimethyl ammonium chloride acrylamide copolymer (Product No. PAS-J-81, Nittobo Medical Co., Ltd., 25 wt% viscosity 900 cp at 20°C), weight-average molecular weight Mw: 180,000
(iii) PAA3: Allylamine hydrochloride dimethylallylamine salt copolymer (Product No. PAA-1112CL, Nittobo Medical Co., Ltd., 15 wt% viscosity 5 cp at 20°C)
(iv) PAA4: Allylamine hydrochloride polymer (Product No. PAA-HC1-10L, Nittobo Medical Co., Ltd., 40 wt% viscosity 1500 cp at 20°C, weight-average molecular weight Mw: 150,000

The polyallylamines (PAA1 to PAA4) of (i) to (iv) above were each dissolved in water to prepare 10% w/v PAA solutions (PAA1 solution, PAA2 solution, PAA3 solution, PAA4 solution).

The AL-ABA (2) prepared in Example 1-2 (Low DS; modification rate (colorimetric): 0.56) was dissolved in physiological saline as a solvent to prepare a 2% w/v AL-ABA solution. The two solutions were mixed so that the aldehyde groups of the AL-ABA solution and the primary amino groups ($-NH_2$) of the PAA solution were in equal amounts (1:1). Specifically, each 10% w/v PAA solution (PAA1 solution, PAA2 solution, PAA3 solution, PAA4 solution) (amino groups: $2.59 \times 10^{-4}$ mol in each) was mixed mechanically with a pipette into 5 mL of the 2% w/v AL-ABA solution (aldehyde groups: $2.59 \times 10^{-4}$ mol). A hydrogel formed within 20 to 30 seconds in all cases (Fig. 38).

6. Tissue adhesive materials using AL-ABA

[Example I-22] Benzaldehyde-modified alginic acid (AL-ABA) as tissue adhesive material (adhesion to submucosa)

<Evaluation of adhesion behavior to mucosa>

The adhesiveness of AL-ABA on submucosa was studied. Because submucosa is rich in collagen, it contains many amino groups. The following test confirmed that crosslinked structures are formed by Schiff bases between the amino groups of the submucosa and the aldehyde groups of the AL-ABA, potentially improving adhesiveness. The specific test procedures are as follows.

(Test procedures)

1. Pig esophagus was cut open lengthwise, and then cut into 2 cm × 2 cm sections. The mucosal layer lining the esophagus was cut away to expose the submucosa. 80 mL security containers were filled with 25 mL each of physiological saline with 0.1% of added sodium benzoate. The esophagus sections with the exposed submucosa were immersed one by one in these, placed in the shaker of a 37°C incubator, and shaken overnight. After being shaken the esophagus sections were weighed (n = 4).
2. AL-ABA and unmodified AL (IL-6G) for purposes of comparison were each dissolved to 2 w/v% in pure water. The AL-ABA (1) prepared in Example 1-1 was used as the AL-ABA.
3. The alginic acid solution (AL-ABA aqueous solution or AL aqueous solution) prepared in 2 above was placed in one side of a double syringe, and 50 mM $CaCl_2$ aqueous solution in the other side.
4. The sections prepared in 1 above were removed from the security containers and placed on petri dishes. 0.5 mL of the alginic acid solution prepared in 3 and 0.5 mL of 50 mM $CaCl_2$ aqueous solution were sprayed simultaneously from above the sections using the double syringe with nitrogen gas. The nitrogen gas flow rate was 2 L/min. This was left standing for 10 minutes at room temperature (25°C) to gel. This treatment caused the benzaldehyde-modified alginic acid (AL-ABA) or alginic acid (AL) to be crosslinked by the calcium ($Ca^{2+}$) and form a hydrogel (AL-ABA gel, AL gel).
5. The gel on the petri dish was rinsed with pure water, moisture on the esophagus section was wiped off, and the esophagus section was weighed. The weight of the gel on the esophagus section was calculated by subtracting the weight of the esophagus section itself (measured in 1 above) from this weight. This was then given as the gel weight after 0 hours.
6. The physiological saline with the 0.1% added sodium benzoate in the security container was replaced with fresh physiological saline. The gel surface of the esophagus section was then immersed in this upside-down. This was placed on a shaker and shaken in a 37°C incubator.
7. The esophagus section was weighed after specified times (after 1, 2, 3, 4, 6, 8, 12, 24 and 48 hours), and the remaining gel weight was calculated by subtracting the weight of the esophagus section itself (measured in 1 above) from this weight. The percentage of residual gel at each time point was determined from the gel weight after 0 hours and the remaining gel weight [(remaining gel weight)/(gel weight after 0 hours) × 100]. The external appearance of the gel was also observed at each time point.

These steps were applied to 4 samples each of AL and AL-ABA, and the average value of the percentage of residual gel at each time point was given as the adhesion rate (%) of the gel.

(Results)

The results are shown in Fig. 39 and Fig. 40. Fig. 39 shows the external appearance of the AL gel and AL-ABA gel at each time point. Fig. 40 shows the adhesion rate (%) of the AL gel and AL-ABA gel at each time point.

As shown in Fig. 39, the gel had peeled off in 3 out of 4 samples in the AL spray group (ALG (IL-6G), AL gel) after 1 hour. In the remaining sample, the gel had peeled off after 2 hours. In the AL-ABA spray group (ALG-ABA, AL-ABA gel), the gel began to peel off gradually after 2 hours, and some residual gel was observed even after 12 hours. Fig. 40 also shows that while the gel adhesion rate (%) reached zero after 3 hours in the AL spray group (ALG (IL-6G), AL gel), in the AL-ABA spray group (ALG-ABA, AL-ABA gel) the gel adhesion rate was still about 40%. These results suggest that adhesiveness can be improved by modifying AL with ABA.

[Example 1-23] AL-ABA as tissue adhesive material (evaluating adhesiveness on esophageal mucosa and submucosa and effects of $Ca^{2+}$ concentration) (37°C)

<Adhesion test on mucosa and submucosa>

The adhesiveness of AL-ABA and AL on esophageal mucosa and submucosa was investigated in a 37°C environment. The samples with mucosa were called Controls, while the samples in which the mucosa was peeled off to expose the submucosa were called ESD samples. Two $Ca^{2+}$ concentrations of 50 M and 100 mM were also tested to investigate changes in adhesiveness at different $Ca^{2+}$ concentrations. The test procedures is shown in Fig. 42. The specific procedures are as follows.

(Materials)

AL-ABA: AL-ABA (modification rate (NMR): 0.052) prepared under the same conditions as the AL-ABA (1) of Example I-1, dissolved to 2 w/v% in pure water
AL: IL-6G (IL-6G, viscosity 50 to 80 mPa·s (1%), manufactured by Kimica Co., Ltd.), dissolved to 2 w/v% in pure water
$CaCl_2$ aqueous solution (for crosslinking): 50 mM or 100 mM
Esophageal sections (Control: with mucosa; ESD: mucosa peeled off to expose submucosa)

(Test procedures)

1. Pig esophagus was cut open lengthwise, and then cut into 2 cm × 2 cm sections. In the submucosa group (ESD), the mucosal layer was removed with scissors.
2. The mass of the cut pig esophageal sections (Control) and the sections with exposed submucosa (ESD) was measured.
3. AL or AL-ABA was dissolved in 2 w/v% of pure water.
4. 2.5 mL syringes were filled with the solution prepared in 3 and a $CaCl_2$ solution, and set in a double syringe (0.5 mL alginic acid solution + 0.5 mL $CaCl_2$ solution per sample).
5. The double syringe was fitted with a spray tip, and each esophageal section was sprayed using nitrogen gas at a rate of 4 L/minute (n = 4 per sample).
6. This was left for 10 minutes to gel. This treatment caused the benzaldehyde-modified alginic acid (AL-ABA) or alginic acid (AL) to be crosslinked by the calcium ($Ca^{2+}$) and form hydrogels (AL-ABA gel, AL gel). The mass of the esophageal section at this point was measured.
7. 25 mL of physiological saline with 0.1% methyl benzoate and 1.25 mM $CaCl_2$ added thereto was placed in a glass petri dish 6 cm in diameter, and each esophageal section was immersed in this and shaken in a shaker. The 1.25 mM $Ca^{2+}$ mimics the $Ca^{2+}$ found in saliva.
8. The esophagus sections were weighed after specified times (after 1, 2, 3, 4, 6, 8, 12, 24, 48 and 72 hours), and the mass of the remaining gel was calculated. The percentage of residual gel at each time point was determined from the gel weight after 0 hours and the remaining gel weight [(remaining gel weight)/(gel weight after 0 hours) × 100]. The external appearance of the gel was also observed at each time point.

These steps were applied to 4 samples each of AL and AL-ABA with $Ca^{2+}$ concentrations of 50 mM and 100 mM using the esophageal sections (Control) and sections with exposed mucosa (ESD), and the average value of the percentage of residual gel at each time point was given as the adhesion rate (%) of the gel.

(Results)

Fig. 43 shows the external appearance of the gels at each time point (after 2, 4, 12, 24, 48 and 72 hours). Fig. 44 shows the percentage of remaining gel (gel adhesion rate, %) at each time point.

The samples with AL-ABA sprayed on submucosa (AL-ABA ESD) and the samples with AL-ABA sprayed on mucosa (AL-ABA Control; AL-ABA Con) maintained gel adhesion for a longer time in comparison with the AL spray groups (AL ESD and AL Control (Con)). This confirms that adhesiveness can be improved by modifying AL with ABA.

In particular, the samples with AL-ABA sprayed on submucosa (AL-ABA ESD) exhibited a greater improvement in adhesive strength than the samples with AL-ABA sprayed on mucosa (AL-ABA Control; AL-ABA Con). It is presumed

that strong adhesion was achieved by binding of the ABA to the amino groups of the submucosa.

Moreover, gel adhesion was also maintained for a longer time at the higher $CaCl_2$ concentration (100 mM). It is believed that the crosslinking density was improved by increasing the amount of Ca ions, thereby increasing the mechanical strength of the gel and prolonging the degradation time. Because cohesive failure occurred at the maximum breaking strength in the tensile test, it appears that adhesive strength increased due to the increased mechanical strength of the gel.

<Adhesiveness evaluation by tensile testing>

Gel peeling and gel dissolution both occurred simultaneously in the above adhesive test. A tensile test was performed to investigate only the adhesiveness of AL-ABA. Fig. 45 shows the procedures in the tensile test.

(Materials)

AL-ABA: AL-ABA (modification rate (NMR): 0.052) prepared by the same methods as in Example 1-1, dissolved to 2 w/v% in pure water
AL: IL-6G (IL-6G , viscosity 50 to 80 mPa s (1%), manufactured by Kimica Co., Ltd.), dissolved to 2 w/v% in pure water
$CaCl_2$ aqueous solution (for crosslinking): 50 mM or 100 mM
Esophageal sections (Control: with mucosa; ESD: mucosa peeled off to expose submucosa)

(Equipment)

Physical property measurement rheometer (Shiro Sangyo, M993R-3000S)

(Test Procedures)

1. Pig esophagus was cut open lengthwise, and then cut into 1 cm × 3 cm sections. In the mucosa group (Control), the cut sections were used as is. In the submucosa (ESD) group, the mucosal layer of each section was peeled off (n = 3).
2. Alginic acid solution (AL-ABA or AL) was added to a 1 cm × 1 cm area of each section.
3. $CaCl_2$ solution was added from above to gel the alginic acid solution.
4. A weight (30 mL of water in 80 mL container) was lowered from above to remove air.
5. This was set for 1 hour in a 37°C thermostatic tank.
6. Breaking strength (N) was measured with the rheometer.
7. The breaking pressure (Pressure, Pa) was calculated from the breaking strength (N) of 6 and the area ($m^2$) of the gel (region where alginic acid solution was added).

(Results)

The results are shown in Fig. 46. In comparison with the AL hydrogel, the AL-ABA hydrogel to have significantly greater adhesive strength on the mucosa and submucosa. Changes in adhesive strength due to the concentration of the crosslinking agent ($Ca^{2+}$ ion) were also confirmed.

[Example 1-24] AL-ABA as tissue adhesive material (evaluating adhesiveness on submucosa and skin tissue by lap shear method)

The adhesive strengths of AL-ABA, AL and a conventional tissue adhesive on living tissue were evaluated by a lap shear test. Specifically, the adhesive strength of each material on living tissue was evaluated by the following procedures using the pregel solution below with pig submucosa and pig skin tissue. Fig. 47 shows the test procedures for the lap shear method.

(Pregel solution)

- AL-ABA: 4 w/v% aqueous solution of AL-ABA (2) prepared in Example 1-2 (High DS; modification rate (colorimetric): 0.64)
- AL500: 4 w/v% AL-500 (sodium alginate, viscosity 400 to 600 mPa·s, manufactured by Mochida Pharmaceutical Co., Ltd.)
- Fibrin Glue: fibrin glue adhesive (Veriplast P, manufactured by CSL Behring Co., Ltd.)
- Hydrofit® (urethane hemostatic; Terumo Japan)
- Dermabond® (cyanoacrylate skin surface adhesive; Johnson and Johnson)

The pregel solutions were gelled as follows.
For the AL-ABA and AL500, the pregel solution (AL-ABA or AL500) was first applied and the biological tissues overlapped, after which 200 μL of a 50 mM or 100 mM $CaCl_2$ aqueous solution was applied to both sides of the biological tissue (200 μL × 2).
The fibrin glue was gelled with a double syringe.
The Hydrofit® is a one-component sealant that was applied as is.
The Dermabond® is a one-component sealant that was applied as is.

(Biological tissue)

Submucosa: Pig esophagus was cut to a length of 40 mm and a width of 10 mm, and the mucosal layer lining the esophagus was cut away to expose the submucosa.
Skin tissue: Pig skin tissue was cut to a length of 40 mm and a width of 10 mm.

(Equipment)

CR3000-EX Dynamic Chemical Analyzer (DMA), manufactured by Sun Scientific Co., Ltd.

(Test procedures for lap shear test)

0.5 mL of pregel solution was coated on the overlapped areas (length 10 mm by width 10 mm) at the ends of two pieces of biological tissue (length 40 mm by width 10 mm), and gelled after overlapping. 20 minutes after gelling, a lap shear test was performed at a rate of 5 mm/minute with a Dynamic Chemical Analyzer (DMA). The adhesive energy was measured by a 180° peel test, and adhesive strength was calculated by the following formula.

$$\text{Adhesive strength} = \text{strength } (N/m^2)/\text{area of overlapping region } (m^2)$$

(Results)

The Results are shown in Fig. 48.
The AL-ABA hydrogel was shown to be useful as a tissue adhesive on biological tissue such as submucosa and skin tissue.

[Example 1-25] AL-ABA as tissue adhesive material (tissue sealant) (evaluating effectiveness as sealant on submucosal tissue and skin tissue by burst test)

To investigate effectiveness as a tissue sealant, a burst test was performed using the device reported in Lei Zhou et al., ADVANCED FUNCTIONAL MATERIALS Vol. 31, Issue 14, 2021, 2007457, "Injectable Self-Healing Natural Biopolymer-Based Hydrogel Adhesive with Thermoresponsive Reversible Adhesion for Minimally Invasive Surgery" with slight modifications. Specifically, a burst test was performed by the following burst test procedures using the following pregel solutions with pig submucosa and pig skin tissue as the biological tissues. The equipment used in the burst test and the burst test procedures are shown in Fig. 49.

(Pregel solution)

- AL-ABA: 4 w/v% aqueous solution of AL-ABA (2) prepared in Example 1-2 (High DS; modification rate (colorimetric): 0.64)

- AL500: 4 w/v% AL-500 (sodium alginate, viscosity 400 to 600 mPa·s, manufactured by Mochida Pharmaceutical Co., Ltd.)
- Fibrin Glue: fibrin glue adhesive (Veriplast P, manufactured by CSL Behring Co., Ltd.)
- Hydrofit® (urethane hemostatic; Terumo Japan)
- Dermabond® (cyanoacrylate skin surface adhesive; Johnson and Johnson)

The pregel solutions were gelled as follows.
For the AL-ABA and AL500, the pregel solution (AL-ABA or AL500) was injected into a puncture site, 500 μL of 100 mM $CaCl_2$ aqueous solution was dripped from above, and crosslinking was performed for 5 to 10 minutes.
The fibrin glue was gelled with a double syringe.
The Hydrofit® is a one-component sealant that was applied as is.
The Dermabond® is a one-component sealant that was applied as is.

(Biological tissue)

Submucosa: Pig esophagus was cut to a length of 50 mm and a width of 50 mm, and the mucosal layer lining the esophagus was cut away to expose the submucosa.
Skin tissue: Pig skin tissue was cut to a length of 50 mm and a width of 50 mm.

(Burst test procedures)

Biological tissue was set on the upper surface of a burst pressure apparatus, and punctured to prepare a hole 2 mm in diameter in the middle of the tissue. 2.5 to 3 mL of pregel solution was injected onto the prepared 2 mm hole (puncture site), and gelled.
The measurement apparatus was connected to a syringe pump filled with PBS solution. Finally, the PBS solution was injected into the apparatus, and the maximum burst pressure (mmHg) was recorded with a digital pressure gauge. The test was repeated 5 times.

(Results)

The results are shown in the following table and Fig. 50.

[Table 4]

| Table 4 Burst test results | | | | | |
|---|---|---|---|---|---|
| Burst pressure (mmHg) | | | | | |
| Biological tissue | AL-ABA | AL500 | Fibrin Glue | Hydrofit | Dermabond |
| Submucosa | 79.5 (±23.59) | 10.49 (±0.97) | 61.9 (±19.59) | 127.4 (±9.67) | Not burst |
| Skin | 53.4 (±13.59) | 14.18 (±2.13) | 64.3 (±20.5) | 134.9 (±34.31) | Not burst |

As shown in Fig. 50, the AL-ABA hydrogen did not perform significantly differently from the fibrin glue, which indicates that it can be used as a tissue sealant.

7. Tissue adhesive material using AL-ABA, AL-AFA, AL-AAP, AL-APA, AL-ANA

[Example 1-26] Modified alginic acid as tissue adhesive material for dura mater tissue (evaluation of adhesion to dura mater tissue according to lap shear method)

In order to evaluate the adhesive properties and adhesive ability of alginic acid derivatives (AL-ABA, AL-AFA, AL-AAP, AL-APA, and AL-ANA), a lap shear test according to the ASTM D1002 test procedure was performed. The adhesive ability of the alginic acid derivative was compared with a plurality of commercially available adhesive materials (Fibrin Glue (Beriplast (registered trademark) P), Duraseal (registered trademark), Dermabond (registered trademark), Hydrofit (registered trademark)) and unmodified sodium alginate (AL-500).

<Sample>

Alginic acid derivatives (AL-ABA, AL-AFA, AL-AAP, AL-APA, and AL-ANA) were prepared in the same method as AL-ABA(2) in Example 1-2, and Example 1-3 to Example 1-6. Here, the following modification rate indicates a modification rate for the carboxyl group (-COOH) of alginic acid calculated from the amount of aldehyde measured using a colorimetric aldehyde assay kit, blue (MAK140).

· AL-ABA alginic acid modified with benzaldehyde (ABA) (a modification rate: 0.56 (56%))
·AL-AFA(AL-ADFBA): alginic acid modified with 4'-4-amino-2,6-difluoro-benzaldehyde (ADFBA) (modification rate: 0.12 (12%))
·AL-AAP: alginic acid modified with 4'-aminoacetophenone (AAP) (modification rate: 0.25 (25%))
·AL-APA(AL-APCA): alginic acid modified with 2-amino-3-pyridinecarboxaldehyde (APA, APCA) (modification rate: 0.31 (31%))
·AL-ANA: alginic acid modified with 6-aminonicotinaldehyde (ANA) (modification rate: 0.17 (17%))
·AL-500: sodium alginate (manufactured by Mochida Pharmaceutical Co., Ltd., viscosity: 400 to 600 mPa·s)
·Fibrin Glue: fibrin glue adhesive (Beriplast (registered trademark) P, manufactured by CSL Behring Co., Ltd.)
·Duraseal (registered trademark) (dura mater sealant material: manufactured by Integra Japan)
·Dermabond (registered trademark) (cyanoacrylate skin surface adhesive; Johnson & Johnson)
·Hydrofit (registered trademark) (urethane-based hemostatic agent; manufactured by Terumo Corporation)

<Biological tissue>

Porcine dura mater was purchased from Tokyo Shibaura Zouki Co. Ltd.
The test was performed according to the procedure shown in Fig. 74(A) as follows. (Test)
First, each alginic acid derivatives and unmodified sodium alginate (AL-500) were dissolved in PBS (pH 7.4) for 48 hours to prepare a 3% w/v solution (pregel solution). Then, the porcine dura mater was thawed and washed in distilled water and cut into a plurality of rectangular pieces with a similar size of 3 cm in length and 1 cm in width to prepare dura mater tissue. About 0.1 mL of a pregel solution (alginic acid derivative or alginate 3%, w/w) was applied to the edge of the dura mater tissue in a 1×1 $cm^2$ area, two pieces of dura mater tissue overlapped to cover the applied area, and the tissue sample was immediately immersed in a 100 mM $CaCl_2$ solution for 2 minutes, and then incubated at 37°C for 1 hour to gel the pregel solution (RCom MARU Delux Max Digital Incubator). Here, during incubation, the tissue sample was kept with several moist tissue papers in order to prevent moisture loss.
The resulting sample was subjected to a lap shear adhesion test using a tensile testing device (CR-3000EX-S; Sun Scientific Co., Ltd.). The sample was fixed between both crumbs of the tensile testing machine, and the test was performed at a speed of 5 mm/min until the two overlapping tissue samples separated from each other. The test was performed three times for each sample. The adhesion strength was calculated from the maximum force when the sample was separated using the following formula.

$$\text{Adhesion strength} = \text{maximum force } (N/m^2)/\text{overlapping region area } (m^2)$$

Here, for commercially available adhesive materials, the test was performed according to the same procedure as above except that the following method was used for gelling in place of immersion in a $CaCl_2$ solution.
Fibrin Glue was gelled using a double syringe.
Duraseal (registered trademark) was gelled using an attached applicator.
Dermabond (registered trademark) was a one-component product and was applied without change.
Hydrofit (registered trademark) was a one-component product and was applied without change.

(Results)

The results are shown in the following table and Fig. 74(B).

[Table 5]

| Table 5 Results of lap shear test using dura mater tissue | | |
|---|---|---|
| Sample | Maximum force (kPa) | Standard error |
| AL-500 | 3.5 | 1.0 |
| AL-ABA | 68.6 | 6.4 |

(continued)

| Table 5 Results of lap shear test using dura mater tissue | | |
|---|---|---|
| Sample | Maximum force (kPa) | Standard error |
| AL-AFA | 75.5 | 11.0 |
| AL-APA | 88.5 | 9.3 |
| AL-AAP | 54.8 | 5.3 |
| AL-ANA | 69.8 | 12.4 |
| Fibrin Glue (Beriplast (registered trademark) P) | 46.8 | 5.1 |
| Duraseal (registered trademark) | 28.7 | 5.8 |
| Hydrofit (registered trademark) | 176.1 | 14.2 |
| Dermabond (registered trademark) | 189.9 | 3.6 |

The measured adhesion data of various alginic acid derivatives according to the lap shear test using dura mater as a substrate exhibited significantly higher adhesion strength to the dura mater. Among the alginic acid derivatives, AL-APA exhibited the maximum adhesion strength (88.5±9.3 kPa), and AL-AAP exhibited the minimum adhesion strength (54.8±5.3 kPa). All alginic acid derivatives exhibited significantly higher adhesion strength than unmodified alginate (AL-500). In addition, all alginic acid derivatives exhibited significantly higher adhesion strength than Duraseal (registered trademark) (28.7±5.8 kPa) as a commercially available dura mater sealant material. In addition, all alginic acid derivatives exhibited significantly higher adhesion strength than Fibrin Glue (46.8±5.1 kPa). On the other hand, all alginic acid derivatives exhibited significantly lower adhesion strength than Hydrofit (registered trademark) (176.1±14.2 kPa) and Dermabond (registered trademark) (189.9±3.6 kPa).

The reason why the alginate exhibited higher adhesion was a Schiff base reaction between the amino group of the dura mater and the aldehyde of the alginic acid derivative. In addition, it was thought that the hydrophobic interaction and the hydrogen bond between the alginic acid derivative and dura mater proteins also contributed to improving the adhesion strength.

[Example 1-27] Alginic acid derivative as tissue adhesive material (tissue sealant) for dura mater tissue (evaluation of sealing effect and adhesive ability on dura mater tissue according to burst test)

In order to compare the sealing effect and adhesive ability of the alginic acid derivatives (AL-ABA, AL-AFA, AL-AAP, AL-APA, and AL-ANA) with a plurality of commercially available adhesive materials (Fibrin Glue (Beriplast (registered trademark) P, may be referred to as "FG"), Duraseal (registered trademark), Dermabond (registered trademark), Hydrofit (registered trademark)), Bio Glue (registered trademark) and unmodified sodium alginate (AL-500), the burst test was performed using a custom-designed device.

As alginic acid derivatives, commercially available adhesive materials other than Bio Glue (registered trademark), and unmodified sodium alginate, those the same as in Example 1-26 were used.

·Bio Glue (registered trademark): glutaraldehyde adhesive (manufactured by Cyiolife)

Porcine dura mater was purchased from Tokyo Shibaura Zouki Co. Ltd.

A 3% w/v solution (pregel solution) of the alginic acid derivative was prepared by dissolving the alginic acid derivative in PBS (pH 7.4) for 48 hours.

The test was performed as follows using the device shown in Fig. 49(A). The test procedure is shown in Fig. 75(A).

(Test 1: test without nonwoven fabric)

Fresh porcine dura mater was gently washed with distilled water, cut into a circle with a diameter of about 50 mm, and a hole was made in the center with a 2 mm biopsy punch (Kai Japan). Then, 2 to 3 mL of a 3% w/v solution (pregel solution) of the alginic acid derivative prepared in advance was applied onto the central hole of the dura mater sample, the sample was transferred into a 100 mM $CaCl_2$ (Wako CAS RN (registered trademark): 10043-52-4, molecular formula: $CaCl_2$, molecular weight: 110.98) solution, and for 5 to 10 minutes, the alginic acid derivative was appropriately crosslinked and the $CaCl_2$ solution was diffused to the deeper surface to form a hydrogel (gelling). Then, the sample was incubated and then kept in the incubator for 5 to 10 minutes, and for the burst test, the sample was fixed firmly between support rings, and the measurement device was connected to a syringe pump filled with a PBS solution. Finally, the PBS (pH 7.4) solution was injected into the device at a flow rate of 10 mL/min. The maximum burst pressure was recorded using a digital pressure

gauge. The pump injected the PBS solution into the burst pressure device and performed PBS injection until the PBS started to leak from the applied hydrogel. After the pump was stopped, the pressure change value was recorded. The test was repeated 4 to 5 times for each sample.

(Test 2: test using nonwoven fabric)

In order to improve burst pressure performance of the alginic acid derivative, a nonwoven fabric Gunze Medical Japan Ltd (GMJ) Neovil (registered trademark) or Ethicon Vicryl (registered trademark) Mesh was used as a support membrane, and a sample containing a nonwoven fabric as a support membrane and an alginic acid derivative (nonwoven fabric-containing sample) was prepared.

Gunze Medical Japan Ltd (GMJ) Neovil (registered trademark): material: glycolic acid ester, thickness: 0.15 to 0.5 mm
Ethicon Vicryl (registered trademark) Mesh: material: glycolic acid/lactic acid polyester (90/10), thickness: 0.1 to 0.5 mm

The nonwoven fabric cut to a size of 3×3 $cm^2$ was used. The test using the nonwoven fabric-containing sample was performed by the following procedure. Specifically, the test was performed by attaching the nonwoven fabric trimmed to a predetermined size onto the tissue defect (the central hole on the dura mater sample), additionally applying the tissue adhesive from above, and then putting it into a $CaCl_2$ solution as in the test 1, and gelling it.
For unmodified sodium alginate (AL-500), the test was performed according to the same procedure (test 1 and test 2) as above except that the unmodified sodium alginate was used in place of the alginic acid derivative.
Here, for commercially available adhesive materials, the test was performed according to the same procedure (test 1 and test 2) as above except that the following method was used for gelling in place of immersion in a $CaCl_2$ solution.
Fibrin Glue was gelled using a double syringe.
Duraseal (registered trademark) was gelled using an attached applicator.
Dermabond (registered trademark) was a one-component product and was applied without change.
Hydrofit (registered trademark) was a one-component product and was applied without change.
Bio Glue (registered trademark) was gelled using an attached applicator.

(Results)

The results are shown in the following table and Figs. 75(B) and 75(C). The burst pressure shown in the table and drawings is the average value of the measured burst pressures.

[Table 6]

| Table 6: Results of burst test using dura mater tissue | | | |
|---|---|---|---|
| Sample | Burst pressure (mmHg) | Stand ard error | Note |
| AL-500 | 13.5 | 3.8 | |
| AL-500 + Neovil | 17.0 | 5.0 | |
| AL-ABA | 32.3 | 12.3 | |
| AL-ABA+ Neovil | 37.5 | 8 | |
| AL-AFA | 35.7 | 2.1 | |
| AL-AFA + Neovil | 43 | 6.5 | |
| AL-APA | 33.6 | 7 | |
| AL-APA+ Neovil | 41 | 17.6 | |
| AL-ANA | 20.9 | 3.0 | |
| AL-ANA+ Neovil | 22.9 | 4.5 | |
| AL-AAP | 20.3 | 10.3 | |
| AL-AAP+ Neovil | 23.1 | 12.4 | |
| Fibrin glue | 43.5 | 9.6 | |

(continued)

| Table 6: Results of burst test using dura mater tissue | | | |
|---|---|---|---|
| Sample | Burst pressure (mmHg) | Stand ard error | Note |
| Fibrin glue + Neovil | 53.3 | 4.2 | |
| DuraSeal | 52.6 | 13.0 | |
| DuraSeal+ Neovil | 51.3 | 9.4 | |
| Dermabond | 250 | 0 | The upper limit was reached each time |
| Dermabond+Neovil | 250 | 0 | The upper limit was reached each time |
| HydroFit | 208.3 | 40.0 | |
| HydroFit+Neovil | 250 | 0 | The upper limit was reached each time |
| Bioglue | 250 | 0 | The upper limit was reached each time |
| Bioglue+Neovil | 250 | 0 | The upper limit was reached each time |
| AL-ABA-Double Vicryl mesh | 88.5 | 8.9 | |
| AL-ABA-Single Vicryl mesh | 77.2 | 6.2 | |
| Fibrin Glue-Double Vicryl mesh | 103.5 | 6.3 | |
| Fibrin Glue-Single Vicryl mesh | 97.4 | 11.5 | |

In the table, samples ending with "+Neovil" are samples in which Neovil (registered trademark) was used as the nonwoven fabric, and samples ending with "-Double Vicryl mesh" and "-Single Vicryl mesh" are samples in which two sheets or one sheet of "Ethicon Vicryl Mesh" was used as the nonwoven fabric, respectively.

All alginic acid derivatives exhibited a sealing effect to some extent. In all alginic acid derivatives, alginates were cross-linked with calcium ions to form hydrogels, and at the same time, aldehyde moieties of the derivatives reacted with free amino groups of the dura mater tissue according to a Schiff base reaction and fixed to the tissue. Overall, the hydrogels were firmly bound to the surface of the tissue according to a covalent bond (C=N), a hydrophobic interaction, and a hydrogen bond.

Among all alginic acid derivatives, AL-APA exhibited a maximum burst pressure of about 33 mmHg (without the support membrane), and AL-AAP exhibited a minimum burst pressure of about 20 mmHg (without the support membrane). Commercially available adhesive materials exhibited a higher burst pressure (that is, fibrin glue 43 mmHg, DuraSeal 52 mmHg, Dermabond>250 mmHg, and HydroFit 208 mmHg) than the alginic acid derivative without the support membrane.

The hydrogel had favorable adhesion to the dura mater tissue, but the value of the burst pressure was lower than that of commercial products. The reason for this was thought to be that, during a gelling procedure of the alginic acid derivative hydrogel, $Ca^{2+}$ ions crosslinked the upper surface first, entry of more $Ca^{2+}$ ions was restricted, and thus the inside of the hydrogel was not sufficiently crosslinked. In this case, when the pressure was applied, burst was likely to occur.

In order to increase the burst pressure, a nonwoven fabric was used as the support membrane. The burst pressure was increased by utilizing the support membrane. When the Neovil support membrane was used, the burst pressure of AL-ABA increased from 32 mmHg to 37 mmHg, and the burst pressure of AL-APA increased from 33 mmHg to 41 mmHg. When the Vicryl mesh was used, the burst pressure of AL-ABA increased from 32 mmHg to 77 mmHg. The Vicryl mesh had a sufficient density to provide the mechanical support, a bulk hydrogel was provided when the Vicryl mesh and the alginic acid derivative were combined, and it was possible to prevent burst at a relatively high pressure level. When two Vicryl meshes were used, the burst pressure could be further increased.

It was shown that the sealing performance could be improved when used in combination with the nonwoven fabric.

[Example 1-28] Viscoelastic analysis of alginic acid derivative as tissue adhesive material (flow curve and frequency sweep test)

<Test method>

(i) Flow curve

Either one of five types of alginic acid derivatives (AL-ABA, AL-AFA, AL-ANA, AL-APA, and AL-AAP), or unmodified alginate AL-500 was dissolved in a phosphate buffer with a pH of 7.4 to prepare a 3% w/v solution. The fluidity of the alginic acid derivative solution was evaluated using a rotational rheometer (Anton Paar MCR 302) with a standard steel plate (parallel geometry) with a diameter of 25 mm. The change in viscosity when the shear rate increased from $10^{-3}$ to $10^{3}$ $sec^{-1}$ was measured, and the shear rate ($sec^{-1}$) was plotted on the X-axis, and the viscosity (mPa·s) was plotted on the Y-axis in a logarithmic graph.

(ii) Frequency sweep test

Either one of five types of alginic acid derivatives (AL-ABA, AL-AFA, AL-ANA, AL-APA, and AL-AAP), or unmodified alginate AL-500 was dissolved in a phosphate buffer with a pH of 7.4 to prepare a 3% w/v solution. A hydrogel was prepared using a 100 mM $CaCl_2$ solution. Using a rotational rheometer (Anton Paar MCR 302) with a standard steel parallel flat shape with a diameter of 25 mm, the frequency sweep rheology test of the hydrogel was performed. The hydrogel was placed on the lower plate, and the thickness was adjusted to 1 mm using a probe in order to move the upper plate. The vibration frequency (ω) increased from 0.1 to 100 rad·$s^{-1}$ at a fixed strain (5%) and temperature (25°C), and G'(storage modulus) and G" (loss modulus) were measured.

As the alginic acid derivatives and unmodified sodium alginate, those the same as in Example 1-26 were used.

(Results)

Fig. 76 and Fig. 77 show the flow curves obtained in (i) and the results of frequency sweep test (ii).

As shown in Fig. 76, AL-ABA, AL-AFA, and AL-AAP exhibited a higher shear thinning property than other AL derivatives and unmodified AL-500. This phenomenon was speculated to be due to the hydrophobic interaction of conjugated (ABA/AFA/AAP) moieties.

As shown in Fig. 77, all hydrogels exhibited a storage modulus (G') (1,800 to 2,800 Pa) and a loss modulus (G") (600 to 1,200 Pa). The parameters G' and G" represent the storage energy and dissipation energy at unit strain, respectively. Both rigidity and dissipation are known to be important for the interaction between biomaterials and tissues, and these are important properties of biomaterials. According to the tissue adhesive material of this aspect, it was possible to adjust these properties by changing the composition (the structure of the polysaccharide derivative, the degree of crosslinking and the like) of the gel.

[Example I-29] Hemolysis test of alginic acid derivative as tissue adhesive material

<Test method>

Hemolysis test of hydrogel

Either one of five types of alginic acid derivatives (AL-ABA, AL-AFA, AL-ANA, AL-APA, and AL-AAP), or unmodified alginate AL-500 was dissolved in a phosphate buffer with a pH of 7.4 to prepare a 3% w/v solution. A hydrogel was prepared using a 100 mM $CaCl_2$ solution.

The blood compatibility test was performed as follows. About 3 mL of blood from the defibrinated sheep was collected. Then, 800 g of the sample was centrifuged for 15 minutes. RBC pellets were re-suspended in a (0.9% NaCl) physiological saline, and collected by centrifugation. This process was repeated to remove any serum. The RBC pellets were diluted in 10 mL of a 0.9% physiological saline to prepare an RBC stock solution. Then, a freshly prepared hydrogel (200 mg each) was washed with a phosphate buffer with a pH of 7.4. Then, the sample was incubated with an RBC stock solution (1 mL) together at 37°C for 3 hours, and after incubation, the sample was centrifuged at 800G for 15 minutes and the supernatant was collected. Hemoglobin released into the supernatant was analyzed at 540 nm using a UV-visible spectrophotometer (V-630BIO UV-Vis spectrometer, Jasco, Tokyo, Japan). The test was performed three times, and regarding hemolysis caused by 0.9% NaCl (physiological saline) (negative control) and distilled water (positive control; 1% w/v in PBS), the hemolysis rate was calculated using the following formula.

[Math. 2]

$$Hemolysis\ (\%) = \frac{Sample_{540\,nm} - Negative\ control\ _{540\,nm}}{Positive\ control_{540\,nm} - Negative\ control_{540\,nm}} \times 100$$

Here, as the alginic acid derivatives and unmodified sodium alginate, those the same as in Example 1-26 were used.

(Results)

The results are shown in Fig. 78. All alginic acid derivative hydrogels exhibited acceptable blood compatibility.

Blood compatibility was also an important criterion for using biomaterials in contact with blood. The amount of hemoglobin released from red blood cells was inversely proportional to the blood compatibility of the hydrogel material. The hemolysis value for blood compatibility was in a range of 10 to 25%.

8. Tissue adhesive material containing AL-ABA and GA-modified amino group-containing polymer

[Example I-30] Combination of AL-ABA and GA-modified amino group-containing polymer as tissue adhesive material for tissue (evaluation of adhesion to tissue according to lap shear method)

(1) Synthesis of dendritic poly(ethyleneimine) (DPIGA) conjugated with gallic acid

[C60]

Reaction scheme for DPIGA synthesis

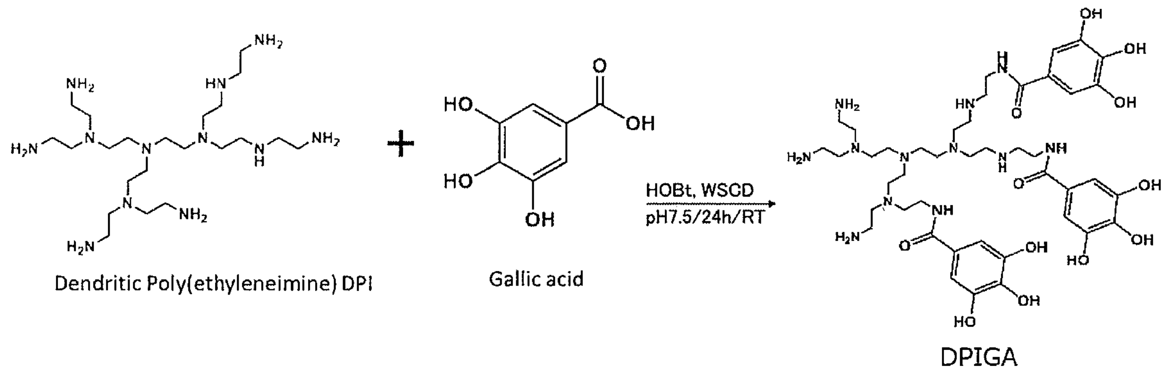


DPIGA was synthesized according to a simple carbodiimide reaction. In this reaction, the primary amine of DPI reacted with the carboxylic acid group of gallic acid. For this synthesis, first, 1 g (0.0058 mol) of gallic acid (GA) (manufactured by Merck, Formula: $(HO)_3C_6H_2CO_2H$ CAS Number: 149-91-7 molecular weight: 170.12) was dissolved in 40 mL of DMSO/water (a volume ratio of 1/3) to prepare a gallic acid solution. Then, 6 g (0.012 mol) of dendritic poly(ethyleneimine) (DPI) (Lupasol (registered trademark) PS, BASF, MW 750,000) was dissolved in 50 mL of distilled water, and the pH was adjusted to 7.4 with 1 N HCl (Wako) to prepare a DPI solution. Then, the gallic acid solution was added dropwise to the DPI solution and stirred for 10 minutes. Then, a sample obtained by dissolving 3.97 g (0.029 mol) of HOBt(1-hydroxybenzotriazole hydrate≥97.0%, Pcode-H0468, Mw135.13, TCI) in 15 mL of DMSO (Wako) was added dropwise, and the pH was again maintained at 7.4. Then, 5.61 g (0.029 mol) of WSCD (WSCD. HCl water-soluble carbodiimide hydrochloride P. Code 1030, Mw. 191, PEPTIDE INSTITUTE INC.) was dissolved in 20 mL of distilled water, and the sample was added to the reaction solution. The pH was maintained at about 7.4 until 4 hours of the reaction, and the reaction was caused at room temperature of 25°C for 24 hours. After the reaction was completed, using a dialysis tube (MWCO 6,000-8,000 kDa) (Spectra/Pro (registered trademark)), the sample was dialyzed against distilled water for 72 hours, and the resulting clear solution was freeze-dried to obtain an amorphous off-white substance at a yield of about 64%. The substance was stored in a dark place at 4°C.

<$^1$H NMR spectrum measurement, UV-visible light absorption spectrum (UV-vis) measurement, and FT-IR spectrum measurement>

DPIGA was subjected to $^1$H NMR spectrum measurement, Fourier transform infrared (FT-IR) spectrum measurement, and UV-vis spectrum measurement. The results are shown in Fig. 79 to Fig. 81.

$^1$H NMR measurement was performed using a JEOL JNM-A500 spectrometer (JEOL, Tokyo, Japan), $D_2O$ was used as a solvent for DPI-GA and PDI, and a DMSO $D^6$ solvent was used as a solvent for gallic acid.

The FT-IR spectrum was recorded on an FT/IR-4200ST spectrometer (JASCO, Tokyo, Japan) using a KBr disk.

The UV-VIS spectrum of gallic acid (GA) in water exhibited the maximum absorbance at 215 nm and 265 nm, but DPI did not exhibit any absorbance in water. DPIGA exhibited an absorbance shift (red shift) to 300 nm. The red shift was thought to be due to an increase in pH of the solution after binding.

Based on these results, it was confirmed that synthesis of DPIGA was successful. The yield was 66%, and the degree of substitution was about 23%. The degree of substitution was calculated using a methyl peak of DPI (2.5 to 3 ppm) and an aromatic peak of gallic acid (7 to 7.5 ppm). The presence of characteristic aromatic peaks of GA in DPI-GA (1,450 to 1,620 $cm^{-1}$) in the FT-IR spectrum also supported the above results. The absorbance at 300 nm in the UV-VIS spectrum of DPI-GA also supported successful synthesis of DPI-GA.

(2) Lap shear test of AL-ABA-DPIGA

In order to evaluate adhesive properties AL-ABA-DPIGA (a crosslinked structure of AL-ABA and DPIGA), a lap shear test according to the ASTM D1002 test procedure was performed.

<Sample>

AL-ABA: AL-ABA(2) prepared in Example 1-2 (Low DS, modification rate (Colorimetric): 0.56)

<Test>

First, PBS was used as a solvent, and a 2% w/v solution (AL-ABA solution) in AL-ABA (a modification rate about 0.56) PBS and a 10% w/v solution (DPIGA solution) in DPIGA PBS were prepared. Then, the porcine esophageal submucosa and skin tissue were thawed and washed with distilled water and cut into a plurality of rectangular pieces with a size of 3 cm in length and 1 cm in width. About 0.1 mL of each of sample solutions in which the AL-ABA solution and the DPIGA solution were mixed in two ratios (3:1 or 1:1) (AL-ABA-DPIGA (3:1) and AL-ABA-DPIGA (1:1), respectively), the AL-ABA solution, and the DPIGA solution was applied to the edge of the tissue in a 1×1 $cm^2$ area, and two pieces of tissues were overlapped to cover the applied area. Then, for the samples using the AL-ABA solution and the DPIGA solution, the tissue samples were immediately immersed in a 100 mM $CaCl_2$ solution for 2 minutes. Each sample was then incubated at 37°C for 1 hour (RCom MARU Delux Max Digital Incubator). Here, during incubation, the tissue sample was kept with several moist tissue papers in order to prevent moisture loss. For uniform effect and adhesion, during incubation, all samples were pressed with a weight of about 100 g.

The resulting sample was subjected to a lap shear adhesion test using a tensile testing device (CR-3000EX-S; Sun Scientific Co., Ltd., Tokyo, Japan). The sample was fixed between both crumbs of the tensile testing machine, and the test was performed at a speed of 5 mm/min until the two overlapping tissue samples separated from each other. The test was performed three times for each sample. The adhesion strength was calculated from the maximum force when the sample was separated using the following formula.

$$\text{Adhesion strength} = \text{maximum force } (N/m^2)/\text{overlapping region area } (m^2)$$

(Results)

The results are shown in the following table and Fig. 82(A).

[Table 7]

| Table 7 Results of lap shear test using skin tissue or submucosa | | | | |
|---|---|---|---|---|
| Material composition | Adhesion strength to pig skin tissue (kPa) | Standard error | Adhesion strength to pig submucosa (kPa) | Standard error |
| AL-ABA | 51.2 | 7.8 | 7.9 | 0.6 |

(continued)

| Table 7 Results of lap shear test using skin tissue or submucosa | | | | |
|---|---|---|---|---|
| Material composition | Adhesion strength to pig skin tissue (kPa) | Standard error | Adhesion strength to pig submucosa (kPa) | Standard error |
| AL-ABA-DPIGA(3:1) | 56.3 | 6.3 | 8.7 | 4.0 |
| AL-ABA-DPIGA(1:1) | 18.4 | 3.2 | 2.2 | 0.2 |
| DPI-GA | 36.4 | 4.1 | 8.3 | 1.4 |

After incubation, all samples formed hydrogels. It was thought that, in the samples in which AL-ABA and DPIGA were mixed, a crosslinked structure of AL-ABA and DPIGA was formed according to crosslinking between the unmodified carboxyl group of AL-ABA and calcium (Ca) ions, and crosslinking due to the formation of a Schiff base between the aldehyde group of AL-ABA and the amino group of DPIGA.

In the results of the lap shear adhesion test using the porcine skin tissue and submucosa, the adhesion strength of AL-ABA-DPIGA (3:1) was increased by about 10% compared to AL-ABA alone. The adhesion strength of AL-ABA-DPIGA (1:1) was lower than that of AL-ABA. Based on these results, it was thought that, in AL-ABA-DPIGA (3:1), a part of DPIGA reacted with the aldehyde group of AL-ABA, and both the gallic acid (GA) moieties of DPIGA and the aldehyde group of AL-ABA reacted with free amino groups of the tissue to form a hydrogen bond and a covalent bond, which led to an increase in adhesion strength. On the other hand, in AL-ABA-DPIGA (1:1), the adhesion strength significantly decreased. The reason for this was thought to be that, all aldehyde groups of AL-ABA reacted with amines of DPI-GA, only GA moieties contributed to adhesion, and as a result, the adhesion strength decreased. As shown in Fig. 82(B), it was thought that AL-ABA-DPIGA adhered to the tissue via a covalent bond (Schiff base) between the aldehyde group of AL-ABA and the amino group of the tissue and a hydrogen bond between the GA (hydroxyl group) of DPIGA and the hydrogen atom of the tissue.

It was shown that the hydrogel formed from AL-ABA and DPIGA (amino group-containing polymer having an amino group and GA (hydroxyl group)) could be used as a tissue adhesive for biological tissues such as submucosa and skin tissue. It was suggested that, when the mixing ratio of AL-ABA and DPIGA (amino group-containing polymer having an amino group and GA (hydroxyl group)), the modification rate of AL-ABA, and the modification rate of DPIGA were adjusted, it was possible to adjust the adhesion strength to the tissue and the crosslinking density of the hydrogel.

II. Hyaluronic acid derivatives

[Example 11-1] Benzaldehyde-modified hyaluronic acid (HA-ABA)

<Synthesis of HA-ABA>

[C61]

Synthesis scheme 3

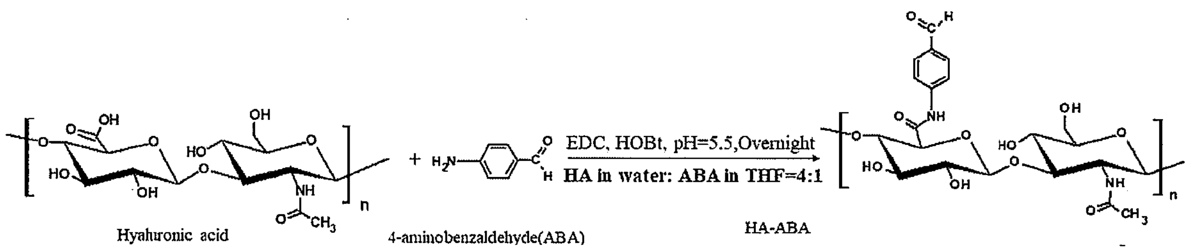


Hyaluronic acid (HA) 4-aminobenzaldehyde(ABA) HA-ABA

Following the above synthesis scheme, HA-ABA was synthesized according to the following procedures by an amidation reaction via carbodiimide.

(Synthesis procedures)

100 mL of HA (Mw: 890 kDa) aqueous solution (2 mg/mL) was dissolved in 25 mL of THF. This was mixed with 121 mg of 4-amino-benzaldehyde (ABA) (corresponding to 2 equivalents of ABA relative to the carboxyl groups of HA). 2 equivalents of 1-ethyl-3-(3-dimethylaminopropyl) carbodiimide hydrochloride (EDC·HCl) and 2 equivalents of 1-hydroxybenzotriazole (HOBt) were added, the pH was adjusted to 5.5, and the mixture was stirred overnight at room temperature. This was then purified by dialysis and freeze dried to obtain HA-ABA.

<Molecular weight measurement>

The weight-average molecular weight (Mw) of the HA was measured under the following conditions by gel filtration chromatography (GPC).

(Measurement procedures)

The molecular weight distribution of the polymer was measured by gel filtration chromatography (GPC). A liquid transport unit for high performance liquid chromatography (LC-10ADVP, Shimadzu Corp.) was used as the pump, and an intelligent refractive index detector (830-RI, JASCO) as the detector. Separation was performed at room temperature using TSK-GEL GMPWXL (molecular weight cutoff 104 to 106, Tosoh) and TSK-GEL-3000 (molecular weight cutoff 103 to 105, Tosoh) as the columns. The flow rate was set at 0.5 mL/min, and the above phosphoric acid buffer (pH 6.7) was used as the eluent. A calibration curve was prepared using dextran (8,000 Da, 15,000 Da, 40,000 Da, 70,000 Da, 500,000 Da, Extrasynthese Co.). The day before measurement, solvent substitution was performed overnight with phosphoric acid buffer at a flow rate of 0.2 mL/min. The calibration curve solutions and sample solutions were each filtered with a 0.22 μm filter. Stabilization of the RI value was confirmed before measurement, measurement was initiated, and after use solvent substitution was performed again overnight with pure water at a flow rate of 0.2 mL/min.

<$^1$H NMR spectrum measurement, UV-visible light absorption spectrum (UV-vis) measurement and FT-IR spectrum measurement>

The HA-ABA was subjected to $^1$H NMR spectrum measurement, UV-visible light absorption spectrum (UV-vis) measurement and FT-IR spectrum measurement. The results are shown in Fig. 51 to Fig. 53.

In the $^1$H NMR spectrum (Fig. 51), peaks derived from ABA (peak a derived from methyl groups of HA, peaks b and c derived from benzene rings and peak d derived from aldehyde) were observed in the HA-ABA. A characteristic peak (at about 250 nm) derived from the benzyl groups of ABA was observed in the UV-vis (Fig. 52) spectrum of the HA-ABA, and absorption (peak at about 2950 $cm^{-1}$) attributable to ABA was also observed in the FT-IR spectrum of the HA-ABA (Fig. 53). These results confirmed synthesis of benzaldehyde-modified hyaluronic acid (HA-ABA) formed by binding between amino groups of 4-aminobenzaldehyde and carboxyl groups of hyaluronic acid. Based on the peaks at 1.95 ppm (a: $-CH_3$ of acetoamide part of N-acetyl-D-glucosamine), 6.75 ppm (b: -CH of benzyl rings), 7.65 ppm (c: -CH of benzyl rings) and 9.44 ppm (d: -CH of aldehyde) in the $^1$H NMR spectrum, the ABA modification rate of the carboxyl group (-COOH) of the hyaluronic acid was calculated as 0.16.

<Cell toxicity evaluation (WST assay) of benzaldehyde-modified hyaluronic acid (HA-ABA)>

(Test procedures)

MeT-5A (human mesothelial cell line), HUVEC (human umbilical vein endothelial cells), RAW264.7 cells (mouse macrophage-like cell line), NIH/3T3 (mouse embryo fibroblasts) and AB22 cells (mouse mesothelioma cells) were seeded on 24-well plates and incubated for 24 hours. The medium was then replaced with media having HA-ABA and HA dissolved at different concentrations (0.01 mg/mL, 0.1 mg/mL, 1 mg/mL). Cell viability (%) 48 hours after sample addition was then measured by WST-8 assay (Cell Counting Kit-8, Dojindo).

(Results)

The results are shown in Fig. 54. The HA-ABA exhibited high cell viability equivalent to HA in most cells. In the NIH/3T3 mouse embryo fibroblasts, cell viability reduced to 20% with 1 mg/mL of HA-ABA. This may be related to different expression of CD44 in different kinds of cells.

[Example II-2] Benzaldehyde-modified hyaluronic acid-pemetrexed conjugate (HA-ABA-PMX)

<Synthesis of HA-ABA-PMX>

[C62]

Synthesis scheme 4

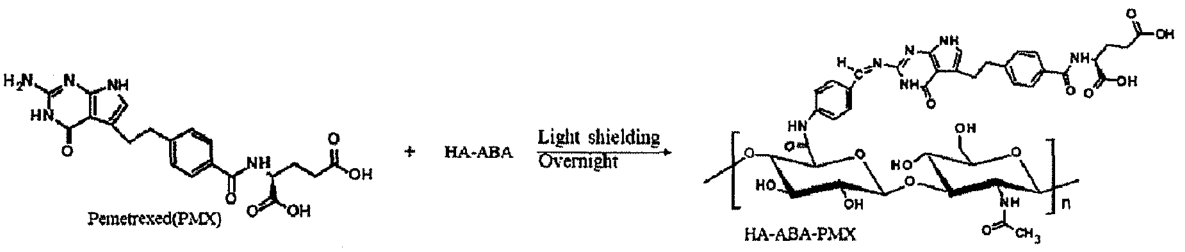


Pemetrexed(PMX)

HA-ABA-PMX

Pemetrexed (PMX) is an anticancer drug having a primary amino group. PMX was linked to HA-ABA according to the above synthesis scheme by a Schiff base reaction between the aldehyde groups of the HA-ABA and the amino groups of the pemetrexed.

Specifically, the HA-ABA obtained in Example II-1 was dissolved in pure water to a concentration of 1 mg/mL, and 1.5 equivalents of PMX dissolved in DMSO were added dropwise. This was reacted overnight at room temperature under shaded conditions. This was then dialyzed and freeze dried to obtain HA-ABA-PMX.

<$^1$H NMR spectrum measurement, and UV-visible light absorption spectrum (UV-vis) measurement>

The resulting HA-ABA-PMX was subjected to $^1$H NMR spectrum measurement, and UV-visible light absorption spectrum (UV-vis) measurement. The results are shown in Fig. 55 and Fig. 56.

In the $^1$H NMR spectrum (Fig. 55), peaks derived from HA-ABA and PMX were observed in the HA-ABA-PMX. In the UV-vis spectrum (Fig. 56), an increase in a characteristic peak of PMX (225 nm) was observed in the HA-ABA-PMX. These results confirm formation of a conjugate between HA-ABA and PMX. Based on the $^1$H NMR spectrum, the conjugate rate of the drug (PMX) to the carboxyl groups (-COOH) of the HA was 11.5%. This shows that 71.8% of the ABA in the HA-ABA reacted with the PMX.

These results show that conjugation of PMX to the HA-ABA was accomplished simply by mixing the HA-ABA and PMX in an aqueous solvent. Since this reaction can be applied to a variety of drugs containing primary amines, HA-ABA is expected to provide a simple and general-purpose platform for developing hyaluronic acid-drug conjugates.

<Release test of PMX from benzaldehyde-modified hyaluronic acid-pemetrexed conjugate (HA-ABA-PMX)>

(Test procedures)

The release behavior or PMX from HA-ABA-PMX at different pH values was investigated using a dialysis tube (MWCO: 1 kDa). 1 mL of an aqueous solution of the HA-ABA-PMX (1 mg/mL) obtained in Example II-2 above was placed in a dialysis tube, and shaken at 50 rpm while being incubated with 79 mL of PBS (37°C, pH = 5.0, 6.0 7.4). The external solution (sustained release solution) was collected at specific time points, and new PBS was substituted for the entire amount. The drug concentration in the collected external solution (sustained release solution) was measured by UV-vis, and the release rate of the drug PMX was calculated.

For purposes of comparison as control tests, the same release test was also performed with the HA-ABA-PMX solution replaced by an aqueous solution of PMX (0.12 mg/mL) (Free PMX), a mixed solution of HA and PMX obtained by dissolving hyaluronic acid (HA) (1 mg/mL) and PMX (0.12 mg/mL) in water (Free PMX mixed with HA), and an aqueous solution of HA-ADH-PMX having the HA bound irreversibly to the PMX by amide bonds.

The HA-ADH-PMX has the following structure. The HA-ADH-PMX was synthesized by the methods described in Amano Y., European Journal of Pharmaceutical Sciences, 2019, 138: 105008.

[C63]

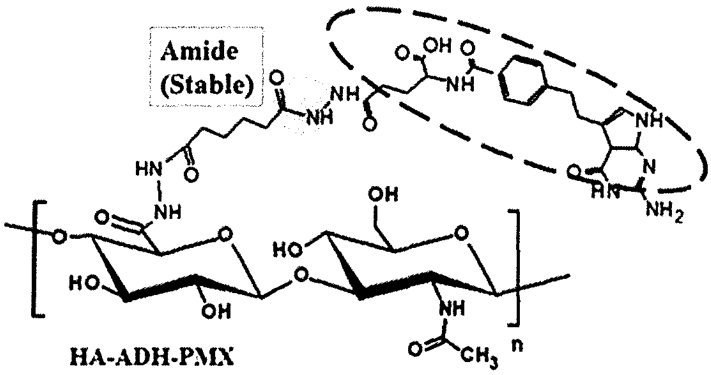

HA-ADH-PMX

(Results)

The results are shown in Fig. 57. Fig. 57 shows the release behavior of pemetrexed (PMX) from a benzaldehyde-modified hyaluronic acid-pemetrexed conjugate solution (HA-ABA-PMX) at different pH values (pH = 5.0, 6.0, 7.4), the release behavior of PMX from a PMX solution (Free PMX), and the release behavior of PMX from a mixed solution of HA and PMX (Free PMX mixed with HA). The vertical axis shows the cumulative release rate of PMX (cumulative drug release, %).

While more than 80% of the PMX was released at pH 5.0 after 24 hours with the HA-ABA-PMX, 40% had not been released at pH 7.4. These results show that PMX is released more rapidly at lower pH values. This is thought to be due to the fact that the imine bonds between HA-ABA and PMX are disassociated more easily at low pH.

In the case of the free PMX solution (Free PMX) and the mixed solution of free PMX and HA (Free PMX mixed with HA), on the other hand, there was no sustained release effect and almost all of the PMX was rapidly released within 2 hours. In the case of the solution of HA-ADH-PMX having irreversible amide bonds, almost no release of PMX from the HA-ADH-PMX was observed. The results suggest that HA-ABA-PMX allows pH dependent sustained drug release due to the formation of Schiff bases.

These results show that in comparison with a control group containing free PMX, the HA-ABA-PMX conjugate allows continuous sustained release of PMX, and also allows pH-dependent changes in the release rate of PMX, with the drug being released selectively at low pH.

[C64]

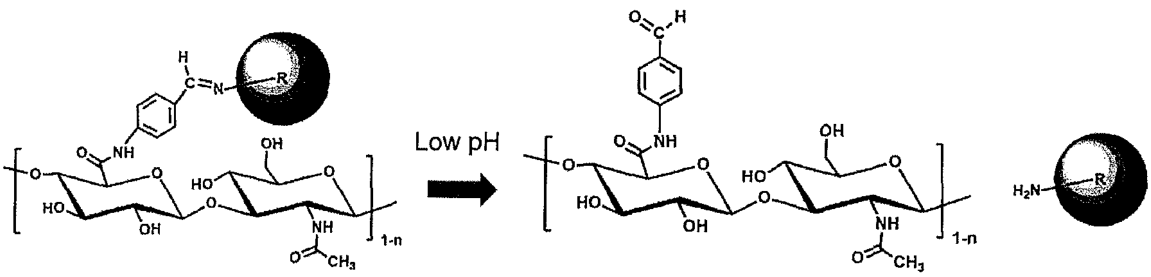


HA-ABA-PMX HA-ABA PMX

<Cell toxicity evaluation (WST assay) of benzaldehyde-modified hyaluronic acid-pemetrexed conjugate (HA-ABA-PMX)>

(Test procedures)

As with the HA-ABA, the cell toxicity of HA-ABA-PMX in MeT-5A (human mesothelial cell line) and AB22 cells (mouse mesothelioma cells) was evaluated by WST assay.

Specifically, MeT-5A (human mesothelial cell line) and AB22 cells (mouse mesothelioma cells) were seeded on 24-well plates and incubated for 24 hours. The medium was then replaced with medium having HA-ABA-PMX dissolved at

different PMX concentrations ($10^{-4}$ μg/mL, $10^{-3}$ μg/mL, $10^{-2}$ μg/mL, $10^{-1}$ μg/mL, 1 μg/mL, 10 μg/mL). Cell viability (%) 48 hours after sample addition was then measured by WST-8 assay (Cell Counting Kit-8, Dojindo).

For purposes of comparison as a control test, cell toxicity was also evaluated using free PMX and HA-ADH-PMX in place of HA-ABA-PMX.

(Results)

The results are shown in Fig. 58. Fig. 58A and Fig. 58B show the cell growth inhibition effects (vertical axis: cell viability (%) of HA-ABA-PMX, free pemetrexed (PMX) and HA-ADH-PMX having PMX irreversibly bound to HA by amide bonds at different PMX concentrations ($10^{-4}$ μg/mL, $10^{-3}$ μg/mL, $10^{-2}$ μg/mL, $10^{-1}$ μg/mL, 1 μg/mL, 10 μg/mL) in AB22 cells (mouse mesothelioma cells) and MeT-5A cells (human mesothelial cell line), respectively.

Both the HA-ABA-PMX and the free PMX exhibited dose-dependent cell growth inhibition effects in AB22 and MeT-5A. Moreover, the HA-ABA-PMX reduced cell viability at lower concentrations than the free PMX and the HA-ADH-PMX having PMX irreversibly bound to HA by amide bonds. These results suggest the strong cell growth inhibition effects of HA-ABA-PMX. This is thought to be due to enhanced cellular uptake via HA-CD44 interactions, along with irreversible dissociation of the conjugated PMX.

[Example 11-3] Benzaldehyde-modified hyaluronic acid-doxorubicin conjugate (HA-ABA-DOX)

<Synthesis of HA-ABA-DOX>

[C65]

Doxorubicin (DOX)

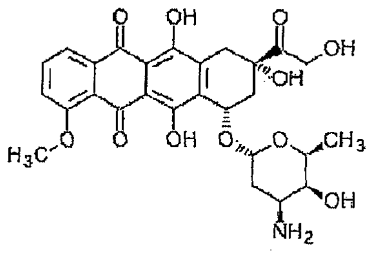

Doxorubicin (DOX) is an anticancer drug having a primary amino group. A benzaldehyde-modified hyaluronic acid-doxorubicin conjugate (HA-ABA-DOX) was synthesized by the same methods as Example 11-2.

Specifically, the HA-ABA obtained in Example II-1 was dissolved in pure water at a concentration of 1 mg/mL, and 1.5 equivalents of DOX hydrochloride dissolved in pure water were added dropwise. This was reacted overnight at room temperature under shaded conditions. This was then dialyzed and freeze dried to obtain HA-ABA-DOX.

<UV-vis absorption spectrum (UV-vis) measurement>

The resulting HA-ABA-DOX was subjected to UV-vis absorption spectrum (UV-vis) measurement. The results are shown in Fig. 59.

In UV-vis (Fig. 59), the HA-ABA-DOX exhibited a peak attributable to doxorubicin (DOX), confirming formation of a conjugate of HA-ABA and DOX.

<Release test of DOX from benzaldehyde-modified hyaluronic acid-doxorubicin conjugate (HA-ABA-DOX)>

(Test procedures)

Release of DOX from HA-ABA-DOX under different pH conditions was investigated in the same manner as in Example II-2. Specifically, 1 mL of an aqueous solution of the HA-ABA-DOX (1 mg/mL) obtained in Example 11-3 above was placed in a dialysis tube (MWCO: 1 kDa), and shaken at 50 rpm while being incubated with 79 mL of PBS (37°C, pH = 5.0, 6.0 7.4). The external solution (sustained release solution) was collected at specific time points, and new PBS was substituted for the entire amount. The drug concentration in the collected external solution (sustained release solution)

was measured by UV-vis, and the release rate of the drug DOX was calculated.

(Results)

The results are shown in Fig. 60. Fig. 60 shows the release behavior of doxorubicin (DOX) from a benzaldehyde-modified hyaluronic acid-doxorubicin conjugate solution (HA-ABA-DOX) at different pH values (pH = 5.0, 6.0, 7.4). The vertical axis shows the cumulative release rate of DOX (cumulative drug release, %).

It was shown that the HA-ABA-DOX conjugate is capable of selective drug release at low pH, and that the DOX release rate changes in a pH-dependent manner.

<Cell toxicity evaluation (WST assay) of benzaldehyde-modified hyaluronic acid-doxorubicin conjugate (HA-ABA-DOX)>

(Test procedures)

As with the HA-ABA, the cell toxicity of HA-ABA-DOX in AB22 cells (mouse mesothelioma cells) was evaluated by WST assay.

Specifically, AB22 cells (mouse mesothelioma cells) were seeded on 24-well plates and incubated for 24 hours. The medium was then replaced with media having HA-ABA-PMX dissolved at different DOX concentrations ($10^{-3}$ µg/mL, $10^{-2}$ µg/mL, $10^{-1}$ µg/mL, 1 µg/mL, 10 µg/mL, 100 µg/mL). Cell viability (%) 48 hours after sample addition was then measured by WST-8 assay (Cell Counting Kit-8, Dojindo).

For purposes of comparison as a control test, cell toxicity was also evaluated using free DOX in place of HA-ABA-DOX.

(Results)

The results are shown in Fig. 61. Fig. 61 shows that HA-ABA-DOX and free DOX both had dose-dependent cell growth inhibition effects on AB22. In comparison with the free DOX, the HA-ABA-DOX depressed cell viability at lower concentrations. These results suggest the strong cell growth inhibition effects of HA-ABA-DOX.

[Example II-4] Benzaldehyde-modified hyaluronic acid-gemcitabine conjugate (HA-ABA-GEM)

<Synthesis of HA-ABA-GEM>

[C66]

Gemcitabine (GEM)

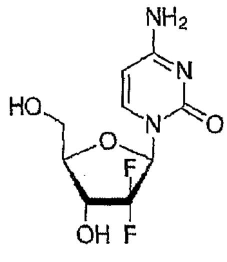

Gemcitabine (GEM) is an anticancer drug having a primary amino group. A benzaldehyde-modified hyaluronic acid-gemcitabine conjugate (HA-ABA-GEM) was synthesized in the same manner as in Example II-2.

Specifically, the HA-ABA obtained in Example II-1 was dissolved in pure water at a concentration of 1 mg/mL, and 1.5 equivalents of GEM dissolved in pure water were added dropwise. This was reacted overnight at room temperature under shaded conditions. This was then dialyzed and freeze dried to obtain HA-ABA-GEM.

<UV-vis absorption spectrum (UV-vis) measurement>

The resulting HA-ABA-GEM was subjected to UV-vis absorption spectrum (UV-vis) measurement. The results

are shown in Fig. 62.

In UV-vis (Fig. 62), the AL-ABA-GEM exhibited a peak attributable to gemcitabine (GEM), confirming the formation of a conjugate of HA-ABA and GEM.

[Example 11-5] Benzaldehyde-modified hyaluronic acid-DNA nucleotide conjugates (HA-ABA-adenine, HA-ABA-cytosine, HA-ABA-guanine)

<Synthesis of HA-ABA-adenine, HA-ABA-cytosine and HA-ABA-guanine>

[C67]

Adenine

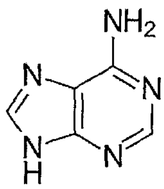

Cytosine

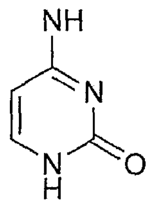

Guanine

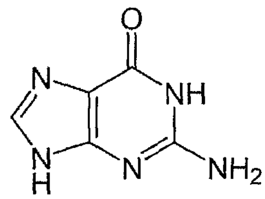

Conjugates of benzaldehyde-modified hyaluronic acid with the DNA nucleotides adenine, cytosine and guanine (HA-ABA-adenine, HA-ABA-cytosine, HA-ABA-guanine) were synthesized in the same manner as in Example II-2.

Specifically, the HA-ABA obtained in Example 8 was dissolved in pure water at a concentration of 1 mg/mL, and 2.5 equivalents of adenine, cytosine or guanine dissolved in pure water were added dropwise. This was reacted overnight at room temperature under shaded conditions, and then dialyzed and freeze dried to obtain HA-ABA-adenine, HA-ABA-cytosine and HA-ABA-guanine.

<$^{1}$H NMR spectrum measurement and FT-IR spectrum measurement>

The resulting HA-ABA-adenine, HA-ABA-cytosine and HA-ABA-guanine were subjected to $^{1}$H NMR spectrum measurement and FT-IR spectrum measurement, with the results shown in Fig. 63 to Fig. 66.

A peak derived from HA (e) and peaks derived from adenine (a to d) were observed in the $^{1}$H NMR spectrum (Fig. 63) of the HA-ABA-adenine.

A peak derived from HA (e) and peaks derived from cytosine (a to d) were observed in the $^{1}$H NMR spectrum (Fig. 64) of the HA-ABA-cytosine.

A peak derived from HA (d) and peaks derived from guanine (a to c) were observed in the $^{1}$H NMR spectrum (Fig. 65) of the HA-ABA-guanine.

Moreover, absorption attributable to binding between HA and ABA (stretching and ring vibration of C=O, C=C, C=N) was observed in the FT-IR spectra (Fig. 66) of the HA-ABA-adenine, HA-ABA-cytosine and HA-ABA-guanine as well as HA-ABA.

These results confirm that conjugates were formed between HA-ABA and DNA nucleotides (adenine, cytosine or guanine). The modification rates (drug introduction rates) of the drugs (DNA nucleotides) were calculated from the $^{1}$H NMR spectra. The conjugate rate of adenine on the carboxyl groups (-COOH) of HA was 8%, indicating that 38% of the ABA in the HA-ABA had reacted with the adenine. The conjugate rate of the cytosine on the carboxyl groups (-COOH) of HA was 16%, indicating that 76.1% of the ABA in the HA-ABA had reacted with the cytosine. The conjugate rate of the guanine on the carboxyl groups (-COOH) of HA was 9%, indicating that 42.8% of the ABA in the HA-ABA had reacted with the guanine.

These results show that a DNA nucleotide can be made to bind (conjugate) with HA-ABA simply by mixing the HA-ABA with the DNA nucleotide in an aqueous medium. HA-ABA is therefore expected to provide a simple and general-purpose platform for developing hyaluronic acid-nucleic acid drug conjugates.

III. Carboxymethyl cellulose derivative

[Example III-1] Benzaldehyde-modified carboxymethyl cellulose (CMC-ABA)

<Synthesis of CMC-ABA>

[C68]

Synthesis scheme 5

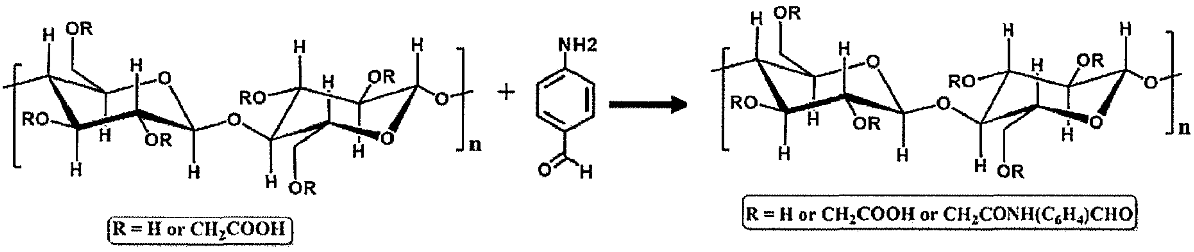


CMC ABA CMC-ABA

Following the above synthesis scheme, CMC-ABA was synthesized by the following procedures by an amidation reaction via carbodiimide.

(Synthesis procedures)

0.3 g of CMC (Sigma-Aldrich, Product No. 419338-100G, product name: Sodium carboxymethyl cellulose, weight-average molecular weight: about 700,000 (catalog value)) was dissolved in 100 mL of distilled water in a 100 mL round-bottom flask, and shaken overnight to prepare a CMC solution. 2.16 g (0.016 mol) of HOBt (1-hydroxybenzotriazole hydrate, Tokyo Chemical Industry Co., Ltd., Product No.: H0468, weight-average molecular weight: 135.13) was dissolved in 10 mL of DMSO and added dropwise to the CMC solution, after which 3.06 g (0.016 mol) of WSCD HCl (Peptide Institute, Inc., Product No. 1030, weight-average molecular weight: 191) was dissolved in 15 mL of water and added dropwise to the CMC solution. The solution was then further stirred for 10 minutes. 1.38 g (0.011 mol) of 4-amino-benzaldehyde (ABA) was dissolved in 12 mL of tetrahydrofuran (THF, Fujifilm Wako Pure Chemical, Product No. 206-08744, weight-average molecular weight: 72.11), and added dropwise to the CMC solution. 1N NaOH (sodium hydroxide, Fujifilm Wako Pure Chemical, Product No. 198-13765, weight-average molecular weight: 40) was added drop by drop to the mixture as the pH was adjusted to a range of 7.5 to 8. The mixture was then stirred for 16 to 20 hours at room temperature. The reaction mixture was centrifuged for 10 minutes at 3,000 rpm. The supernatant was collected and dialyzed for 72 hours with pure water using a dialysis membrane (MWCO 6-8 kDa, Spectra/Pro®), and freeze dried to obtain CMC-ABA.

<$^1$H NMR spectrum measurement and FT-IR spectrum measurement>

The CMC-ABA was subjected to $^1$H NMR spectrum measurement and FT-IR spectrum measurement, with the results shown in Fig. 67 and Fig. 68.

A peak derived from CMC (e) and peaks derived from ABA (a to d) were observed in the $^1$H NMR spectrum (Fig. 67). Absorption attributable to binding between CMC and ABA (C=O stretching of amide bond, C-H stretching of aldehyde and N-H stretching) was also observed in the FT-IR spectrum (Fig. 68) of the CMC-ABA. Moreover, based on the $^1$H NMR spectrum the ABA modification rate of the carboxyl groups (-COOH) of the CMC was calculated to be 0.4866 (86.6 mol%).

IV. Carboxymethyl dextran derivatives

[Example IV-1] Benzaldehyde-modified carboxymethyl dextran (CMDX-ABA)

<Synthesis of CMDX-ABA>

[C69]

Synthesis scheme 6

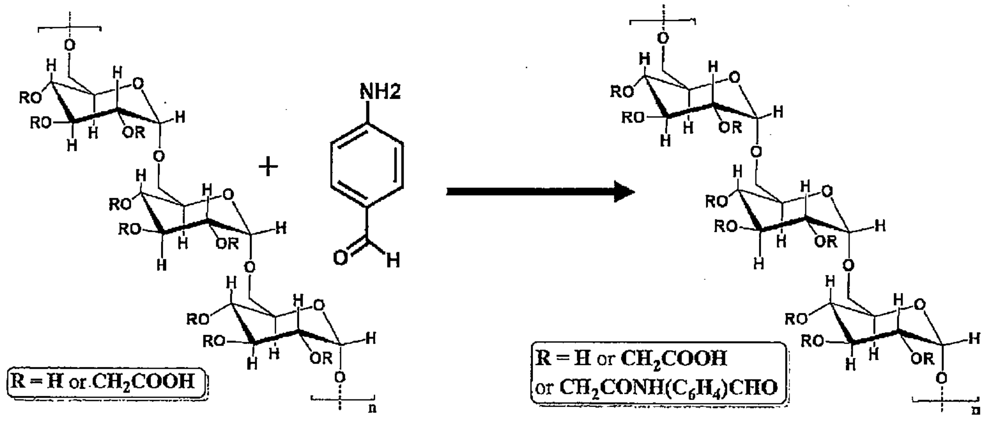


CMDX ABA CMDX-ABA

Following the above synthesis scheme, CMDX-ABA was synthesized by the following procedures by an amidation reaction via carbodiimide.

(Synthesis procedures)

0.5 g (0.0030 mol) of CMDX (Meito Industry Co., Ltd., Product name: carboxymethyl dextran, Product No. CMD-500-0613, weight-average molecular weight: 580,000 (catalog value)) was dissolved in 50 mL of pure water in a 100 mL round-bottom flask, and shaken overnight to prepare a CMDX solution. 2.83 g (0.021 mol) of HOBt was dissolved in 10 mL of DMSO and added dropwise to the CMDX solution, after which 4.01 g (0.021 mol) of WSCD HCl was dissolved in 20 mL of water and added dropwise to the CMDX solution. The solution was then further stirred for 10 minutes. 1.81 g (0.015 mol) of 4-amino-benzaldehyde (ABA) was dissolved in 12 mL of THF, and added dropwise to the CMDX solution. 1N NaOH was added drop by drop as the pH of the mixture was adjusted to a range of 7.5 to 8. The mixture was then stirred for 16 to 20 hours at room temperature. The reaction mixture was centrifuged for 10 minutes at 3,000 rpm. The supernatant was dialyzed for 72 hours with pure water using a dialysis membrane (MWCO 6-8 kDa, Spectra/Pro®), and freeze dried to obtain CMDX-ABA.

<$^1$H NMR spectrum measurement and FT-IR spectrum measurement>

The CMDX-ABA was subjected to $^1$H NMR spectrum measurement and FT-IR spectrum measurement, with the results shown in Figs. 69 and 70.

Peaks derived from CMDX (e, f) and peaks derived from ABA (a to d) were observed in the $^1$H NMR spectrum (Fig. 69). Absorption attributable to binding between CMDX and ABA (C=O stretching of amide bond, C-H stretching of aldehyde and N-H stretching) was also observed in the FT-IR spectrum (Fig. 70) of the CMDX-ABA. Moreover, based on the $^1$H NMR spectrum the ABA modification rate of the carboxyl groups (-COOH) of the CMDX was calculated to be 0.43 (43 mol%).

[Example IV-2] Benzaldehyde-modified carboxymethyl dextran-DNA nucleotide conjugates (CMD-ABA-adenine, CMD-ABA-cytosine, CMD-ABA-guanine)

<Synthesis of CMD-ABA-adenine, CMD-ABA-cytosine and CMD-ABA-guanine>

Conjugates of benzaldehyde-modified carboxymethyl dextran with the DNA nucleotides adenine, cytosine and guanine (CMD-ABA-adenine, CMD-ABA-cytosine, CMD-ABA-guanine) were synthesized.

Specifically, the CMDX-ABA obtained in Example IV-1 was dissolved in pure water at a concentration of 1 mg/mL, and 2.5 equivalents of adenine, cytosine or guanine dissolved in pure water were added dropwise. This was reacted overnight at room temperature under shaded conditions, and then dialyzed and freeze dried to obtain CMD-ABA-adenine, CMD-ABA-cytosine and CMD-ABA-guanine.

<FT-IR spectrum measurement>

The resulting CMD-ABA-adenine, CMD-ABA-cytosine and CMD-ABA-guanine were subjected to FT-IR spectrum measurement, with the results shown in Fig. 71.

Absorption from C=N stretching of imine bonds at 1740 $cm^{-1}$, from C=O stretching of amide bonds at 1640-1690 $cm^{-1}$, and from N-H stretching at 1550-1640 $cm^{-1}$ was observed in the FT-IR spectra (Fig. 71) of the CMD-ABA-adenine, CMD-ABA-cytosine and CMD-ABA-guanine. These results that conjugates were formed between the CMD-ABA and the DNA nucleotides (adenine, cytosine or guanine).

V. Chitosan derivative

[Example V-1] Benzaldehyde-modified chitosan (Chitosan-CBA)

<Synthesis of Chitosan-CBA>

[C70]

Synthesis scheme

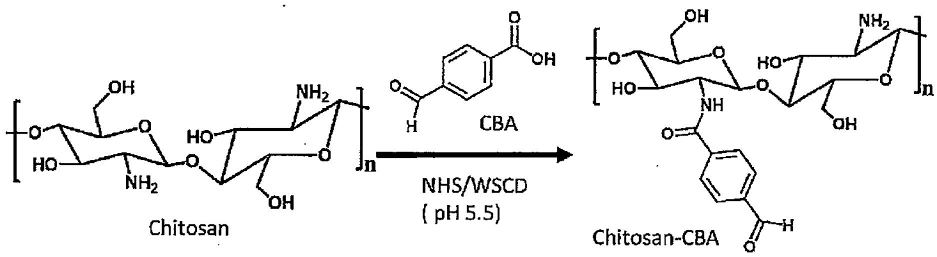


Following the above synthesis scheme, Chitosan-CBA was synthesized by the following procedures by an amidation reaction via carbodiimide.

1 g (0.0062 mol) of chitosan (Sigma Aldrich, Product No. 448877, CAS No. 9012-76-4, 75% to 85% deacetylated, weight-average molecular weight MW: 190,000 to 310,000 Da) was dissolved in 150 mL of 1.5% acetic acid solution to obtain a chitosan solution. 0.93 g (0.0062 mol) of 4-carboxybenzaldehyde (CBA) was then dissolved in 20 mL of water. 3.7 g (0.027 mol) of HOBt was dissolved in 10 mL of DMSO and added dropwise to the CBA solution, after which 5.32 g (0.027 mol) of WSCD/HCl was dissolved in 10 mL of distilled water and added dropwise to the CBA solution, which was then stirred for 15 to 20 minutes. The CBA solution was then added dropwise for 5 to 7 minutes to the chitosan solution, and the reaction mixture was stirred at room temperature for 20 to 24 hours with the pH maintained at about 5.5. The solution was then dialyzed with deionized water for 72 hours using a dialysis tube (MWCO: 6-8 kDa, Spectra/Pro®) and freeze dried to obtain Chitosan-CBA.

<$^1$H NMR spectrum measurement and FT-IR spectrum measurement>

The Chitosan-CBA was subjected to $^1$H NMR spectrum measurement and FT-IR spectrum measurement, with the results shown in Figs. 72 and 73. These results confirm synthesis of CBA-modified chitosan (Chitosan-CBA) by binding between the carboxyl groups of CBA and the amino groups of chitosan.

Based on the $^1$H NMR spectrum, the CBA modification rate of the chitosan amino groups ($-NH_2$) was calculated to be 0.20.

[Example V-2] Tissue adhesive material using benzaldehyde-modified chitosan (CH-CBA)

<Synthesis of CH-CBA>

[C71]

Synthesis scheme

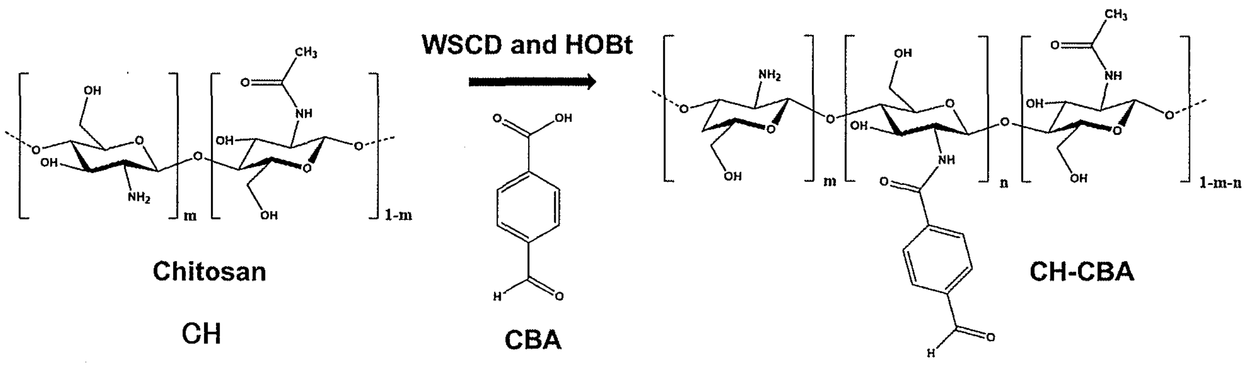


Chitosan

CH

CBA

CH-CBA

According to the synthesis scheme, CH-CBA was synthesized according to an amidation reaction via carbodiimide by the following procedure.

1 g of chitosan (Mw: 190 to 310 kDa; Sigma Aldrich; a degree of deacetylation of 75-85%) was dissolved in 200 mL of a 2% acetic acid solution to prepare a chitosan solution. 1 equivalent (eq.) of 4-carboxybenzaldehyde (CBA; Mw: 150.13 g/mol, manufactured by Tokyo Chemical Industry Co., Ltd.) with respect to the amino groups in chitosan was dissolved in 20 mL of dimethyl sulfoxide (DMSO) to prepare a CBA solution. Then, 6 equivalents of 1-hydroxybenzotriazole (HOBt; hydrate≥97.0%, Pcode-H0468, Mw: 135.12 g/mol, manufactured by Tokyo Chemical Industry Co., Ltd.) dissolved in 20 mL of DMSO and water-soluble carbodiimide hydrochloride (WSCD/HCl; Pcode 1030, Mw. 191.7 g/mol, PEPTIDE INSTITUTE INC) dissolved in 10 mL of distilled water were added dropwise to the CBA solution. After stirring for 10 minutes, the chitosan solution was added, and the pH was adjusted to 5 using hydrochloric acid (HCl) or sodium hydroxide (NaOH). After reacting overnight, the resulting solution was filtered, purified by dialysis (MWCO: 6,000 to 8,000 kDa, Spectra/Pro) against sodium chloride (NaCl) for 1 day and against distilled water for 2 days, and then freeze-dried.

<$^1$H NMR spectrum measurement, UV-visible light absorption spectrum (UV-vis) measurement, and FT-IR spectrum measurement>

The synthesis of CH-CBA was confirmed using $^1$H NMR (α500, JEOL, Japan), Fourier transform infrared (FT-IR) spectrum measurement, and UV-vis spectrum measurement (UV-1900i; SHIMADZU). The results are shown in Figs. 83(A) to 83(C).

In the NMR spectrum of CH-CBA, a hydrogen signal belonging to the benzene ring was observed at around 7.6 ppm and a signal belonging to the aldehyde group was observed at around 9.8 ppm. These signals were not confirmed in the spectrum of pure chitosan. In addition, from the $^1$H NMR spectrum, the CBA modification rate for the amino group ($-NH_2$) of chitosan was calculated to be 0.045 (4.5%). In the FT-IR spectrum, a drop in the $-NH_2$ peak at about 1,595 $cm^{-1}$ and a C=C stretching signal belonging to the benzene ring at about 1,530 $cm^{-1}$ were observed. In addition, in the UV-Vis spectrum of CH-CBA, binding of CBA to chitosan was confirmed. These results indicated that the synthesis of CH-CBA was successful.

<Preparation of CH-CBA hydrogel>

A 3% (w/v) CH-CBA hydrogel was prepared by dissolving a polymer (CH-CBA) in PBS and the pH was then adjusted to a desired value using HCl or NaOH. A gelling test was performed at different pH values using the vial inversion method. The resulting CH-CBA hydrogel was freeze-dried and then observed using a scanning electron microscope (SEM; TM3030Plu, Hitachi Co., Ltd.).

<Test method>

The prepared CH-CBA hydrogel was subjected to a test according to a vial inversion method viscoelastic analysis, a tack test, a lap shear test, and a cell toxicity assay. The test methods were as follows.

(Measurement of gelling time according to vial inversion method)

In the gelling test at different pH values, a vial was used for checking. First, 3% (w/v) CH-CBA dissolved in PBS was put into a vial. Then, the pH was adjusted to a desired value using HCl or NaOH. Gelling was evaluated by visual observation after the vial was inverted. It was determined that a gel was formed if the mixture was not added dropwise when the vial was inverted.

Gel formation: the mixture was not added dropwise
No gel formation: the mixture was added dropwise

(Viscoelastic analysis)

In order to evaluate viscoelastic properties, a rheometer (MCR302; Anton Paar, Graz, Austria) was used. CH-CBA hydrogels with a diameter size of 25 mm and at different pH values were placed on the rheometer, a 25 mm probe was brought in contact with the hydrogel sample, and the thickness space was adjusted to 1 mm. The storage modulus (G') and the loss modulus (G") were measured at an angular frequency ($\omega$) of $10^{-1}$ to $10^{2}$ rad/s and a shear strain set to 5%. The loss factor (tan$\delta$) was defined as a ratio of G' to G". The viscosity curve was measured using a 25 mm sawtooth probe. The viscosity ($\eta$) (mPa s) was measured at a shear rate of $10^{-3}$ to $10^{3}$ 1/sec and a frequency set to 1 Hz. The temperature for all tests was set to 25°C.

(Probe tack test)

A rheometer was also used for the probe tack test. Similar to viscoelastic analysis, CH-CBA hydrogels with a diameter size of 25 mm and with different pH values were placed on the rheometer, a 25 mm probe was brought in contact with the CH-CBA hydrogel, and the thickness space was adjusted to 0.25 mm. Then, the probe was raised quickly at a speed of 5 mm/s, and the normal force (N) was recorded. The maximum normal stress ($F_{max}$) (N) received by the probe was the tack force, and the area under the curve exhibited the work of adhesion ($W_{adh}$)(mJ) of the material.

(Lap shear test)

The test was performed according to the procedure shown in Fig. 90. First, various tissues including mucosa, submucosa tissue and skin were cut into rectangular pieces (1 ×3 cm). Then, 100 μL of CH-CBA hydrogel with various pH values was applied onto a 1 $cm^2$ area (1×1 cm) of the tissue. FibrinGlue, BioGlue, albumin/GTA and pure chitosan solutions were used as control groups. After two pieces of tissues were glued together to cover the applied area, the sample was cured at 37°C for 1 hour, the test was then performed using a tensile testing device (CR-3000 EX-S; Sun Scientific Co., Ltd.). All tests were performed at a tensile speed of 5 mm/min. The adhesion strength was calculated from the maximum force when the sample was separated using the following formula.

$$\text{Adhesion strength} = \text{maximum force } (N/m^2)/\text{overlapping region area } (m^2)$$

(Cell toxicity assay)

The cell viability was evaluated by (A) WST-8 assay and (B) live/dead assay. Specifically, the cell viability of NIH/3T3 (mouse embryonic fibroblasts) was measured in the presence of CH-CBA hydrogel using WST-8 assay (Cell CountingKit-8, Dojindo) and Live/Dead cell viability (Lonza, Switzerland).

(A) WST-8 assay

Fibroblasts were grown and maintained in DMEM low glucose (Wako, Japan) containing a 10% newborn calf serum (NCF; Wako, USA) and a 1% penicillin-streptomycin-amphotericin B suspension (PSA; Wako, Japan) in a 10 cm petri dish at 37°C under 5% $CO_2$. When cells reached a confluence of 70 to 80%, they were separated with trypsin-EDTA (Wako, Japan), and the number of cells was measured with a hemocytometer. The cell suspension was seeded in a 24-well plate at an initial density of 50,000 cells/well and incubated overnight at 37°C under 5% $CO_2$. Then, the culture medium was replaced with a medium containing different volumes of CH-CBA hydrogel (pH 7.4) so that the final polymer concentration after degradation thereof was 0.1, 0.2, and 0.3% (w/v). In addition, Fibrin Glue and albumin/glutaraldehyde (Albumin/GTA) were used as control groups.
WST-8 assay was performed 24 hours after treatment. In the test, the medium was replaced with 500 μL of a

culture medium containing a 10% WST-8 reagent, and incubated at 37°C for 4 hours. Next, the absorbance at 450 nm was measured using a plate reader (2030 ARVOV3; PerkinElmer, Waltham, MA, USA). In order to calculate the cell viability (Cell viability (%)), the absorbance value of each well was normalized with respect to the absorbance value of a control well to which no test substance was added.

(B) Live/Dead cell viability

The Live/Dead cell viability was also measured 24 hours after treatment. The medium was replaced with PBS containing 2 μM calcein-AM and 4 μM ethdium homodimer-1 (EthD-1), incubated for 1 hour, and then observed using a confocal laser scanning microscope (FLUOVIEW FV3000; OLYMPUS, Japan).

<Results>

(1) pH-responsive self-crosslinking property

The pH-responsive self-crosslinking property of CH-CBA was evaluated by the vial inversion method and rheological analysis. Fig. 84 shows the results of a gelling test at different pH values measured by the vial inversion method. Fig. 85 shows the results of viscoelastic analysis.

As shown in Fig. 84, when the pH was less than 5, CH-CBA remained in a solution state. When the pH was 5 or more, it was observed that CH-CBA became a gel. As a result of viscoelastic analysis shown in Fig. 85(A), when the pH was 4, G" was higher than G', indicating a solution state, and when the pH was more than 5, G' was higher than G", indicating a gel state. This result matched the observation result according to the vial inversion method. In addition, as shown in Fig. 85(B), a group with a high pH (a pH of 5 to 8) exhibited a low loss factor (tanδ), indicating a more elastic property. These results demonstrated the pH-responsive self-crosslinking property of CH-CBA.

(2) Shear thinning property

From the viscosity curve results in Fig. 85(C), CH-CBA hydrogel exhibited shear thinning. The major interaction in the CH-CBA gel included a dynamic Schiff base (an interaction between -CHO and $-NH_2$) and a hydrophobic interaction (an interaction between - $NH_2$ and $-NH_2$) (Fig. 88(A)). Under high shear stress, breakage of dynamic imine bonds and weaker hydrophobic interactions lead to a decrease in viscosity.

In addition, the shear thinning property was evaluated based on the viscosity ratio ($\eta_{0.001}/\eta_{1000}$) of a viscosity ($\eta_{0.001}$) at a shear rate of $10^{-3}$ $s^{-1}$ to a viscosity ($\eta_{1000}$) at a shear rate of $10^3$ $s^{-1}$ (Fig. 86). As the pH increased from 5 to 7, the viscosity ratio ($\eta_{0.001}/\eta_{1000}$) increased, indicating an improvement in the shear thinning property. In a pH range of 7 to 8, no clear difference in the viscosity ratio was observed. This was thought to be because most - $NH_3^+$ groups became $-NH_2$ groups, and as a result, the increase in the interactions within the hydrogel became smaller.

(3) Scanning electron microscope image

Fig. 87 shows SEM images of CH-CBA hydrogel. At a pH of 4, a lay-by-lay (lay-by-lay) structure was shown, suggesting little interaction between CH-CBA polymers. When the pH was higher than 5, a porous structure was observed. At a pH of 8, a porous structure with a smaller pore size was observed, suggesting the improved hydrophobic interaction.

(4) Cell toxicity evaluation (WST assay) of CH-CBA

Fig. 89(A) shows the cell toxicity test results (WST-8 assay results). There was no clear difference between the control group not treated with a gel and the CH-CBA group. However, in the results of WST-8 assay, there was no obvious difference between the control group not treated with a gel and the CH-CBA group. On the other hand, the albumin/GTA group exhibited lower cell viability probably due to the toxicity of GTA.

Fig. 89(B) shows the Live/Dead cell viability assay results. The CH-CBA gel group exhibited favorable viability, fewer dead cell signals were observed, and the results matched the results of WST-8 assay.

(5) Adhesion strength evaluation

The adhesion strength was evaluated by a tack test and a lap shear test. The results of the tack test are shown in Figs. 91(A) and 91(B). The results of the lap shear test are shown in Figs. 91(C) to 91(E).

As a result of the tack test, it was confirmed that the tack force and the work of adhesion increased as the pH increased. An increase in the tack force and the work of adhesion exhibited stronger adhesive power.

As a result of the lap shear test, the adhesion strength to the mucous tissue (Fig. 91(C)) decreased as the pH increased. This was thought to be because the electrostatic attractive force between $-NH_3^+$ of CH-CBA and $-COO^-$ of mucin decreased as the pH increased. At a pH of 7.4, CH-CBA exhibited adhesion strength equivalent to that of FibrinGlue and BioGlue and exhibited higher adhesion strength than that of the albumin/GTA group.

In the case of submucosa (Fig. 91(D)) and the outer surface of the skin (Fig. 91(E)), as the pH increased, higher adhesion strength was exhibited. It was thought that aggregation within the hydrogel became stronger as the pH increased, and higher adhesion strength was exhibited. At a pH of 7.4, CH-CBA exhibited significantly higher adhesion strength than the fibrin adhesive.

It was shown that the CH-CBA hydrogel could be used as a tissue adhesive for biological tissues such as mucosa, submucosa and skin tissue. It was shown that the CH-CBA hydrogel had favorable adhesion (excellent tack performance) in a wide range of pH (a pH of 5 to 8).

It was shown that CH-CBA exhibited a pH-dependent self-crosslinking property, and became a solution at a pH of 4 or less, but a hydrogel was formed at a pH of 5 or more. In addition, it was confirmed that, when the pH was adjusted, the viscosity ratio ($\eta_{0.001}/\eta_{1000}$) of CH-CBA increased, and the shear thinning property was improved. In certain embodiments, the viscosity ratio ($\eta_{0.001}/\eta_{1000}$) of CH-CBA was in a range of $10^5$ to $10^7$.

CH-CBA was also excellent in terms of solubility in water. Unmodified chitosan was insoluble in water at a neutral pH, and was only soluble in an acidic aqueous solution, but the material of this form was useful because it was soluble in water at a neutral pH due to modification of CBA.

## Claims

1. A tissue adhesive material used for hard and thick tissue and/or connective tissue, comprising a polysaccharide derivative in which a group represented by Formula (A) below is introduced into an acidic, basic or amphoteric polysaccharide:

[C72]

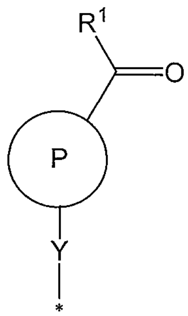

(A)

wherein $R^1$ represents a hydrogen atom or a $C_{1-4}$ alkyl group,
ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
Y represents a divalent group selected from the group consisting of -NH-, -C(=O)-, - S-, -O-, a $C_{1-6}$ alkylene group, $-(CH_2CH_2O)_n-$, and any combinations of these, and n is an integer from 1 to 9, and
* represents a linkage with the polysaccharide.

2. The tissue adhesive material according to claim 1, wherein the polysaccharide is selected from alginic acid, derivatives thereof or salts of these, hyaluronic acid, derivatives thereof or salts of these, carboxymethyl cellulose, derivatives thereof or salts of these, carboxymethyl dextran, derivatives thereof or salts of these, carboxymethyl starch, derivatives thereof or salts of these, heparin, derivatives thereof or salts of these, heparan sulfate, derivatives thereof or salts of these, chondroitin sulfate, derivatives thereof or salts of these, dermatan sulfate, derivatives thereof or salts of these, chitosan, derivatives thereof or salts of these, regenerated oxidized cellulose, derivatives thereof or salts of these, and pectic acid, derivatives thereof or salts of these.

3. The tissue adhesive material according to claim 1 or 2, wherein

the polysaccharide is a polysaccharide containing a carboxyl group, the group represented by Formula (A) is a

group represented by Formula (A-1) below, and the group represented by Formula (A-1) is introduced into the polysaccharide by substituting -OH of the carboxyl group of the polysaccharide to form an amide bond; or; the polysaccharide is a polysaccharide containing an amino group, the group represented by Formula (A) is a group represented by Formula (A-2) below, and the group represented by Formula (A-2) is introduced into the polysaccharide by substituting a hydrogen atom of the amino group of the polysaccharide to form an amide bond:

[C73]

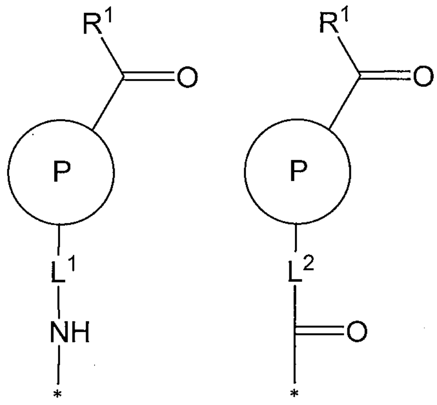

(A-1) (A-2)

wherein in formulae (A-1) and (A-2), $R^1$ represents a hydrogen atom or $C_{1-4}$ alkyl,
ring P is a phenyl ring or pyridine ring, and the phenyl ring or pyridine ring is optionally substituted with one or more substituents independently selected from the halogen atoms and $-CF_3$, $-NO_2$, carboxyl and $-SO_3H$ groups,
$L^1$ is a single bond or represents a divalent group selected from the group consisting of -C(=O)-, -S-, -O-, the $C_{1-6}$ alkylenes and $-(CH_2CH_2O)_n-$ and any combinations of these,
$L^2$ is a single bond or represents a divalent group selected from the group consisting of -NH-, -S-, -O-, the $C_{1-6}$ alkylenes and $-(CH_2CH_2O)_n-$ and any combinations of these,
n is an integer from 1 to 9, and
* represents a linkage with the polysaccharide.

**4.** The tissue adhesive material according to any one of claims 1 to 3, wherein

the polysaccharide is alginic acid, a derivative thereof or a salt thereof, and
the polysaccharide derivative contains a constituent unit represented by Formula (c11) and/or (c12) below:

[C74]

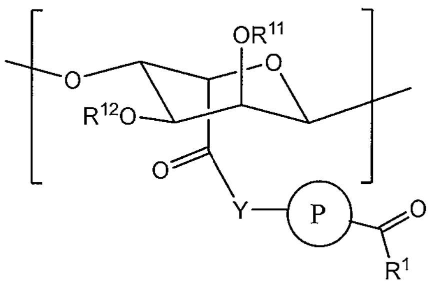

(c11)

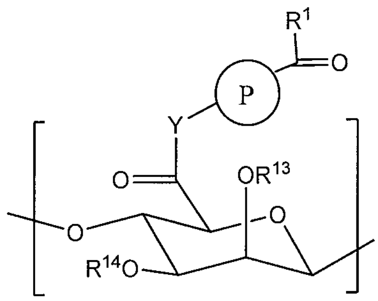

(c12)

wherein $R^{11}$, $R^{12}$, $R^{13}$, and $R^{14}$ are each independently selected from among a hydrogen atom, a $C_{1-6}$ alkyl group, and a $-C(=O)-C_{1-6}$ alkyl group,
$R^1$ represents a hydrogen atom or a $C_{1-4}$ alkyl group,
ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,

Y represents $-L^1-NH-$, in which $L^1$ is bound to ring P,
$L^1$ is selected from among a single bond, a $C_{1-6}$ alkylene group, and $-(CH_2CH_2O)_n-$, and
n is an integer from 1 to 9.

**5.** The tissue adhesive material according to any one of claims 1 to 4, wherein

the group represented by Formula (A) is selected from the group consisting of the following formulae:

[C75]

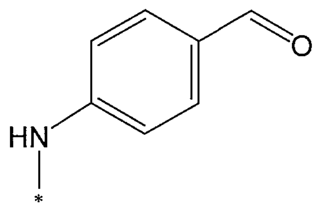
,
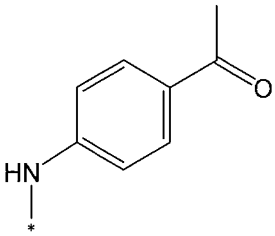
,
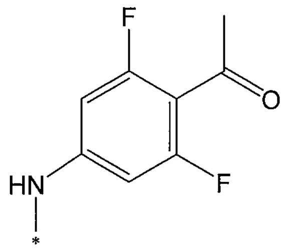
,

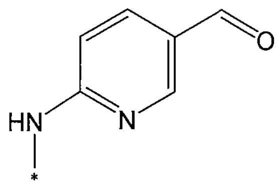
, and
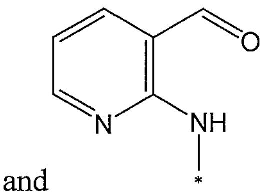
,

wherein * represents a linkage with the polysaccharide.

**6.** A tissue adhesive material used for hard and thick tissue and/or connective tissue, having a crosslinked structure in which at least a part of a polysaccharide derivative in which a group represented by Formula (A) below is introduced into an acidic, basic or amphoteric polysaccharide is crosslinked:

[C76]

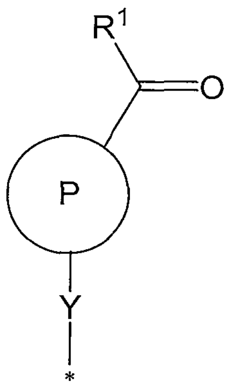
(A)

wherein $R^1$ represents a hydrogen atom or a $C_{1-4}$ alkyl group,
ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
Y represents a divalent group selected from the group consisting of -NH-, -C(=O)-, - S-, -O-, a $C_{1-6}$ alkylene group, $-(CH_2CH_2O)_n-$, and any combinations of these, and n is an integer from 1 to 9, and
* represents a linkage with the polysaccharide.

**7.** The tissue adhesive material according to any one of claims 1 to 6, which is in the form of a gel, sponge, sheet, or film.

**8.** The tissue adhesive material according to any one of claims 1 to 7, further comprising a nonwoven fabric, sheet, or film.

**9.** A crosslinked structure, comprising:

(a) a polysaccharide derivative in which a group represented by Formula (A) below is introduced into an acidic, basic or amphoteric polysaccharide; and
(b) at least one of an amino group-containing polymer and an amino group-containing low-molecular-weight compound, which contain two or more primary amino groups, a hydrazide group, or an aminoxy group, and a group represented by Formula (x1) below, wherein

the primary amino groups, the hydrazide group, or the aminoxy group contained in the amino group-containing polymer and the amino group-containing low-molecular-weight compound and the group represented by Formula (A) contained in the polysaccharide derivative are crosslinked by a covalent bond via a Schiff base:

[C77]

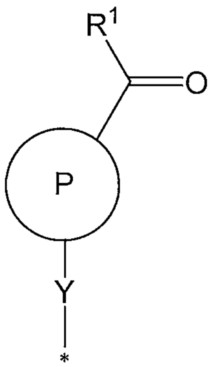

(A)

wherein in Formula (A), $R^1$ represents a hydrogen atom or a $C_{1-4}$ alkyl group,
ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
Y represents a divalent group selected from the group consisting of -NH-, -C(=O)-, - S-, -O-, a $C_{1-6}$ alkylene group, $-(CH_2CH_2O)_n-$, and any combinations of these, and n is an integer from 1 to 9, and
* represents a linkage with the polysaccharide.

[C78]

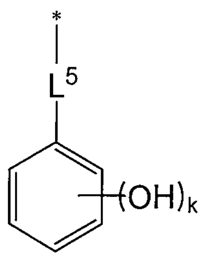

(x1)

wherein in Formula (x1), k is an integer from 2 to 5,
$L^5$ is a single bond or a divalent group selected from the group consisting of - C(=O)-, -S-, -O-, an alkylene group, $-(CH_2CH_2O)_n-$, and any combinations of these,
n is an integer from 1 to 9, and
* represents a linkage with the amino group-containing polymer or the amino group-containing low-molecular-weight compound.

**10.** The crosslinked structure according to claim 9, wherein

the amino group-containing polymer is at least one selected from among linear, branched or dendritic polyamines; polyalkylene glycols substituted with an amino group, a hydrazide group, or an aminoxy group; polyallylamines; polyvinylamines; polyacrylamines; amino group-containing polysaccharides; amino group-containing proteins; and polyamino acids.

**11.** The crosslinked structure according to claim 9 or 10, wherein

the polysaccharide is alginic acid, a derivative thereof or a salt thereof, and
the polysaccharide derivative contains a constituent unit represented by Formula (c11) and/or (c12) below:

[C79]

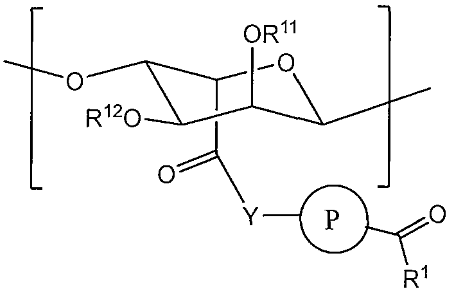

(c11)

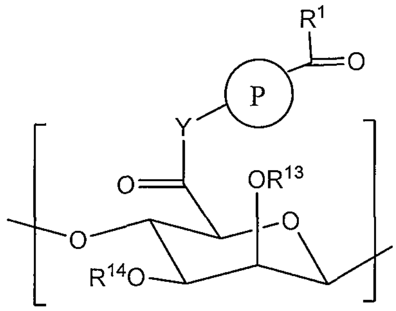

(c12)

wherein $R^{11}$, $R^{12}$, $R^{13}$, and $R^{14}$ are each independently selected from among a hydrogen atom, a $C_{1-6}$ alkyl group, and a $-C(=O)-C_{1-6}$ alkyl group,
$R^1$ represents a hydrogen atom or a $C_{1-4}$ alkyl group,
ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
Y represents $-L^1-NH-$, in which $L^1$ is bound to ring P,
$L^1$ is selected from among a single bond, a $C_{1-6}$ alkylene group, and $-(CH_2CH_2O)_n-$, and
n is an integer from 1 to 9.

**12.** A tissue adhesive material having the crosslinked structure according to any one of claims 9 to 11.

**13.** A tissue adhesive material containing a polysaccharide derivative in which a group represented by Formula (A-2) below is introduced into a polysaccharide containing an amino group, wherein

the group represented by Formula (A-2) is introduced into the polysaccharide by substituting a hydrogen atom of the amino group of the polysaccharide to form an amide bond, and
the tissue adhesive material is in the form of a gel:

[C80]

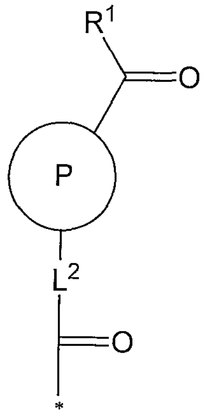

(A-2)

wherein in Formula (A-2),
$R^1$ represents a hydrogen atom or a $C_{1-4}$ alkyl group,
ring P is a phenyl ring or pyridine ring, and the phenyl ring or pyridine ring is optionally substituted with one or more substituents independently selected from among a halogen atom, $-CF_3$, $-NO_2$, a carboxyl group and $-SO_3H$,
$L^2$ is a single bond or represents a divalent group selected from the group consisting of -NH-, -S-, -O-, a $C_{1-6}$ alkylene group, $-(CH_2CH_2O)_n-$, and any combinations of these,
n is an integer from 1 to 9, and
* represents a linkage with the polysaccharide.

**14.** The composition according to claim 13, wherein
the ratio ($\eta_{0.001}/\eta_{1000}$) of a viscosity ($\eta_{0.001}$) at a shear rate of 0.001 $s^{-1}$ to a viscosity ($\eta_{1000}$) at a shear rate of 1,000 $s^{-1}$ measured at 25°C is 10 to $10^9$.

**15.** The tissue adhesive material according to claim 13 or 14, wherein
the polysaccharide derivative forms a crosslinked structure when the amino group contained in the polysaccharide derivative and the group represented by Formula (A-2) are crosslinked by forming a covalent bond via a Schiff base.

**16.** The tissue adhesive material according to any one of claims 13 to 15, wherein

the polysaccharide is chitosan, a derivative thereof or a salt thereof, and
the polysaccharide derivative contains a constituent unit represented by Formula (c16) below:

[C81]

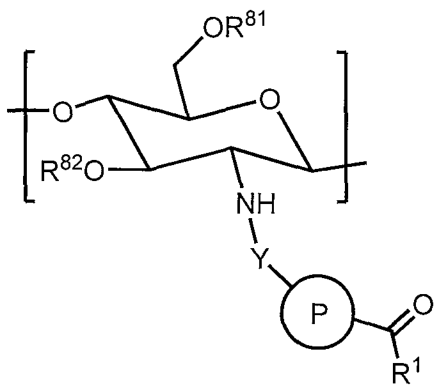

(c16)

wherein each of $R^{81}$ and $R^{82}$ is independently selected from a hydrogen atom, a $C_{1-6}$ alkyl and a -C(=O)-$C_{1-6}$ alkyl,
$R^1$ represents a hydrogen atom or $C_{1-4}$ alkyl,
ring P is a phenyl ring or pyridine ring, and the phenyl ring or pyridine ring is optionally substituted with one or more substituents independently selected from the halogen atoms and $-CF_3$, $-NO_2$, carboxyl and $-SO_3H$ groups,
Y represents -$L^2$-C(=O)-, in which $L^2$ is bound to ring P,
$L^2$ is selected from a single bond, a $C_{1-6}$ alkylene, $-(CH_2CH_2O)_n-$, $-(CH_2)_{m1}-(CH_2CH_2O)_n-(CH_2)_{m2}-$ and $-(CH_2)_{m1}-O-(CH_2CH_2O)_n-(CH_2)_{m2}$,
n is an integer from 1 to 9, and
each of $m_1$ and $m_2$ is independently an integer from 1 to 9.

[Fig. 1]

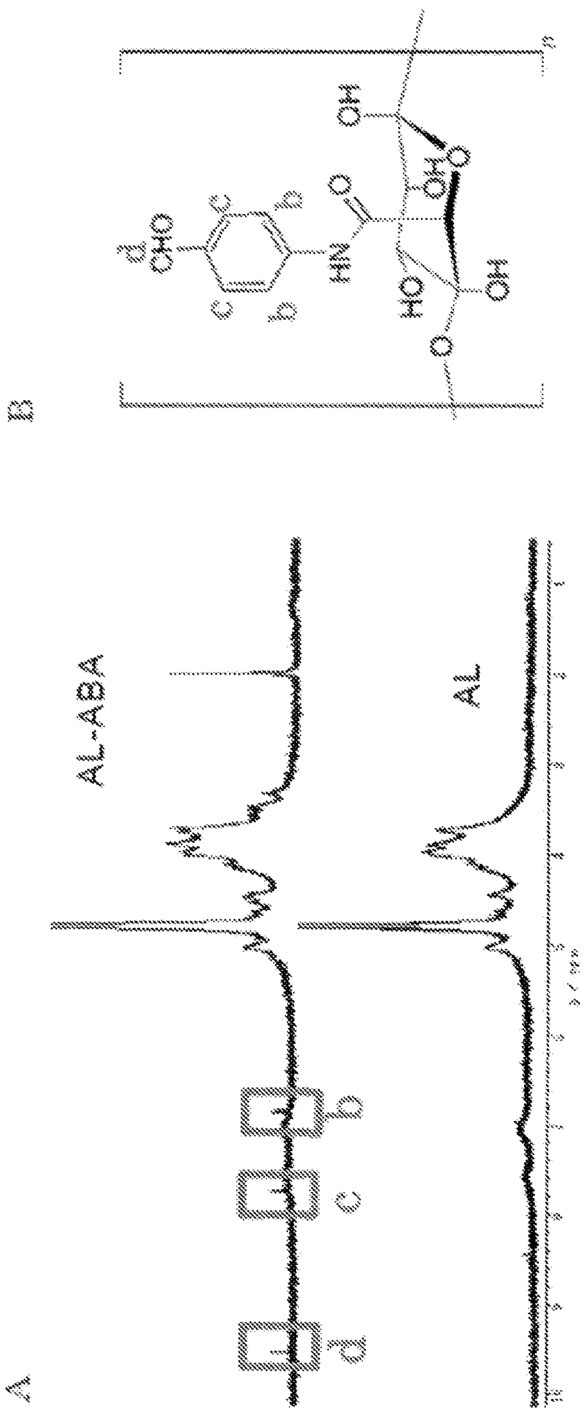

[Fig. 2]

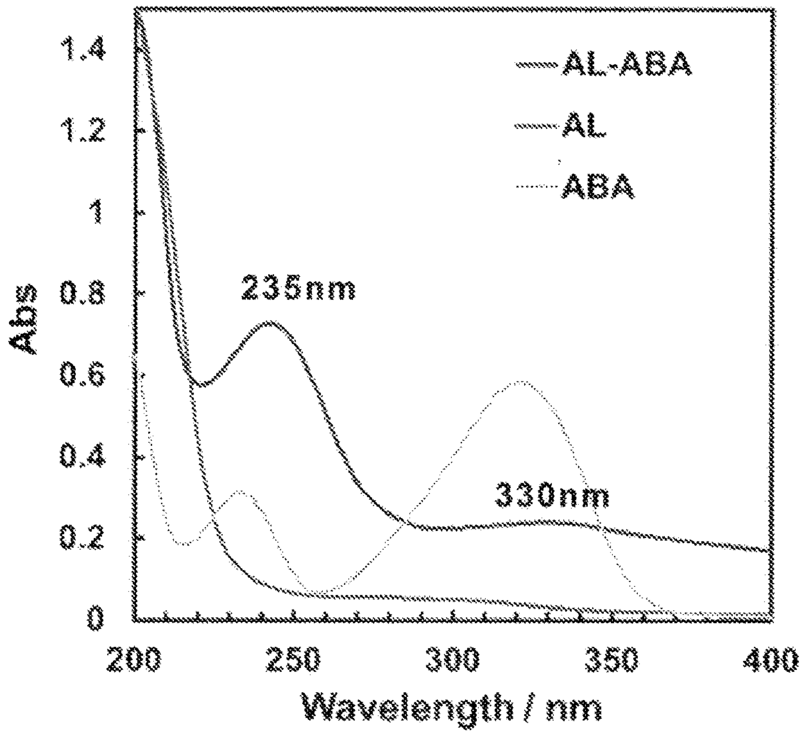
AL-ABA
AL
ABA
235nm
330nm
Abs
1.4
1.2
1
0.8
0.6
0.4
0.2
0
200
250
300
350
400
Wavelength / nm

[Fig. 3]

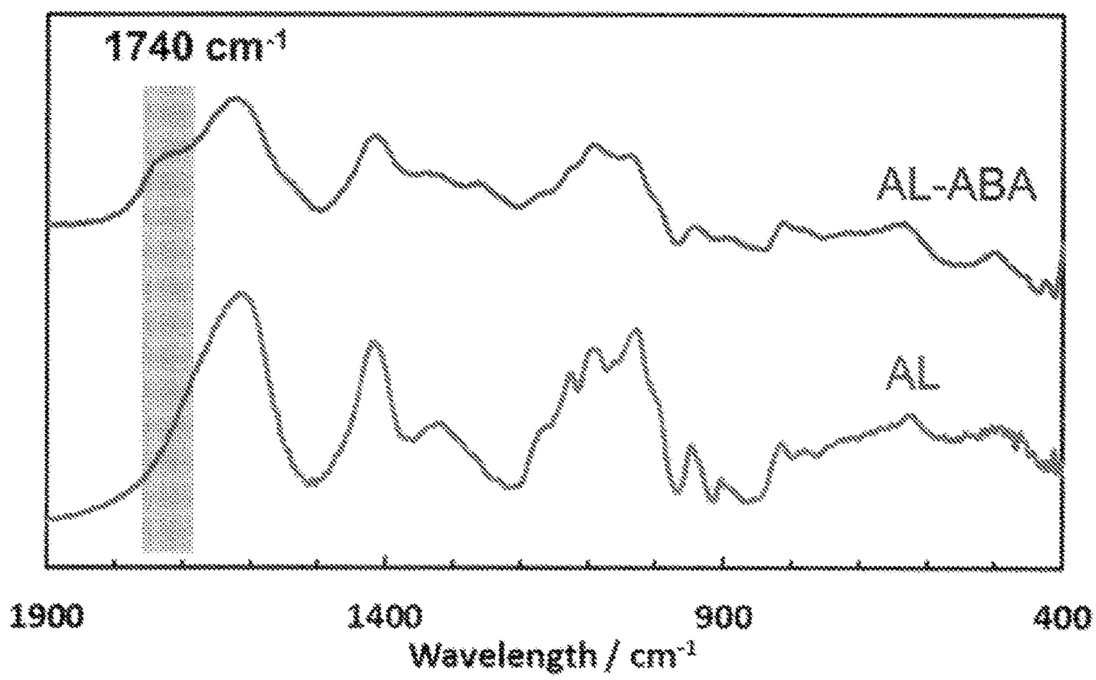
1740 cm-1
AL-ABA
AL
1900
1400
900
400
Wavelength / cm-1

[Fig. 4]

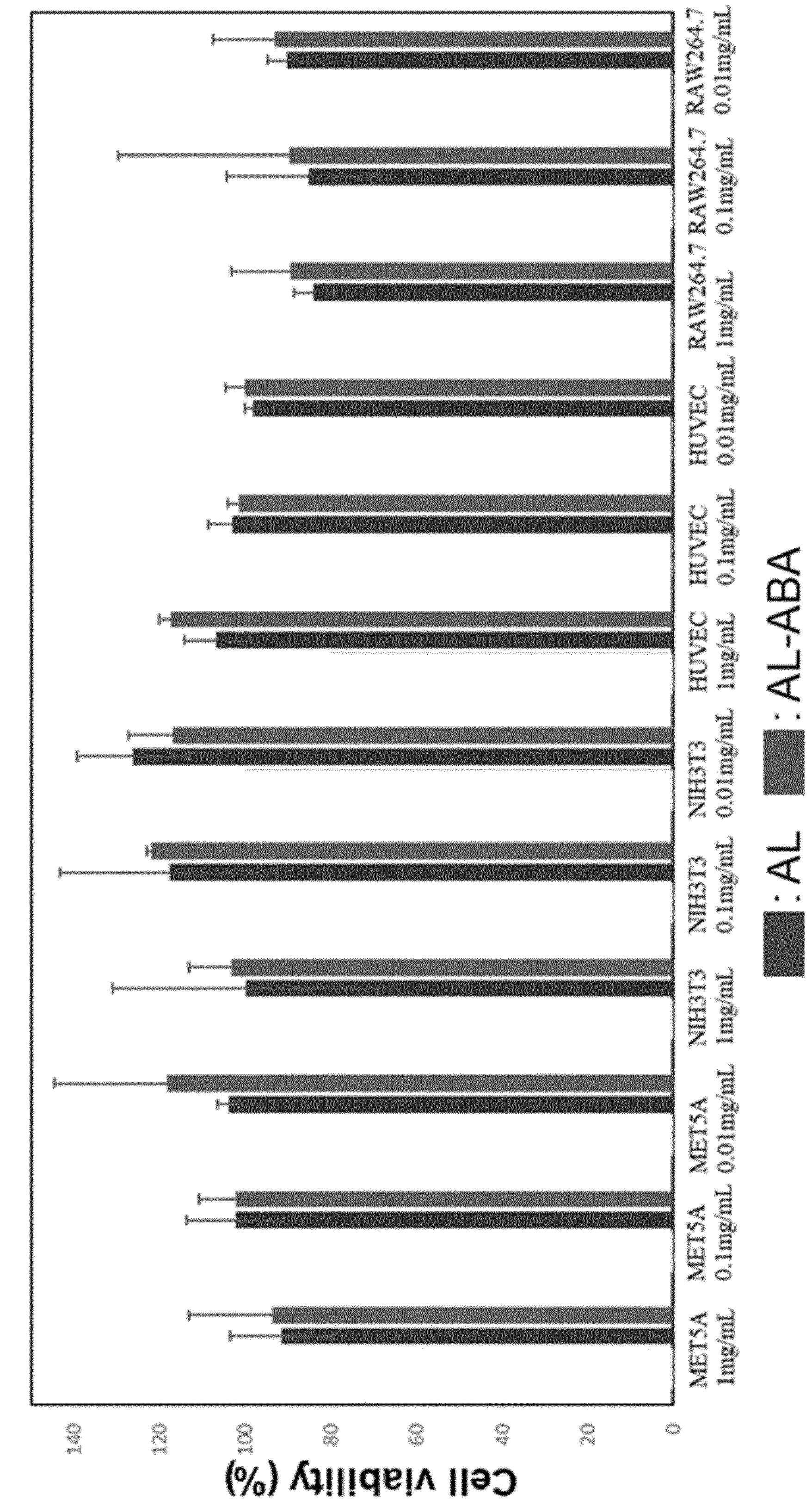
Cell viability (%)
0
20
40
60
80
100
120
140
MET5A 1mg/mL
MET5A 0.1mg/mL
MET5A 0.01mg/mL
NIH3T3 1mg/mL
NIH3T3 0.1mg/mL
NIH3T3 0.01mg/mL
HUVEC 1mg/mL
HUVEC 0.1mg/mL
HUVEC 0.01mg/mL
RAW264.7 1mg/mL
RAW264.7 0.1mg/mL
RAW264.7 0.01mg/mL
: AL
: AL-ABA

[Fig. 5]

AL-AAP

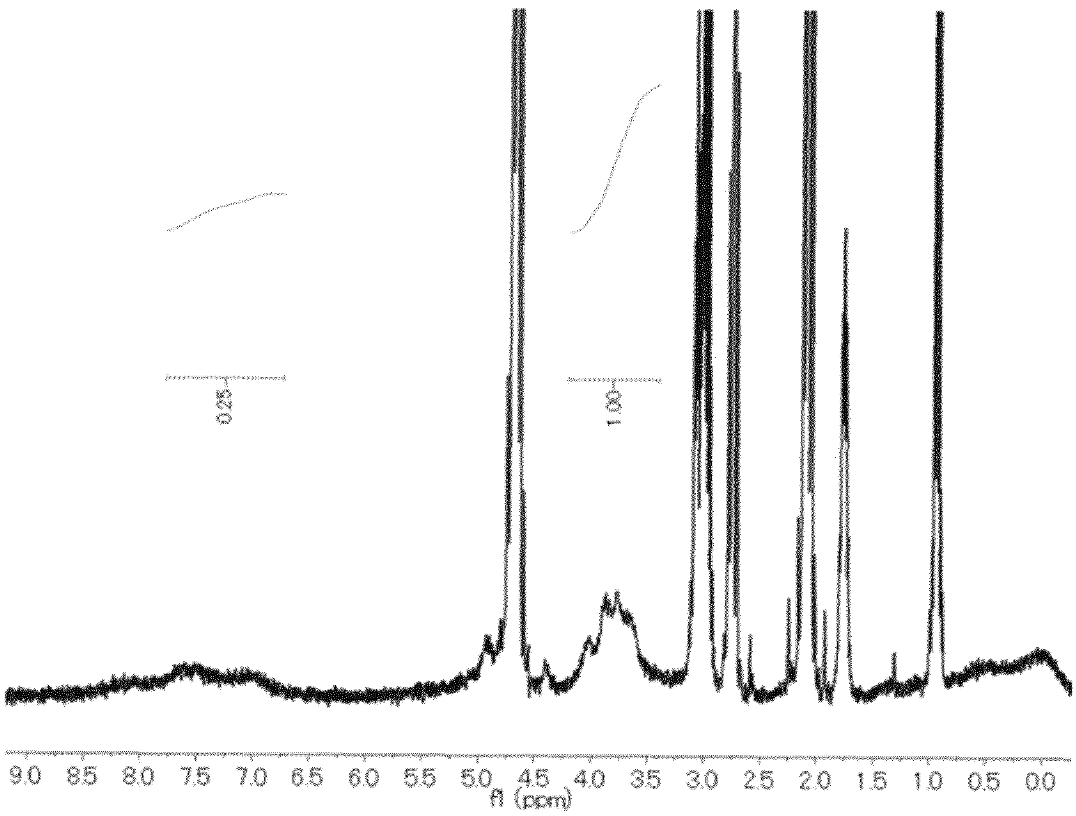


[Fig. 6]

AL-AAP

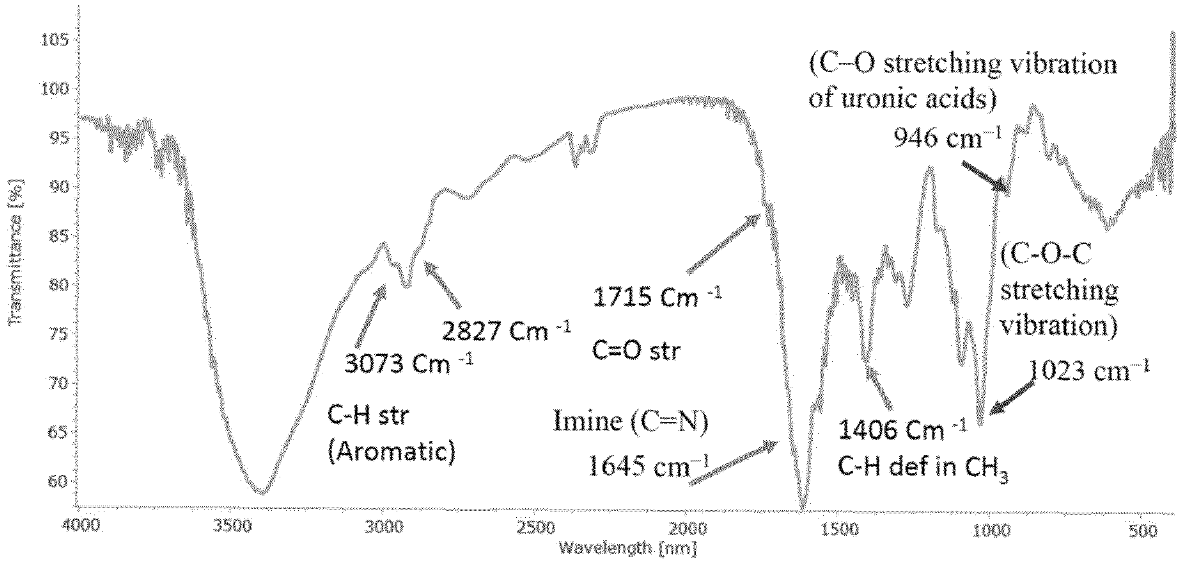

[Fig. 7]

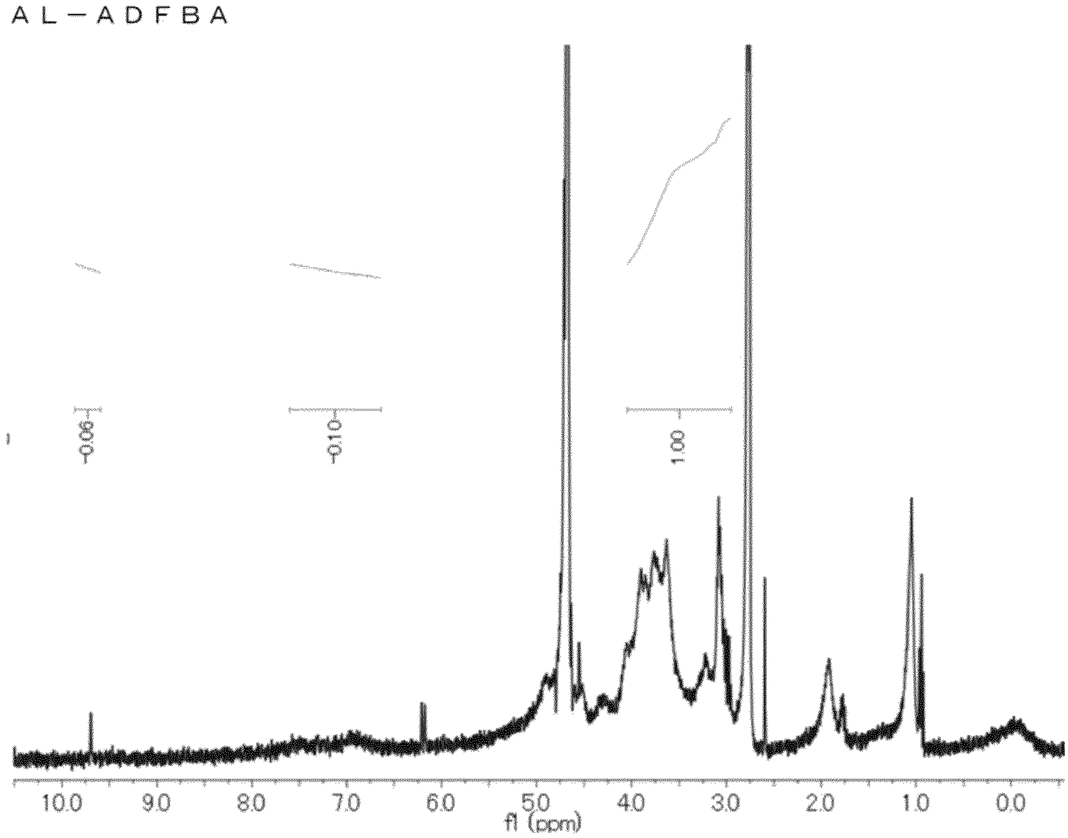
AL-ADFBA
0.06
0.10
1.00
10.0
9.0
8.0
7.0
6.0
5.0
4.0
3.0
2.0
1.0
0.0
f1 (ppm)

[Fig. 8]

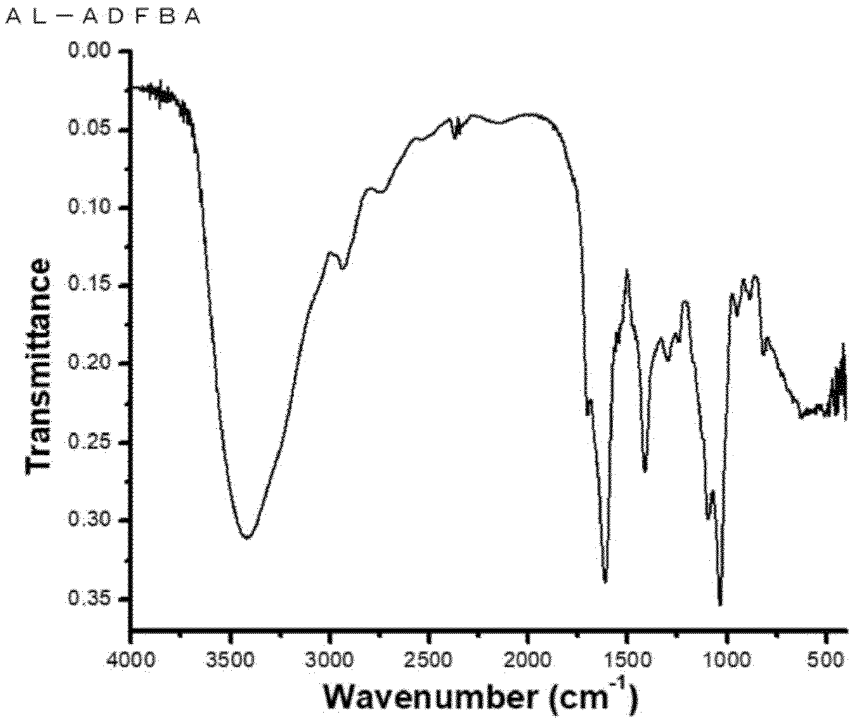
AL-ADFBA
Transmittance
0.00
0.05
0.10
0.15
0.20
0.25
0.30
0.35
4000
3500
3000
2500
2000
1500
1000
500
Wavenumber (cm-1)

[Fig. 9]

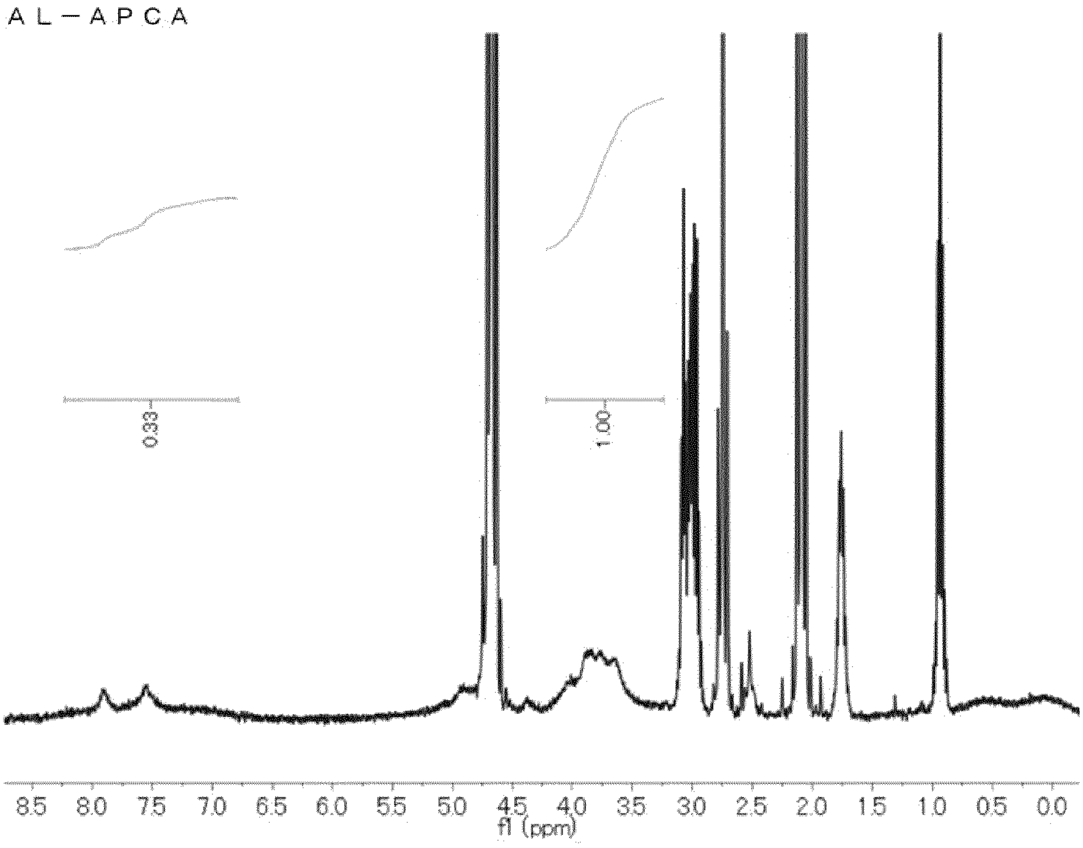
AL-APCA
0.33
1.00
8.5 8.0 7.5 7.0 6.5 6.0 5.5 5.0 4.5 4.0 3.5 3.0 2.5 2.0 1.5 1.0 0.5 0.0
f1 (ppm)

[Fig. 10]

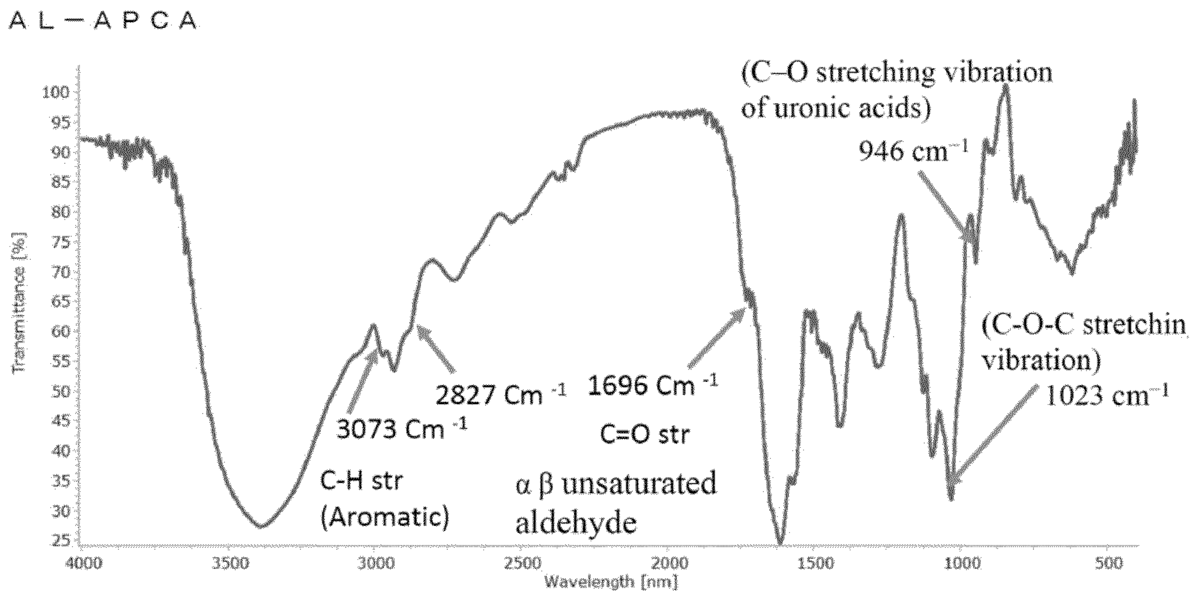
AL-APCA
(C–O stretching vibration of uronic acids)
946 cm⁻¹
(C-O-C stretchin vibration)
1023 cm⁻¹
3073 Cm -1
C-H str (Aromatic)
2827 Cm -1
1696 Cm -1
C=O str
α β unsaturated aldehyde
Transmittance [%]
Wavelength [nm]
4000 3500 3000 2500 2000 1500 1000 500

[Fig. 11]

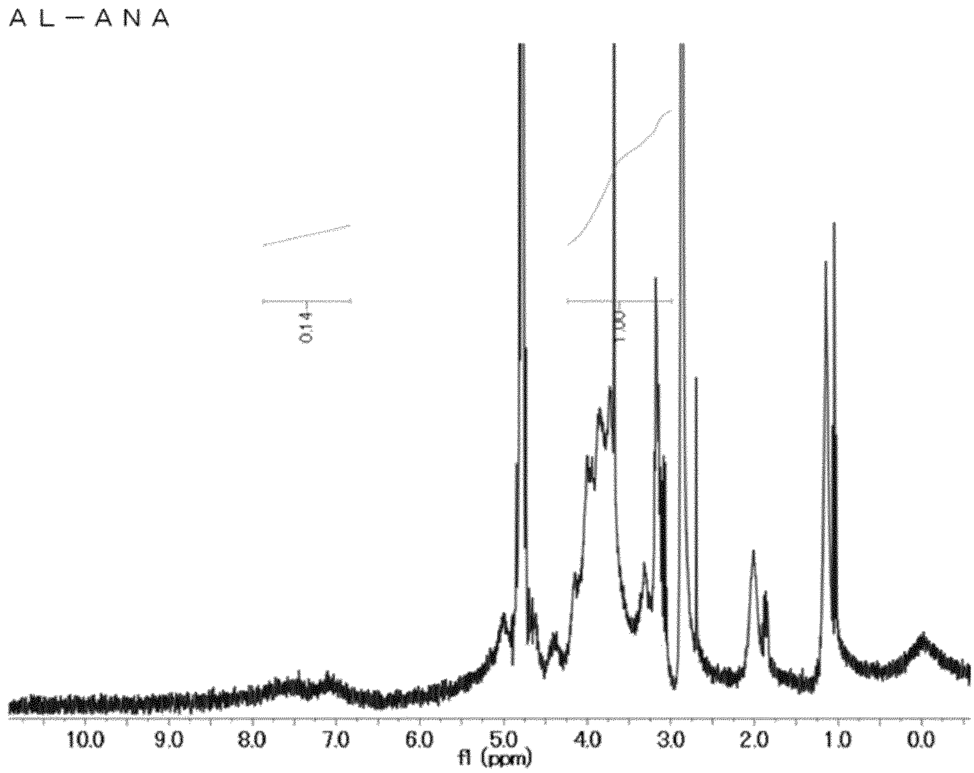
AL-ANA
0.14
1.00
10.0
9.0
8.0
7.0
6.0
5.0
4.0
3.0
2.0
1.0
0.0
f1 (ppm)

[Fig. 12]

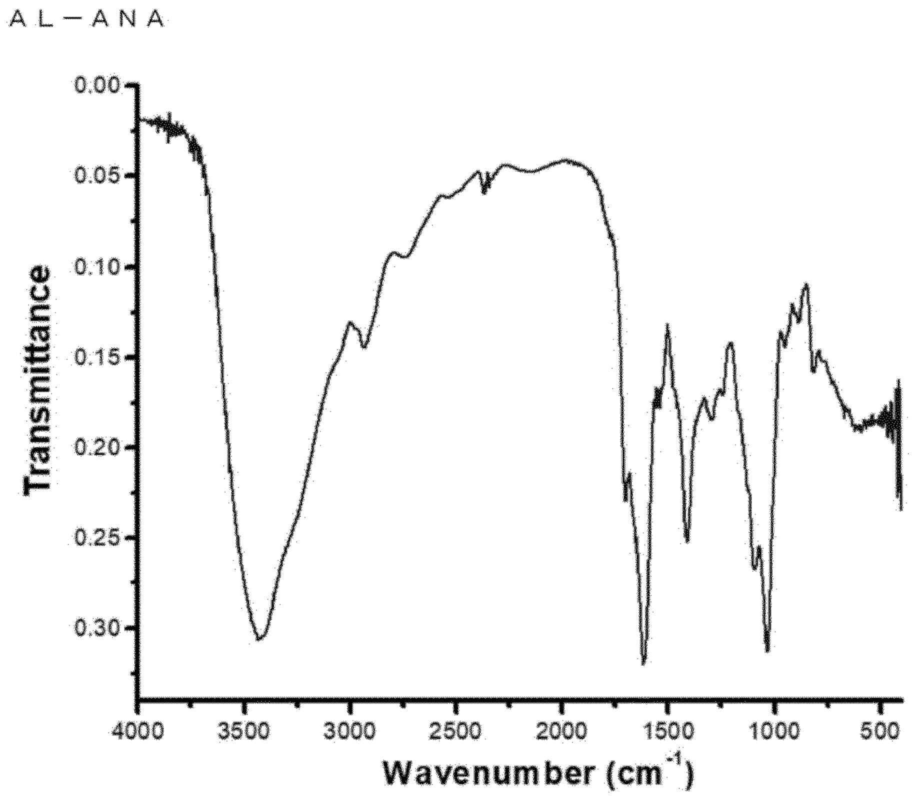
AL-ANA
Transmittance
0.00
0.05
0.10
0.15
0.20
0.25
0.30
4000
3500
3000
2500
2000
1500
1000
500
Wavenumber (cm-1)

[Fig. 13]

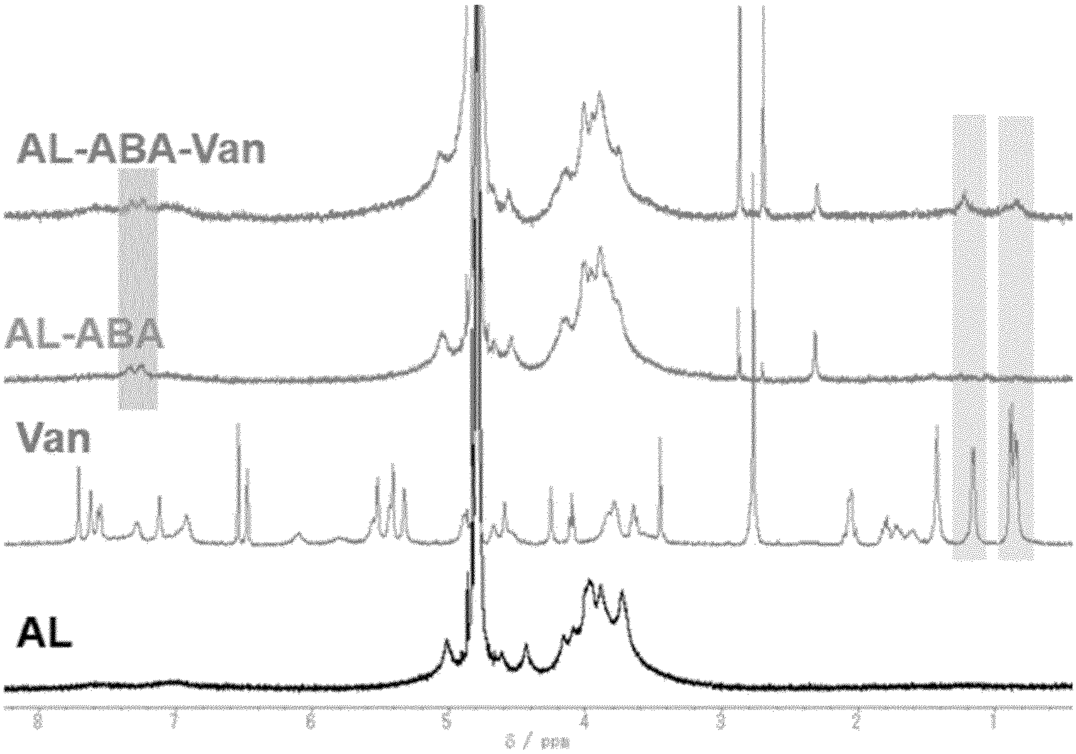
AL-ABA-Van
AL-ABA
Van
AL
8
7
6
5
4
3
2
1
δ / ppm

[Fig. 14]

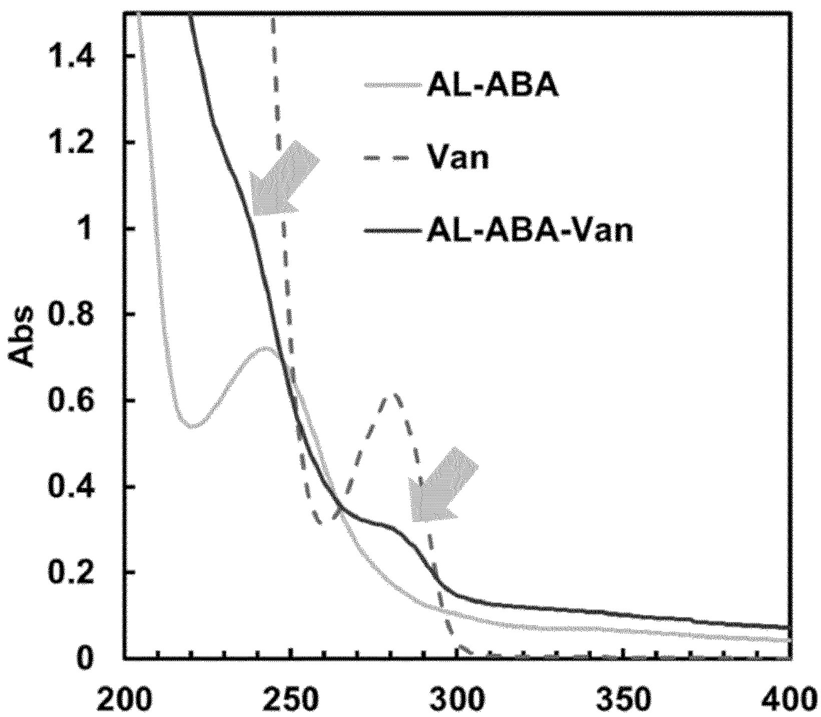
AL-ABA
Van
AL-ABA-Van
Abs
1.4
1.2
1
0.8
0.6
0.4
0.2
0
200
250
300
350
400
Wavelength / nm

[Fig. 15]

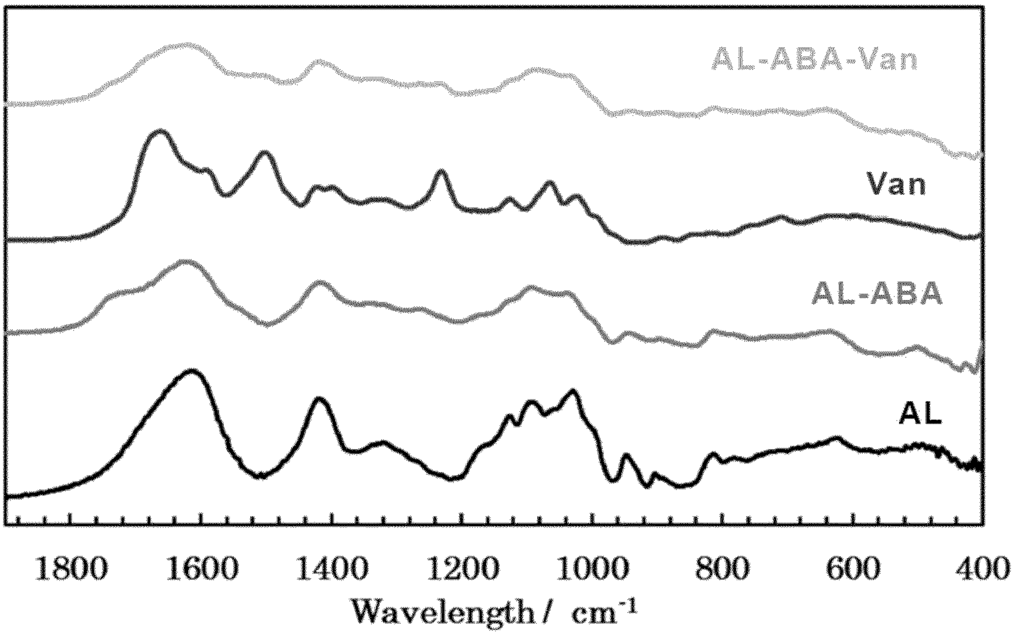


[Fig. 16A]

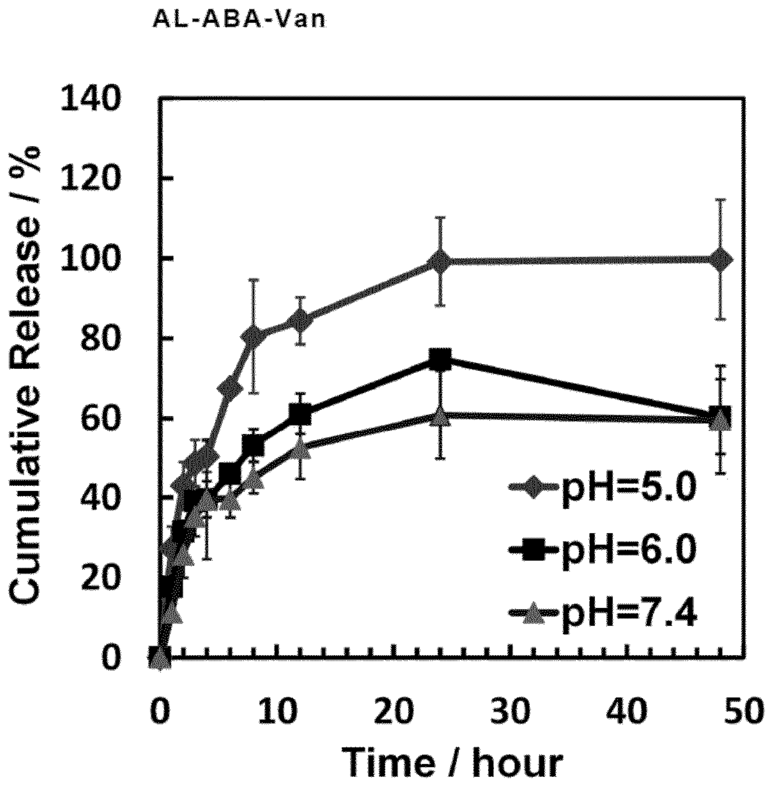

[Fig. 16B]

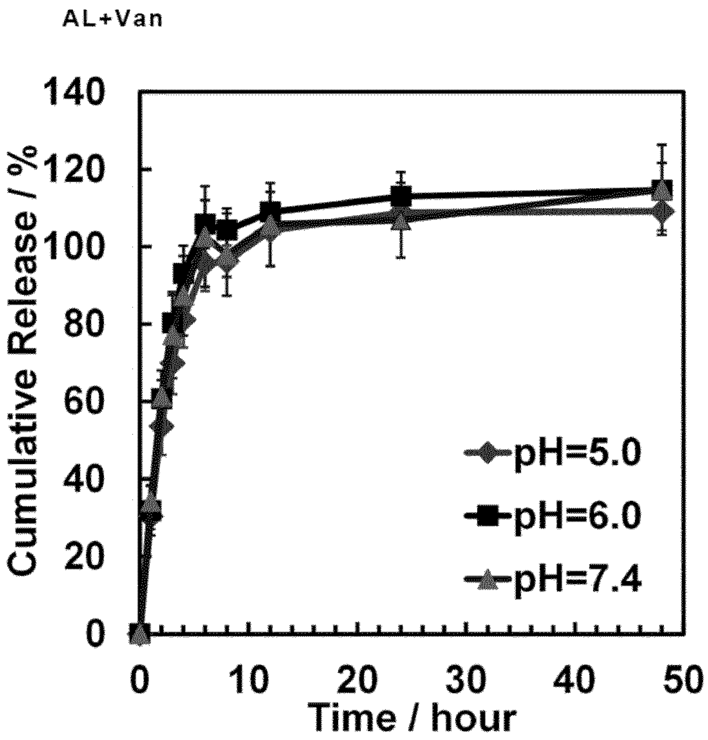


[Fig. 16C]

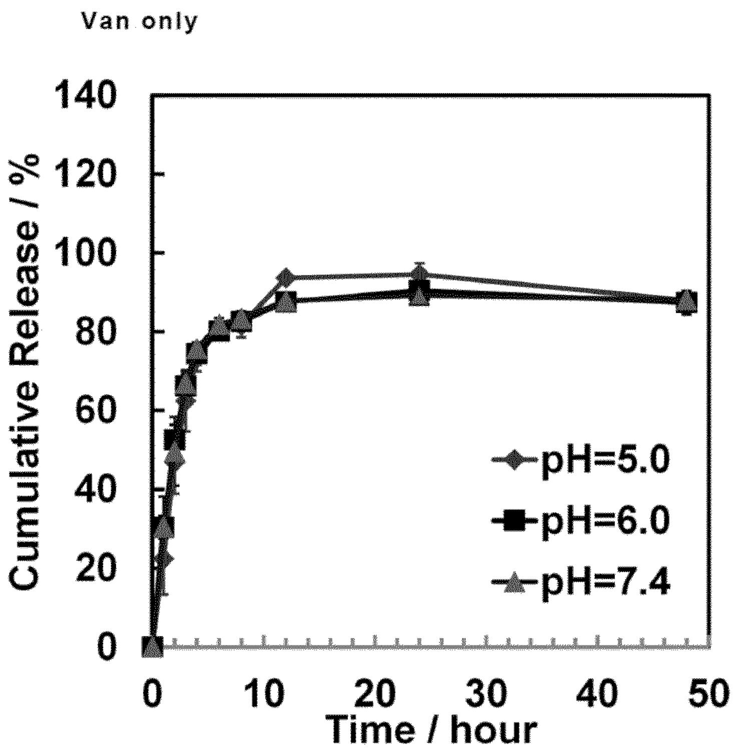

[Fig. 17]

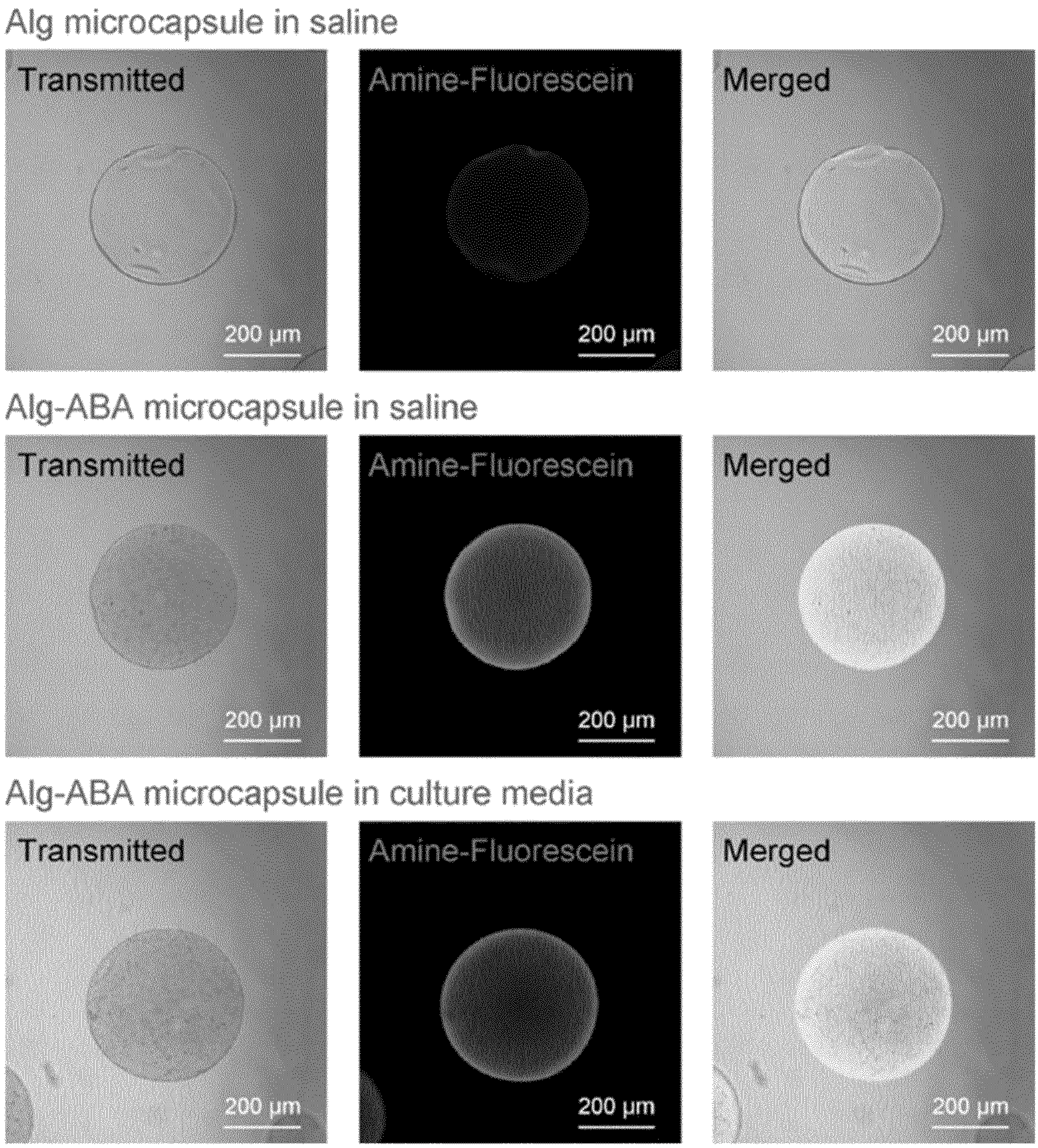
Alg microcapsule in saline
Transmitted
Amine-Fluorescein
Merged
200 μm
Alg-ABA microcapsule in saline
Transmitted
Amine-Fluorescein
Merged
200 μm
Alg-ABA microcapsule in culture media
Transmitted
Amine-Fluorescein
Merged
200 μm

[Fig. 18]

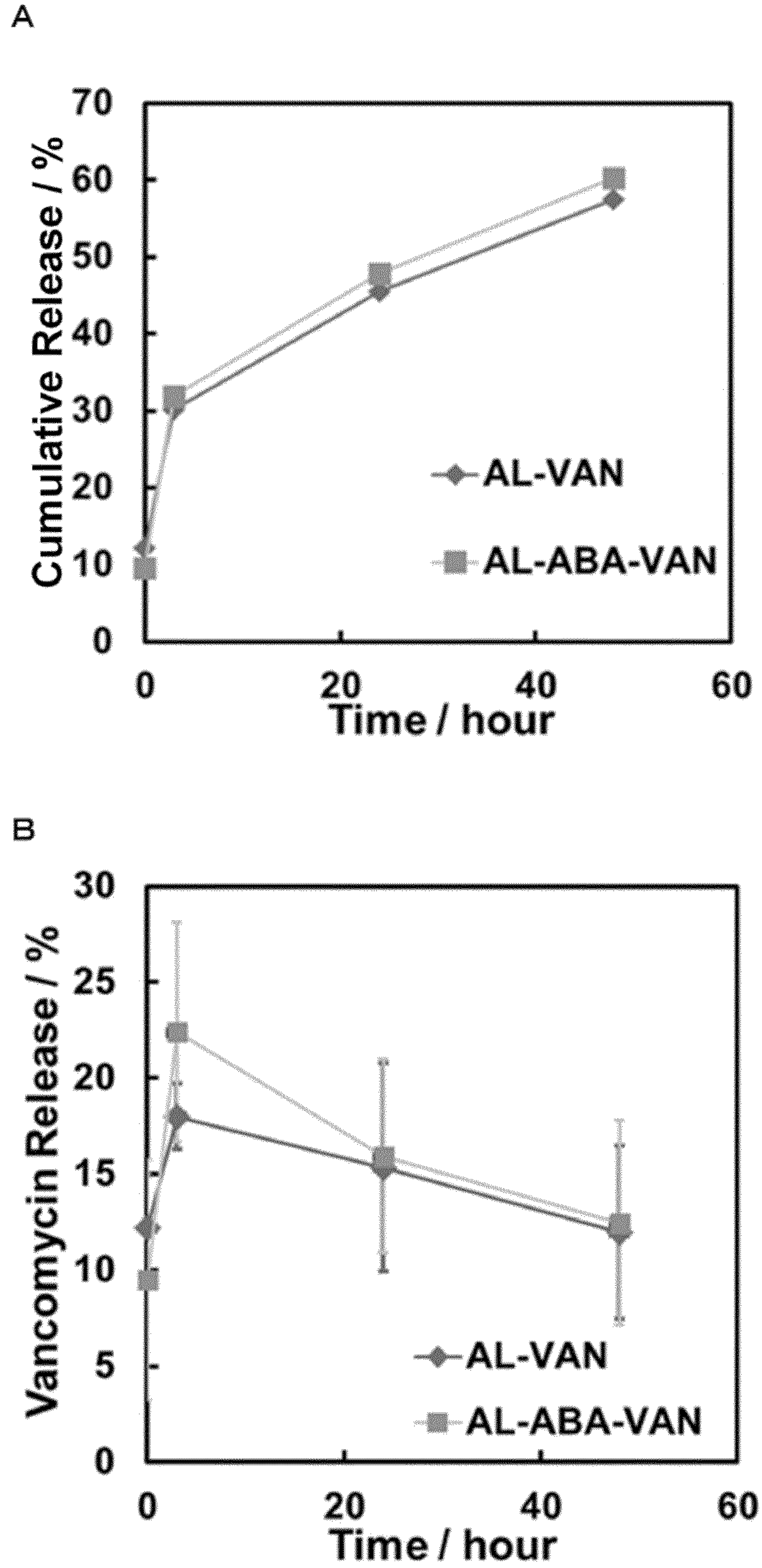
A
Cumulative Release / %
70
60
50
40
30
20
10
0
AL-VAN
AL-ABA-VAN
0
20
40
60
Time / hour
B
Vancomycin Release / %
30
25
20
15
10
5
0
AL-VAN
AL-ABA-VAN
0
20
40
60
Time / hour

[Fig. 19]

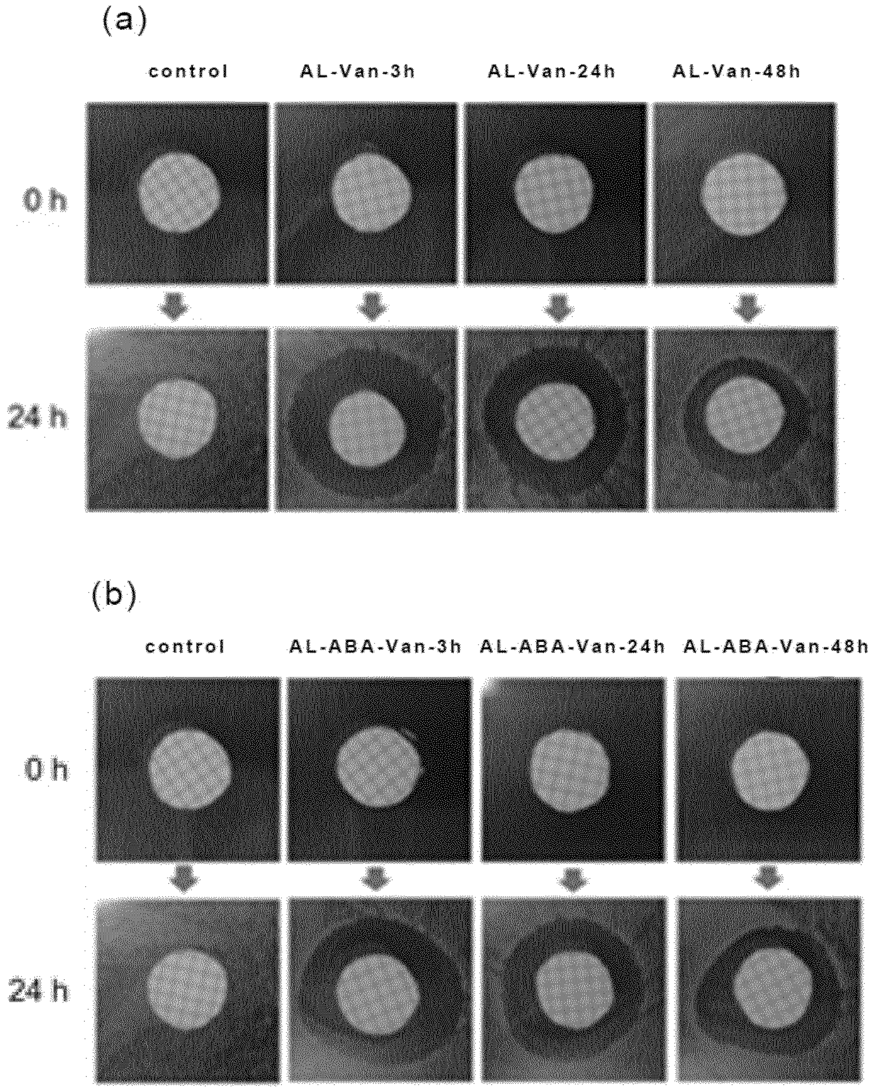
(a)
control
AL-Van-3h
AL-Van-24h
AL-Van-48h
0 h
24 h
(b)
control
AL-ABA-Van-3h
AL-ABA-Van-24h
AL-ABA-Van-48h
0 h
24 h

[Fig. 20]

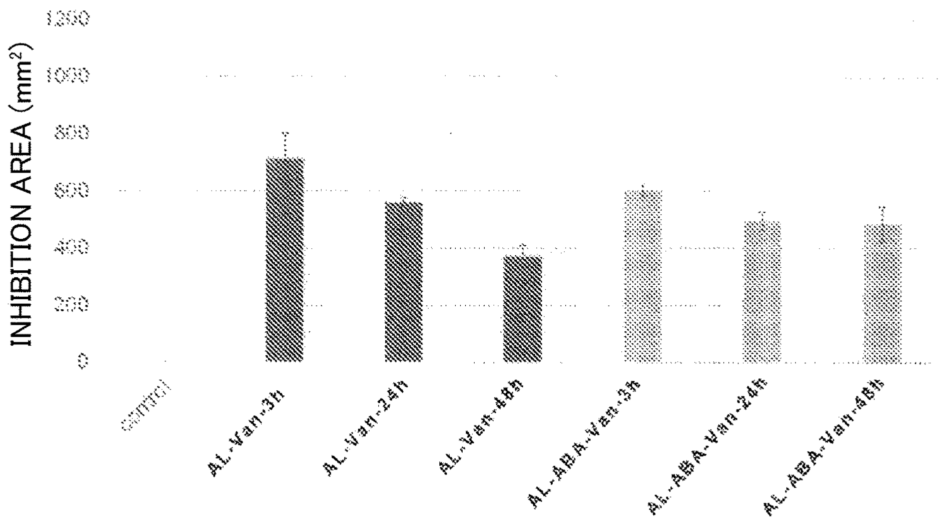
INHIBITION AREA (mm²)
1200
1000
800
600
400
200
0
control
AL-Van-3h
AL-Van-24h
AL-Van-48h
AL-ABA-Van-3h
AL-ABA-Van-24h
AL-ABA-Van-48h

[Fig. 21]

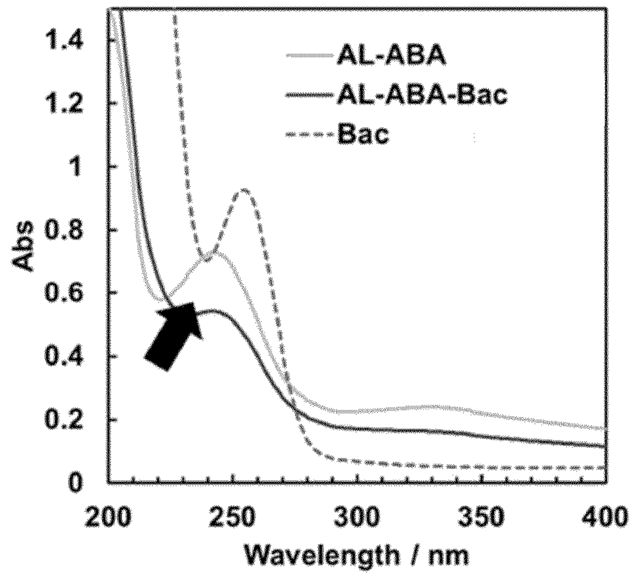
AL-ABA
AL-ABA-Bac
Bac
Abs
1.4
1.2
1
0.8
0.6
0.4
0.2
0
200
250
300
350
400
Wavelength / nm

[Fig. 22]

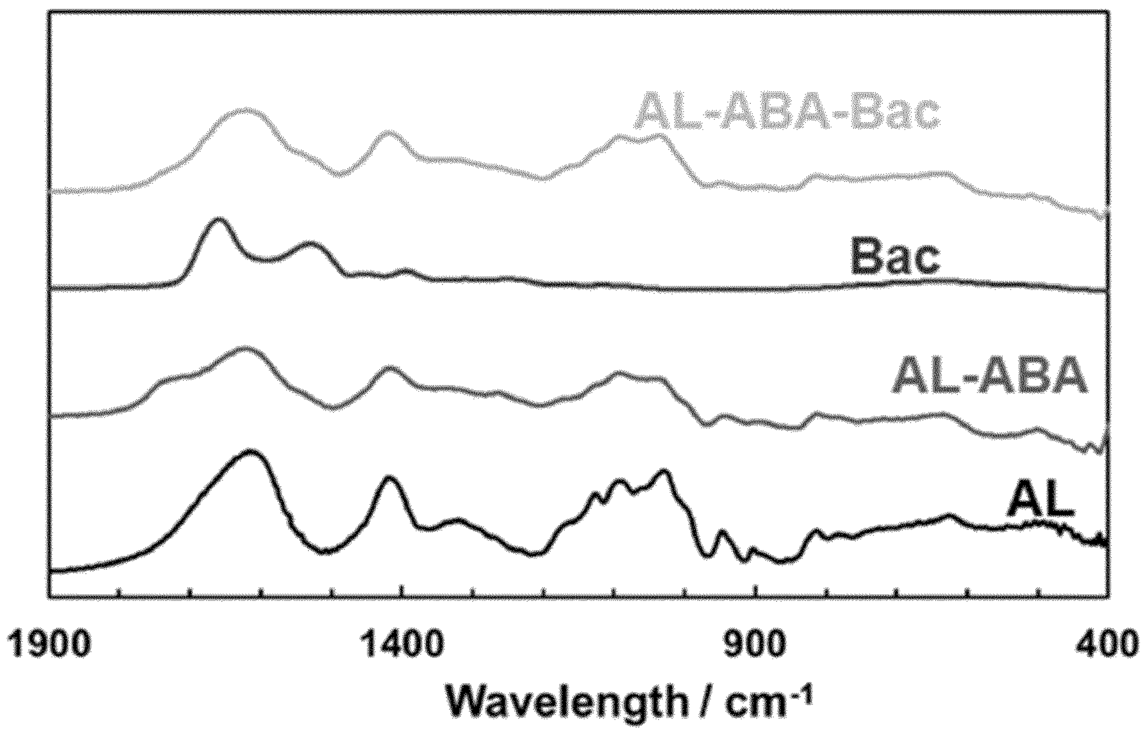
AL-ABA-Bac
Bac
AL-ABA
AL
1900
1400
900
400
Wavelength / cm-1

[Fig. 23]

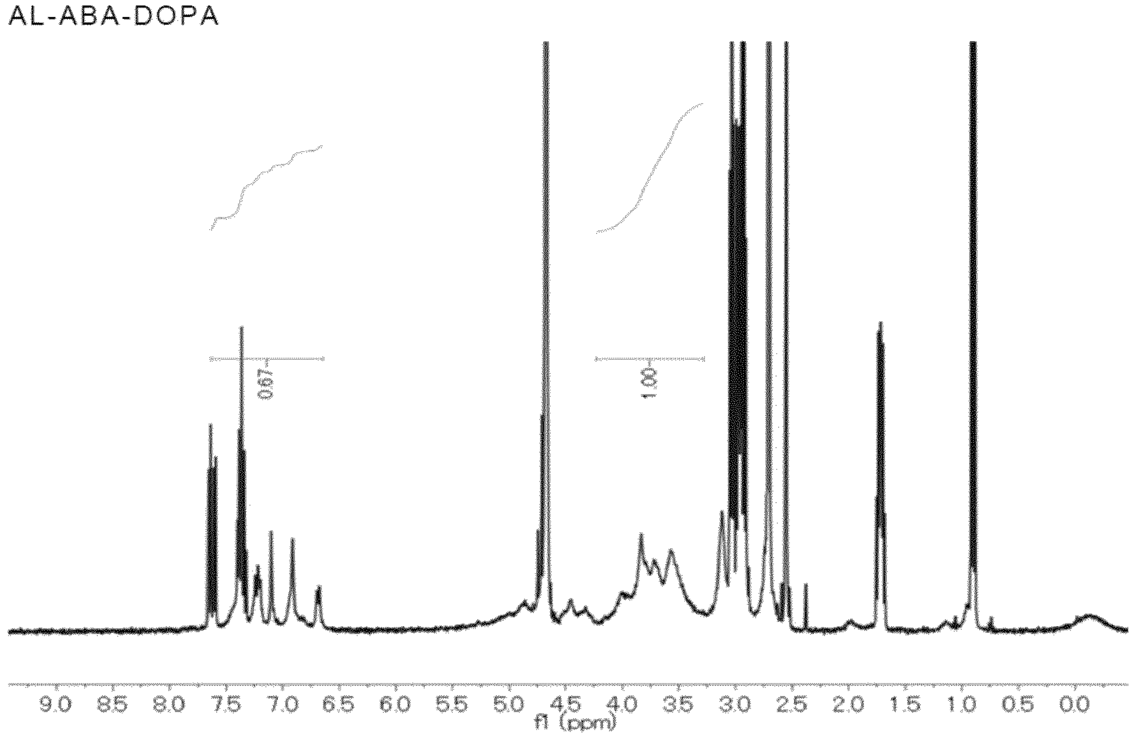
AL-ABA-DOPA
0.67
1.00
9.0 8.5 8.0 7.5 7.0 6.5 6.0 5.5 5.0 4.5 4.0 3.5 3.0 2.5 2.0 1.5 1.0 0.5 0.0
f1 (ppm)

[Fig. 24]

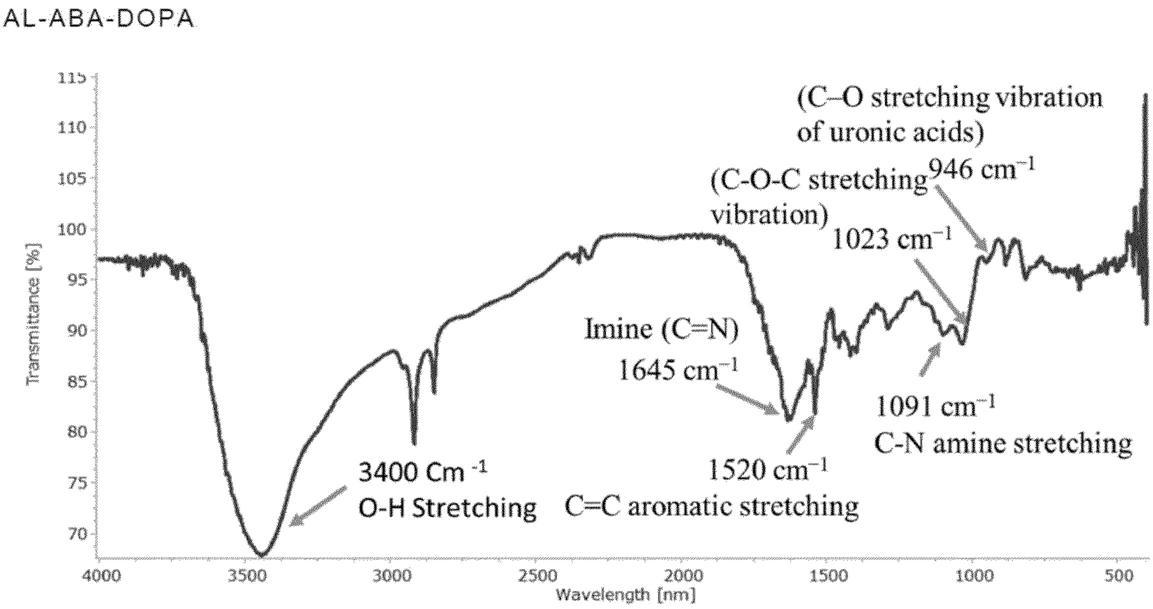
AL-ABA-DOPA
(C–O stretching vibration of uronic acids)
946 cm⁻¹
(C-O-C stretching vibration)
1023 cm⁻¹
Imine (C=N)
1645 cm⁻¹
1091 cm⁻¹
C-N amine stretching
1520 cm⁻¹
C=C aromatic stretching
3400 Cm ⁻¹
O-H Stretching
Transmittance [%]
Wavelength [nm]
115 110 105 100 95 90 85 80 75 70
4000 3500 3000 2500 2000 1500 1000 500

[Fig. 25]

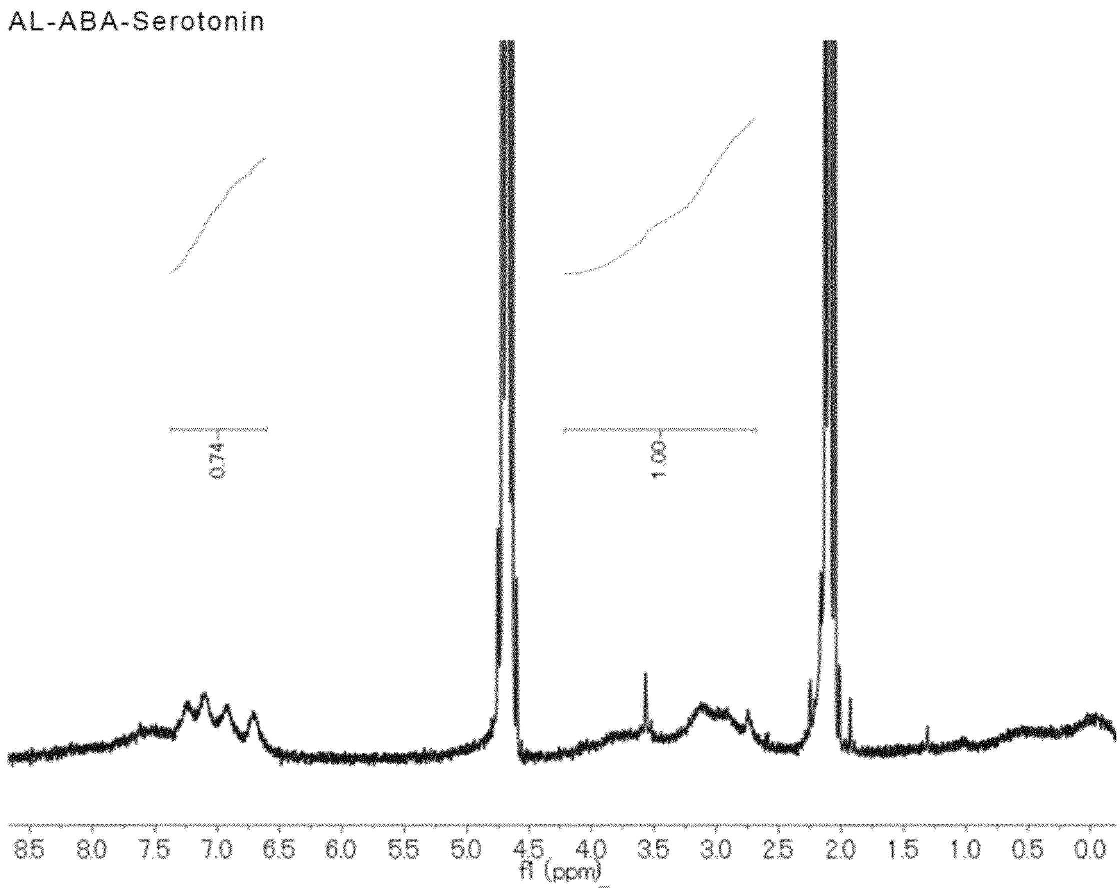
AL-ABA-Serotonin
0.74
1.00
8.5 8.0 7.5 7.0 6.5 6.0 5.5 5.0 4.5 4.0 3.5 3.0 2.5 2.0 1.5 1.0 0.5 0.0
f1 (ppm)

[Fig. 26]

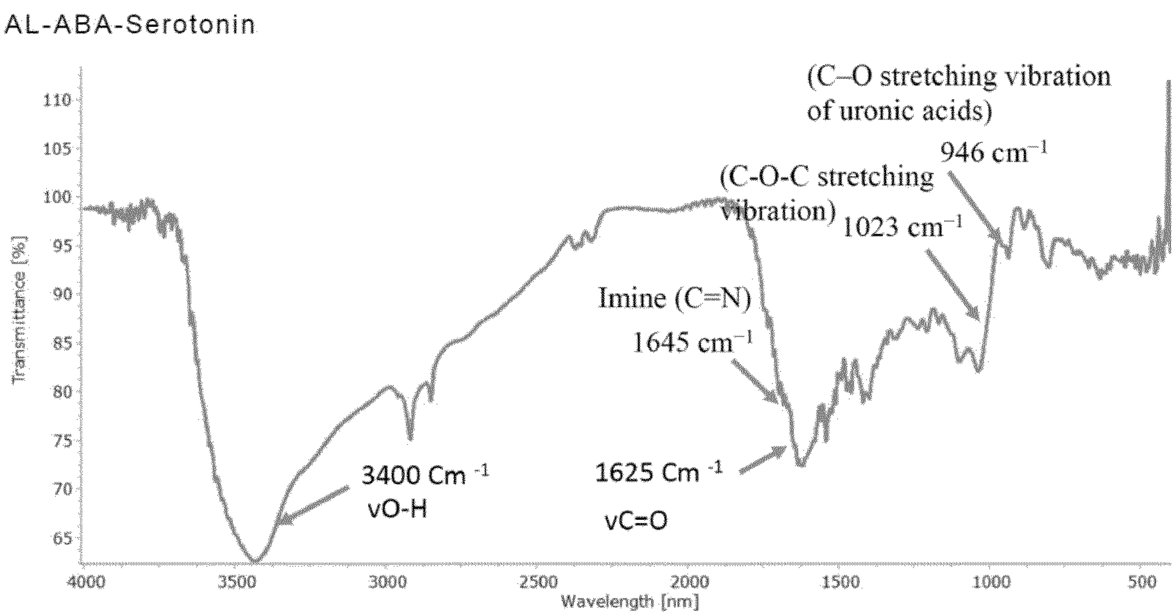
AL-ABA-Serotonin
(C–O stretching vibration of uronic acids)
946 cm$^{-1}$
(C-O-C stretching vibration)
1023 cm$^{-1}$
Imine (C=N)
1645 cm$^{-1}$
3400 Cm $^{-1}$
vO-H
1625 Cm $^{-1}$
vC=O
Transmittance [%]
Wavelength [nm]

[Fig. 27]

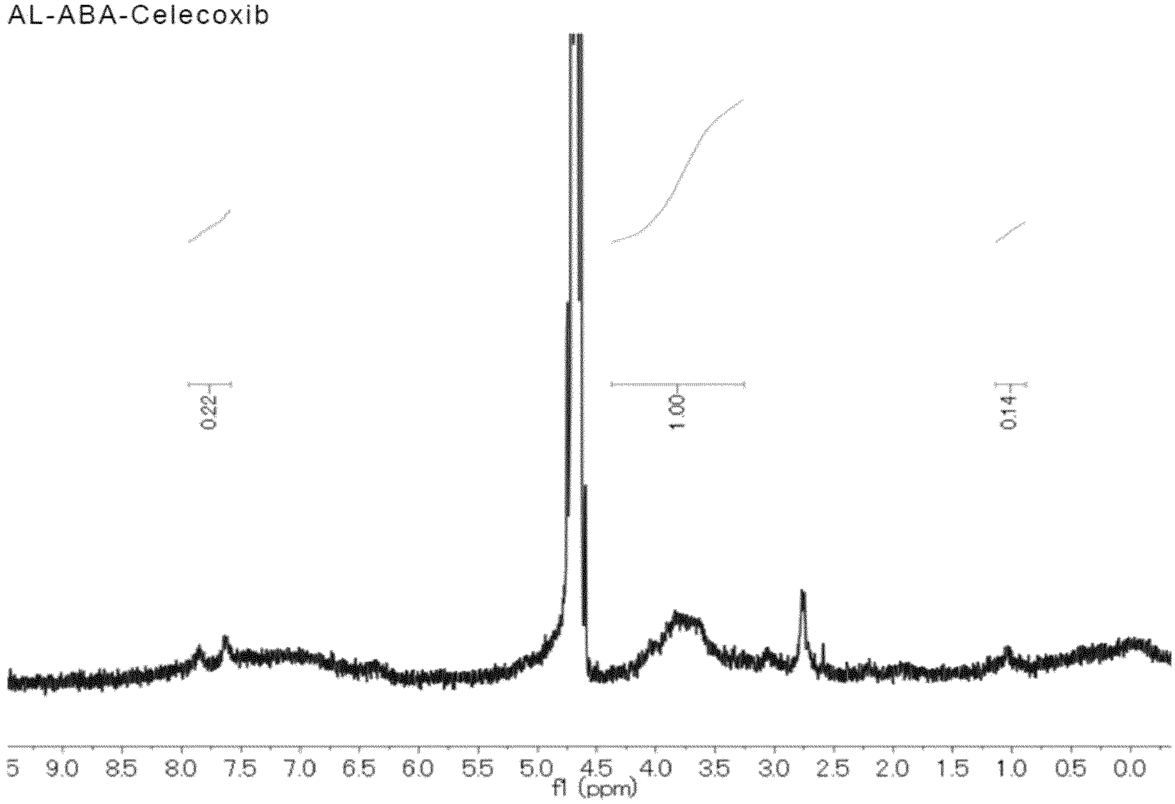
AL-ABA-Celecoxib
0.22
1.00
0.14
5 9.0 8.5 8.0 7.5 7.0 6.5 6.0 5.5 5.0 4.5 4.0 3.5 3.0 2.5 2.0 1.5 1.0 0.5 0.0
f1 (ppm)

[Fig. 28]

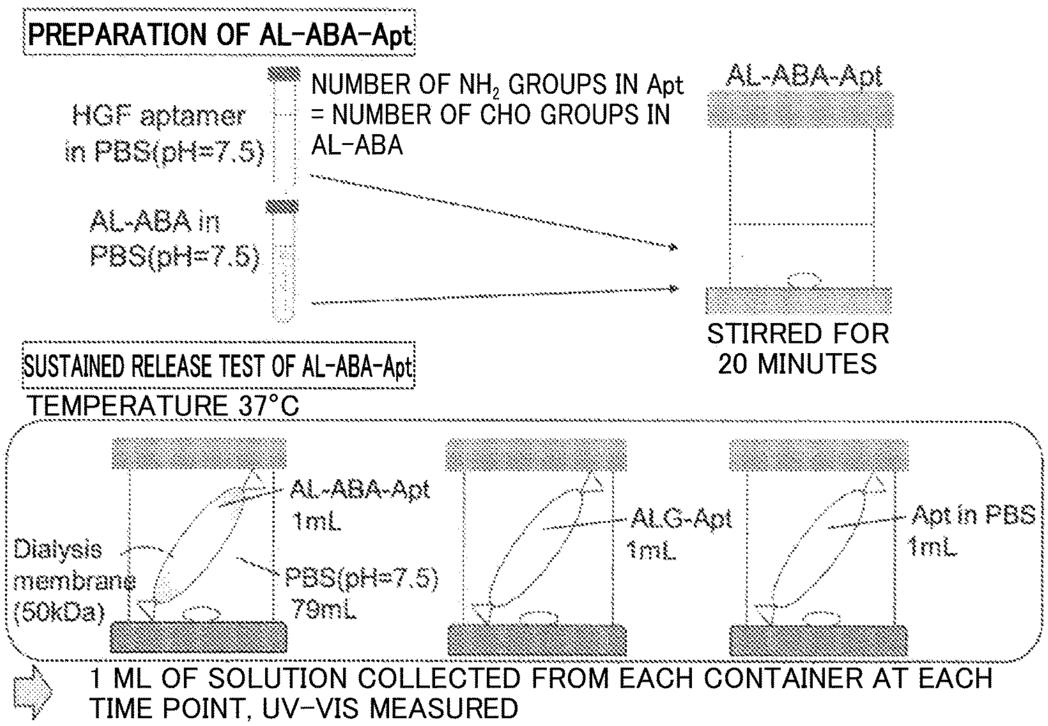
PREPARATION OF AL-ABA-Apt
HGF aptamer in PBS(pH=7.5)
NUMBER OF NH2 GROUPS IN Apt = NUMBER OF CHO GROUPS IN AL-ABA
AL-ABA in PBS(pH=7.5)
AL-ABA-Apt
STIRRED FOR 20 MINUTES
SUSTAINED RELEASE TEST OF AL-ABA-Apt
TEMPERATURE 37°C
AL-ABA-Apt 1mL
Dialysis membrane (50kDa)
PBS(pH=7.5) 79mL
ALG-Apt 1mL
Apt in PBS 1mL
1 ML OF SOLUTION COLLECTED FROM EACH CONTAINER AT EACH TIME POINT, UV-VIS MEASURED

[Fig. 29]

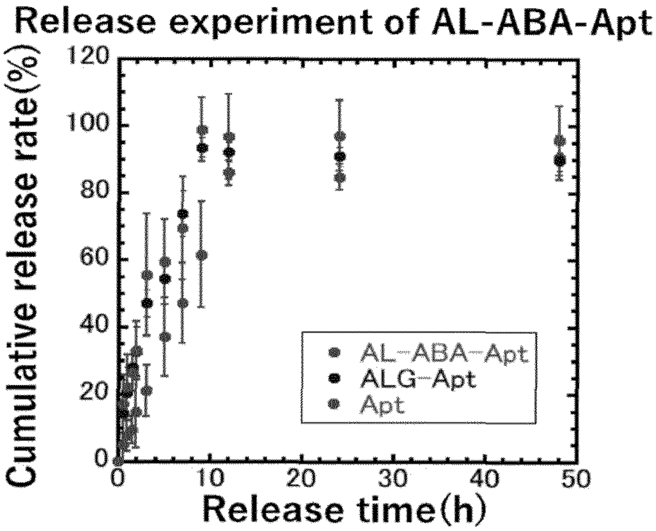


[Fig. 30]

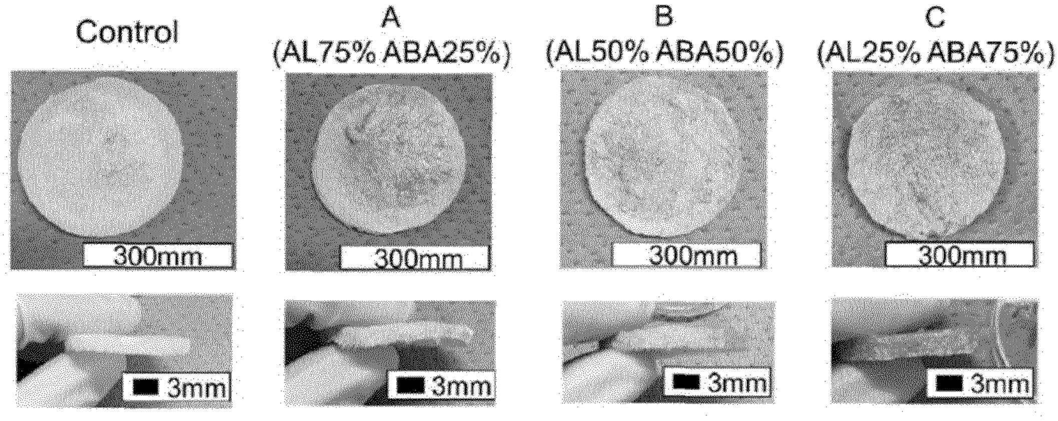


[Fig. 31]

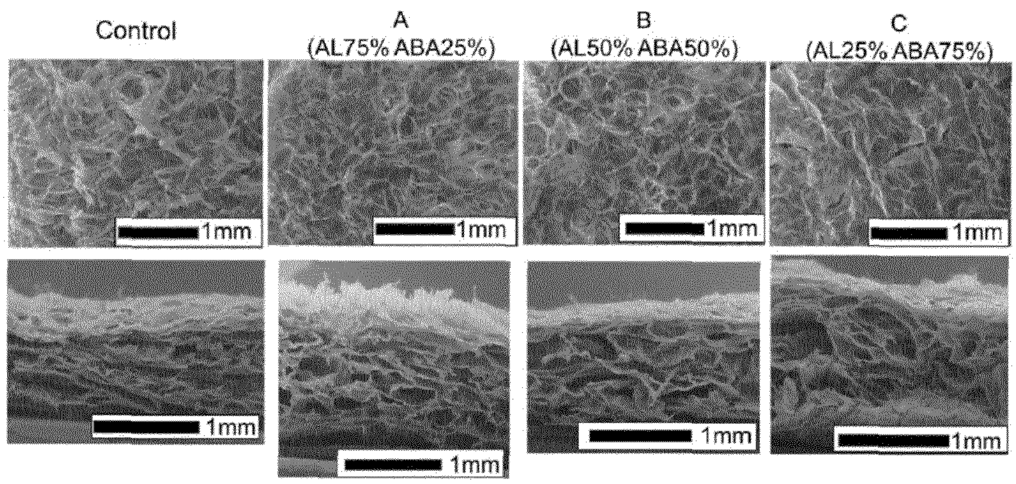

[Fig. 32]

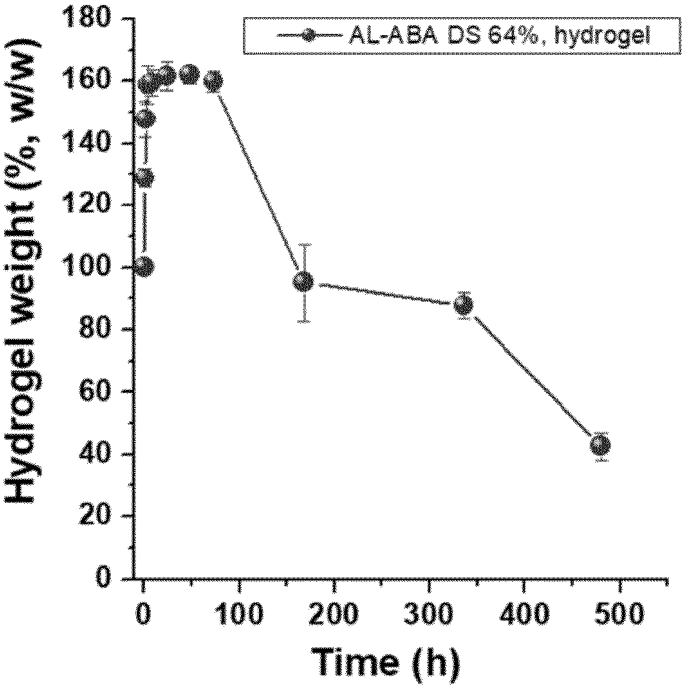
AL-ABA DS 64%, hydrogel
Hydrogel weight (%, w/w)
180
160
140
120
100
80
60
40
20
0
0
100
200
300
400
500
Time (h)

[Fig. 33]

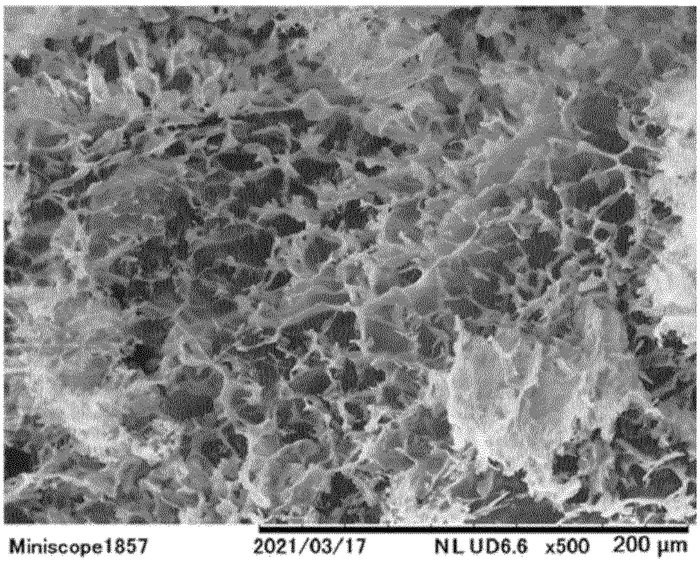
Miniscope1857
2021/03/17
NL UD6.6 x500 200 µm

[Fig. 34]

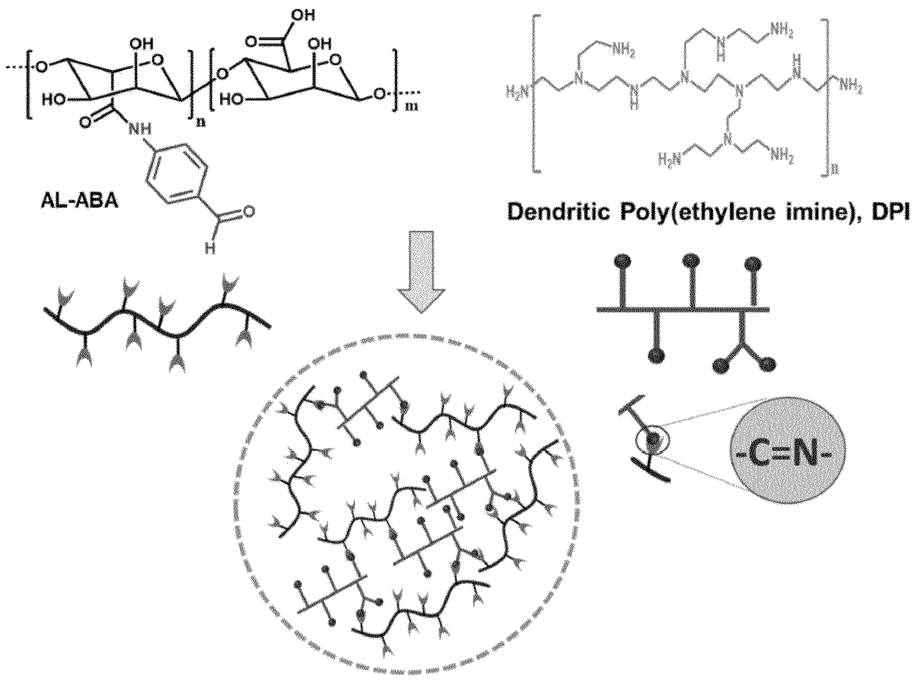
AL-ABA
Dendritic Poly(ethylene imine), DPI


[Fig. 35]

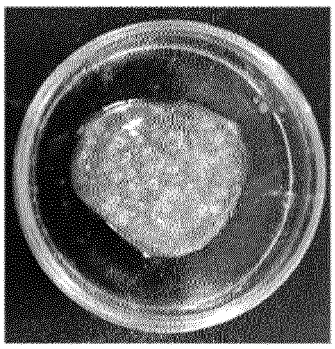

[Fig. 36A]

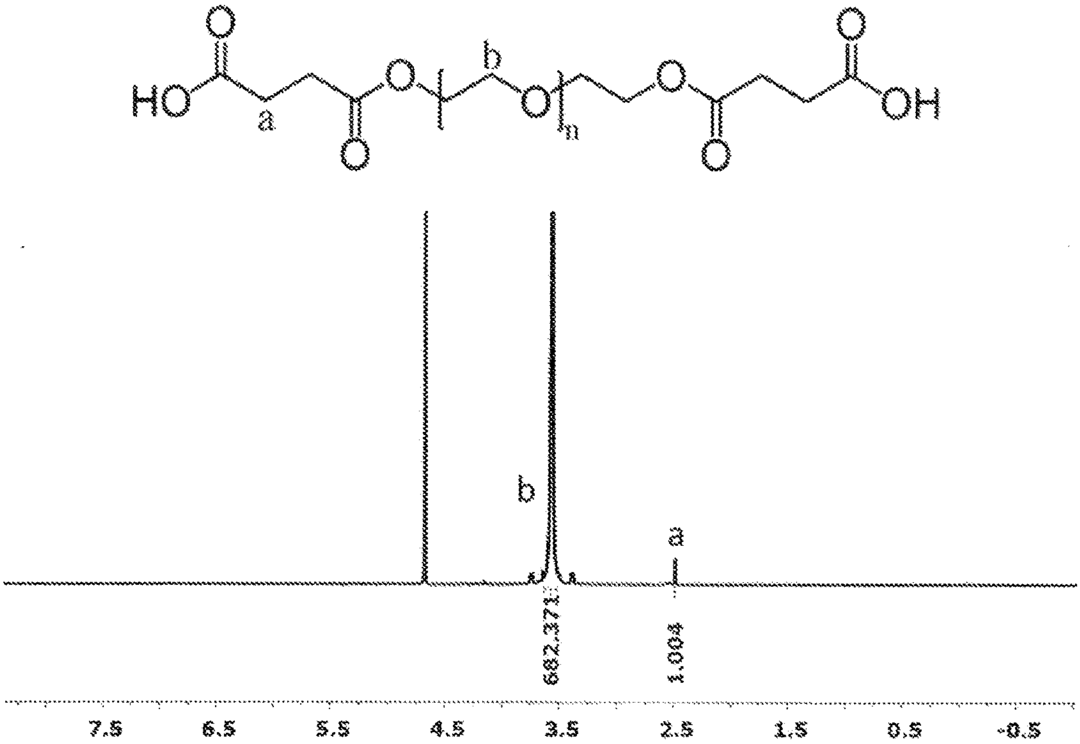


[Fig. 36B]

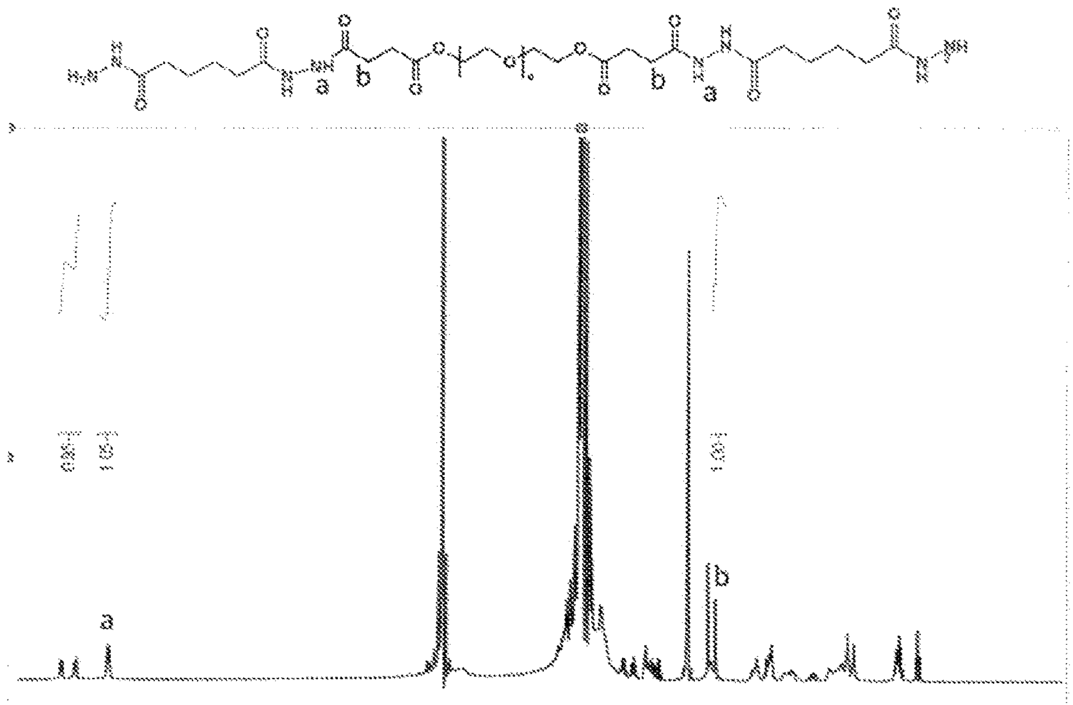

[Fig. 36C]

AL-ABA-PEG dihydrazide gel

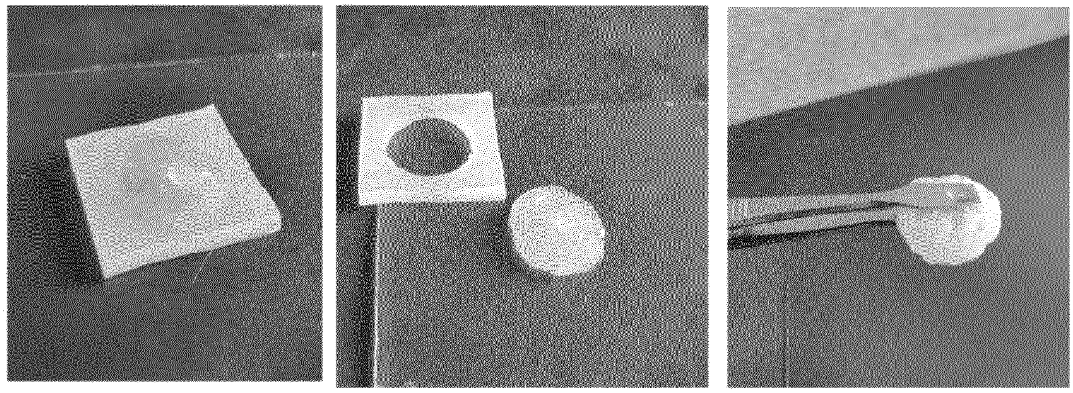

[Fig. 37]

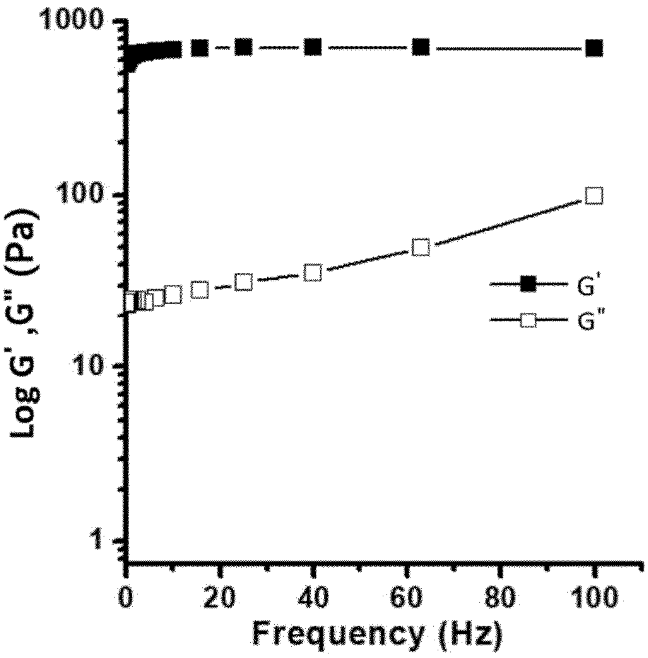


[Fig. 38]

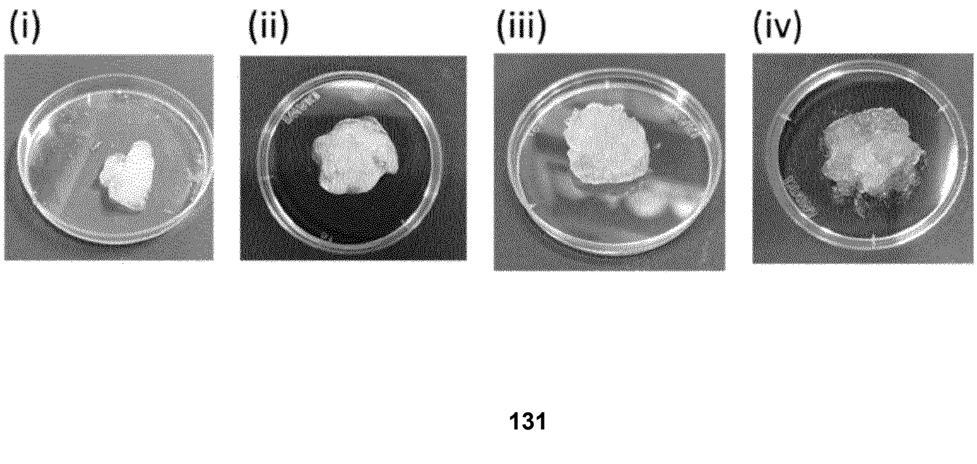

[Fig. 39]

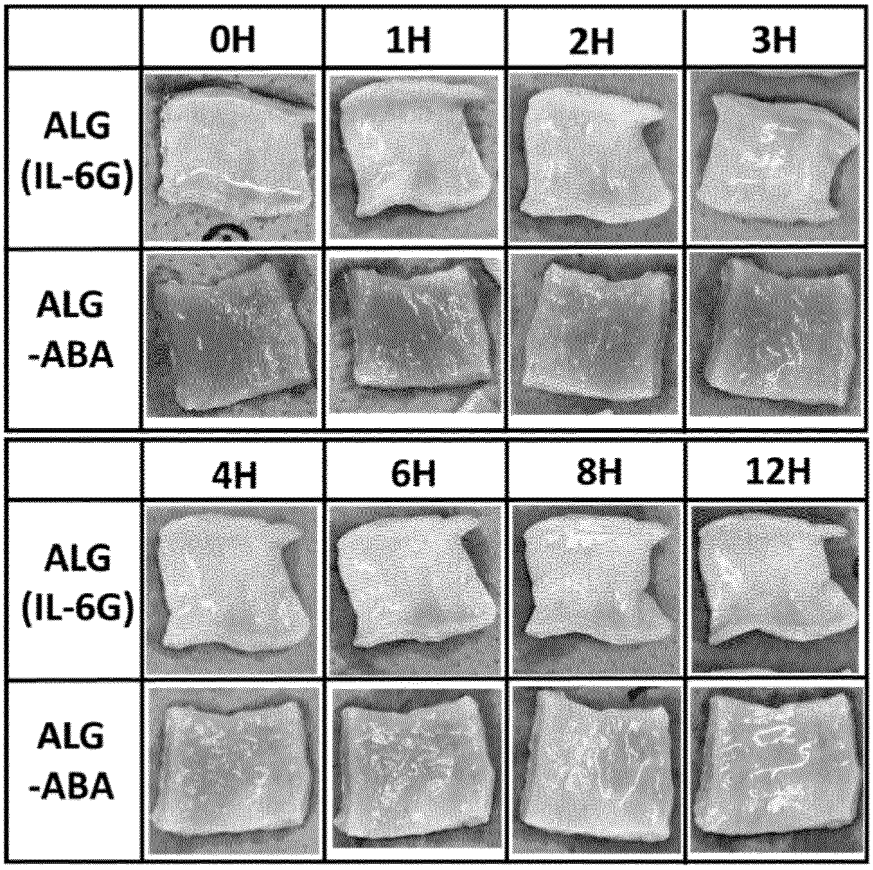
0H
1H
2H
3H
ALG (IL-6G)
ALG -ABA
4H
6H
8H
12H
ALG (IL-6G)
ALG -ABA

[Fig. 40]

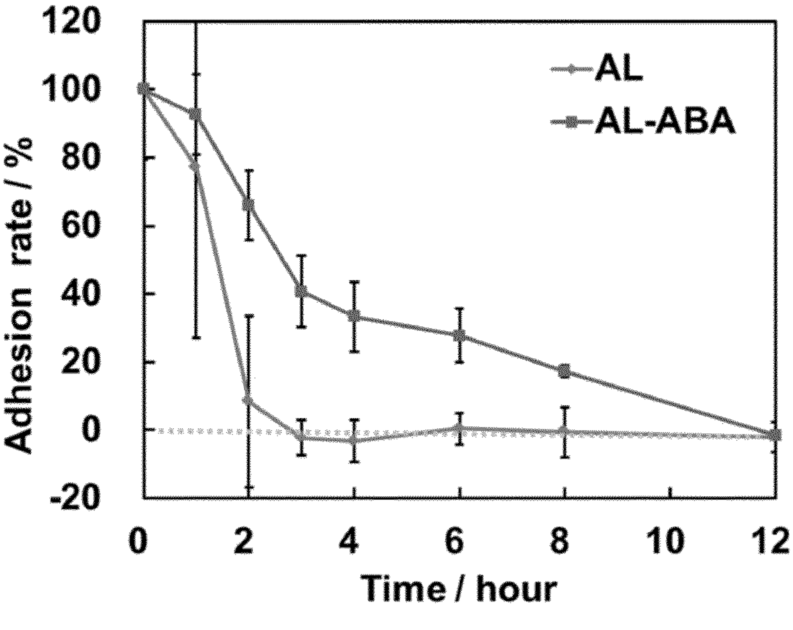
AL
AL-ABA
Adhesion rate / %
Time / hour
120
100
80
60
40
20
0
-20
0
2
4
6
8
10
12

[Fig. 41]

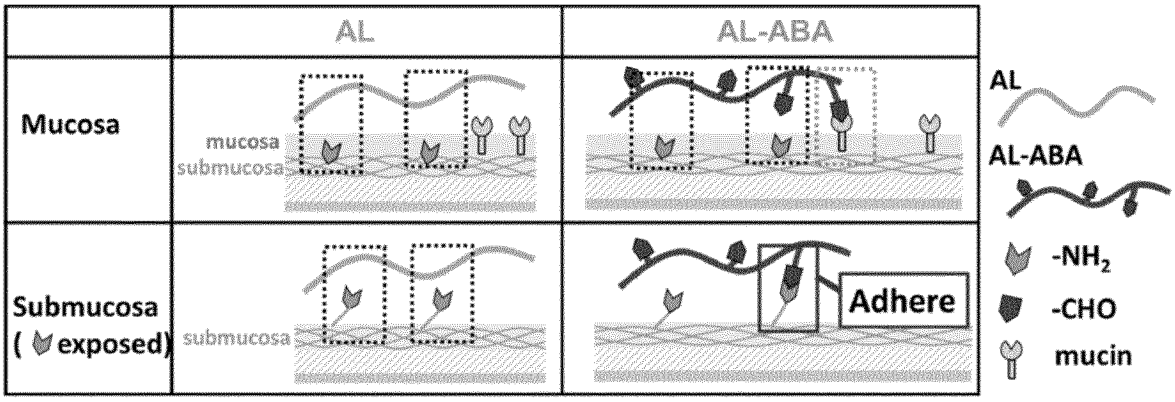
AL
AL-ABA
Mucosa
mucosa
submucosa
Submucosa
( exposed)
submucosa
Adhere
AL
AL-ABA
-NH$_2$
-CHO
mucin

[Fig. 42]

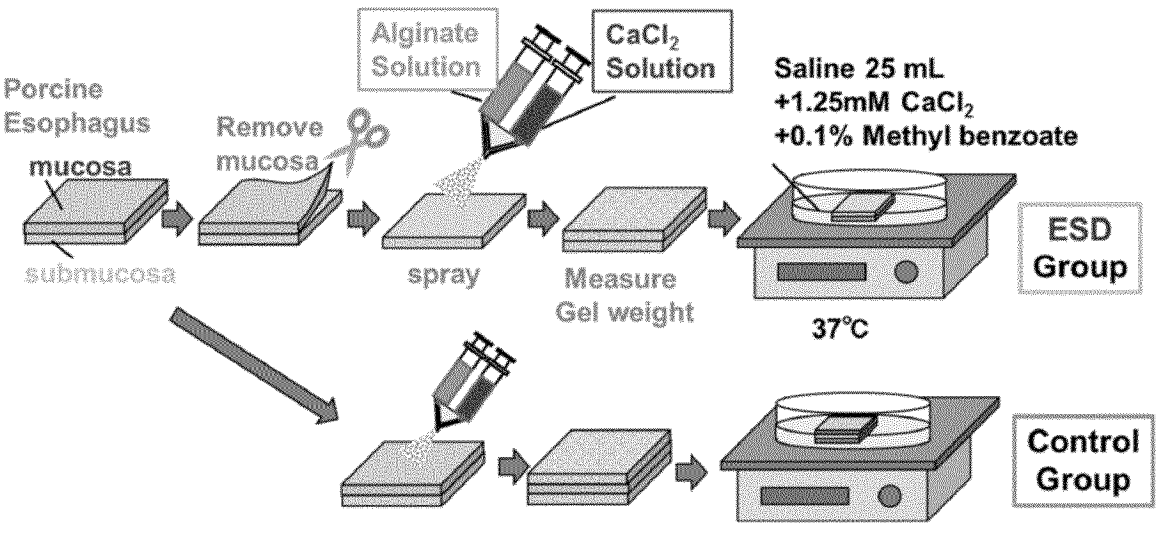
Alginate Solution
CaCl$_2$ Solution
Porcine Esophagus
mucosa
submucosa
Remove mucosa
spray
Measure Gel weight
Saline 25 mL
+1.25mM CaCl$_2$
+0.1% Methyl benzoate
ESD Group
37°C
Control Group

[Fig. 43]

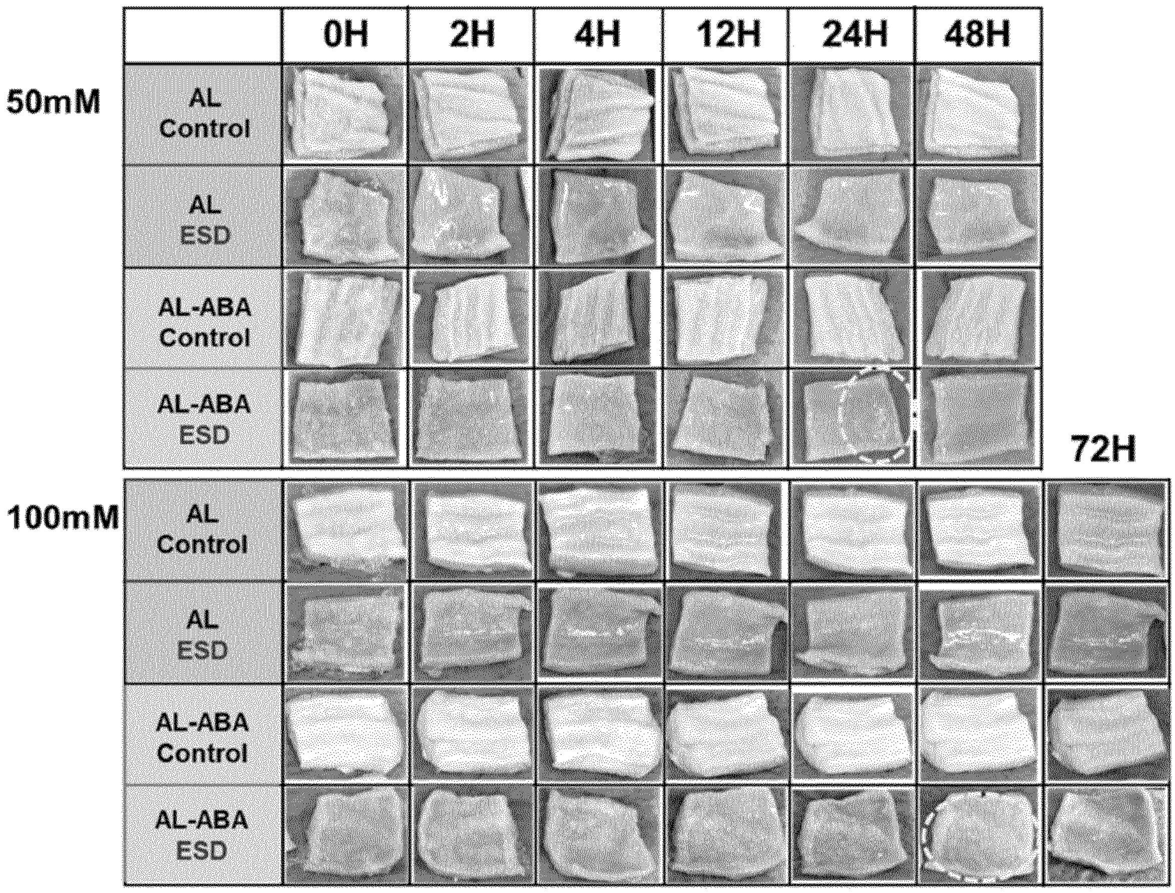
0H
2H
4H
12H
24H
48H
72H
50mM
100mM
AL Control
AL ESD
AL-ABA Control
AL-ABA ESD

[Fig. 44]

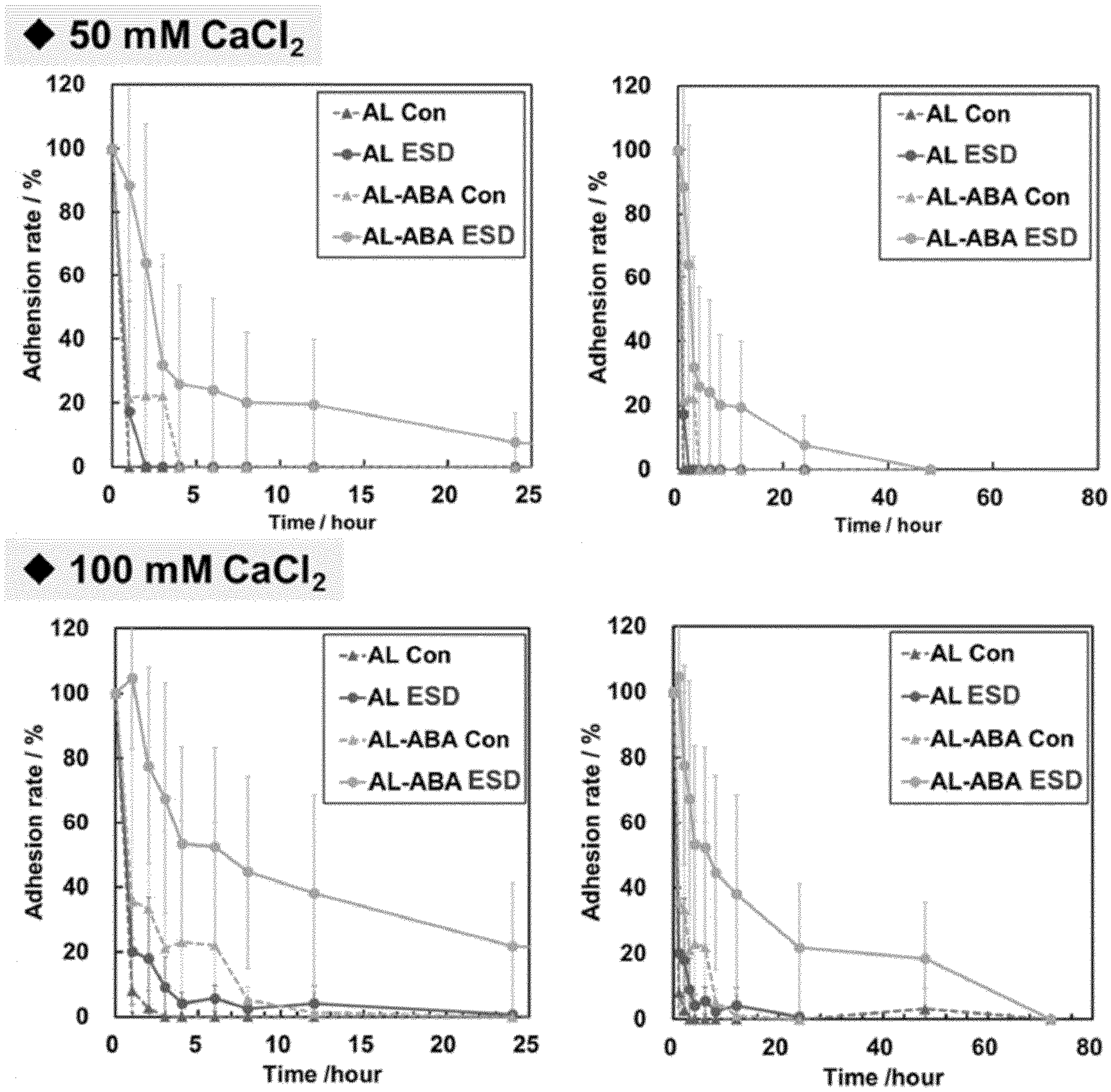
◆ 50 mM $CaCl_2$
AL Con
AL ESD
AL-ABA Con
AL-ABA ESD
Adhesion rate / %
Time / hour
◆ 100 mM $CaCl_2$
AL Con
AL ESD
AL-ABA Con
AL-ABA ESD
Adhesion rate / %
Time /hour

[Fig. 45]

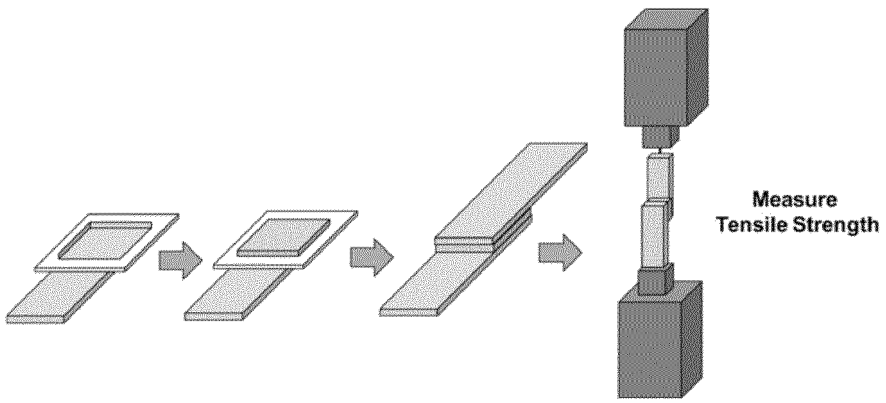
Measure
Tensile Strength

[Fig. 46]

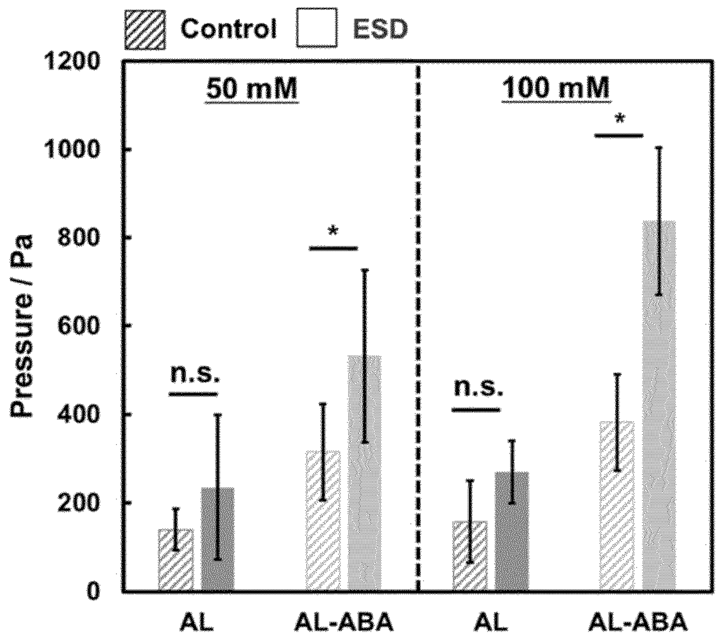
Control
ESD
50 mM
100 mM
Pressure / Pa
1200
1000
800
600
400
200
0
n.s.
*
n.s.
*
AL
AL-ABA
AL
AL-ABA

[Fig. 47]

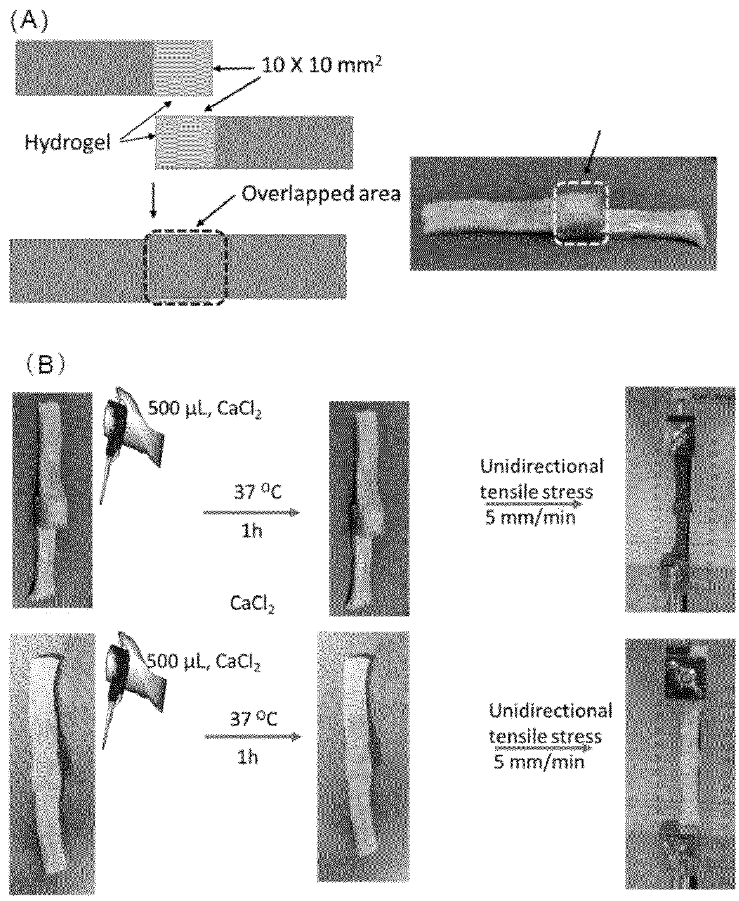
(A)
10 X 10 mm2
Hydrogel
Overlapped area
(B)
500 μL, CaCl2
37 °C
1h
Unidirectional
tensile stress
5 mm/min
CaCl2
500 μL, CaCl2
37 °C
1h
Unidirectional
tensile stress
5 mm/min

[Fig. 48]

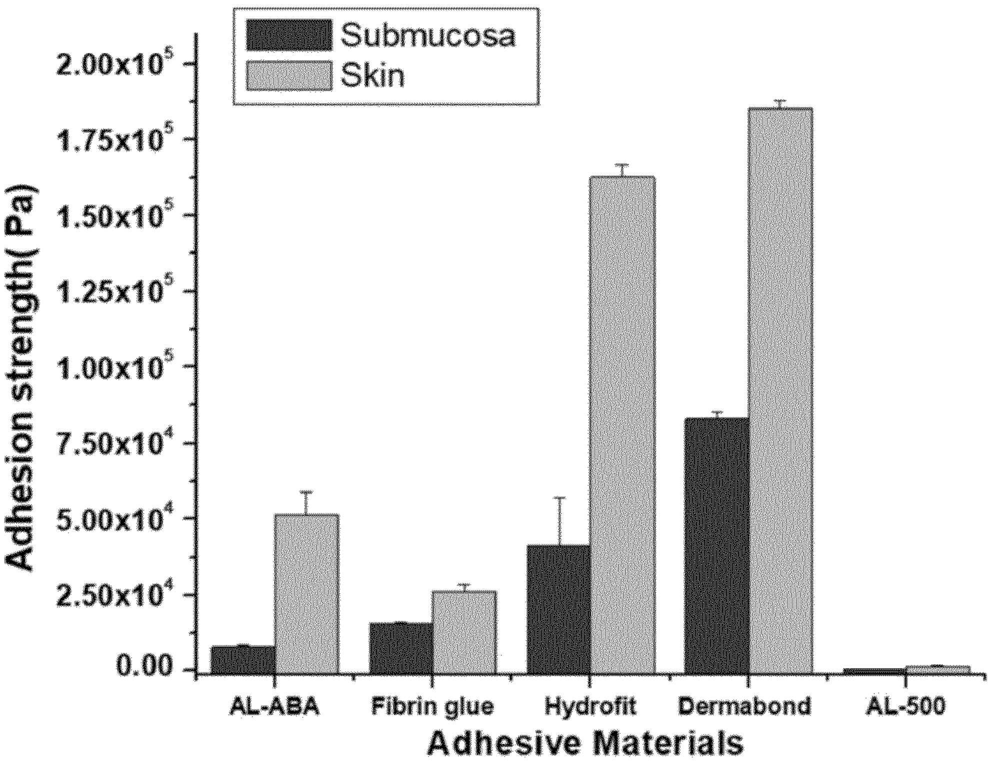
Submucosa
Skin
Adhesion strength( Pa)
2.00x10^5
1.75x10^5
1.50x10^5
1.25x10^5
1.00x10^5
7.50x10^4
5.00x10^4
2.50x10^4
0.00
AL-ABA
Fibrin glue
Hydrofit
Dermabond
AL-500
Adhesive Materials

[Fig. 49]

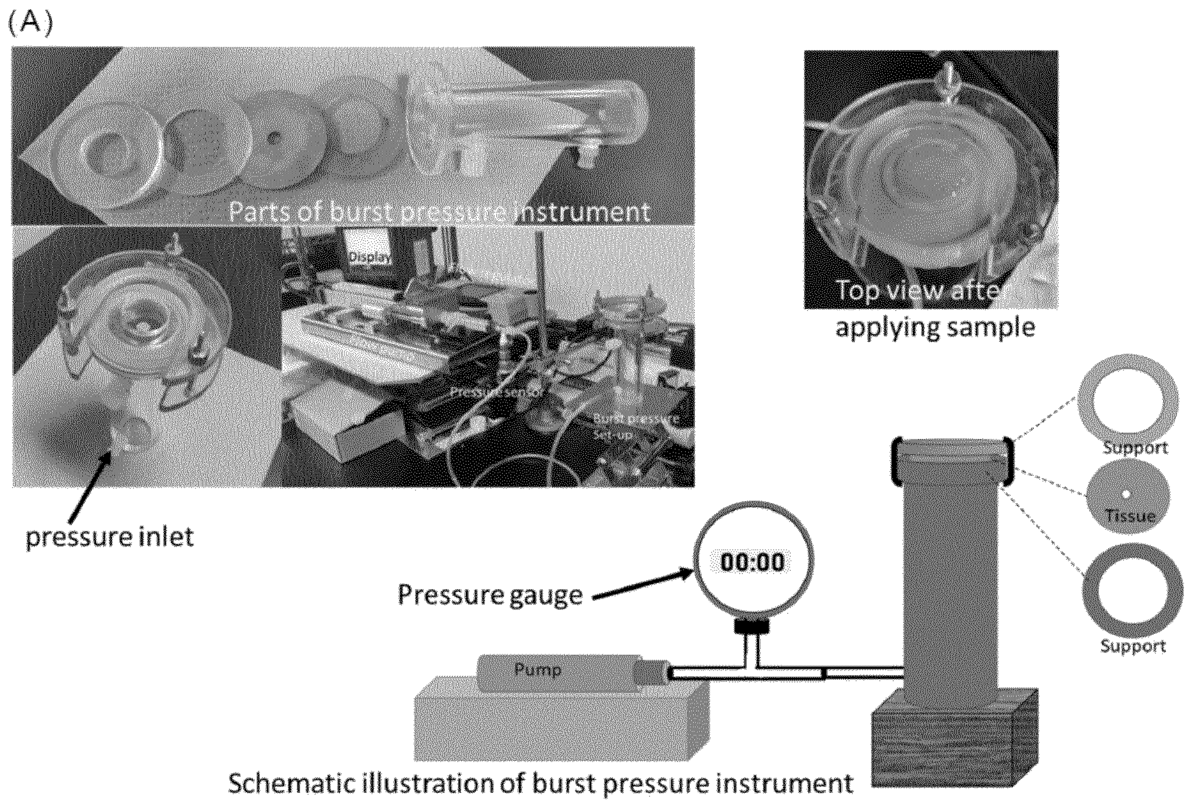
(A)
Parts of burst pressure instrument
Display
Pressure sensor
Burst pressure Set-up
Top view after applying sample
pressure inlet
Support
Tissue
Support
00:00
Pressure gauge
Pump
Schematic illustration of burst pressure instrument

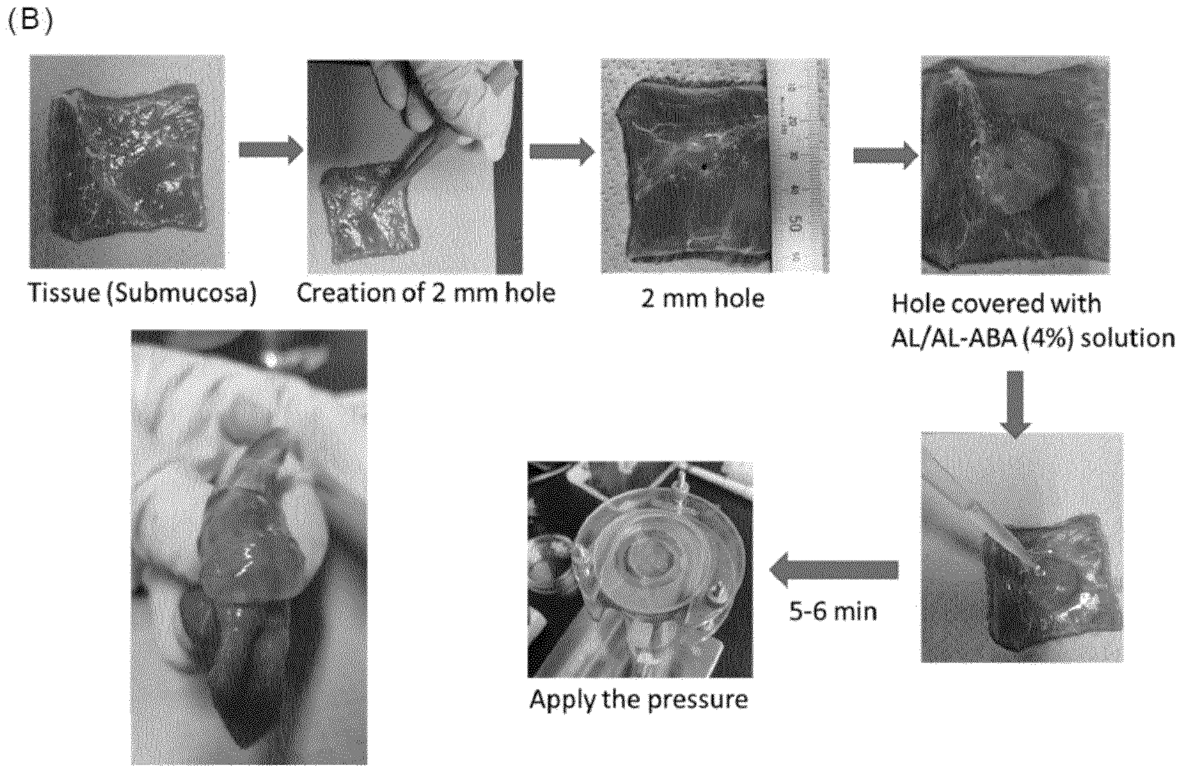
(B)
Tissue (Submucosa)
Creation of 2 mm hole
2 mm hole
Hole covered with
AL/AL-ABA (4%) solution
5-6 min
Apply the pressure

[Fig. 50]

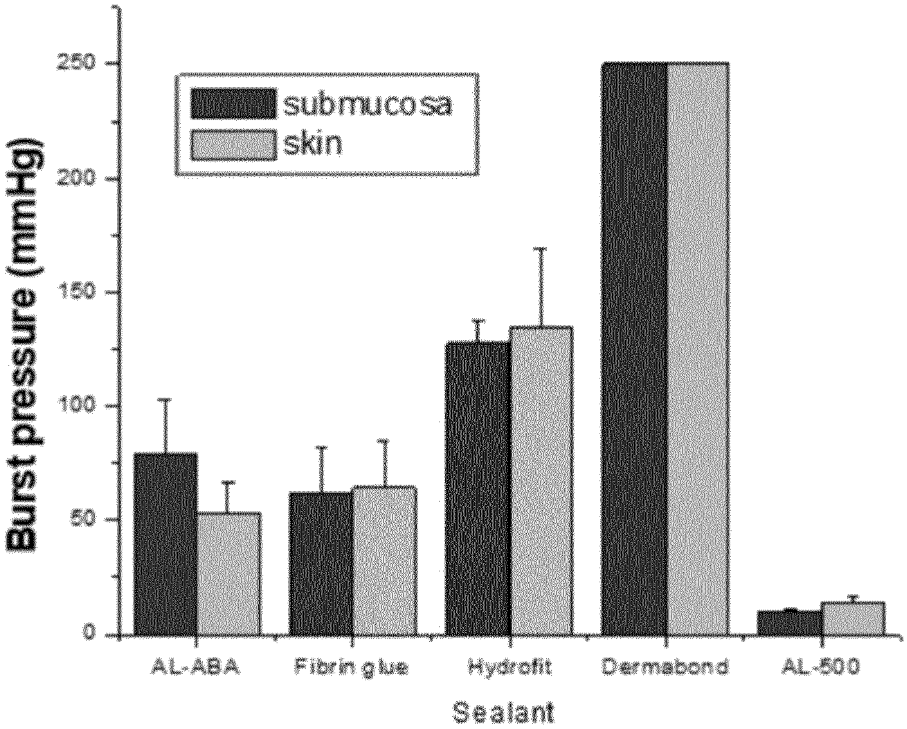
submucosa
skin
Burst pressure (mmHg)
0
50
100
150
200
250
AL-ABA
Fibrin glue
Hydrofit
Dermabond
AL-500
Sealant

[Fig. 51]

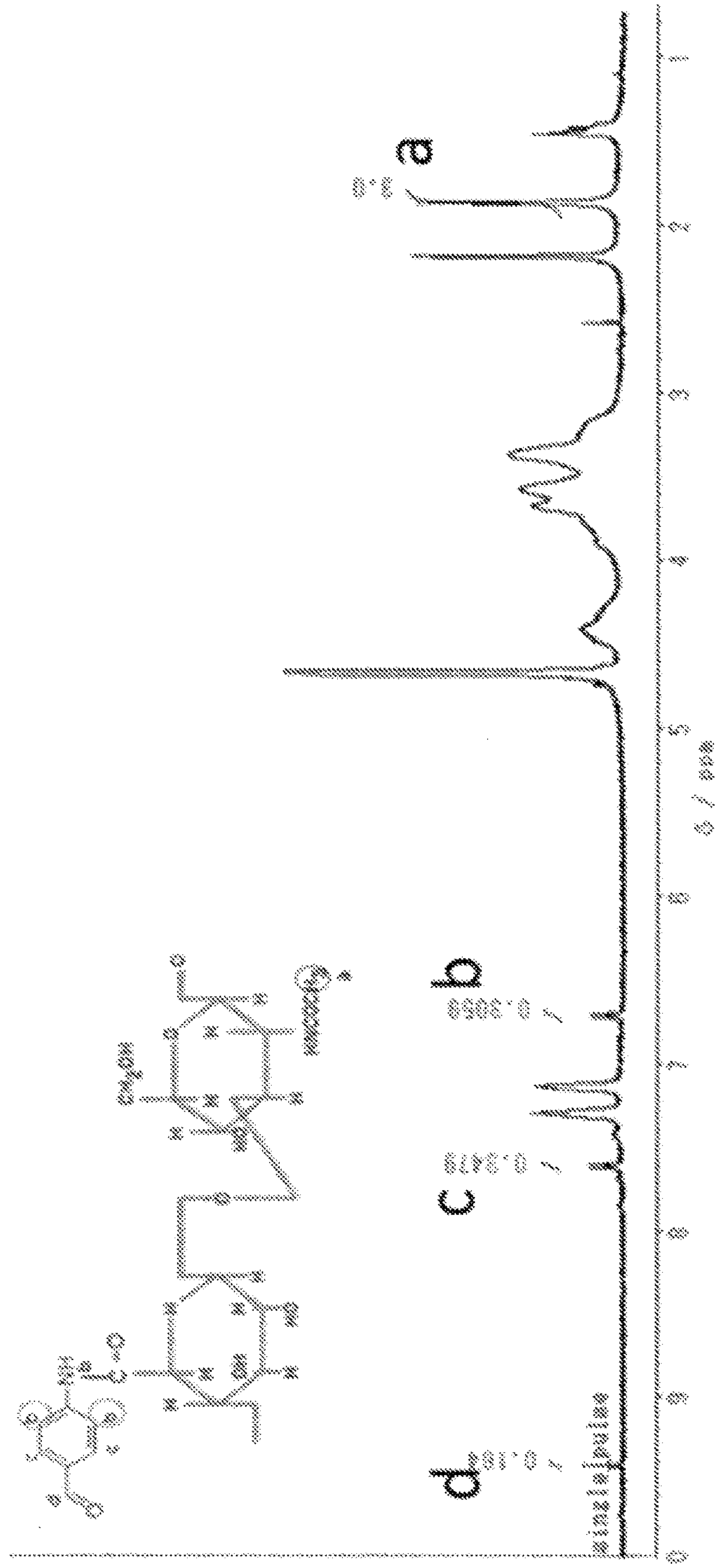

[Fig. 52]

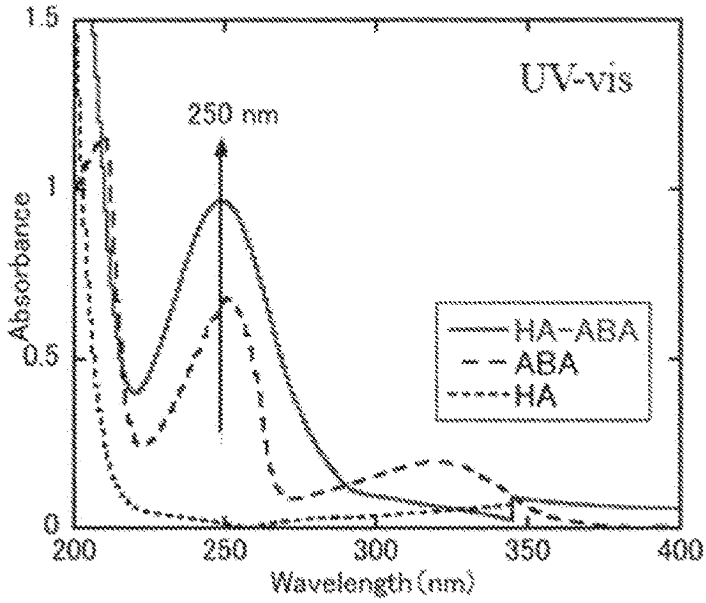
UV-vis
250 nm
Absorbance
HA-ABA
ABA
HA
1.5
1
0.5
0
200
250
300
350
400
Wavelength (nm)

[Fig. 53]

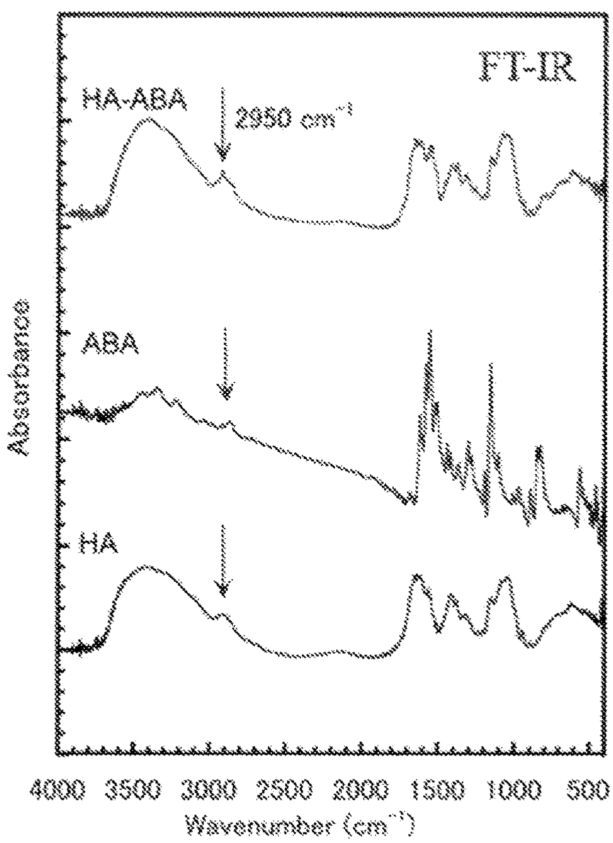
FT-IR
HA-ABA
2950 cm⁻¹
ABA
HA
Absorbance
4000
3500
3000
2500
2000
1500
1000
500
Wavenumber (cm⁻¹)

[Fig. 54]

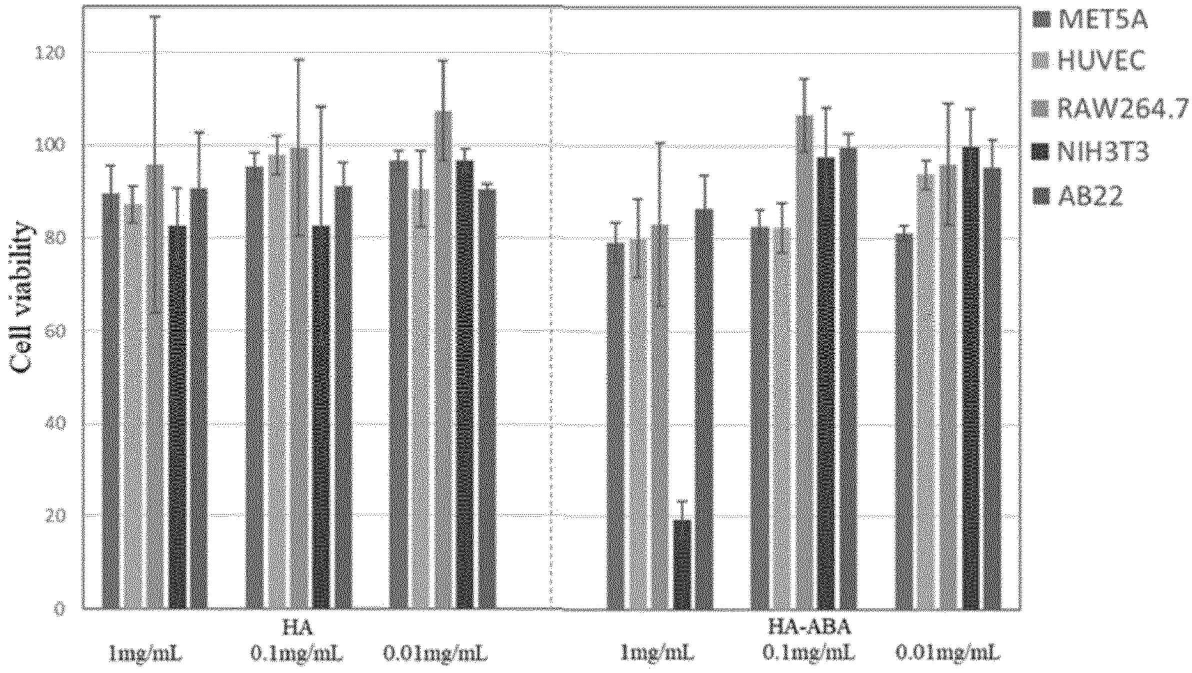
MET5A
HUVEC
RAW264.7
NIH3T3
AB22
Cell viability
0
20
40
60
80
100
120
HA
1mg/mL
0.1mg/mL
0.01mg/mL
HA-ABA
1mg/mL
0.1mg/mL
0.01mg/mL

[Fig. 55]

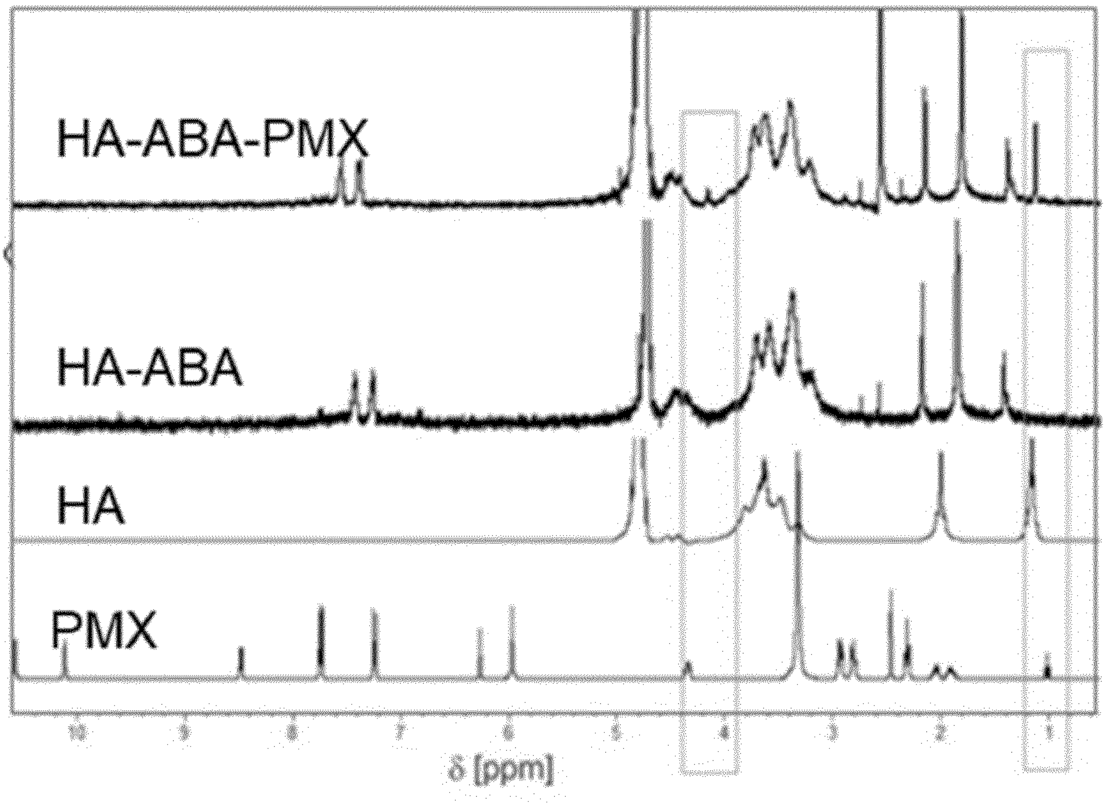
HA-ABA-PMX
HA-ABA
HA
PMX
δ [ppm]

[Fig. 56]

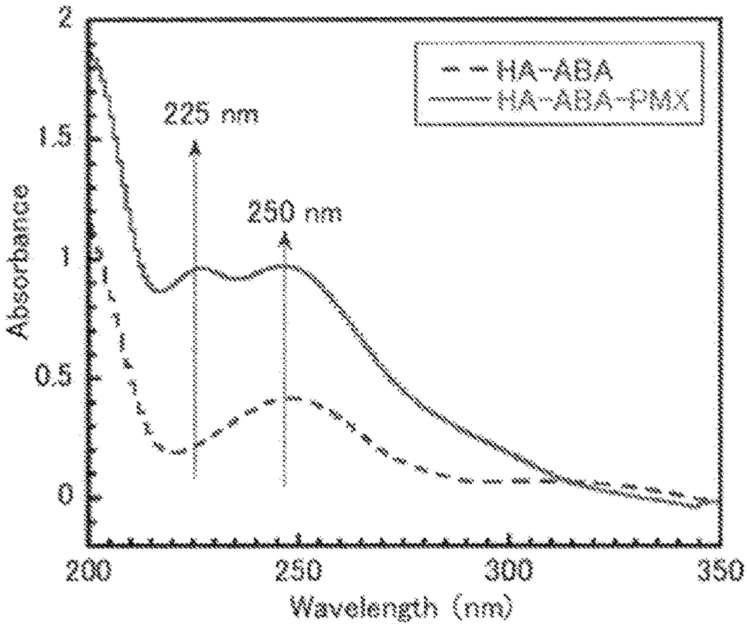
HA-ABA
HA-ABA-PMX
225 nm
250 nm
Absorbance
Wavelength (nm)
2
1.5
1
0.5
0
200
250
300
350

[Fig. 57]

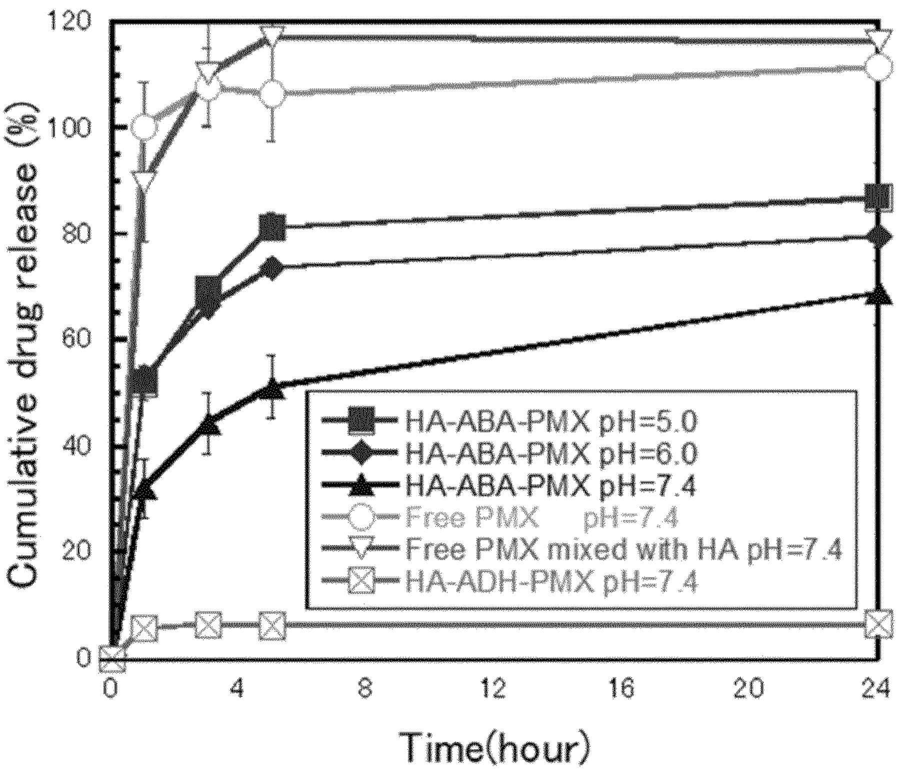
Cumulative drug release (%)
Time(hour)
HA-ABA-PMX pH=5.0
HA-ABA-PMX pH=6.0
HA-ABA-PMX pH=7.4
Free PMX pH=7.4
Free PMX mixed with HA pH=7.4
HA-ADH-PMX pH=7.4
120
100
80
60
40
20
0
0
4
8
12
16
20
24

[Fig. 58]

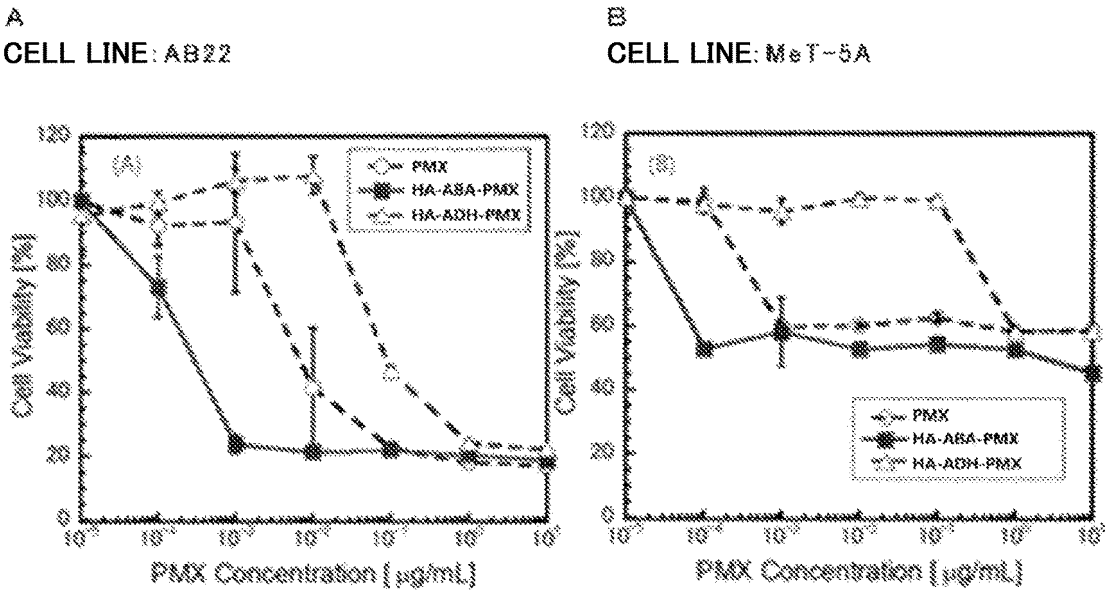
A
CELL LINE: AB22
B
CELL LINE: MeT-5A
PMX
HA-ABA-PMX
HA-ADH-PMX
Cell Viability [%]
PMX Concentration [μg/mL]

[Fig. 59]

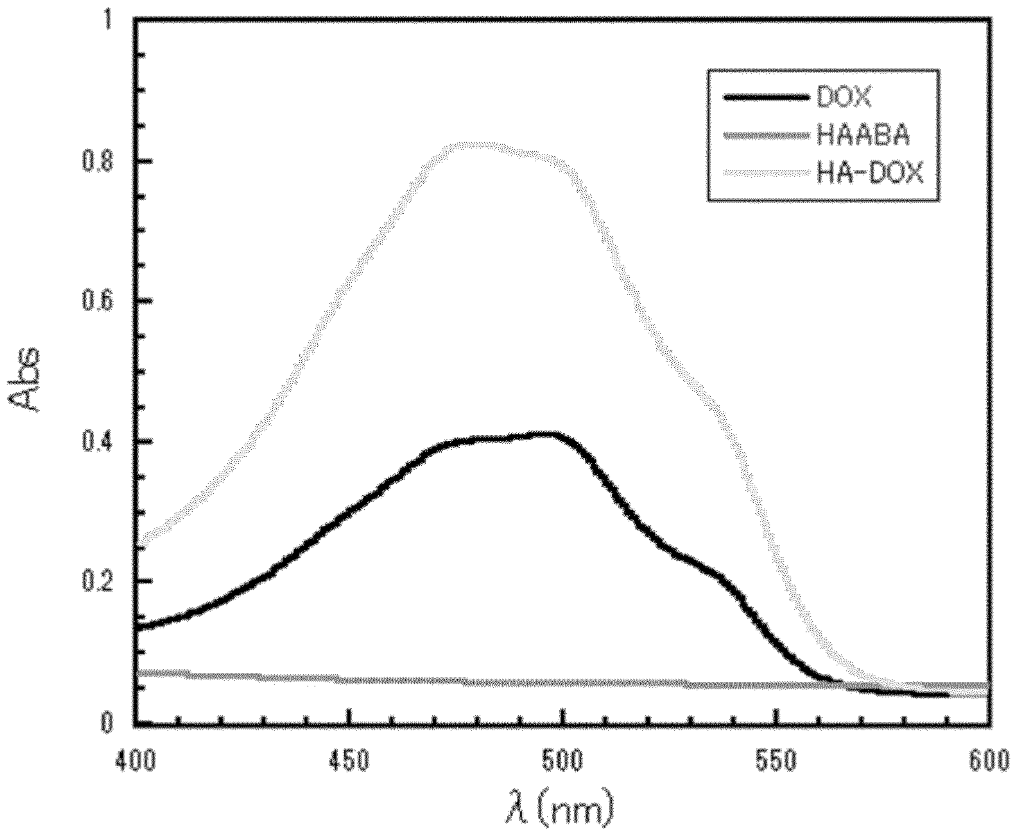
DOX
HAABA
HA-DOX
Abs
λ (nm)
0
0.2
0.4
0.6
0.8
1
400
450
500
550
600

[Fig. 60]

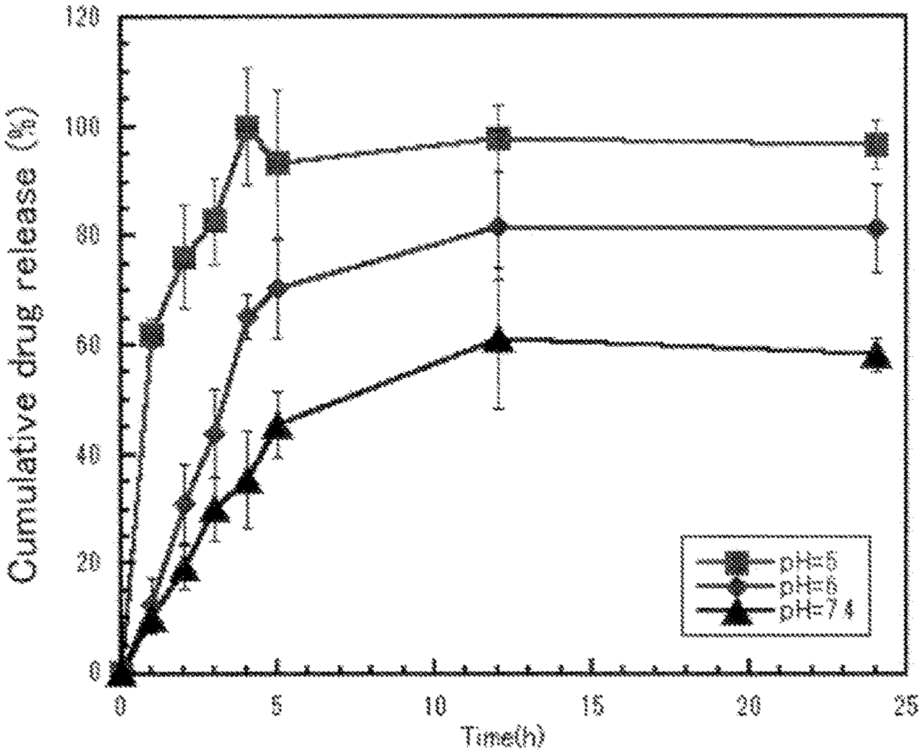
Cumulative drug release (%)
Time(h)
pH=5
pH=6
pH=7.4
0
20
40
60
80
100
120
0
5
10
15
20
25

[Fig. 61]

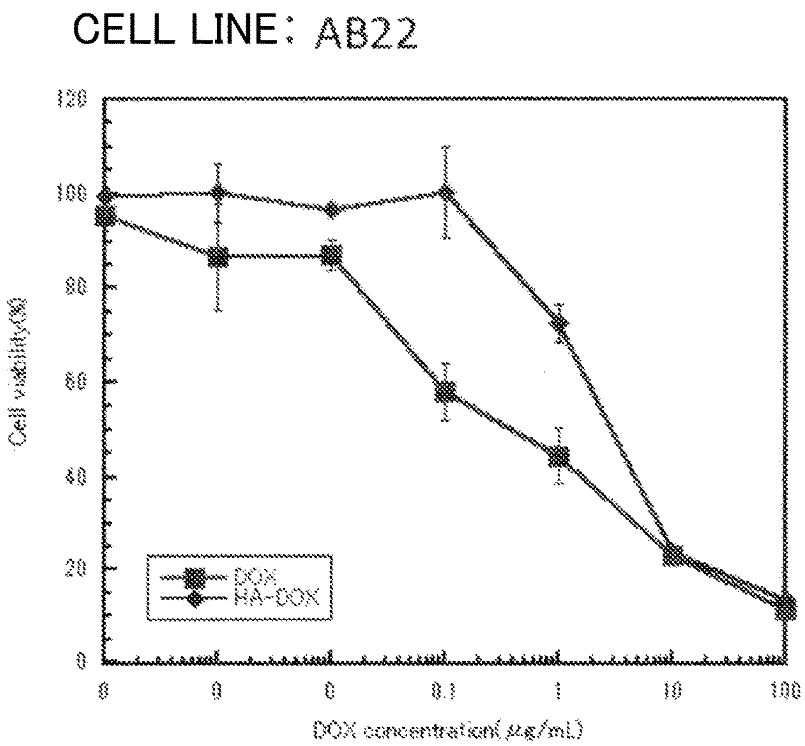
CELL LINE: AB22
Cell viability(%)
DOX concentration( μg/mL)
DOX
HA-DOX
0
20
40
60
80
100
120
0
0
0
0.1
1
10
100

[Fig. 62]

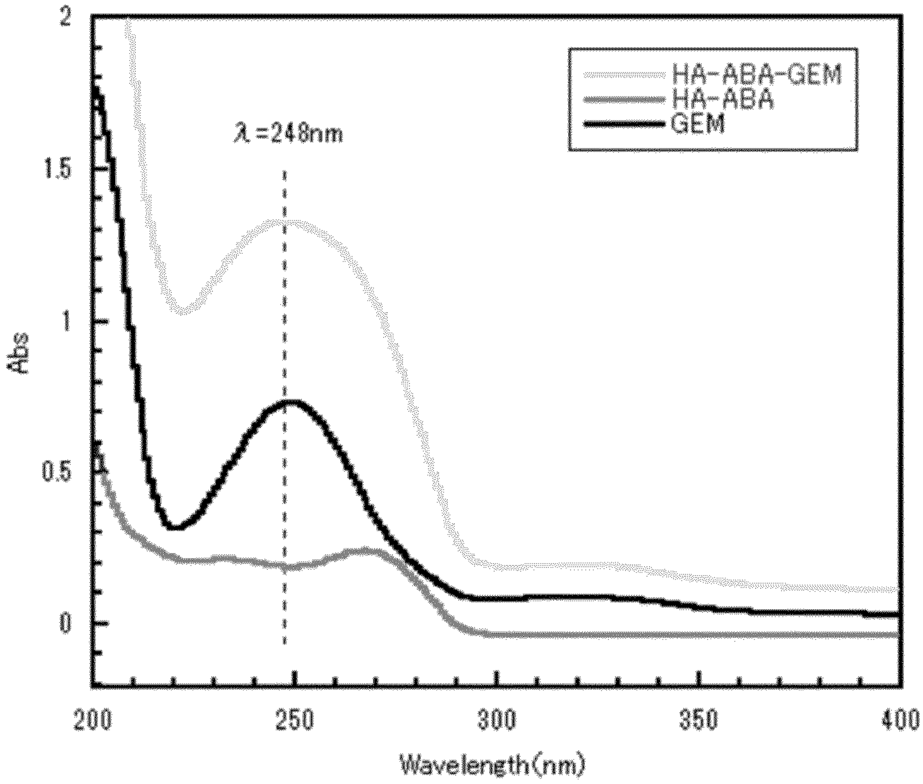
HA-ABA-GEM
HA-ABA
GEM
λ=248nm
Abs
2
1.5
1
0.5
0
200
250
300
350
400
Wavelength(nm)

[Fig. 63]

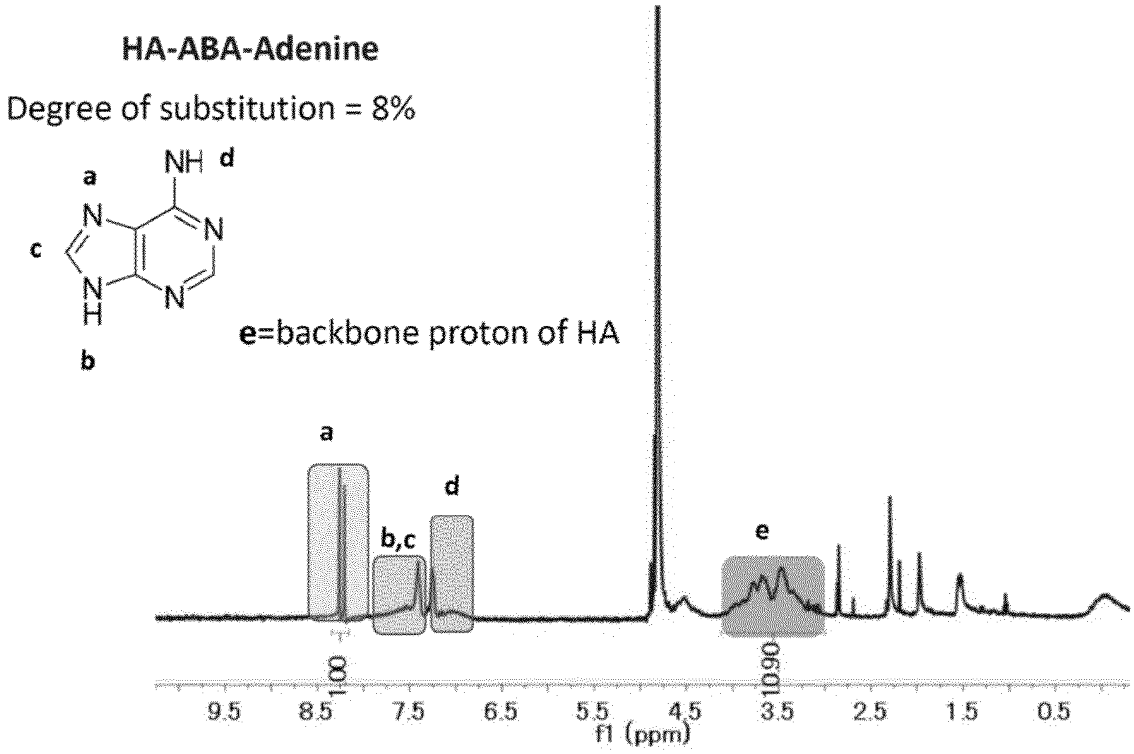
HA-ABA-Adenine
Degree of substitution = 8%
e=backbone proton of HA
a
b,c
d
e
1.00
10.90
9.5
8.5
7.5
6.5
5.5
4.5
3.5
2.5
1.5
0.5
f1 (ppm)

[Fig. 64]

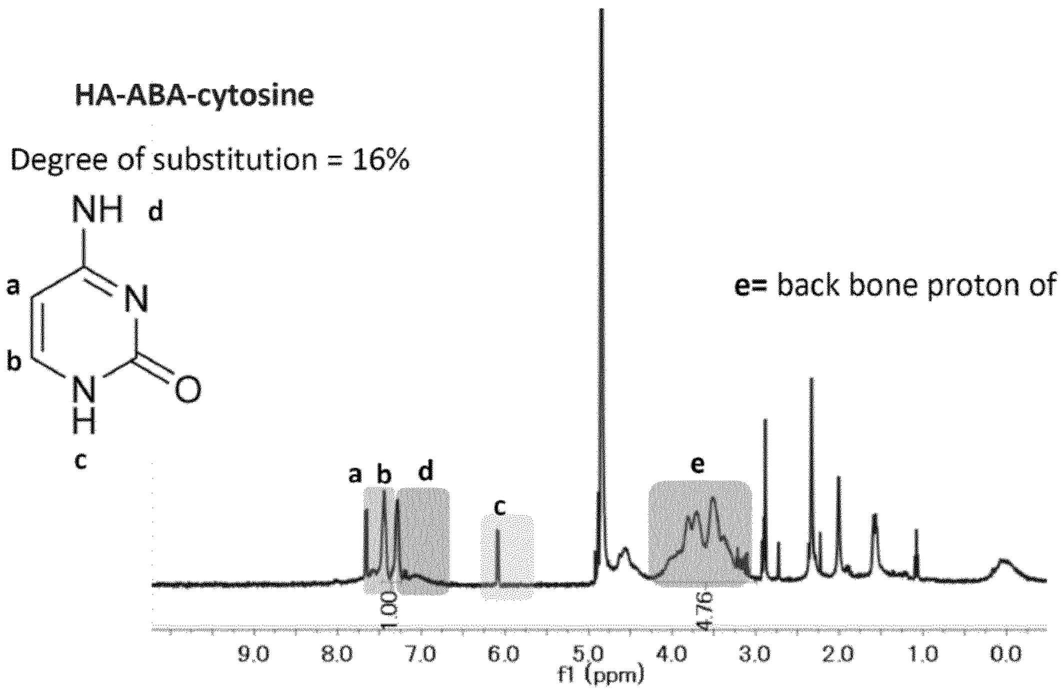
HA-ABA-cytosine
Degree of substitution = 16%
e= back bone proton of HA
a b d
c
e
1.00
4.76
9.0 8.0 7.0 6.0 5.0 4.0 3.0 2.0 1.0 0.0
f1 (ppm)

[Fig. 65]

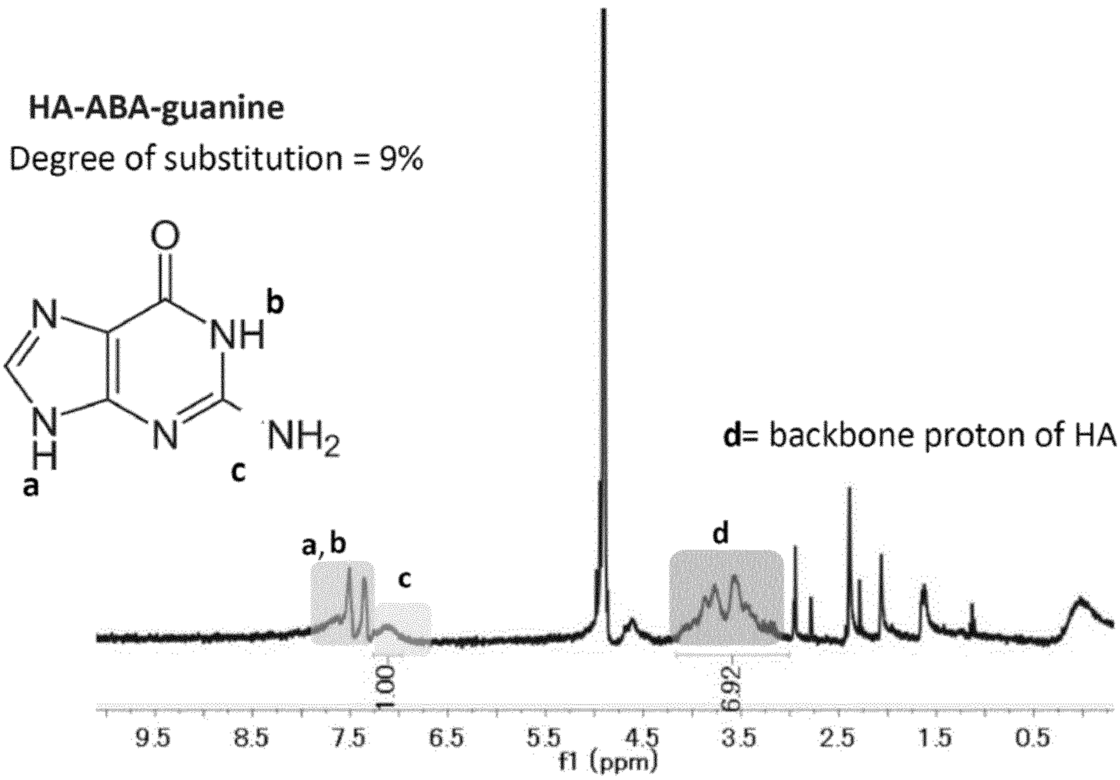
HA-ABA-guanine
Degree of substitution = 9%
d= backbone proton of HA
a, b
c
d
1.00
6.92
9.5 8.5 7.5 6.5 5.5 4.5 3.5 2.5 1.5 0.5
f1 (ppm)

[Fig. 66]

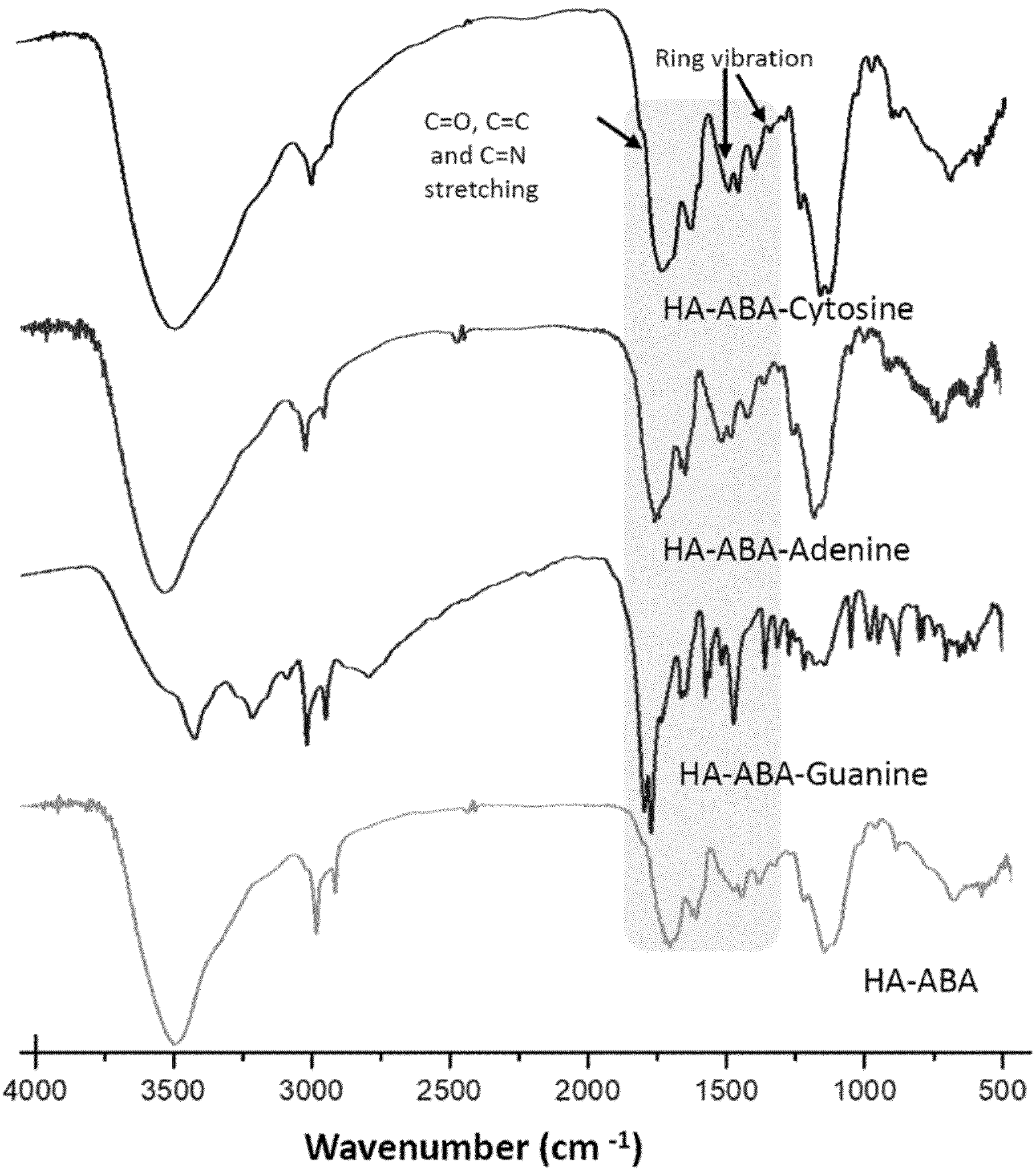
Ring vibration
C=O, C=C
and C=N
stretching
HA-ABA-Cytosine
HA-ABA-Adenine
HA-ABA-Guanine
HA-ABA
4000
3500
3000
2500
2000
1500
1000
500
Wavenumber (cm -1)

[Fig. 67]

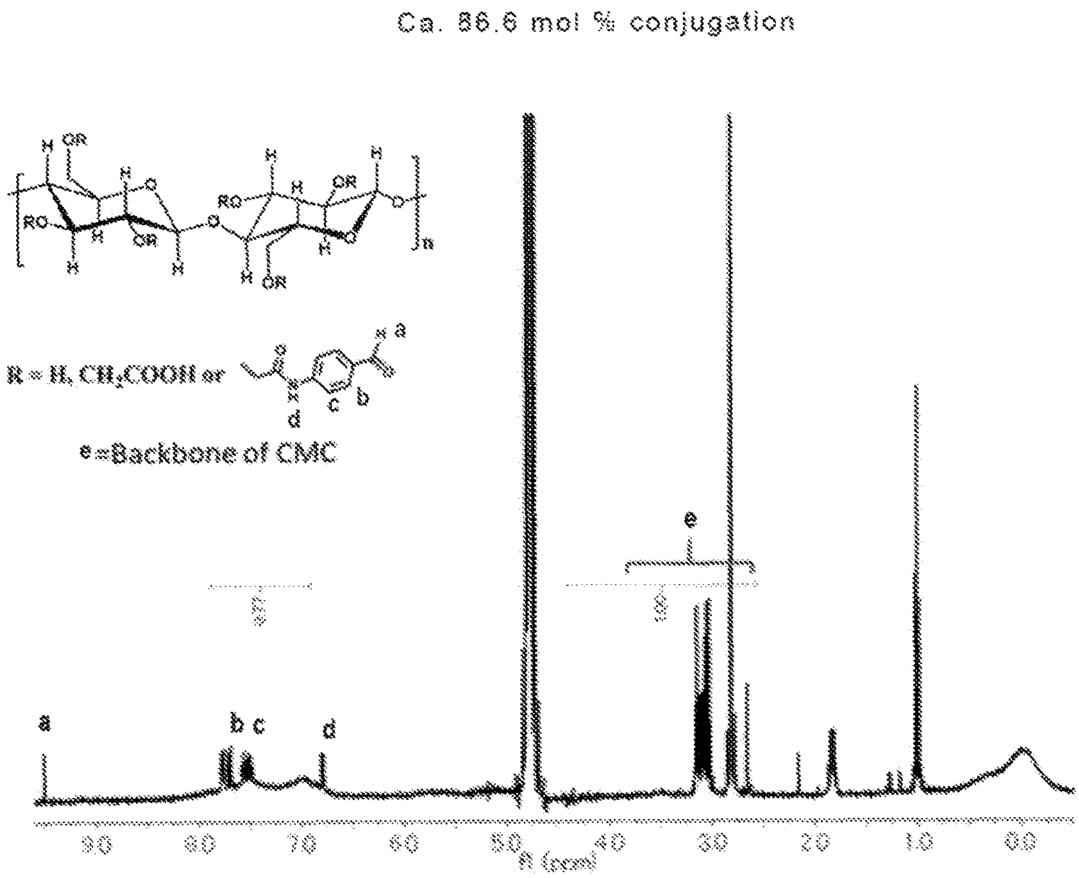
Ca. 86.6 mol % conjugation
R = H, CH2COOH or
e =Backbone of CMC
a
b c
d
e
9.0
8.0
7.0
6.0
5.0
4.0
3.0
2.0
1.0
0.0
f1 (ppm)

[Fig. 68]

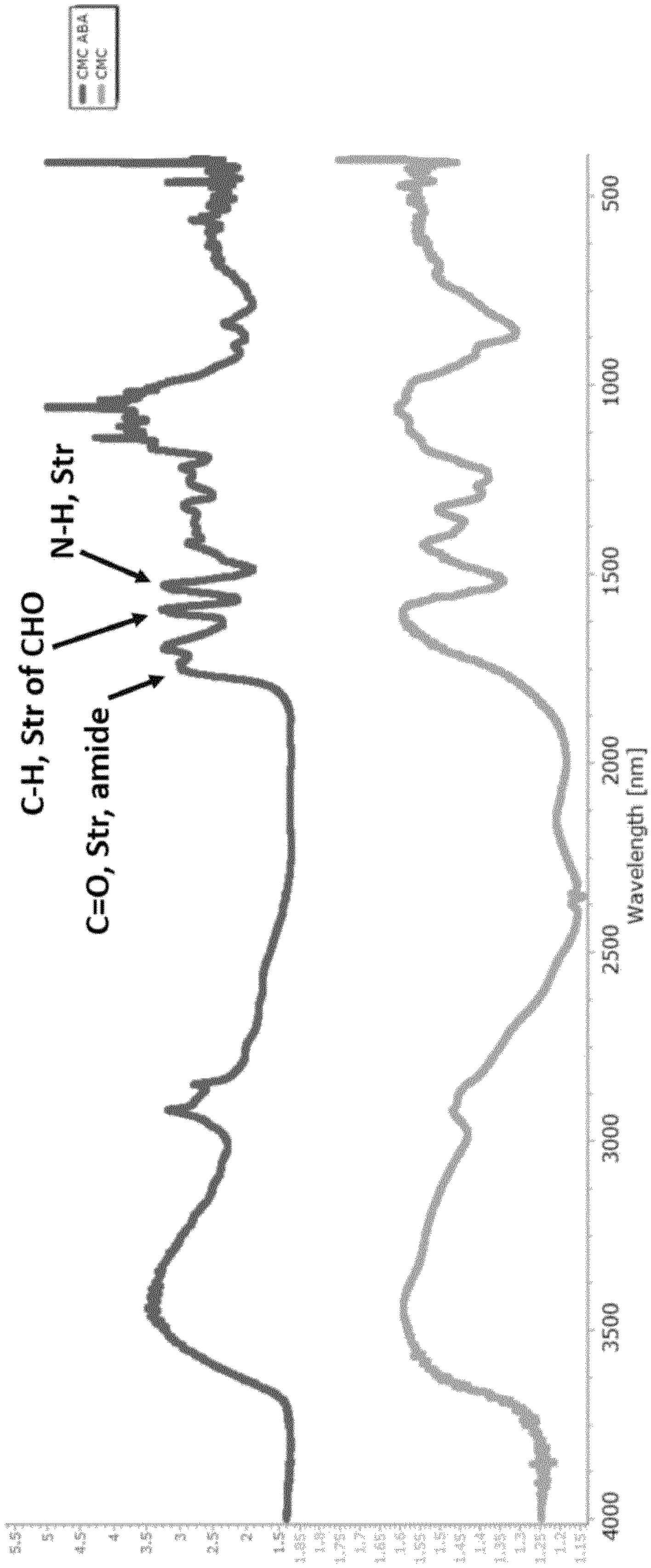
CMC ABA
CMC
N-H, Str
C-H, Str of CHO
C=O, Str, amide
Wavelength [nm]
500
1000
1500
2000
2500
3000
3500
4000

[Fig. 69]

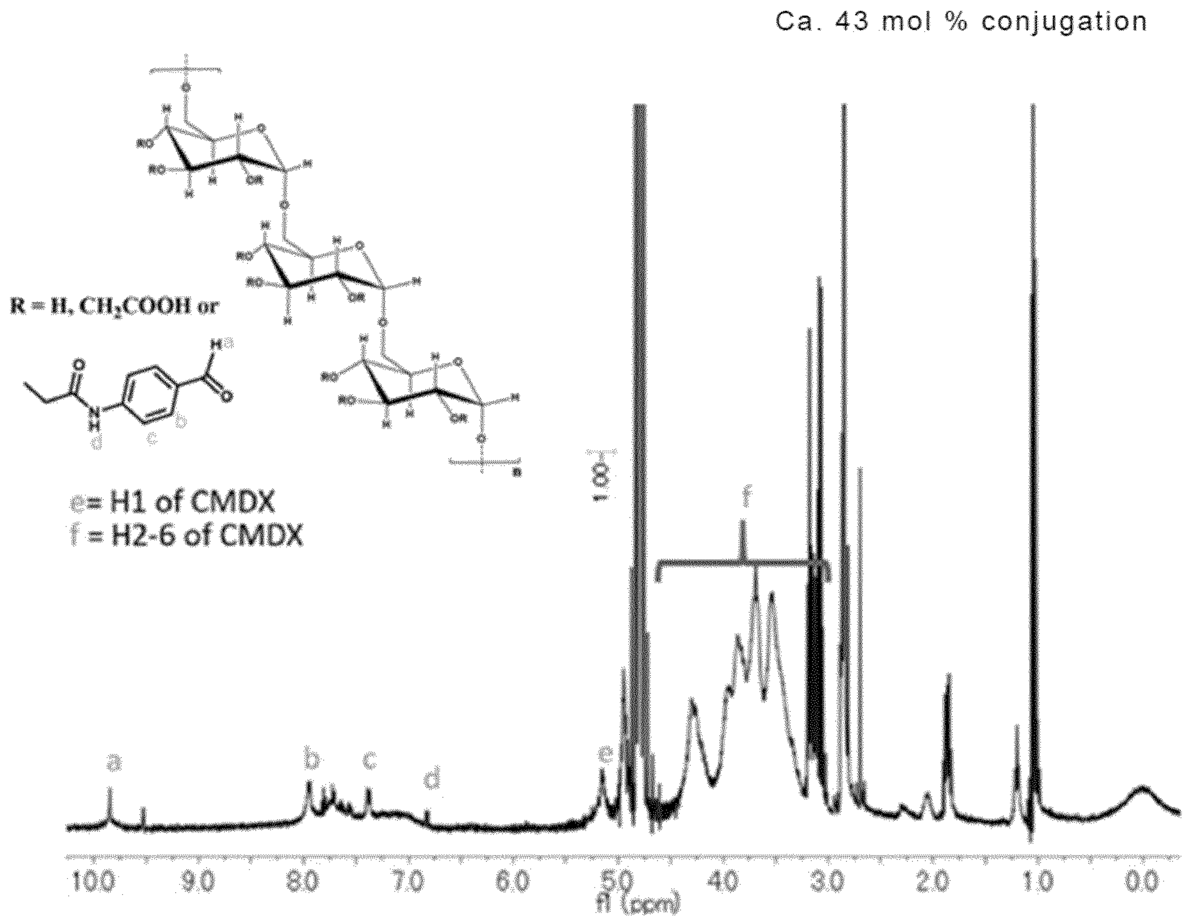
Ca. 43 mol % conjugation
R = H, $CH_2COOH$ or
e= H1 of CMDX
f = H2-6 of CMDX
a
b
c
d
e
f
1.00
10.0
9.0
8.0
7.0
6.0
5.0
4.0
3.0
2.0
1.0
0.0
f1 (ppm)

[Fig. 70]

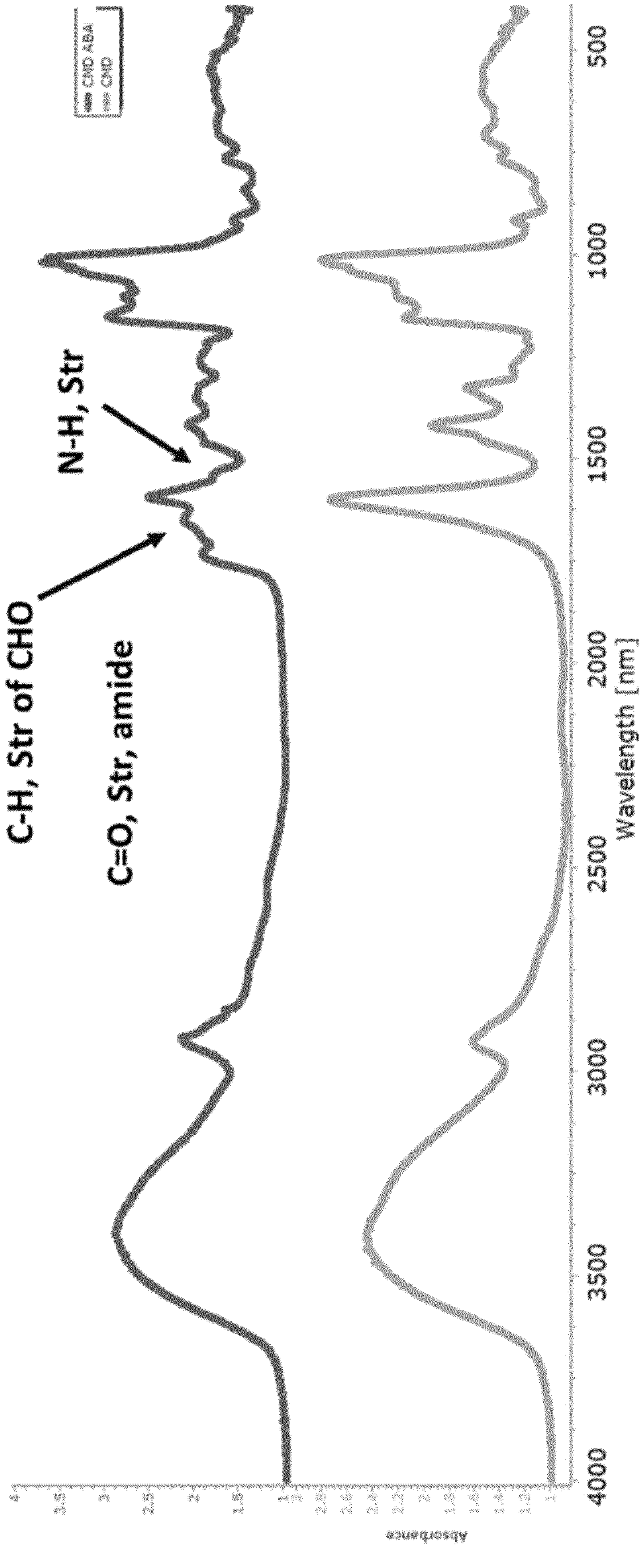
CMD ABA
CMD
N-H, Str
C-H, Str of CHO
C=O, Str, amide
Absorbance
Wavelength [nm]
500
1000
1500
2000
2500
3000
3500
4000

[Fig. 71]

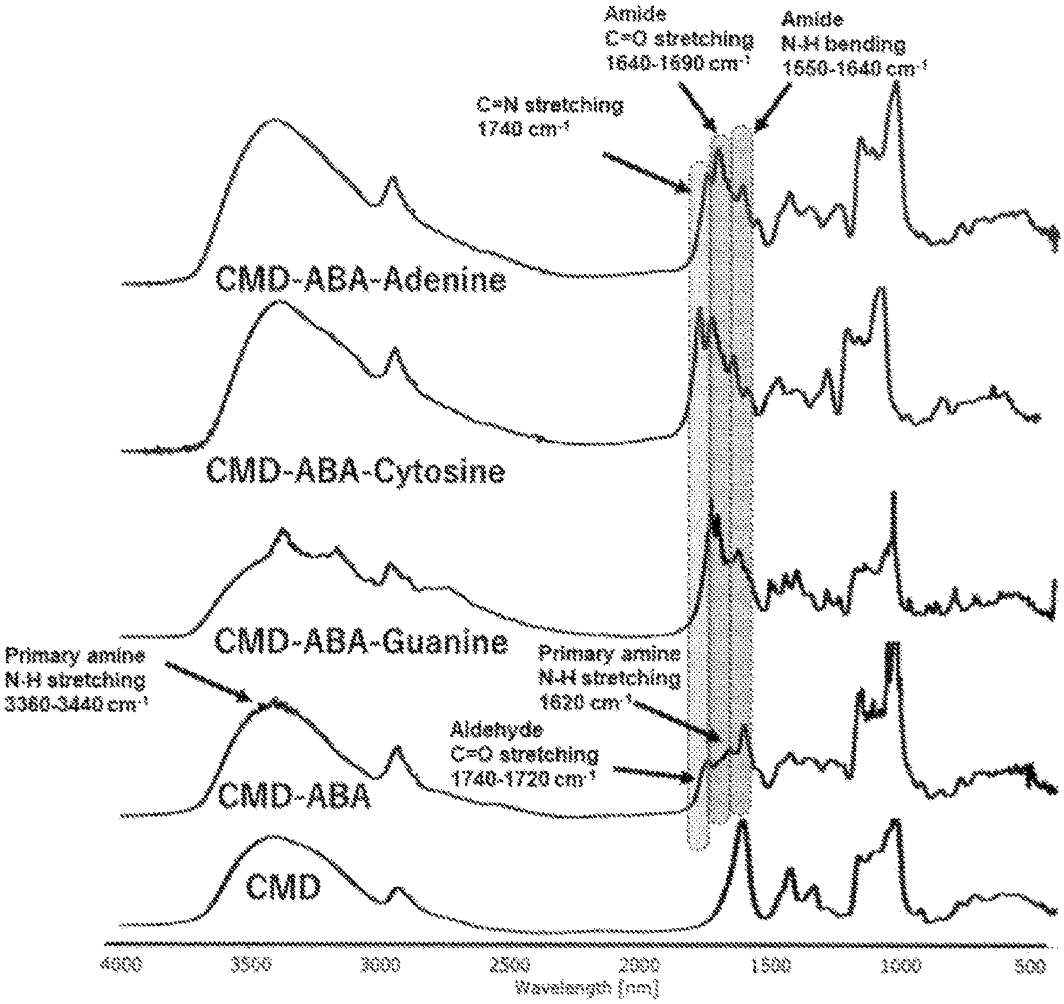
Amide
C=O stretching
1640-1690 cm-1
Amide
N-H bending
1550-1640 cm-1
C=N stretching
1740 cm-1
CMD-ABA-Adenine
CMD-ABA-Cytosine
CMD-ABA-Guanine
Primary amine
N-H stretching
3360-3440 cm-1
Primary amine
N-H stretching
1620 cm-1
Aldehyde
C=O stretching
1740-1720 cm-1
CMD-ABA
CMD
4000
3500
3000
2500
2000
1500
1000
500
Wavelength [nm]

[Fig. 72]

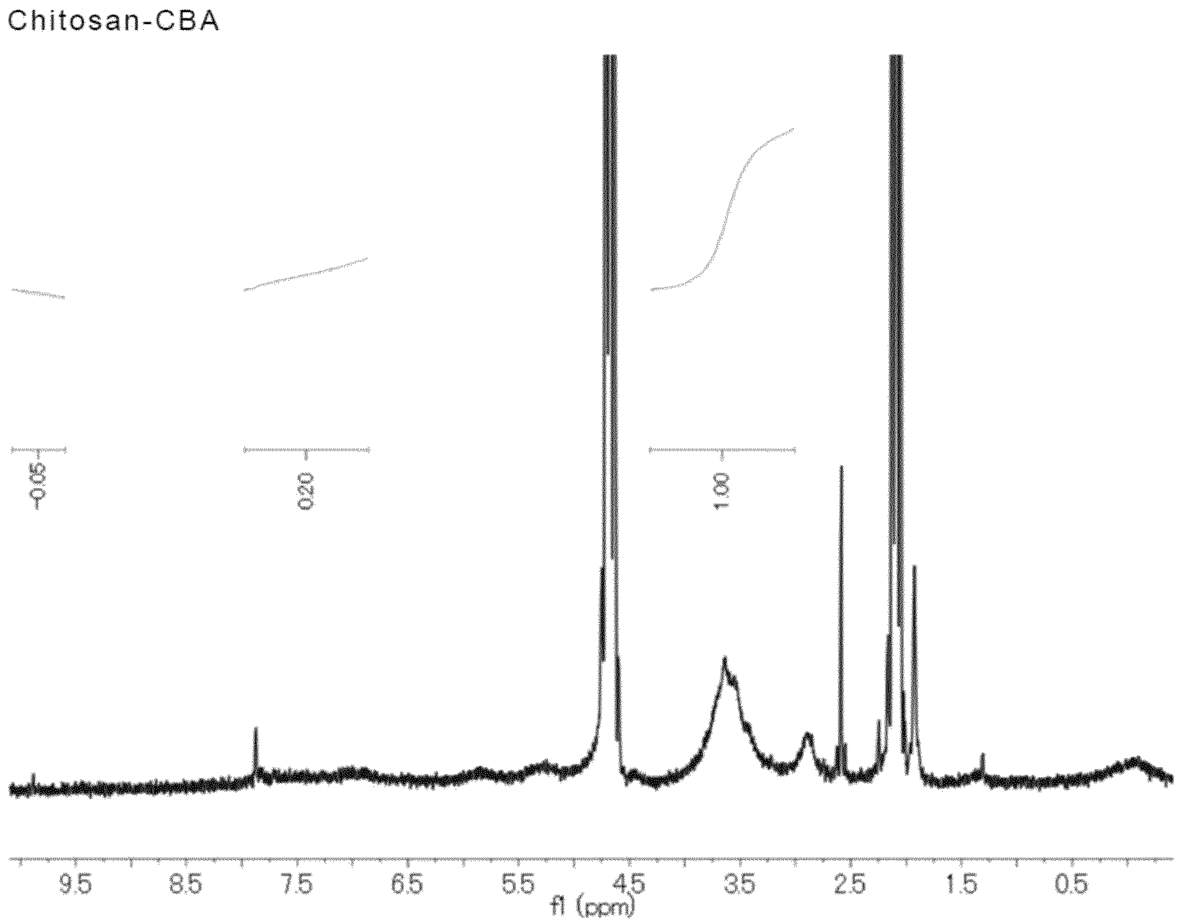
Chitosan-CBA
-0.05
0.20
1.00
9.5
8.5
7.5
6.5
5.5
4.5
3.5
2.5
1.5
0.5
f1 (ppm)

[Fig. 73]

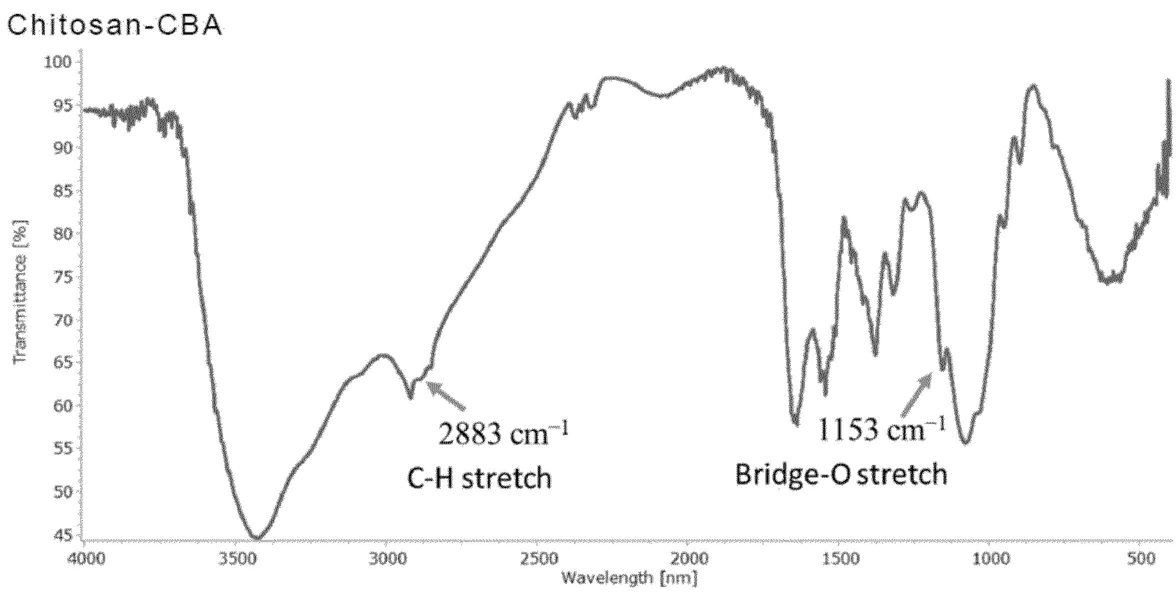
Chitosan-CBA
Transmittance [%]
100
95
90
85
80
75
70
65
60
55
50
45
2883 cm⁻¹
C-H stretch
1153 cm⁻¹
Bridge-O stretch
4000
3500
3000
2500
2000
1500
1000
500
Wavelength [nm]

[Fig. 74]

(A)

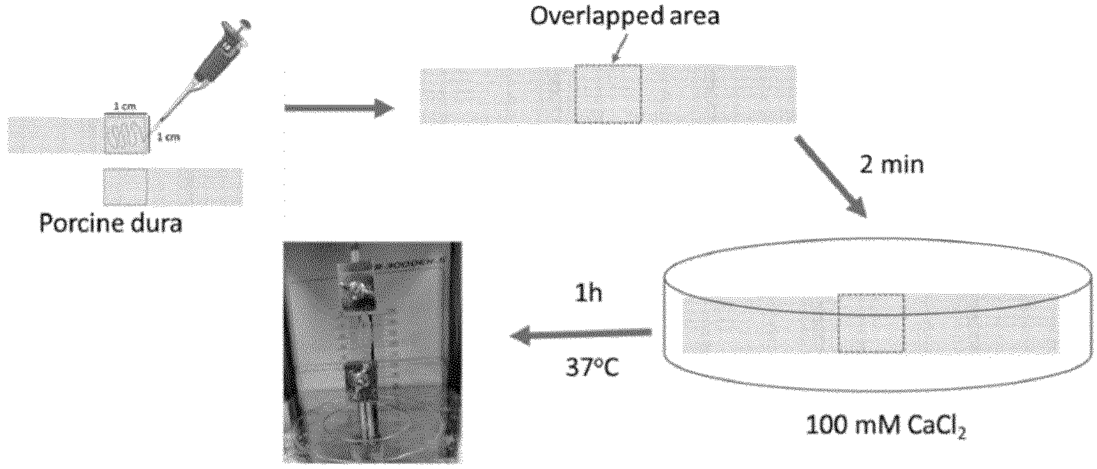
Overlapped area
1 cm
1 cm
Porcine dura
2 min
1h
37°C
100 mM CaCl2

(B)

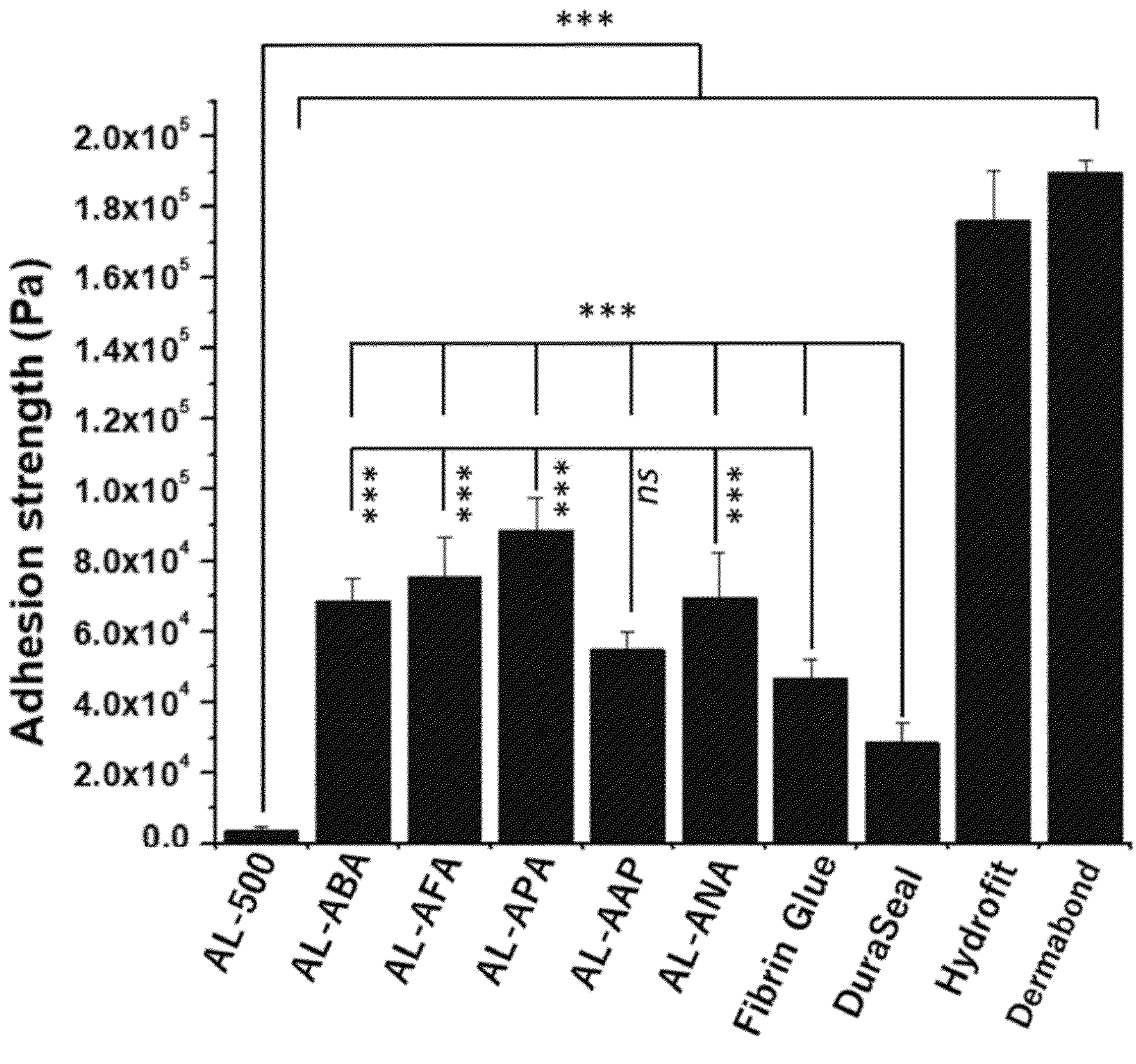
***
***
Adhesion strength (Pa)
2.0x10^5
1.8x10^5
1.6x10^5
1.4x10^5
1.2x10^5
1.0x10^5
8.0x10^4
6.0x10^4
4.0x10^4
2.0x10^4
0.0
***
***
***
ns
***
AL-500
AL-ABA
AL-AFA
AL-APA
AL-AAP
AL-ANA
Fibrin Glue
DuraSeal
Hydrofit
Dermabond

[Fig. 75(A)]

(A)

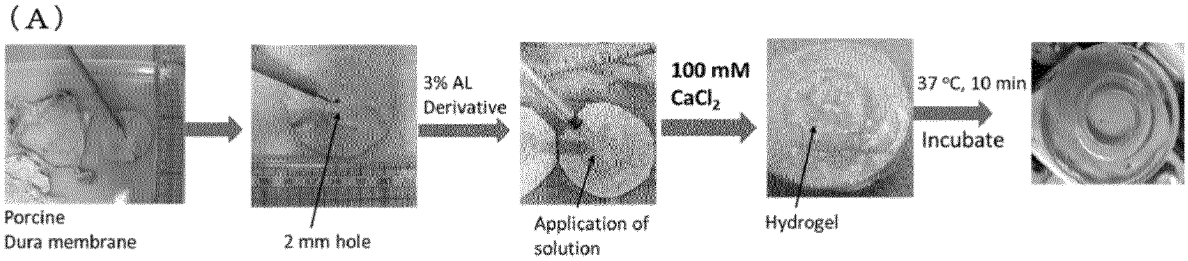


Process of sample preparation for burst pressure experiment

(B)

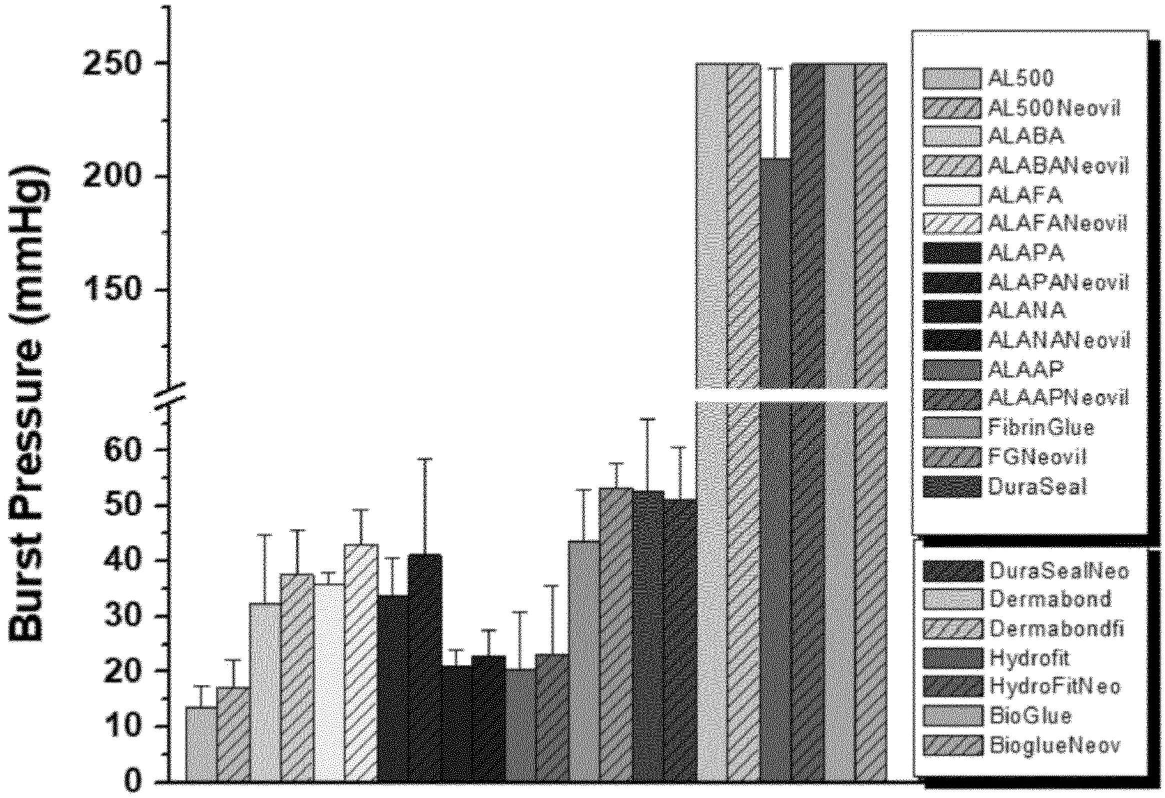

[Fig. 75(B)]

(C)

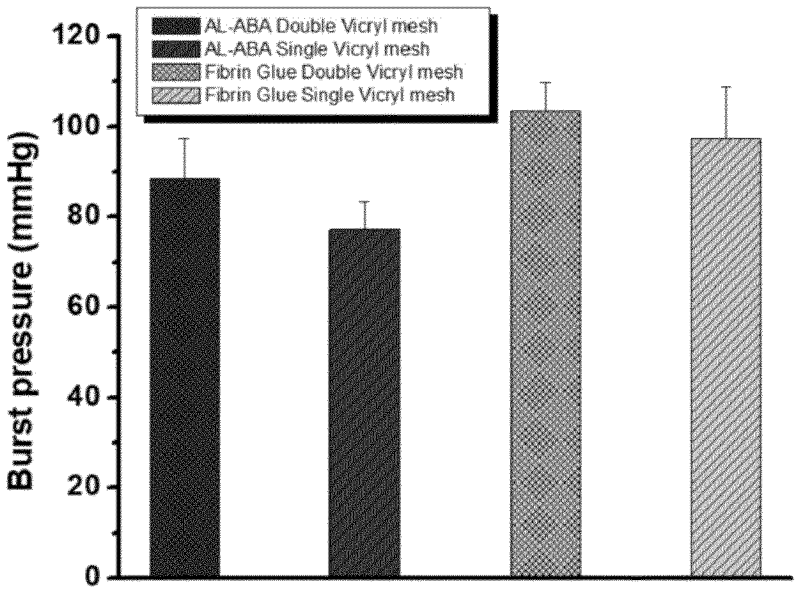


[Fig. 76]

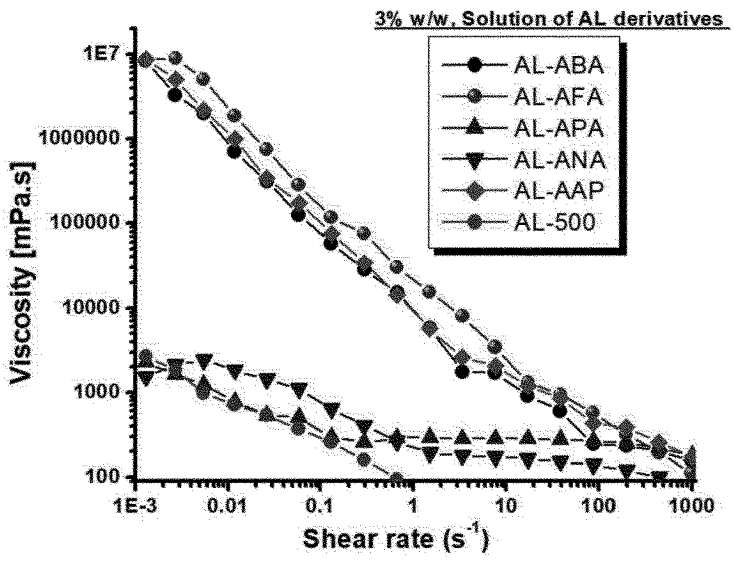

[Fig. 77]

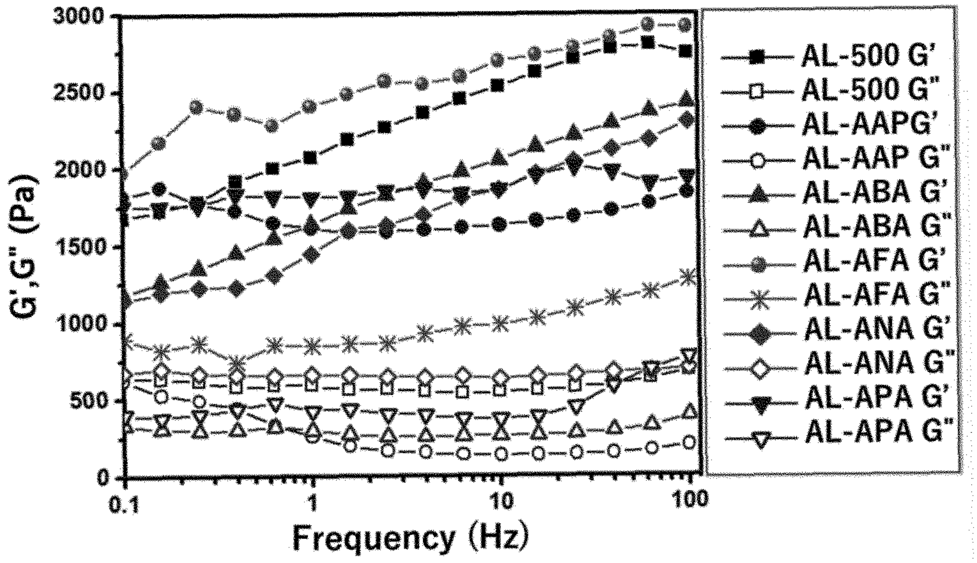
3000
2500
2000
1500
1000
500
0
G',G" (Pa)
0.1
1
10
100
Frequency (Hz)
AL-500 G'
AL-500 G"
AL-AAPG'
AL-AAP G"
AL-ABA G'
AL-ABA G"
AL-AFA G'
AL-AFA G"
AL-ANA G'
AL-ANA G"
AL-APA G'
AL-APA G"

[Fig. 78]

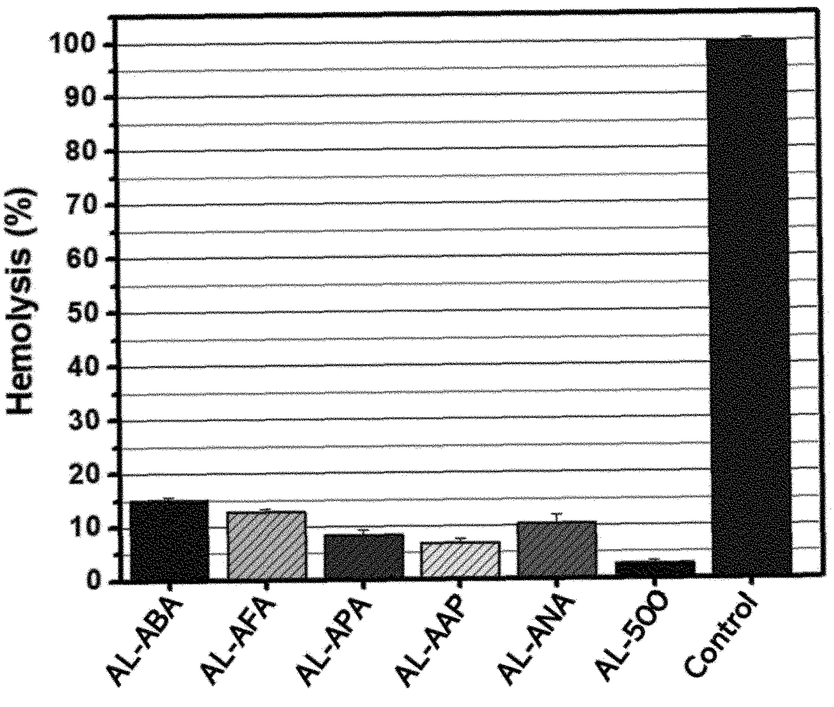
100
90
80
70
60
50
40
30
20
10
0
Hemolysis (%)
AL-ABA
AL-AFA
AL-APA
AL-AAP
AL-ANA
AL-500
Control

[Fig. 79]

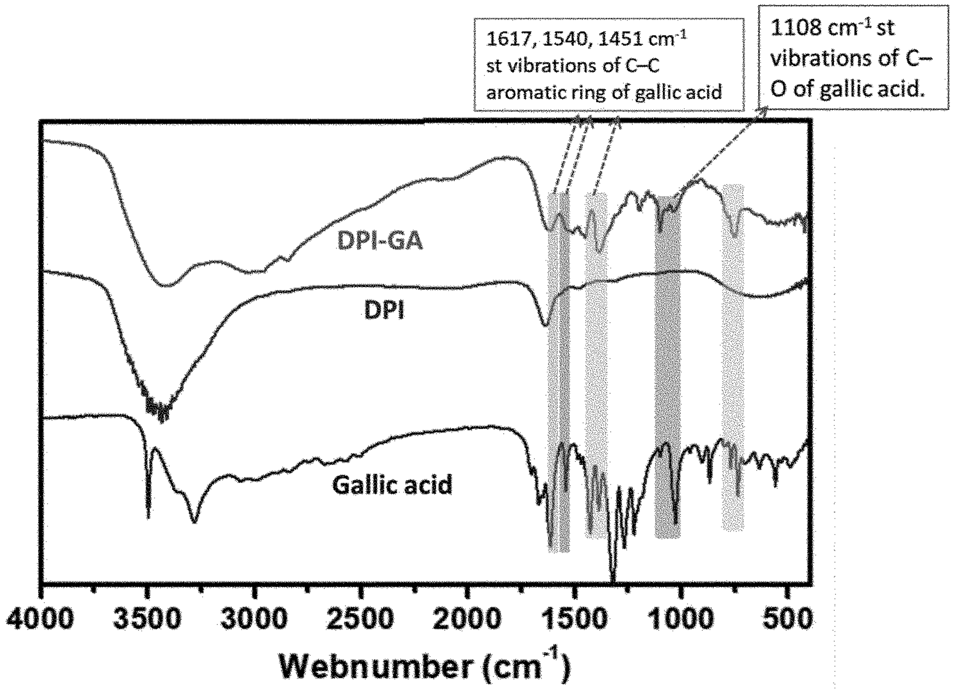
1617, 1540, 1451 cm-1 st vibrations of C–C aromatic ring of gallic acid
1108 cm-1 st vibrations of C–O of gallic acid.
DPI-GA
DPI
Gallic acid
4000
3500
3000
2500
2000
1500
1000
500
Webnumber (cm-1)

[Fig. 80]

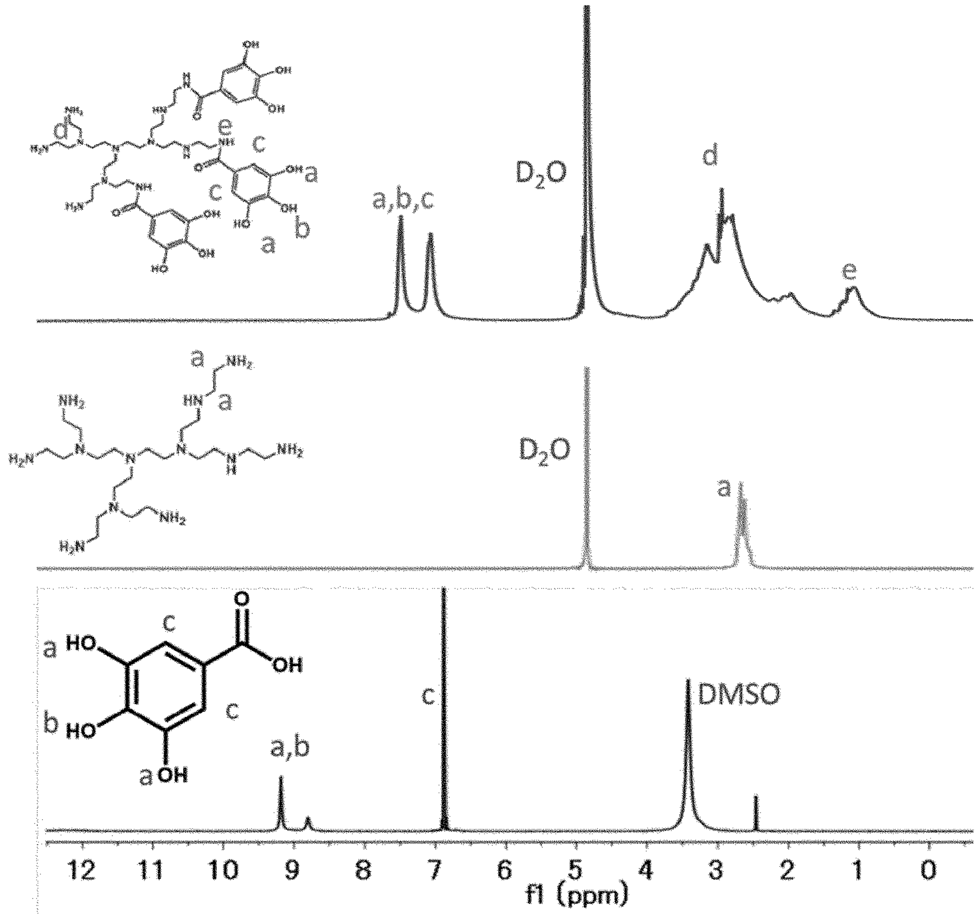

[Fig. 81]

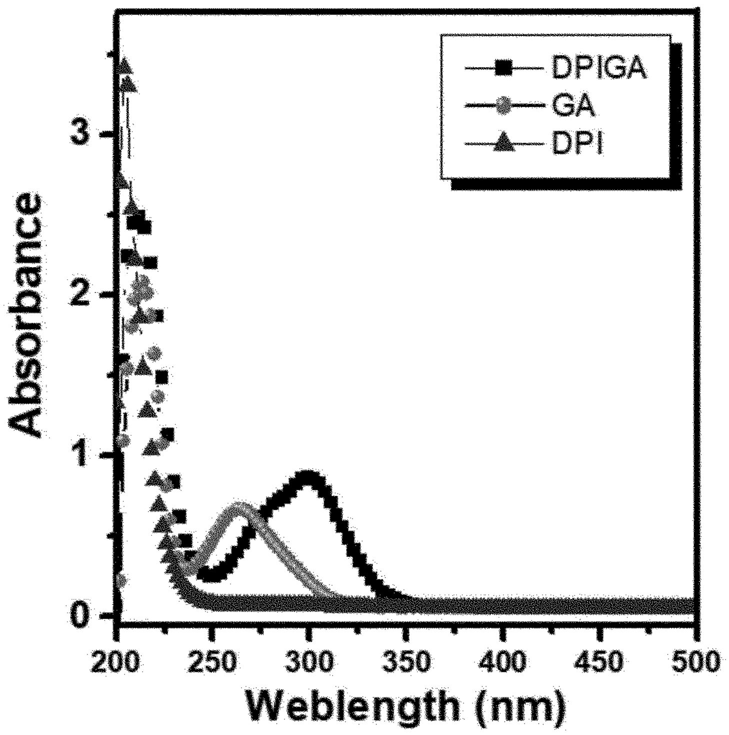
DPIGA
GA
DPI
Absorbance
Weblength (nm)
0
1
2
3
200
250
300
350
400
450
500

[Fig. 82]

(A)

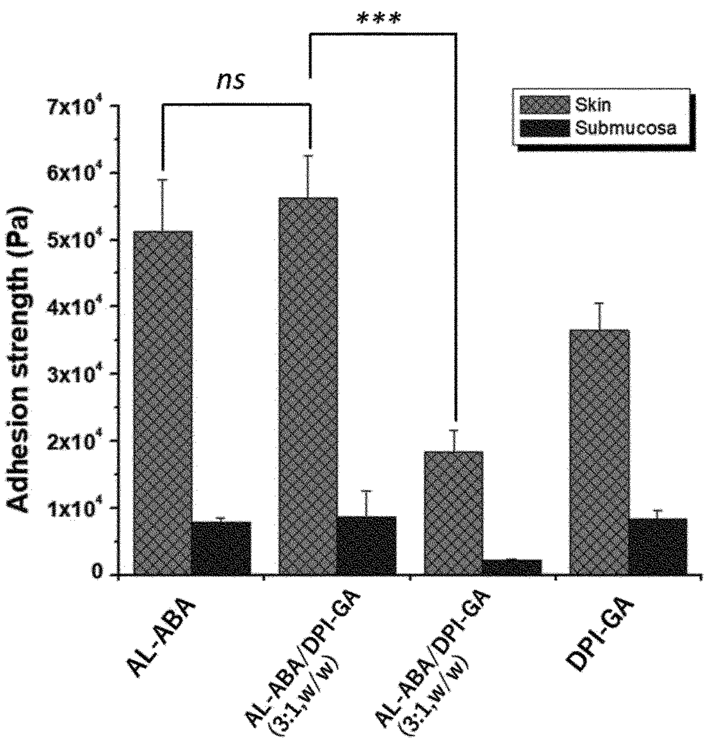
***
ns
Skin
Submucosa
Adhesion strength (Pa)
$7x10^4$
$6x10^4$
$5x10^4$
$4x10^4$
$3x10^4$
$2x10^4$
$1x10^4$
0
AL-ABA
AL-ABA/DPI-GA (3:1,w/w)
AL-ABA/DPI-GA (3:1,w/w)
DPI-GA

(B)

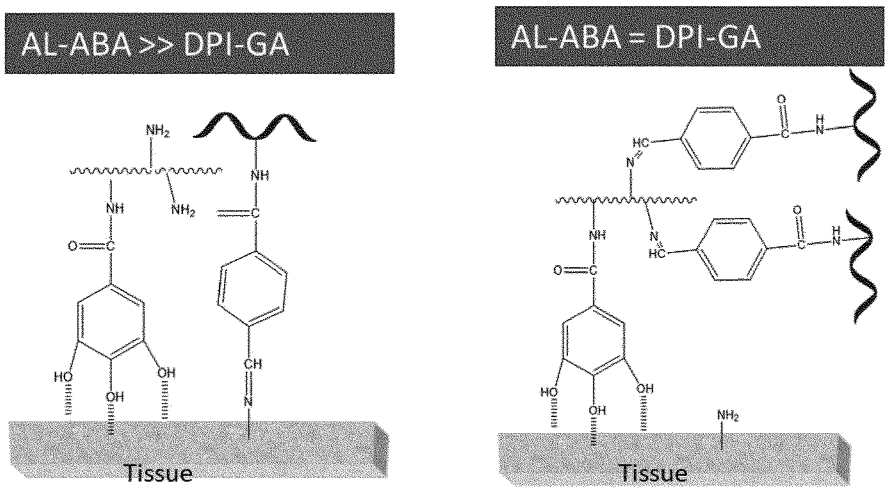
AL-ABA >> DPI-GA
AL-ABA = DPI-GA
Tissue
Tissue

[Fig. 83]

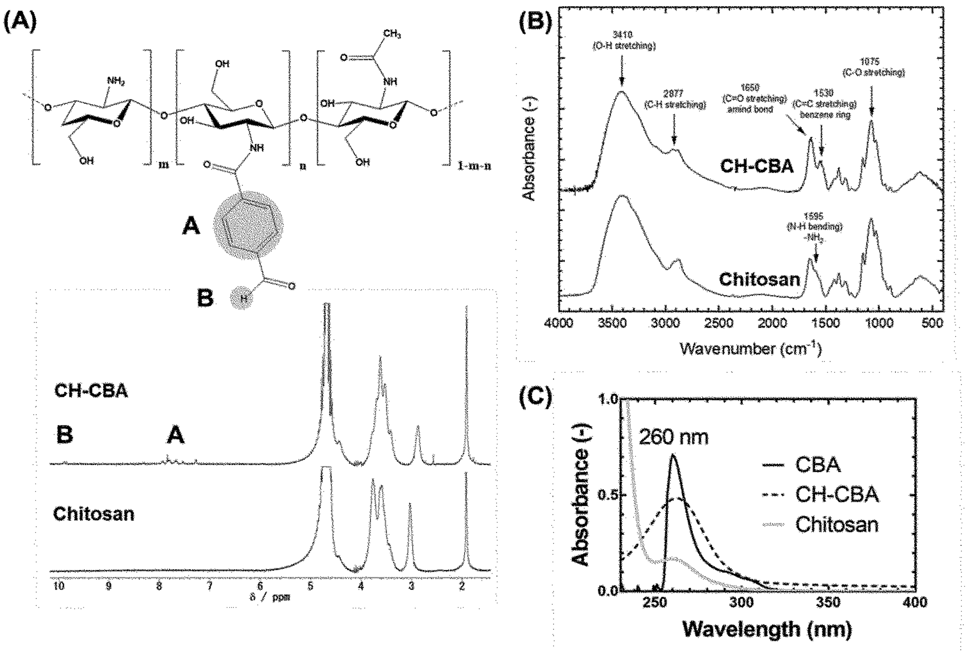


[Fig. 84]

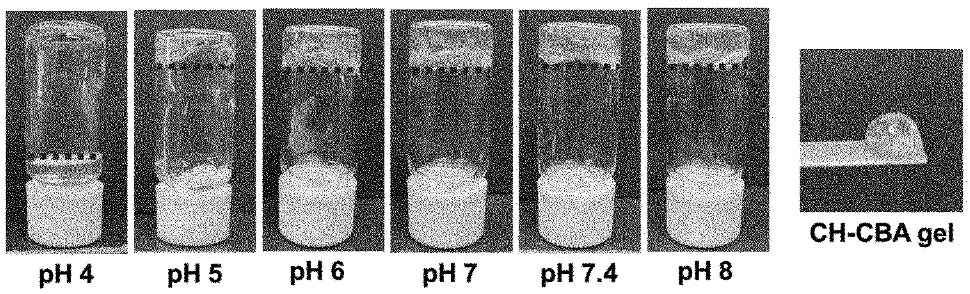


FIGURE pH RESPONSIVE PROPERTY MEASURED ACCORDING TO VIAL INVERSION METHOD

[Fig. 85]

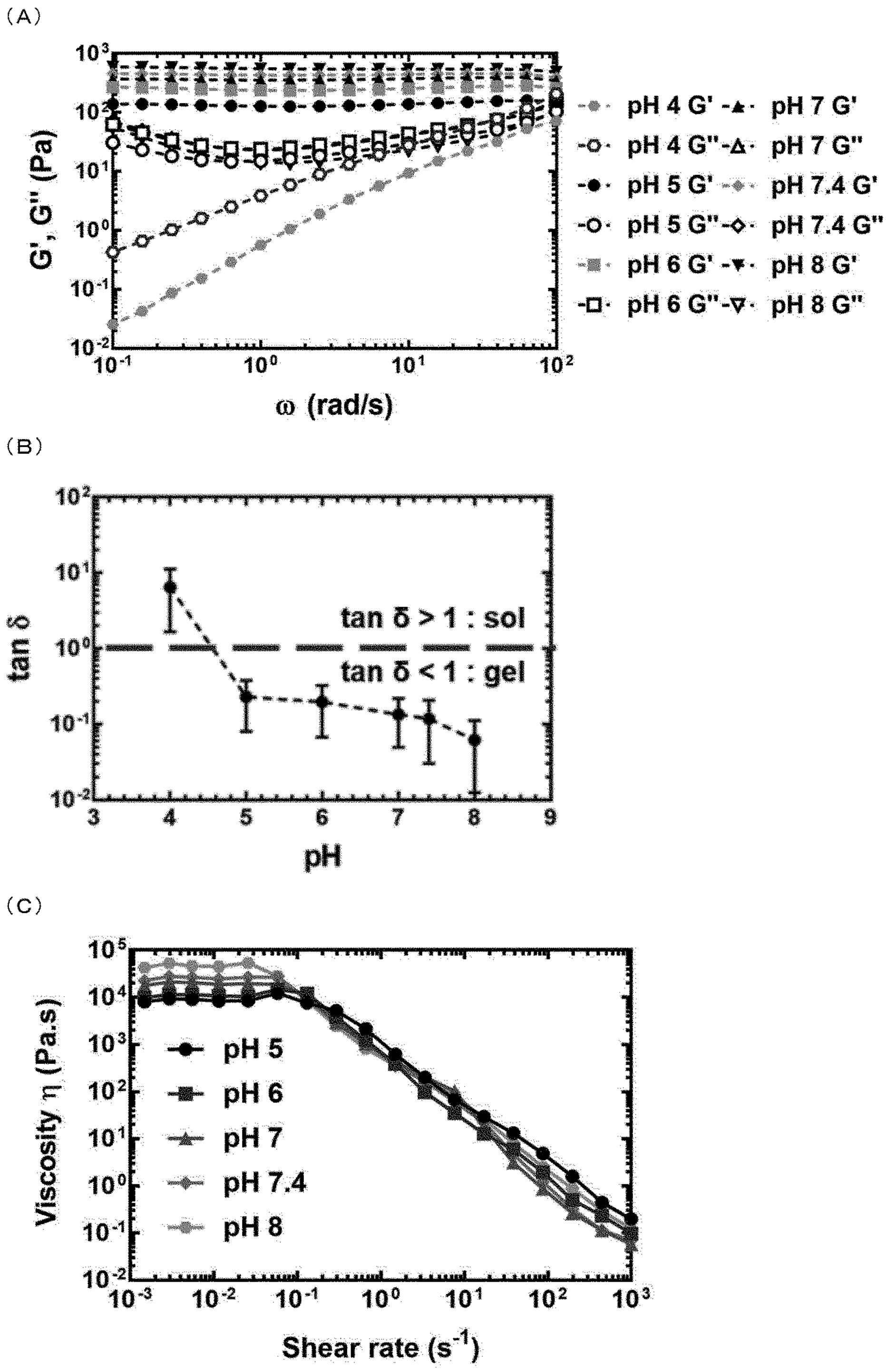
(A)
G', G" (Pa)
ω (rad/s)
pH 4 G'
pH 4 G"
pH 5 G'
pH 5 G"
pH 6 G'
pH 6 G"
pH 7 G'
pH 7 G"
pH 7.4 G'
pH 7.4 G"
pH 8 G'
pH 8 G"
(B)
tan δ
tan δ > 1 : sol
tan δ < 1 : gel
pH
(C)
Viscosity η (Pa.s)
pH 5
pH 6
pH 7
pH 7.4
pH 8
Shear rate (s-1)

[Fig. 86]

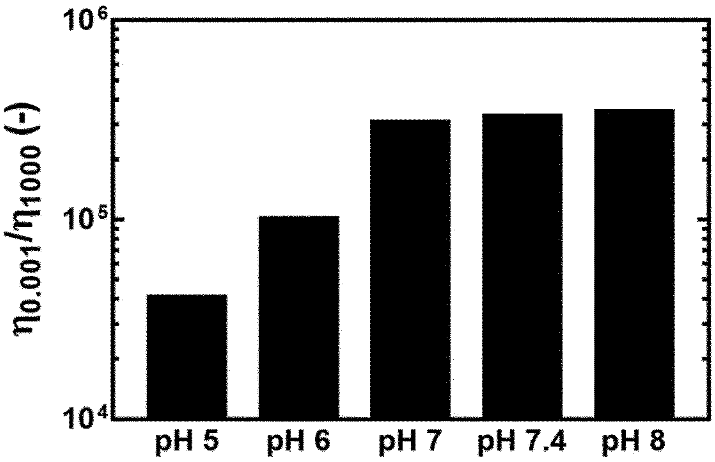
$10^6$
$10^5$
$10^4$
$\eta_{0.001}/\eta_{1000}$ (-)
pH 5
pH 6
pH 7
pH 7.4
pH 8

[Fig. 87]

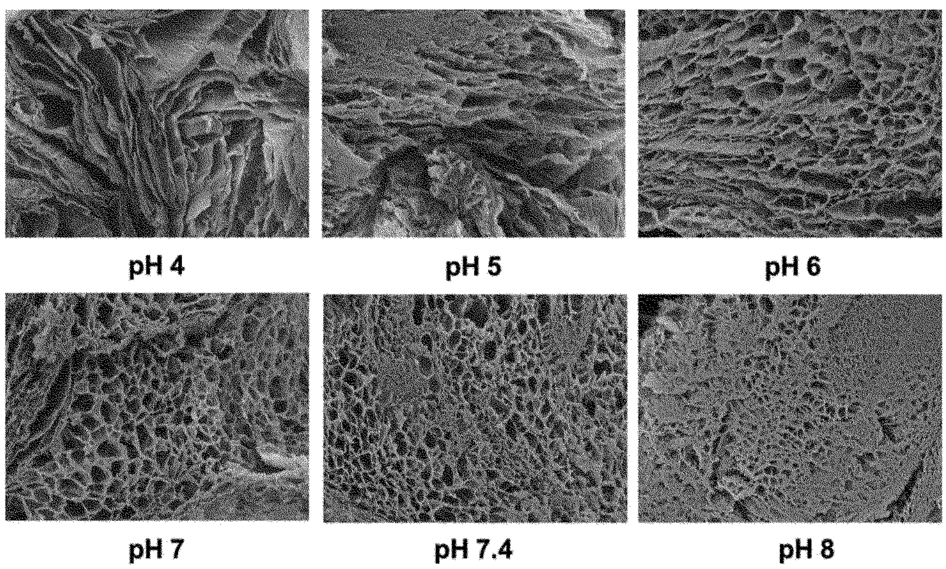
pH 4
pH 5
pH 6
pH 7
pH 7.4
pH 8

[Fig. 88]

(A)

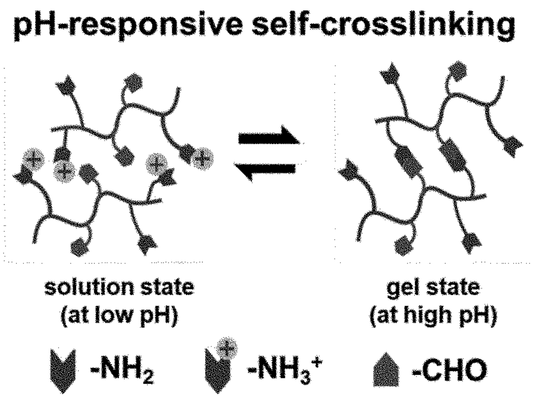
pH-responsive self-crosslinking
solution state
(at low pH)
gel state
(at high pH)
-NH2
-NH3+
-CHO

(B)

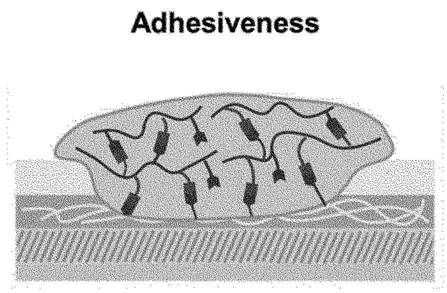
Adhesiveness

[Fig. 89]

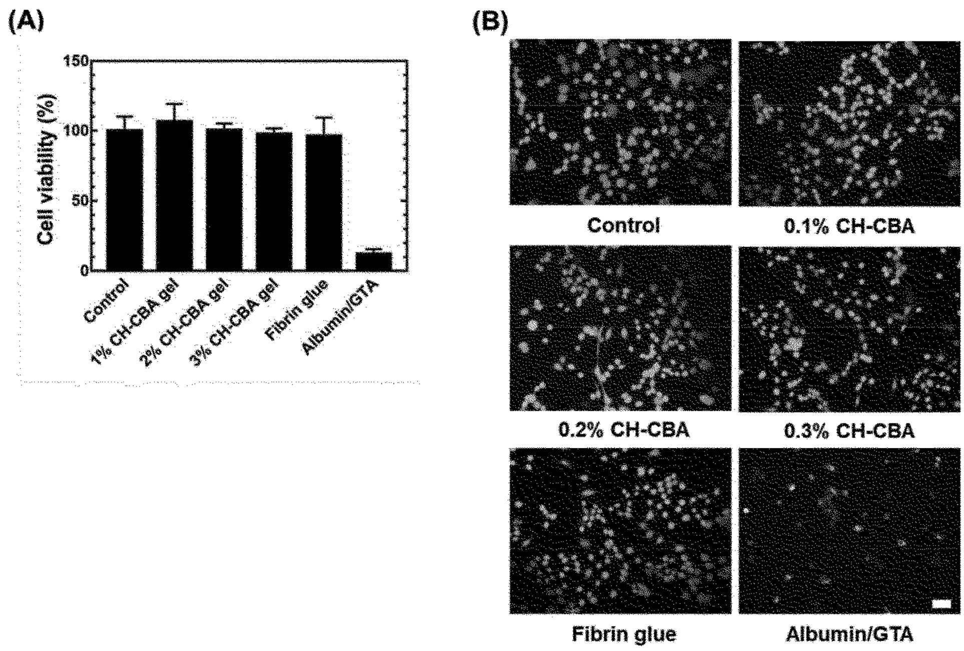
(A)
150
100
50
0
Cell viability (%)
Control
1% CH-CBA gel
2% CH-CBA gel
3% CH-CBA gel
Fibrin glue
Albumin/GTA
(B)
Control
0.1% CH-CBA
0.2% CH-CBA
0.3% CH-CBA
Fibrin glue
Albumin/GTA

[Fig. 90]

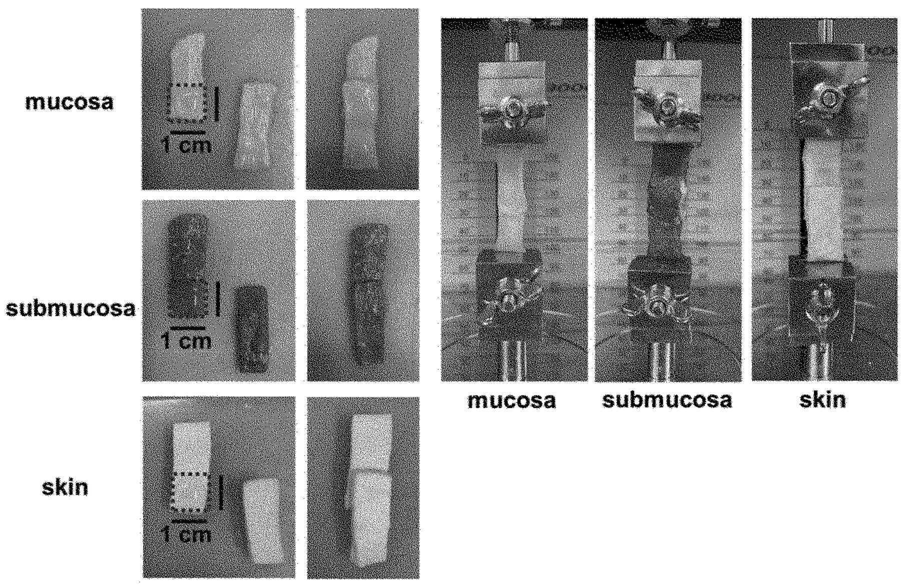
mucosa
submucosa
skin
1 cm
1 cm
1 cm
mucosa
submucosa
skin

[Fig. 91(A)]

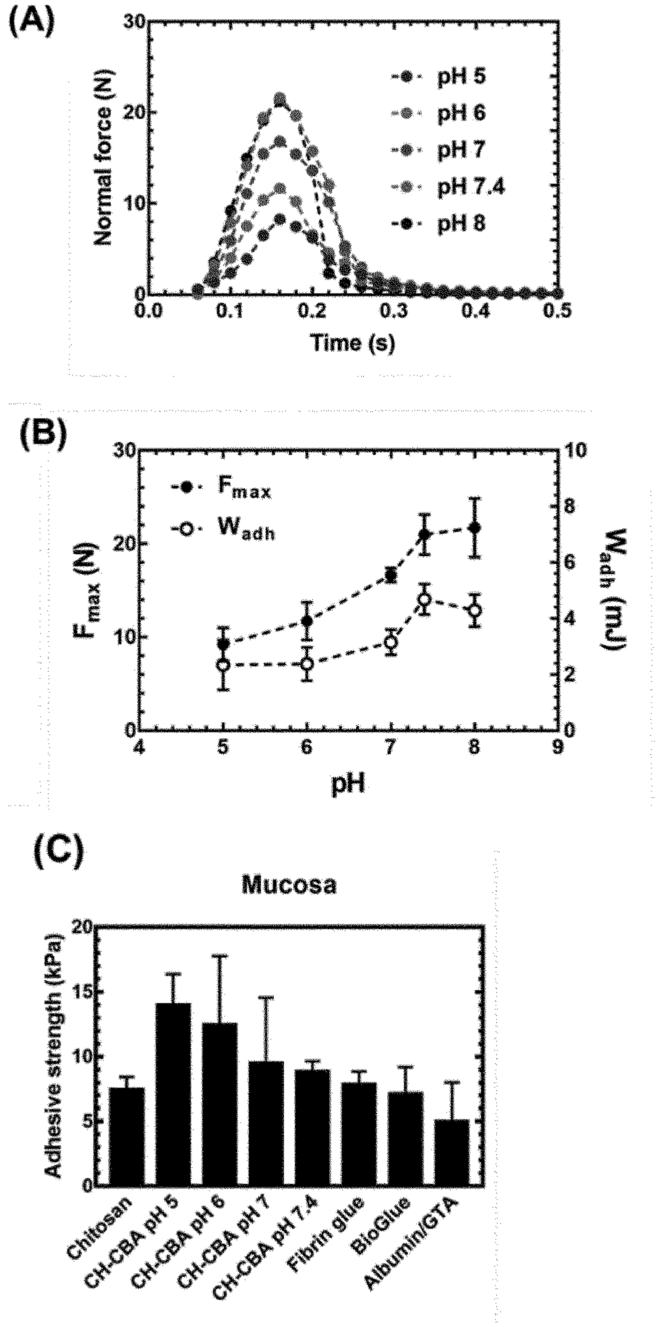
(A)
Normal force (N)
Time (s)
pH 5
pH 6
pH 7
pH 7.4
pH 8
(B)
$F_{max}$ (N)
$W_{adh}$ (mJ)
pH
$F_{max}$
$W_{adh}$
(C)
Mucosa
Adhesive strength (kPa)
Chitosan
CH-CBA pH 5
CH-CBA pH 6
CH-CBA pH 7
CH-CBA pH 7.4
Fibrin glue
BioGlue
Albumin/GTA

[Fig. 91(B)]

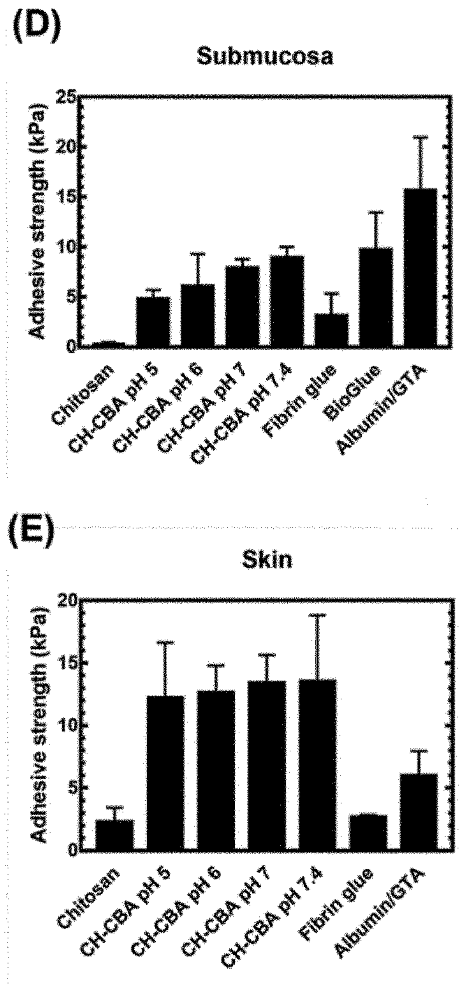
(D)
Submucosa
Adhesive strength (kPa)
0
5
10
15
20
25
Chitosan
CH-CBA pH 5
CH-CBA pH 6
CH-CBA pH 7
CH-CBA pH 7.4
Fibrin glue
BioGlue
Albumin/GTA
(E)
Skin
Adhesive strength (kPa)
0
5
10
15
20
Chitosan
CH-CBA pH 5
CH-CBA pH 6
CH-CBA pH 7
CH-CBA pH 7.4
Fibrin glue
Albumin/GTA

## INTERNATIONAL SEARCH REPORT

International application No.
**PCT/JP2022/006265**

**A. CLASSIFICATION OF SUBJECT MATTER**

***A61L 24/08***(2006.01)i; ***C08B 37/02***(2006.01)i; ***C08B 37/04***(2006.01)i; ***C08B 37/08***(2006.01)i
FI: A61L24/08; C08B37/02; C08B37/04; C08B37/08

According to International Patent Classification (IPC) or to both national classification and IPC

**B. FIELDS SEARCHED**

Minimum documentation searched (classification system followed by classification symbols)

A61L24/08; C08B37/02; C08B37/04; C08B37/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| E, X | WO 2022/034889 A1 (UNIVERSITY OF TOKYO) 17 February 2022 (2022-02-17) entire text | 1-16 |
| A | SONG, F. et al., Chitosan-based multifunctional flexible hemostatic bio-hydrogel, Acta Biomaterialia, 02 October 2021, 136, pp. 170-183 entire text | 1-16 |
| A | LI, Z. et al., Hydrogel Cross-Linked with Dynamic Covalent Bonding and Micellization for Promoting Burn Wound Healing, ACS Appl. Mater. Interfaces, 2018, 10, pp. 25194-25202 entire text | 1-16 |
| A | JIA, Z. et al., Repair of osteochondral defects using injectable chitosan-based hydrogel encapsulated synovial fluid-derived mesenchymal stem cells in a rabbit model, Materials Science & Engineering C, 2019, 99, pp. 541-551 entire text | 1-16 |
| A | DELLACHERIE, E. et al., A new approach to aldehydic dextrans, Polymer Bulletin, 1993, 31, pp. 145-149 entire text | 1-16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 April 2022** | **17 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/006265

C. DOCUMENTS CONSIDERED TO BE RELEVANT

| Category* | Citation of document, with indication, where appropriate, of the relevant passages | Relevant to claim No. |
|---|---|---|
| A | LIU, C. et al., Multifunctional PEG-grafted chitosan copolymer possessing amino and carboxyl [or formyl] groups, Cent. Eur. J. Chem., 2010, 8(3), pp. 576-581<br>entire text | 1-16 |
| A | BUFFA, R. et al., Conjugates of modified hyaluronic acid with amino compounds for biomedical applications, Carbohydrate Polymers, 2018, 189, pp. 273-279<br>entire text | 1-16 |
| A | LI, N,-N. et al., Using casein and oxidized hyaluronic acid to form biocompatible composite hydrogels for controlled drug release, Materials Science and Engineering C, 2014, 36, pp. 287-293<br>entire text | 1-16 |

Form PCT/ISA/210 (second sheet) (January 2015)

**INTERNATIONAL SEARCH REPORT**
**Information on patent family members**

International application No.
**PCT/JP2022/006265**

| Patent document cited in search report | Publication date (day/month/year) | Patent family member(s) | Publication date (day/month/year) |
|---|---|---|---|
| WO 2022/034889 A1 | 17 February 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021029573 W
- WO 2019189330 A
- JP 2001233786 A


### Non-patent literature cited in the description


- *Materials Science and Engineering: C*, 2014, vol. 36, 287-293
- *Carbohydrate Polymers*, 2015, vol. 134, 293-299
- *Carbohydrate Polymers*, 2018, vol. 189, 273-279
- *Carbohydrate Polymers*, 2019, vol. 216, 63-71
- *Materials Chemistry Frontiers*, 2018, vol. 2 (10), 1765-1778
- *J. Biomed. Nanotechnol.*, 2017, vol. 13, 1647-1659
- *Polymer Bulletin*, 1993, vol. 31, 145-149
- *Carbohydrate Polymers*, 2013, vol. 92, 2163-2170
- *Journal of Controlled Release*, 2016, vol. 236, 79-89
- *European Journal of Pharmaceutical Sciences*, 2019, vol. 138, 105008
- *Materials Chemistry Frontiers*, 2018, vol. 2, 1765-1778
- **AKIRA TAKAHASHI et al.** *Biomacromolecules*, 2013, vol. 14 (10), 3581-3588
- **VIANNEY DELPLACE et al.** Nonswelling, Ultralow Content Inverse Electron-Demand Diels-Alder Hyaluronan Hydrogels with Tunable Gelation Time: Synthesis and In Vitro Evaluation. *Advanced Functional Materials*, 2020, vol. 30 (14)
- **NIMMO, CHELSEA M. et al.** *Biomacromolecules*, 2011, vol. 12 (3), 824-830
- *RSC Adv.*, 2018, vol. 8, 11036-11042
- **CHIKAKO YOMOTA et al.** *Bull. Natl. Health Sci.*, 1999, vol. 117, 135-139
- **CHIKAKO YOMOTA et al.** *Bull. Natl. Inst. Health Sci.*, 2003, vol. 121, 30-33
- **GREG T. HERMANSON**. Bioconjugate Techniques. 2013
- Experimental Chemistry Course 5th Edition 16, Synthesis of Organic Compounds IV. Carboxylic Acids and Derivatives, 35-70
- *Acid Amides and Acid Imides*, 118-154
- *Amino Acids and Peptides*, 2007, 258-283
- **LEI ZHOU et al.** Injectable Self-Healing Natural Biopolymer-Based Hydrogel Adhesive with Thermoresponsive Reversible Adhesion for Minimally Invasive Surgery. *ADVANCED FUNCTIONAL MATERIALS*, 2021, vol. 31 (14), 2007457
- **AMANO Y.** *European Journal of Pharmaceutical Sciences*, 2019, vol. 138, 105008